(19) Europäisches Patentamt
European Patent Office
Office européen des brevets

(11) EP 1 888 615 B1

(12) EUROPEAN PATENT SPECIFICATION

(45) Date of publication and mention of the grant of the patent:
**16.05.2012 Bulletin 2012/20**

(21) Application number: **06763273.7**

(22) Date of filing: **24.05.2006**

(51) Int Cl.:
***C07J 41/00*** *(2006.01)* ***C07J 71/00*** *(2006.01)*
***C07J 43/00*** *(2006.01)* ***A61K 31/565*** *(2006.01)*
***A61K 31/58*** *(2006.01)* ***A61P 5/32*** *(2006.01)*

(86) International application number:
**PCT/EP2006/062587**

(87) International publication number:
**WO 2006/125800 (30.11.2006 Gazette 2006/48)**

(54) **17 -HSD1 AND STS INHIBITORS**

17-HSD1- UND STS-HEMMER

INHIBITEURS DE 17-HSD1 ET STS

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**HR**

(30) Priority: **26.05.2005 EP 05104534**

(43) Date of publication of application:
**20.02.2008 Bulletin 2008/08**

(73) Proprietor: **Abbott Products GmbH**
**30173 Hannover (DE)**

(72) Inventors:
- **MESSINGER, Josef**
**31319 Sehnde (DE)**
- **THOLE, Heinrich-Hubert**
**30655 Hannover (DE)**
- **HUSEN, Bettina**
**30173 Hannover (DE)**
- **KOSKIMIES, Pasi**
**FI-20740 Turku (FI)**
- **PIRKKALA, Lila**
**FI-20780 Kaarina (FI)**
- **WESKE, Michael**
**31303 Burgdorf (DE)**

(74) Representative: **Modiano, Micaela Nadia et al**
**Modiano & Partners (DE)**
**Thierschstrasse 11**
**80538 München (DE)**

(56) References cited:
**EP-A- 0 367 576 WO-A-2005/047303**

- **POIRIER D ET AL: "SYNTHESIS OF 17BETA-ESTRADIOL DERIVATIVES WITH N-BUTYL, N-METHYL ALKYLAMIDE SIDE CHAIN AT POSITION 15" TETRAHEDRON, ELSEVIER SCIENCE PUBLISHERS, AMSTERDAM, NL, vol. 47, no. 37, 1991, pages 7751-7766, XP001122350 ISSN: 0040-4020**
- **POIRIER D ET AL: "D-ring alkylamide derivatives of estradiol: effect on ER-binding affinity and antiestrogenic activity" BIOORGANIC & MEDICINAL CHEMISTRY LETTERS, OXFORD, GB, vol. 6, no. 21, 5 November 1996 (1996-11-05), pages 2537-2542, XP004135909 ISSN: 0960-894X**
- **TREMBLAY M R ET AL: "OVERVIEW OF A RATIONAL APPROACH TO DESIGN TYPE I 17BETA-HYDROXYSTEROID DEHYDROGENASE INHIBITORS WITHOUT ESTROGENIC ACTIVITY: CHEMICAL SYNTHESIS AND BIOLOGICAL EVALUATION" JOURNAL OF STEROID BIOCHEMISTRY, PERGAMON PRESS PLC, GB, vol. 66, no. 4, 1998, pages 179-191, XP001055919 ISSN: 0022-4731**
- **SAM K-M ET AL: "C16 AND C17 DERIVATIVES OF ESTRADIOL AS INHIBITORS OF 17BETA - HYDROXYSTEROID DEHYDROGENASE TYPE 1: CHEMICAL SYNTHESIS AND STRUCTURE-ACTIVITY RELATIONSHIPS" DRUG DESIGN AND DISCOVERY, HARWOOD ACADEMIC PUBLISHERS GMBH, vol. 15, 1998, pages 157-180, XP009024879 ISSN: 1055-9612**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- POIRIER D ET AL: "A 6(beta)-(thiaheptanamide) derivative of estradiol as inhibitor of 17 (beta)-hydroxysteroid dehydrogenase Type 1" JOURNAL OF STEROID BIOCHEMISTRY AND MOLECULAR BIOLOGY, ELSEVIER SCIENCE LTD., OXFORD, GB, vol. 64, no. 1/2, 1998, pages 83-90, XP002269407 ISSN: 0960-0760
- POIRIER D: "INHIBITORS OF 17BETA-HYDROXYSTEROID DEHYDROGENASES" CURRENT MEDICINAL CHEMISTRY, BENTHAM SCIENCE PUBLISHERS BV, BE, vol. 10, 2003, pages 453-477, XP009024878 ISSN: 0929-8673

## Description

FIELD OF INVENTION

The present invention relates to novel substituted steroid derivatives which represent selective inhibitory compounds of the 17β-hydroxysteroid dehydrogenase type I (17β-HSD1) enzyme, and, in addition, which may represent inhibitors of the steroid sulphatase, as well as to the salts of these compounds, to pharmaceutical preparations containing these compounds and to processes for the preparation of these compounds. Furthermore, the invention concerns the therapeutic use of said novel substituted steroid derivatives, particularly their use in the treatment or prevention of steroid hormone dependent diseases or disorders, such as steroid hormone dependent diseases or disorders requiring the inhibition of the 17β-HSD1 enzyme and/or steroid sulphatase enzymes and/or requiring the lowering of the endogenous 17β-estradiol concentration.

BACKGROUND

Mammalian 17β-hydroxysteroid dehydrogenases (17β-HSDs) are NAD(H) or NADP(H) dependent enzymes which convert inactive 17-keto-steroids into their active 17β-hydroxy-forms or catalyse the oxidation of the 17β-hydroxy-forms into the 17-keto-steroids. Because both estrogens and androgens have the highest affinity for their receptors in the 17β-hydroxy form, 17β-HSD enzymes play an essential role in the tissue-selective regulation of the activity of sex steroid hormones. At present, 10 human members of the 17β-HSD enzyme family have been described (types 1-5, 7, 8, 10-12)., whereby each type of 17β-HSD has a selective substrate affinity, directional (reductive or oxidative) activity in intact cells, and a particular tissue distribution.

Due to their essential role in the tissue-selective regulation of the activity of sex steroid hormones, 17β-HSDs can be involved in the occurrence and development of estrogen-sensitive pathologies (f. ex. breast, ovarian, and endometrium cancers etc.) and androgen-sensitive pathologies (f. ex. prostate cancer, benign prostatic hyperplasia, acne, hirsutism, etc). Furthermore, many types of 17β-HSD have been shown to be involved in the pathogenesis of particular human disorders such as pseudohermaphroditism (17β-HSD3), polycystic kidney disease (17β-HSD8) and bifunctional enzyme deficiency (17β-HSD4) [reviewed by: Mindnich et al (2004)]. Therefore treatment of sex steroid-sensitive diseases by administration of specific inhibitors of the 17β-HSDs enzymes have been suggested, optionally in combination with potent and specific anti-estrogens and antiandrogens [Labrie et al. (1997)].

The best characterized member of the 17β-HSD family is the 17β-HSD1 [EC 1.1.1.62]. The 17β-HSD1 enzyme catalyzes in vitro the reduction and the oxidation between estrone (E1) and estradiol (E2). However, under physiological in vivo conditions the enzyme only catalyses the reductive reaction from the estrone (E1) to the estradiol (E2). The 17β-HSD1 was found to be expressed in a variety of hormone-dependent tissues, e.g. placenta, mammary gland tissue or uterus and endometrium tissue, respectively.

Estradiol itself is, especially in comparison to the significantly less active estrone, a very potent hormone, which regulates the expression of a variety of genes by binding to the nuclear estrogen receptor and plays an essential role in the proliferation and differentiation of the target cell. Physiological as well as pathological cell proliferations can be estradiol dependent. Especially many breast cancer cells are stimulated by a locally raised estradiol concentration. Furthermore, the occurrence or course of benign pathologies such as endometriosis, uterine leiomyomas (fibroids or myomas), adenomyosis, menorrhagia, metrorrhagia and dysmenorrhoea is dependent from the existence of significantly high estradiol levels.

Endometriosis is a well-known gynaecological disorder that affects 10 to 15% of women in the reproductive age. It is a benign disease defined as the presence of viable endometrial gland and stroma cells outside the uterine cavity. It is most frequently found in the pelvic area. In women developing endometriosis, the endometrial cells entering the peritoneal cavity by retrograde menstruation (the most likely mechanism) have the capacity to adhere to and invade the peritoneal lining, and are then able to implant and grow. The implants respond to steroid hormones of the menstrual cycle in a similar way as the endometrium in the uterus. The infiltrating lesions and the blood from these lesions which are unable to leave the body cause inflammation of the surrounding tissue. The most common symptoms of endometriosis are dysmenorrhoea, dyspareunia and (chronic) abdominal pain. The occurrence of these symptoms is not related to the extent of the lesions. Some women with severe endometriosis are asymptomatic, while women with mild endometriosis may have severe pain. Up to now, no reliable non-invasive test is available to diagnose endometriosis. Laparoscopy has to be performed to diagnose the disease. Endometriosis is classified according to the 4 stages set up by the American Fertility Society (AFS). Stage I corresponds to minimal disease while stage IV is severe, depending on the location and the extent of the endometriosis. Endometriosis is found in up to 50% of the women with infertility. However, currently no causal relation has been proven between mild endometriosis and infertility. Moderate to severe endometriosis can cause tubal damage and adhesions leading to infertility. The aims of treatment of endometriosis are pain relief, resolution of the endometriotic tissue and restoration of fertility (if desired). The two common treatments are surgery or anti-inflammatory and/or hormonal therapy or a combination thereof.

Uterine leiomyomas (fibroids or myomas), benign clonal tumours, arise from smooth muscle cells of the human uterus. They are clinically apparent in up to 25% of women and are the single, most common indication for hysterectomy. They cause significant morbidity, including prolonged and heavy menstrual bleeding, pelvic pressure and pain, urinary problems, and, in rare cases, reproductive dysfunction. Myomas are found submucosally (beneath the endometrium), intramurally (within the myometrium) and subserosally (projecting out of the serosal compartment of the uterus), but mostly are mixed forms of these 3 different types. The presence of estrogen receptors in leiomyoma cells has been studied by Tamaya et al. [Tamaya et al. (1985)]. They have shown that the ratios of estrogen receptor compared to progesterone and androgen receptor levels were higher in leiomyomas than in the corresponding normal myometrium. Surgery has long been the main treatment for myomas. Furthermore, medical therapies that have been proposed to treat myomas include administration of a variety of steroids such as the androgenic steroids danazol or gestrinone and progestogens, or of compounds modulating the steroid hormone plasma levels like e.g. GnRH agonists and GnRH antagonists, whereby the administration is often associated a variety of serious side-effects.

Everything that has been said above in relation to the treatment of uterine leiomyomas and endometriosis equally applies to other benign gynaecological disorders, notably adenomyosis, functional menorrhagia and metrorrhagia. These benign gynaecological disorders are all estrogen sensitive and are treated in a comparable way as described herein before in relation to uterine leiomyomas and endometriosis. The available pharmaceutical treatments, however, suffer from the same major drawbacks, i.e. they have to be discontinued once the side-effects become more serious than the symptoms to be treated and symptoms reappear after discontinuation of the therapy.

Since the aforementioned malign and benign pathologies are all 17β-estradiol dependent, a reduction of the endogenous 17β-estradiol concentration in the respective tissue will result in an impaired or reduced proliferation of 17β-estradiol responsive cells in said tissues. Therefore, selective inhibitors of the 17β-HSD1 enzyme are well suited for their use to impair endogenous productions of estrogens, in particular of 17β-estradiol, in myomas, endometriotic, adenomyotic and endometrial tissue. The application of a compound acting as selective inhibitor on the 17β-HSD1 which preferentially catalyses the reductive reaction will result in a lowered intracellular estradiol-concentration, since the reductive conversion of the estrone into the active estradiol is reduced or suppressed. Therefore, reversible or even irreversible inhibitors of the 17β-HSD1 may play a significant role in the prophylaxis and/or treatment of steroid-hormone, in particular 17β-estradiol, dependent disorders or diseases. Furthermore, the reversible or even irreversible inhibitors of the 17β-HSD1 should have no or only pure antagonistic binding activities to the estradiol receptor, in particular to the estrogen receptor α subtype, since agonistic binding of the estrogen receptor would lead to activation and subsequently to the proliferation and differentiation of the target cell. In contrast, antagonists of the estrogen receptor, so called anti-estrogens, bind competitively to the specific receptor protein thus preventing access of endogenous estrogens to their specific binding site.

At present it is described in the literature that several malignant disease as breast cancer, prostate carcinoma, ovarian cancer, uterine cancer, endometrial cancer and endometrial hyperplasia may be treated by the administration of a selective 17β-HSD1 inhibitor. Furthermore, a selective 17β-HSD1 inhibitor may be useful for the prevention of the aforementioned hormone-dependent cancers, especially breast cancer (e.g. WO 2004/080271). Furthermore, international patent application WO 03/017973 describes the use of a selective estrogen enzyme modulator (SEEM) in the manufacture of a drug delivery vehicle for intravaginal administration to treat or prevent a benign gynaecological disorder such as endometriosis in a mammalian female.

Another known target for estrogen deprivation is the steroid sulphatase enzyme (STS) (E.C. 3.1.6.2), which regulates the local production of estrogens and androgens from systemic precursors in several tissues [reviewed by Reed et al (2005)]. The enzyme catalyzes the hydrolysis of the sulphate esters of 3-hydroxy steroids, which are inactive transport or precursor forms of the active 3-hydroxy steroids. In particular, STS hydrolyzes in-active estron-sulphate into estrone, which is then further converted into the active estradiol by action of the above described 17β-HSD1 enzyme. Therefore, STS has a pivotal role in regulating the formation of biologically active steroids. The enzyme is widely distributed throughout the body and its action is implicated in physiological processes and pathological conditions, such as hormone-dependent tumors. STS expression is increased in breast tumors and has prognostic significance. The role of STS in supporting tumor growth of the breast and prostate prompted the development of potent STS inhibitors, since STS inhibitors are expected to block the local production and, consequently, to reduce the local levels of the hormones. Therefore, they are considered as potential therapeutic agents for the treatment of estrogen- and androgen-dependent disorders in general. Indications may range from cancers of the breast, endometrium and prostate to disorders of the pilosebaceous unit, e. g. acne, androgenetic alopecia, and hirsutism. Furthermore, STS inhibitors may be useful as immunosuppressive agents, and have been shown to enhance memory when delivered to the brain.

Acne is a polyetiological disease caused by the interplay of numerous factors, such as inheritance, sebum, hormones, and bacteria. The most important causative factor in acne is sebum production; in almost all acne patients sebaceous glands are larger and more sebum is produced than in persons with healthy skin. The development of the sebaceous gland and the extent of sebum production is controlled hormonally by androgens; therefore, androgens play

a crucial role in the pathogenesis of acne. In man, there are two major sources supplying androgens to target tissues: (i) the gonades which secrete testosterone, (ii) the adrenals producing dehydroepiandrosterone (DHEA) which is secreted as the sulfate conjugate (DHEAS). Testosterone and DHEAS are both converted to the most active androgen, dihydrotestosterone (DHT), in the target tissue, e. g. in the skin. There is evidence that these pathways of local synthesis of DHT in the skin are more important than direct supply with active androgens from the circulation. Therefore, reduction of endogeneous levels of androgens in the target tissue by specific inhibitors should be of therapeutic benefit in acne and seborrhoea. Furthermore, it opens the perspective to treat these disorders through modulation of local androgen levels by topical treatment, rather than influencing circulating hormone levels by systemic therapies.

Androgenetic male alopecia is very common in the white races, accounting for about 95% of all types of alopecia. Male-pattern baldness is caused by an increased number of hair follicles in the scalp entering the telogen phase and by the telogen phase lasting longer. It is a genetically determined hair loss affected through androgens. Elevated serum DHEA but normal testosterone levels have been reported in balding men compared with non-balding controls, implying that target tissue androgen production is important in androgenetic alopecia.

Hirsutism is the pathological thickening and strengthening of the hair which is characterized by a masculine pattern of hair growth in children and women. Hirsutism is androgen induced, either by increased formation of androgens or by increased sensitivity of the hair follicle to androgens.

The presence of the STS enzyme in keratinocytes and in skin-derived fibroblasts has been described, and the potential use of STS inhibitors for the reduction of endogenous levels of steroid hormones in the skin was confirmed using known steroid sulfatase inhibitors, such as EMATE. Additionally, it has been described that inhibitors of placenta steroid sulfatase also inhibit steroid sulfatase in human keratinocyte or human skin-derived fibroblast cell lines. Therefore, STS inhibitors may be used to reduce androgen and estrogen levels in the skin, e.g. for the local treatment of androgen-dependent disorders of the pilosebaceous unit (such as acne, seborrhoea, androgenetic alopecia, hirsutism). STS inhibitors are also useful for the treatment of cancer, especially for the treatment of estrogen-and androgen-dependent cancers, such as cancer of the breast and endometrium, squamous cell carcinoma, and cancer of the prostata.

In addition, STS inhibitors may be useful for the prevention and treatment of further estrogen- or androgen-dependent diseases or disorders and/or diseases or disorders requiring the lowering of the endogeneous estrogen or androgen concentration in a generalized or tissue-specific manner, such as inflammatory and autoimmune diseases, e.g. rheumatoid arthritis, type I and II diabetes, systemic lupus erythematosus, multiple sclerosis, myastenia gravis, thyroiditis, vasculitis, ulcerative colitis, and Crohn's disease, psoriasis, contact dermatitis, graft versus host disease, eczema, asthma and organ rejection following transplantation. STS inhibitors are also useful for the enhancement of cognitive function, especially in the treatment of senile dementia, including Alzheimer's disease, by increasing the DHEAS levels in the central nervous system.

Several reversible or irreversible inhibitors of the 17β-HSD1 enzyme or of the steroid sulphatase of steroidal and even non-steroidal origin are already known from the literature. The characteristics of the inhibitory molecules of the 17β-HSD1 enzyme, which mainly have a substrate or cofactor-like core structure, have been reported in the literature [reviewed in: Poirier D. (2003)]. The characteristics and structure-activity relationship of known irreversible as well as reversible STS inhibitors have been reviewed in the literature [reviewed by Nussbaumer & Billich (2004) and (2003)]. Even dual inhibitors of the 17β-HSD1 enzyme and of the steroid sulphatase have been described in international patent application WO 02/32409.

The following compounds or compound classes have already been described as 17β-HSD1 inhibitors: For example, Tremblay and Poirier describe an estradiol derivative, 16-[carbamoyl-(bromo-methyl)-alkyl]-estradiol, and tested the same in respect of its inhibition of the estradiol formation catalysed by the enzyme 17β-HSD1 [Tremblay & Poirier (1998)]. Poirier and colleagues describe a 6β-thiaheptan-butyl-methyl-amide derivative of estradiol as a potent and selective inhibitor of the 17HSD1 enzyme [Poirier et al. (1998)]. Furthermore, Poirier and colleagues describe new derivatives of 17β-estradiol with long N-butyl, N-methyl alkylamide side chains of three different lengths (n=8, 10 or 12) at position 15, which might be potential inhibitors of the 17β-HSD1 enzyme [Poirier et al. (1991)]. Similar compounds were also disclosed within European patent application EP0367576. However, the biological activity of these compounds was only tested with regard to estrogen receptor binding affinity, estrogenic and anti-estrogenic activity [Poirier et al. (1996)], but not with regard to their ability to inhibit the 17β-HSD1 enzyme. In addition, Pelletier and Poirier describe novel 17β-estradiol derivatives with different bromo-alkyl side chains, which might be potential inhibitors of the 17β-HSD1 enzyme [Pelletier & Poirier (1996)]. Sam and colleagues describe several estradiol derivatives with a halogenated alkyl side chain in 16α or 17α position of the steroidal D-ring which possess 17β-HSD1 inhibiting properties [Sam et al. (1998)]. Furthermore, the finding that some anti-estrogens, such as tamoxifen, possess weak 17β-HSD1 inhibiting properties suggested that it may be possible to develop a potent 17β-HSD1 inhibitor that is also anti-estrogenic [reviewed in: Poirier D. (2003)]. Several of the aforementioned already known compounds also display anti-estrogenic properties (e.g. the 6β-thiaheptan-butyl-methyl-amide derivative of estradiol described by Poirier and colleagues [Poirier et al. (1998)]). None of the aforementioned compounds has been clinically used so far.

Furthermore, the international patent application WO 2004/085457 discloses a variety of estron derivatives

with different substituents in C2, C3, C6, C16 and/or C17 position as potent 17β-HSD1 inhibitors. For some of the compounds it was shown that the substitution of steroid based 17β-HSD1 inhibitors at the C2 position with small hydrophobic groups renders the compounds less estrogenic and are favourable for 17β-HSD1 over 17β-HSD2 discrimination [Lawrence et al (2005)].

The international application WO 2005/047303, published on the filing date of the priority application of the present invention, discloses new 3, 15 substituted 17-estradiol derivatives with different kind of side chains at position 15, which are potent and selective 17β-HSD1 inhibitors.

Additional compounds representing potential 17β-HSD1 inhibitors were disclosed within international applications WO 2006/003012 and WO 2006/003013 in the form of novel 2-substituted D-homo-estra-1,3,5(10)-trienes and novel 2-substituted estra-1,3,5(10)-trien-17-ones.

The synthesis of different B-, C- and D-ring substituted estradiol carboxylic esters was described by Labaree et al. [Labaree et al. (2003)]. However, these esters were only analysed with regard to their estrogenic potential. The related international patent application WO 2004/085345 discloses 15α substituted estradiol compounds bearing a $-(CH_2)_m-CO-O-R$ side chain, wherein R is H, a $C_1$-$C_5$ alkyl group, optionally substituted with at least one halogen group, such as $CH_2CH_2F$, or other group (e.g. $CH_2CHF_2$, $CH_2CF_3$ or $CF_3$ group); and m is from 0-5. These 15α estradiol esters are described as locally active estrogens without significant systemic action.

Furthermore, international application WO 2006/027347 discloses 15β substituted estradiol derivatives having selective estrogen receptor activity towards the estrogen receptor α-subtype.

Several compounds and compound classes have already been identified as STS inhibitors. They all share the common structural feature of an aromatic ring bearing a substituent that mimics the phenolic A-ring of the enzyme substrate, estrone-sulphate. On the development of steroidal inhibitors, a wide variety of chemical groups have been introduced at C3, of which the 3-0-sulfamate was found to be the most potent for the estrone molecule. The resulting compound, estrone-3-O-sulfamate ("EMATE") led to the identification of the aryl-O-sulphamate structure as an active pharmacophore required for potent inhibition of STS (as disclosed in international patent application WO 93/05064). EMATE was shown to inhibit steroid sulphate activity in a time-and concentration-dependent manner and was active in vivo on oral administration. It was however revealed to be highly estrogenic which raised the need to design STS inhibitors devoid of agonist activity on the human estrogen receptor. For example, the recently published international patent application WO 2004/085459 discloses a variety of estron derivatives with different subsituents in C2, C3, C4 and/or C17 position as potent STS inhibitors.

Accordingly, there is still a need for the development of compounds which are suited for the treatment and/or prevention of steroid hormone dependent diseases or disorders such as breast cancer, endometriosis and uterine leiomyomas by selectively inhibiting the 17β-HSD1 enzyme and preferably additionally inhibiting the STS enzyme, while desirably failing to substantially inhibit other members of the 17β-HSD protein family or other catalysts of sex steroid degradation or activation. In particular, it is an aim of the present invention to develop selective inhibitors of the 17β-HSD1 enzyme, whereby in addition the compounds have no or only pure antagonistic binding affinities to the estrogen receptor (both subtypes α and β) and have favourably no residual activity on the 17β-HSD2 enyme. Furthermore, an increased metabolic stability of the compounds, in particular of the C17 keto position of the steroidal core, would be desirable, in order to prevent conversion of the estron to the respective estradiol derivative, which shows less inhibitory potential on the 17β-HSD1 enzyme.

## SUMMARY OF THE INVENTION

Therefore, it is an object of the present invention to develop novel inhibitors of the enzyme 17β-HSD1 and preferably also of the enzyme STS, which have valuable pharmacological properties and which are suited for the treatment of estrogen dependent diseases and disorders.

It has now been found that novel 3, 15 substituted estrone derivatives bearing a side chain of the amide, ester, carbonyl, hydrazone, alcohol, ether, urea, carbamate, "retro"-amide, sulfonyl urea, sulfamide, sulfamate, "retro"-sulfonamide, "retro"-carbamate, "retro"-ester or sulfonylcarbamate type in position C15 and being additionally modified by substitution in position C2, C3, C16 and/or C17 position of the estron core, would be valuable in therapy, especially in the treatment or prevention of steroid hormone dependent diseases or disorders requiring the lowering of the endogeneous estradiol concentration, in humans and other mammals. In particular, compounds of formula (I) represent potent inhibitors of the 17β-HSD1 enzyme and optionally of the STS enzyme, and possess valuable pharmacological properties for the treatment and/or prophylaxis of malignant steroid dependent diseases or disorders such as breast cancer, prostate carcinoma, ovarian cancer, uterine cancer, endometrial cancer and endometrial hyperplasia, but also for the treatment and/or prophylaxis of benign steroid dependent diseases or disorders such as endometriosis, uterine fibroids, uterine leiomyoma, adenomyosis, dysmenorrhoea, menorrhagia, metrorrhagia, prostadynia, benign prostatic hyperplasia, urinary dysfunction or lower urinary tract syndrome. Further estrogen-dependent diseases which may be treated and/or prevented with an effective amount of a compound of the invention are multiple sclerosis, rheumatoid arthritis, colon

cancer, tissue wounds, skin wrinkles and cataracts. The compounds of the present invention have been developed as improved inhibitors of the enzyme 17β-HSD1, in addition showing no or only pure antagonistic binding affinities to the estrogen receptor (both subtypes α and β) and having favourably no residual activity on the 17β-HSD2 enyme, and/ or showing an increased metabolic stability of the C17 keto function of the steroidal core.

Accordingly, the present invention relates to a compound having the structural formula I I

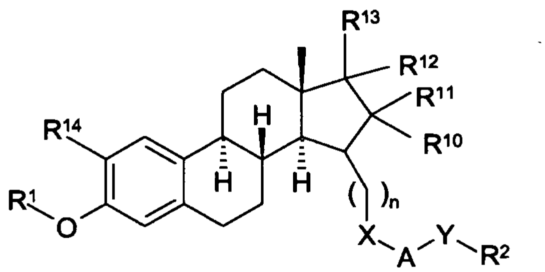

(I)

wherein

(i) X represents:

(a) a bond,
(b) -NH-, or
(c) -O-;

A represents:

(a) -CO-, or
(b) under the proviso that X represents -NH-, A represents $-SO_2-$;

Y represents:

(a) $-NR^4-$,
(b) -O-, under the proviso that X represents a bond or -NH-,
(c) a bond,
(d) $-NH-SO_2-$, under the proviso that X represents -NH- and A represents -CO-,
(e) $-NH-SO_2-NR^4-$, under the proviso that X represents -O-, or
(f) $-NH-NR^4-$, under the proviso that X represents a bond;

or
(ii) X-A-Y- together represent -O-;

and wherein
$R^1$ is selected from:

(a) -H,
(b) $-(C_1-C_6)$alkyl, which is optionally substituted with halogen, nitril, $-OR^6$, $-SR^6$, or $-COOR^6$; the number of said substituents being up to three for halogen, and up to two for any combination of said halogen, nitril, $-OR^6$, $-SR^6$, or $-COOR^6$ moieties,
(c) -phenyl, which is optionally substituted with halogen, nitril, $-OR^6$, $-SR^6$, $-R^6$, or $-COOR^6$, the number of said substituents being up to perhalo for halogen, and up to two for any combination of said halogen, nitril, $-OR^6$, $-SR^6$, $-R^6$ or $-COOR^6$ moieties,
(d) $-(C_1-C_4)$alkyl-phenyl, in which the alkyl portion is optionally substituted with up to three halogens; and the phenyl portion is optionally substituted with halogen, nitril, $-OR^6$, $-SR^6$, $-R^6$ or $-COOR^6$, the number of substituents on said phenyl portion being up to perhalo for halogen, and up to two for any combination of said halogen, nitril, $-OR^6$, $-SR^6$, $-R^6$ or $-COOR^6$ moieties,
(e) $-SO_2-NR^3R^{3'}$,
(f) $-CO-NR^3R^{3'}$,
(g) $-PO(OR^{16})-R^{3'}$,

(h) $-PS(OR^{16})-R^3$,
(i) $-PO(OR^{16})-O-R^3$
(j) $-SO_2-R^3$, and
(k) $-SO_2-O-R^3$;

wherein
$R^6$ represents H, $-(C_1-C_4)$alkyl or halogenated $-(C_1-C_4)$alkyl;
$R^3$ and $R^{3'}$ are independently selected from H, alkyl, aryl and arylalkyl, or $R^3$ and $R^{3'}$ form together with the nitrogen atom, to which $R^3$ and $R^{3'}$ are attached, a heterocyclic 4-, 5-, 6-, 7-or 8-memberred ring, which is optionally saturated, partly unsaturated, or aromatic; which optionally contains up to three additional heteroatoms selected from N, O or S, the number of additional N atoms being 0, 1, 2 or 3 and the number of O and S atoms each being 0, 1 or 2; and
$R^{16}$ represents -H, alkyl, or arylalkyl;
$R^2$ and $R^4$ are independently selected from:

(a) -H, wherein if X represents a bond, A represents -CO- and Y represents -O- or a bond, then $R^2$ is different from -H;
(b) optionally substituted alkyl,
(c) optionally substituted acyl, under the proviso that Y represents $-NH-NR^4-$,
(d) optionally substituted aryl,
(e) optionally substituted heteroaryl, and
(f) optionally substituted cycloheteroalkyl,

or, under the proviso that Y represents $-NR^4-$, $-NH-NR^4-$ or $-NH-SO_2-NR^4-$,
$R^2$ and $R^4$ form together with the nitrogen atom, to which $R^2$ and $R^4$ are attached, a heterocyclic 4-, 5-, 6-, 7- or 8-memberred ring, which is optionally saturated, partly unsaturated, or aromatic; which optionally contains up to three additional heteroatoms selected from N, O or S, the number of additional N atoms being 0, 1, 2 or 3 and the number of O and S atoms each being 0, 1 or 2; and which ring is optionally part of a multiple condensed ring-system, wherein the ring or the ring-system is optionally substituted;
the substituents $R^{10}$, $R^{11}$, $R^{12}$ and $R^{13}$ together with the carbon atoms, to which they are attached, form a structure $-CR^{13}R^{12}-CR^{11}R^{10}-$, wherein

(a) $R^{10}$ and $R^{11}$ both represent -H and $R^{12}$ and $R^{13}$ together represent a group selected from =O, $=CF_2$, =N-O-alkyl, and =N-OH, or
(b) $R^{10}$ and $R^{11}$ both represent -H, and $R^{12}$ and $R^{13}$ both individually represent -F, or
(c) $R^{10}$, $R^{11}$ and $R^{13}$ all represent -H, and $R^{12}$ is selected from -OH, -CN, -F, $-CF_3$, and $-CF_2H$, or
(d) $R^{10}$ represents -H, $R^{11}$ together with $R^{13}$ forms a bond, and $R^{12}$ is selected from -CN, -F, $-CF_3$, and $-CF_2H$; or
(e) $R^{10}$ represents -H, $R^{11}$ represents -CHO, and $R^{12}$ and $R^{13}$ together represent =O;

or, the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring, which is partly unsaturated or aromatic, which contains one, two or three heteroatoms independently selected from N, O or S, the number of N atoms being 0, 1, 2 or 3 and the number of O and S atoms each being 0, 1 or 2, wherein one heteroatom is directly attached to the C17 C-atom of the steroidal core;
and which ring is optionally substituted with an alkyl group;
$R^{14}$ represents an alkyl, alkoxy, or alkoxy-alkyl group, or
under the proviso that at least

(i) $R^1$ represents $-SO_2-NR^3R^{3'}$ $-CO-NR^3R^{3'}$, $-PO(OR^{16})-R^3$, $-PS(OR^{16})-R^3$, $-PO(OR^{16})-OR^3$, $-SO_2-R^3$, or $-SO_2-OR^3$; or
(ii) $R^{10}$ or $R^{11}$ is different from -H, or
(iii) $R^{10}$, $R^{11}$ and $R^{13}$ all represent -H, and $R^{12}$ does not represent -OH, or
(iv) $R^{12}$ and $R^{13}$ together do not represent =O,

then $R^{14}$ may represent -H; and
n represents 0, 1, 2, 3, 4, 5 or 6, wherein, if X represents -NH- or -O-, then n is different from 0, and all stereoisomers and, pharmacologically acceptable salts thereof.
Accordingly, the present inventions relates to a compound of the general formula I, wherein -X-A-Y- together represent a group selected from

(a) $-CO-NR^4-$,
(b) -CO-O-,
(c) -CO-,
(d) $-CO-NH-NR^4-$,
(e) $-NH-CO-NR^4-$, preferably -NH-CO-NH-,
(f) -NH-CO-O-,
(g) -NH-CO-,
(h) $-NH-CO-NH-SO_2-$,
(i) $-NH-SO_2-NR^4-$, preferably $-NH-SO_2-NH-$,
(j) $-NH-SO_2-O-$,
(k) $-NH-SO_2-$
(l) $-O-CO-NR^4-$, preferably -O-CO-NH-,
(m) -O-CO-,
(n) $-O-CO-NH-SO_2-NR^4-$, and
(o) -O-;

n represents 1, 2, 3, 4, 5 or 6, or, if -X-A-Y- represents $-CO-NR^4-$, -CO-O-, -CO-, or $-CO-NH-NR^4-$, then n may also represent 0;
$R^1$ is selected from:

(a) -H,
(b) $-(C_1-C_6)$alkyl, which is optionally substituted with halogen, nitril, $-OR^6$, $-SR^6$, or $-COOR^6$; the number of said substituents being up to three for halogen, and up to two for any combination of said halogen, nitril, $-OR^6$, $-SR^6$, or $-COOR^6$ moieties,
(c) -phenyl, which is optionally substituted with halogen, nitril, $-OR^6$, $-SR^6$, $-R^6$, or $-COOR^6$, the number of said substituents being up to perhalo for halogen, and up to two for any combination of said halogen, nitril, $-OR^6$, $-SR^6$, $-R^6$ or $-COOR^6$ moieties,
(d) $-(C_1-C_4)$alkyl-phenyl, in which the alkyl portion is optionally substituted with up to three halogens; and the phenyl portion is optionally substituted with halogen, nitril, $-OR^6$, $-SR^6$, $-R^6$ or $-COOR^6$, the number of substituents on said phenyl portion being up to perhalo for halogen, and up to two for any combination of said halogen, nitril, $-OR^6$, $-SR^6$, $-R^6$ or $-COOR^6$ moieties,
(e) $-SO_2NR^3R^{3'}$,
(f) $-CO-NR^3R^{3'}$,
(g) $-PO(OR^{16})-R^3$,
(h) $-PS(OR^{16})-R^3$,
(i) $-PO(OR^{16})-O-R^3$
(j) $-SO_2-R^3$, and
(k) $-SO_2-O-R^3$;

wherein
$R^6$ represents H, $-(C_1-C_4)$alkyl or halogenated $-(C_1-C_4)$alkyl;
$R^3$ and $R^{3'}$ are independently selected from H, alkyl, aryl and arylalkyl, or $R^3$ and $R^{3'}$ form together with the nitrogen atom, to which $R^3$ and $R^{3'}$ are attached, a heterocyclic 4-, 5-, 6-, 7-or 8-memberred ring, which is optionally saturated, partly unsaturated, or aromatic; which optionally contains up to three additional heteroatoms selected from N, O and S, the number of additional N atoms being 0, 1, 2 or 3 and the number of O and S atoms each being 0, 1 or 2; and
$R^{16}$ represents -H, alkyl, or arylalkyl;
$R^2$ and $R^4$ are independently selected from:

(a) -H,
(b) optionally substituted alkyl,
(c) optionally substituted acyl, under the proviso that -X-A-Y- represent $-CO-NH-NR^4-$,
(d) optionally substituted aryl or arylalkyl,
(e) optionally substituted heteroaryl or heteroarylalkyl, and
(f) optionally substituted cycloheteroalkyl or cycloheteroalkyl-alkyl,

or $R^2$ and $R^4$ form together with the nitrogen atom, to which $R^2$ and $R^4$ are attached, a heterocyclic 4-, 5-, 6-, 7- or 8-memberred ring, which is optionally saturated, partly unsaturated, or aromatic; which optionally contains up to three additional heteroatoms selected from N, O and S, the number of additional N atoms being 0, 1, 2 or 3 and the number

of O and S atoms each being 0, 1 or 2; and which ring is optionally part of a multiple condensed ring-system, wherein the ring or the ring-system is optionally substituted;
the substituents $R^{10}$, $R^{11}$, $R^{12}$ and $R^{13}$ together with the carbon atoms, to which they are attached, form a structure

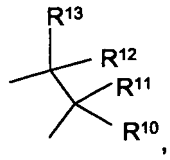

which is selected from the group of

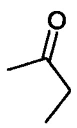
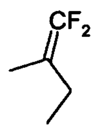
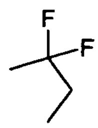
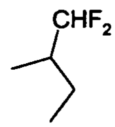
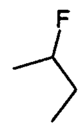

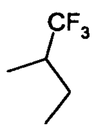
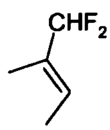
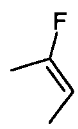
and
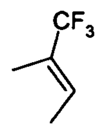

or, the substituents $R^{10}$, $R^{11}$, $R^{12}$ and $R^{13}$ together with the carbon atoms, to which they are attached, form a heterocyclic 5- or 6-membered ring, which is partly unsaturated or aromatic, which contains one, two or three heteroatoms independently selected from N, O and S, the number of N atoms being 0, 1, 2 or 3 and the number of O and S atoms each being 0, 1 or 2, wherein one heteroatom is directly attached to the C17 C-atom of the steroidal core; and which ring is optionally substituted with an alkyl group;
$R^{14}$ represents an alkyl, alkoxy, or alkoxy-alkyl group, or
$R^{14}$ may also represent -H, under the proviso that at least

(i) $R^1$ represents $-SO_2-NR^3R^{3'}$, $-CO-NR^3R^{3'}$, $-PO(OR^{16})-R^3$, $-PS(OR^{16})-R^3$, $-PO(OR^{16})-OR^3$, $-SO_2-R^3$, or $-SO_2-OR^3$; or
(ii)

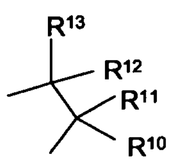
is different from
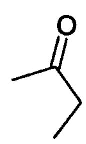

and all pharmacologically acceptable salts thereof.

In one embodiment, the present invention relates to a compound of the general formula I, which is an optically pure 15α enantiomer having the formula (II)

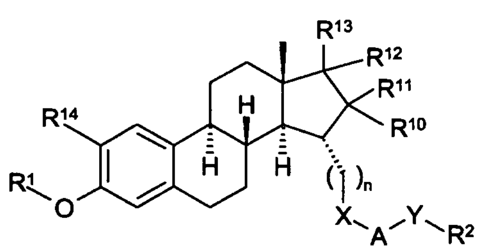
(II)

or a physiologically acceptable salt thereof. In a further embodiment, the present invention relates to the 15α enantiomer having formula (II), wherein n represents 1, 2, 3 or 4.

In another embodiment, the present invention relates to a compound of the general formula I, which is an optically pure 15β enantiomer having the formula (III)

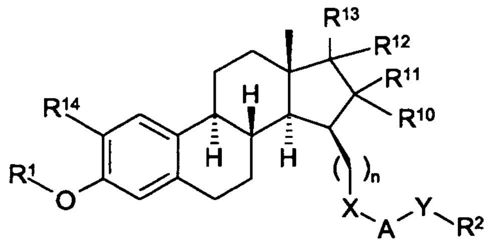

(III)

or a physiologically acceptable salt thereof. In a further embodiment, the present invention relates to the 15β enantiomer having formula (III), wherein n represents 2, 3, 4, or 5.

One embodiment of the present invention relates to compounds of formula I, wherein $R^1$ is selected from:

(a) $-SO_2-NR^3R^{3'}$,
(b) $-CO-NR^3R^{3'}$,
(c) $-PO(OR^{16})-R^3$,
(d) $-PS(OR^{16})-R^3$,
(e) $-PO(OR^{16})-O-R^3$,
(f) $-SO_2-R^3$; and
(g) $-SO_2-O-R^3$;

wherein
$R^3$ and $R^{3'}$ are independently selected from H, alkyl, aryl and arylalkyl, or $R^3$ and $R^{3'}$ form together with the nitrogen atom, to which $R^3$ and $R^{3'}$ are attached, a heterocyclic 4-, 5-, 6-, 7- or 8-memberred ring, which is optionally saturated, partly unsaturated, or aromatic; which optionally contains up to three additional heteroatoms selected from N, O or S, the number of additional N atoms being 0, 1, 2 or 3 and the number of O and S atoms each being 0, 1 or 2; and
$R^{16}$ represents -H, alkyl, or arylalkyl.

In a preferred subgroup of this embodiment, $R^{10}$ and $R^{11}$ both represent -H and $R^{12}$ and $R^{13}$ together represent =O; and/or $R^{14}$ represents -H, $-(C_1-C_8)$alkyl, $-O-(C_1-C_8)$alkyl, or $-(C_1-C_8)$alkyl-$O-C_1-C_8)$alkyl.

In this embodiment, $R^3$ and $R^{3'}$ are preferably independently selected from -H, $-(C_1-C_8)$alkyl, phenyl and $-(C_1-C_4)$alkyl-phenyl, or
$R^3$ and $R^{3'}$ together with the nitrogen atom, to which $R^3$ and $R^{3'}$ are attached, form a heterocyclic 4-, 5-, 6-, 7- or 8-memberred ring, which is selected from the group consisting of

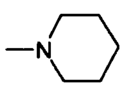 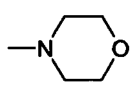 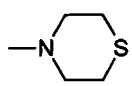 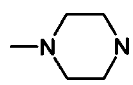

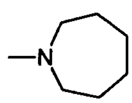 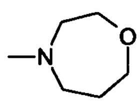 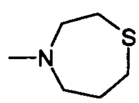 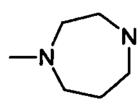

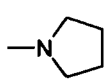 and 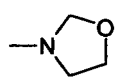 ,

and
$R^{16}$ represents -H, $-(C_1-C_4)$alkyl, or $-(C_1-C_4)$alkyl-phenyl, preferably -H.

Particularly preferred compounds are those, wherein $R^1$ is selected from $-SO_2-NR^3R^{3'}$, $CO-NR^3R^{3'}$, $-PO(OR^{16})-R^3$, and $-SO_2-R^3$; preferably $-SO_2-NR^3R^{3'}$, wherein $R^3$ and $R^{3'}$ together with the nitrogen atom, to which $R^3$ and $R^{3'}$ are attached, form a heterocyclic ring selected from morpholine, thiomorpholine and piperazyl, and even more preferred $-SO_2-NH_2$. Preferably, those compounds carry a substituent $R^{14}$ representing -H.

One embodiment of the present invention relates to compounds of formula I, wherein $R^{14}$ represents an alkyl, alkoxy, or alkoxy-alkyl group.

In a further embodiment, the invention relates compounds of the general formula I, wherein
$R^1$ represents -H, $(C_1\text{-}C_4)$alkyl, or $-(C_1\text{-}C_4)$alkyl-phenyl;
$R^{10}$ and $R^{11}$ both represent -H and $R^{12}$ and $R^{13}$ together represent =O; and
$R^{14}$ represents $-(C_1\text{-}C_8)$alkyl, $-O-(C_1\text{-}C_8)$alkyl, or $-(C_1\text{-}C_8)$alkyl-$O-(C_1\text{-}C_8)$alkyl.

In this context, mostly preferred are compounds of the general formula I, wherein $R^{14}$ represents $-(C_1\text{-}C_4)$alkyl, $-O(C_1\text{-}C_4)$alkyl, or $-(C_1\text{-}C_4)$alkyl-$O\text{-}C_1\text{-}C_4)$alkyl, preferably ethyl, propyl, methoxyethyl, methoxy, ethoxy or methoxyethoxy, and $R^1$ is independently selected from -H, $(C_1\text{-}C_4)$alkyl, preferably methyl, and phenyl$(C_1\text{-}C_4)$alkyl, preferably benzyl, most preferred -H.

A further preferred embodiment of the present invention relates to compounds of the general formula I, wherein the substituents $R^{10}$, $R^{11}$, $R^{12}$ and $R^{13}$ together with the carbon atoms, to which they are attached, form a structure

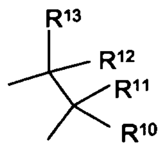

which is selected from the group of

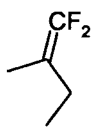 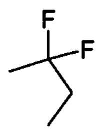 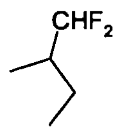 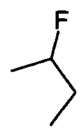

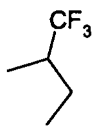 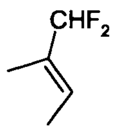 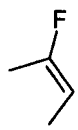 and 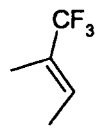.

In a subgroup of this embodiment, additionally $R^{14}$ represents -H, $-(C_1\text{-}C_8)$alkyl, $-O-(C_1\text{-}C_8)$alkyl, or $-(C_1\text{-}C_8)$alkyl-$O-(C_1\text{-}C_8)$alkyl, and $R^1$ represents -H, $(C_1\text{-}C_4)$alkyl, or $-(C_1\text{-}C_4)$alkylphenyl;

In this context are those compounds preferred, wherein the substituents $R^{10}$, $R^{11}$, $R^{12}$ and $R^{13}$ together with the carbon atoms, to which they are attached, form a structure

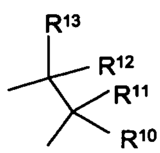,

which is selected from the group of

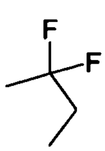 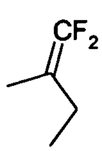 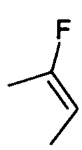 and 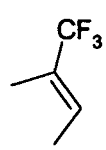.

Preferably, those compounds carry substituents $R^1$ and $R^{14}$ which are each individually -H.

In an additional embodiment of the present invention, compounds of the general formula I are disclosed, wherein the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring, which is partly unsaturated or aromatic, which contains one, two or three heteroatoms independently selected from N, O or S, the number of N atoms being 0, 1, 2 or 3 and the number of O and S atoms each being 0, 1 or 2, wherein one heteroatom is directly attached to the C17 C-atom of the steroidal core; and which ring is optionally substituted with an alkyl group; and

In a subgroup of this embodiment, additionally $R^1$ represents -H, $(C_1\text{-}C_4)$alkyl, or $-(C_1\text{-}C_4)$alkyl-phenyl, and $R^{14}$ represents -H, $-(C_1\text{-}C_8)$alkyl, $-O-(C_1\text{-}C_8)$alkyl, or $-(C_1\text{-}C_8)$alkyl-$O-(C_1\text{-}C_8)$alkyl.

In this context, the present invention preferably relates to compounds, wherein the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring to provide a compound of one of the following formulas

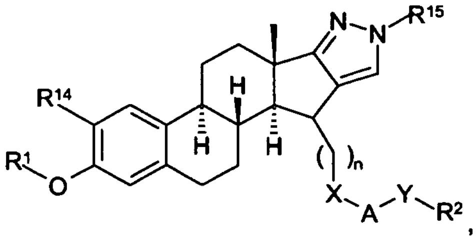

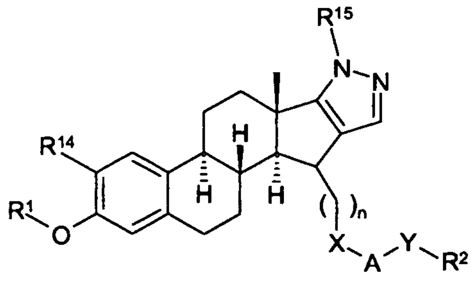

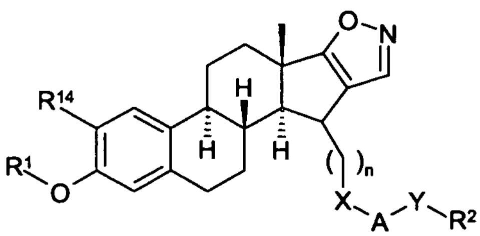

, or

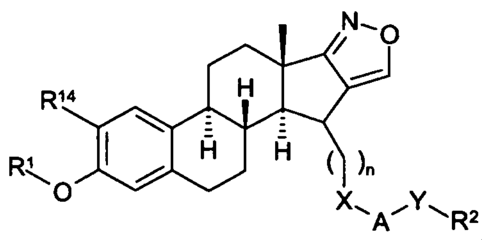

wherein $R^{15}$ represents -H or $-(C_1\text{-}C_4)$alkyl, preferably methyl or -H. Preferably, those compounds carry substituents $R^1$ and $R^{14}$ each individually representing -H.

One embodiment of the present invention relates to compounds of formula I, wherein

- $R^1$ is selected from:

  (a) -H,
  (b) $-(C_1\text{-}C_6)$alkyl,
  (c) -phenyl,
  (d) $-(C_1\text{-}C_4)$alkyl-phenyl,
  (e) $-SO_2-NR^3R^{3'}$,
  (f) $-CO-NR^3R^{3'}$,
  (g) $-PO(OH)-R^3$,
  (h) $-PS(OH)-R^3$,
  (i) $-PO(OH)-O-R^3$
  (j) $-SO_2-R^3$, and
  (k) $-SO_2-O-R^3$;

  wherein
  $R^6$ represents H, $-(C_1\text{-}C_4)$alkyl or halogenated $-(C_1\text{-}C_4)$alkyl;
  $R^3$ and $R^{3'}$ are independently selected from -H, $-(C_1\text{-}C_8)$alkyl, phenyl and $-(C_1\text{-}C_4)$alkylphenyl, or $R^3$ and $R^{3'}$ together with the nitrogen atom, to which $R^3$ and $R^{3'}$ are attached, form a heterocyclic 4-, 5-, 6-, 7- or 8-memberred ring, which is selected from the group consisting of

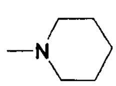 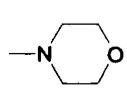 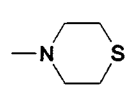 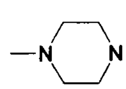

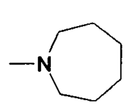 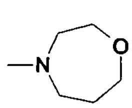 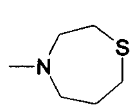 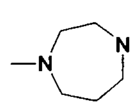

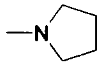 and 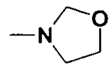 ,

even more preferred selected from morpholine, thiomorpholine and piperazyl,

- the substituents $R^{10}$, $R^{11}$, $R^{12}$ and $R^{13}$ together with the carbon atoms, to which they are attached, form a structure

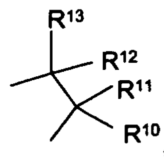,

which is selected from the group of

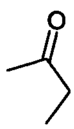 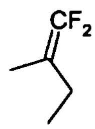 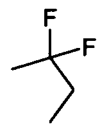 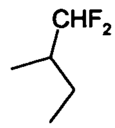 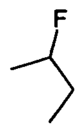

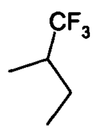 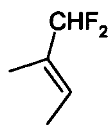 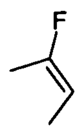 and 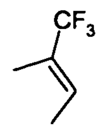

or, the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring to provide a compound of one of the following formulas

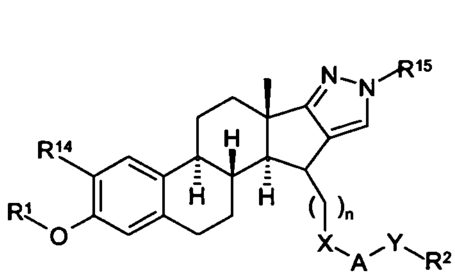, 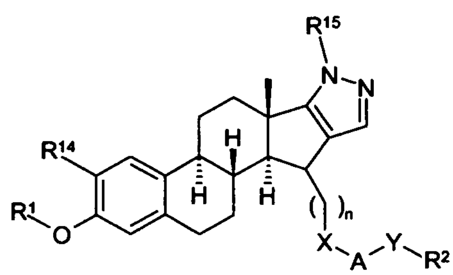,

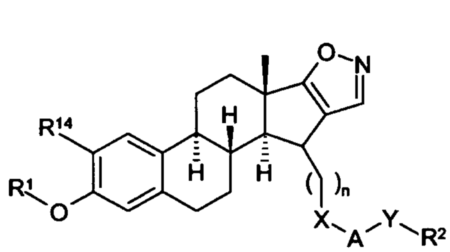, or 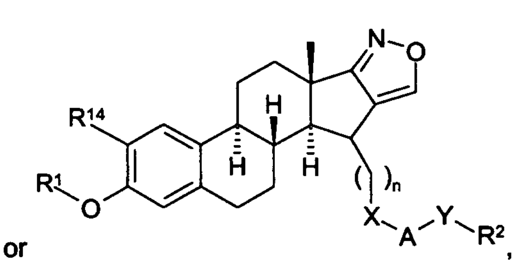,

wherein $R^{15}$ represents -H or -($C_1$-$C_4$)alkyl; and

- $R^{14}$ represents -($C_1$-$C_4$)alkyl, -O-($C_1$-$C_4$)alkyl, or -($C_1$-$C_4$)alkyl-O-($C_1$-$C_4$)alkyl, or $R^{14}$ may also represent -H, under the proviso that at least

(i) $R^1$ represents -$SO_2$-$NR^3R^{3'}$, -CO-$NR^3R^{3'}$, -PO(OH)-$R^3$, -PS(OH)-$R^3$, -PO(OH)-$OR^3$, -$SO_2$-$R^3$, or -$SO_2$-$OR^3$; or

(ii)

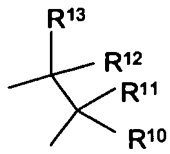 is different from 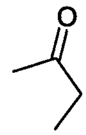

A further embodiment of the present invention relates to compounds of the formula (I), wherein
$R^1$ represents -H or $-SO_2-NH_2$,
the substituents $R^{10}$, $R^{11}$, $R^{12}$ and $R^{13}$ together with the carbon atoms, to which they are attached, form a structure

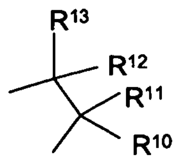,

which is selected from the group of

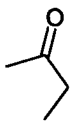 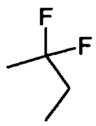 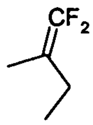 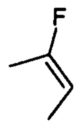 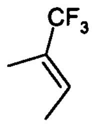 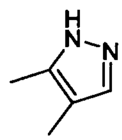 and 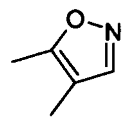 ;

and
$R^{14}$ represents -H, $-(C_1-C_4)$alkyl, $-O-(C_1-C_4)$alkyl, or $-(C_1-C_4)$alkyl-$O-(C_1-C_4)$alkyl.
$R^{14}$ in particular may represent -H, when at least

(i) $R^1$ represents $-SO_2-NH_2$, or
(ii)

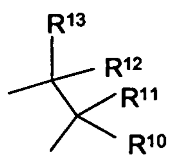 is different from 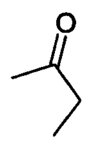

A preferred embodiment of the present invention relates to compounds of formula I, wherein $R^2$ and $R^4$ are independently selected from:

(a) -H, wherein if -X-A-Y- together represents -CO-O- or -CO-, then $R^2$ is different from -H,
(b) $-(C_1-C_{12})$alkyl, optionally substituted with up to five substituents independently selected from the group consisting of halogen, hydroxyl, thiol, nitrile, alkoxy, aryloxy, arylalkyloxy, amino, amido, alkylthio, arylthio, arylalkylthio, sulfamoyl, sulfonamide, acyl, carboxyl, acylamino,
aryl, which aryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, carboxyl-$(C_1-C_6)$alkyl, thiol, nitrile, sulfamoyl, sulfonamide, carboxyl, aryloxy, arylalkyloxy, $(C_1-C_6)$alkylthio, arylthio, arylalkylthio, amino, amido, acyl, acylamino and heteroaryl; or which aryl is optionally substituted by two groups which are attached to adjacent carbon atoms and are combined into a saturated or partly unsaturated cyclic 5, 6, 7, or 8-membered ring system, optionally containing up to three heteroatoms selected from N, O and S, the number of N atoms being 0, 1, 2 or 3 and the number of O and S atoms each being being 0, 1 or 2;
heteroaryl, which heteroaryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, carboxyl-$(C_1-C_6)$alkyl, thiol, nitrile, sulfamoyl, sulfonamide, carboxyl, aryloxy, arylalkyloxy, $(C_1-C_6)$alkylthio, arylthio, arylalkylthio, amino, amido, acyl, acylamino, aryl-$(C_1-C_4)$-alkyl and aryl;
whereby each aryl group is optionally substituted with up to three substituents independently selected from the group consisting of hydroxyl, halogen, $(C_1-C_6)$alkoxy, $(C_1-C_6)$alkyl, halogenated $(C_1-C_6)$alkyl and halogenated

$(C_1-C_6)$alkoxy; and
cycloheteroalkyl, which cycloheteroalkyl group is optionally substituted with up to three substituents independently selected from the group consisting of oxo, $(C_1-C_8)$-alkyl, aryl, aryl-$(C_1-C_4)$-alkyl, hydroxyl, $(C_1-C_6)$alkoxy, carboxyl-$(C_1-C_6)$alkyl, thiol, nitrile, sulfamoyl, sulfonamide, carboxyl, aryloxy, arylalkyloxy, $(C_1-C_6)$alkylthio, arylthio, arylalkylthio, amino, amido, acyl, and acylamino,
whereby each aryl group is optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, and halogenated $(C_1-C_4)$-alkoxy);
(c) acyl -(C=O)-R', wherein R' represents hydrogen, $(C_1-C_4)$alkyl, aryl, or aryl-$(C_1-C_4)$alkyl, or heteroaryl-$(C_1-C_4)$alkyl; which aryl or aryl-$(C_1-C_4)$alkyl is optionally substituted in the aryl, preferably phenyl, moiety with up to three substituents independently selected from the group consisting of hydroxyl, halogen, $(C_1-C_4)$alkoxy, $(C_1-C_4)$-alkyl or halogenated $(C_1-C_4)$alkyl;
(d) aryl,
which aryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, carboxyl-$(C_1-C_6)$alkyl, thiol, nitrile, nitro, sulfamoyl, sulfonamide, carboxyl, aryloxy, arylalkyloxy, $(C_1-C_6)$alkylsulfonyl, arylsulfonyl, $(C_1-C_6)$alkylthio, arylthio, arylalkylthio, amino, amido, acyl, acylamino and heteroaryl; or
which aryl is optionally substituted by two groups which are attached to adjacent carbon atoms and are combined into a saturated or partly unsaturated cyclic 5, 6, 7, or 8-membered ring system, optionally containing up to three heteroatoms selected from N, O and S, the number of N atoms being 0, 1, 2 or 3 and the number of O and S atoms each being 0, 1 or2;
(e) heteroaryl,
which heteroaryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, carboxyl-$(C_1-C_6)$alkyl, thiol, nitrile, sulfamoyl, sulfonamide, arylsulfoxy, carboxyl, aryloxy, arylalkyloxy, $(C_1-C_6)$alkylsulfonyl, arylsulfonyl, $(C_1-C_6)$alkylthio, arylthio, arylalkylthio, amino, amido, acyl, acylamino, aryl-$(C_1-C_4)$-alkyl and aryl,
whereby each aryl group is optionally substituted with up to three substituents independently selected from the group consisting of hydroxyl, halogen, $(C_1-C_6)$alkoxy, $(C_1-C_6)$alkyl, halogenated $(C_1-C_6)$alkyl and halogenated $(C_1-C_6)$alkoxy; or
(f) cycloheteroalkyl,

which cycloheteroalkyl is optionally substituted with up to three substituents independently selected from the group consisting of oxo, $(C_1-C_{14})$-alkyl, aryl, aryl-$(C_1-C_4)$-alkyl, hydroxyl, $(C_1-C_6)$alkoxy, carboxyl-$(C_1-C_6)$alkyl, thiol, nitrile, sulfamoyl, sulfonamide, carboxyl, aryloxy, arylalkyloxy, $(C_1-C_6)$alkylthio, arylthio, arylalkylthio, amino, amido, acyl, and acylamino,
whereby each aryl group is optionally further substituted with up to three substituents independently selected from the group consisting of hydroxyl, halogen, $(C_1-C_4)$-alkyl, $(C_1-C_4)$-alkoxy, halogenated $(C_1-C_4)$-alkyl, and halogenated $(C_1-C_4)$-alkoxy;
or wherein, $R^2$ and $R^4$ form together with the nitrogen atom, to which $R^2$ and $R^4$ are attached, a heterocyclic 4-, 5-, 6-, 7- or 8-memberred ring, which is optionally saturated or partly unsaturated; which optionally contains up to three additional heteroatoms selected from N, O and S, the number of additional N atoms being 0, 1, 2 or 3 and the number of O and S atoms each being 0, 1 or 2; and which ring is optionally part of a multiple condensed ring-system,
wherein the ring or the ring-system is optionally substituted

(i) with up to three substituents independently selected from the group consisting of $(C_1-C_8)$-alkyl, halogen, hydroxyl, carboxyl, thiol, nitrile, $(C_1-C_6)$-alkoxy, carboxyl-$(C_1-C_6)$alkyl, aryloxy, arylalkyloxy, amino, amido, alkylthio, arylthio, arylalkylthio, sulfamoyl, sulfonamide, aryl, aryl-$(C_1-C_4)$-alkyl, heteroaryl, and cycloheteroalkyl,
wherein the $(C_1-C_8)$-alkyl group is optionally substituted with up to three substituents independently selected among hydroxyl, halogen, $(C_1-C_4)$-alkoxy, or halogenated $(C_1-C_4)$-alkoxy,
whereby the alkyl-chain of the $(C_1-C_4)$-alkoxy moiety is optionally substituted with hydroxyl;
wherein the aryl group or aryl moiety is optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, halogenated $(C_1-C_4)$-alkoxy and carboxyl-$(C_1-C_6)$alkyl, or wherein the aryl moiety is optionally substituted by two groups which are attached to adjacent carbon atoms and are combined into a saturated or partly unsaturated cyclic 5, 6, 7, or 8 membered ring system, optionally containing up to three heteroatoms selected from N, O and S, the number of N atoms being 0, 1, 2 or 3 and the number of O and S atoms each being 0, 1 or 2;
wherein the heteroaryl group is optionally substituted with up to three substituents independently selected from the

group consisting of hydroxyl, halogen, $(C_1-C_4)$-alkyl, $(C_1-C_4$-alkoxy, halogenated $(C_1-C_4)$-alkyl, halogenated $(C_1-C_4$-alkoxy) and carboxyl-$(C_1-C_6)$alkyl;

wherein the cycloheteroalkyl is optionally substituted with up to three substituents independently selected from the group consisting of oxo, $(C_1-C_8)$-alkyl, aryl, aryl-$(C_1-C_4)$-alkyl, hydroxyl, $(C_1-C_6)$alkoxy, carboxyl-$(C_1-C_6)$alkyl, and carboxyl,

whereby each aryl group is optionally further substituted with up to three substituents independently selected from the group consisting of hydroxyl, halogen, $(C_1-C_4)$-alkyl, $(C_1-C_4)$-alkoxy, halogenated $(C_1-C_4)$-alkyl, and halogenated $(C_1-C_4)$-alkoxy); or

(ii) by two groups which are attached to the same carbon atom and are combined into a saturated or partly unsaturated cyclic 4, 5, 6, 7, or 8-membered ring system, optionally containing up to three heteroatoms selected from N, O and S, the number of N atoms being 0, 1, 2 or 3 and the number of O and S atoms each being 0, 1 or 2,

whereby the cyclic ring system is optionally substituted by up to two substituents independently selected from oxo, $(C_1-C_6)$-alkyl, aryl and aryl-$(C_1-C_4)$-alkyl;

and wherein n represents

(a) 1, 2, 3, 4, 5 or 6, if -X-A-Y- together represent -NH-CO-NH-, -NH-CO-O-, -NH-CO-, -NH-CO-NH-$SO_2$-, -NH-$SO_2$-NH-, -NH-$SO_2$-O-, -NH-$SO_2$-, -O-CO-NH-, -O-CO-, -O-CO-NH-$SO_2$-$NR^4$-, or -O-, or
(b) 0, 1, 2, 3, 4, or 5, if -X-A-Y- together represent -CO-$NR^4$-, -CO-O-, -CO-, or -CO-NH-$NR^4$-.

In one preferred embodiment of the present invention, the residues $R^2$ and $R^4$ in the compounds of the general formula I may independently represent -H, wherein, if -X-A-Y- together represents -CO-O- or -CO-, then $R^2$ is different from -H.

In a further embodiment of the present invention relates to compounds of formula I, wherein $R^2$ and $R^4$ are independently selected from:

(a) -$(C_1-C_{12})$alkyl, optionally substituted with up to five substituents independently selected from the group consisting of halogen, hydroxyl, nitrile, -O-$R^7$; -O-$Ar^1$, -O-$(C_1-C_4)$alkyl-$Ar^1$, alkylamino, alkylamido, -S-$R^7$, -S-$Ar^1$, -S-$(C_1-C_4)$alkyl-$Ar^1$, -(C=O)-$OR^8$, aryl, heteroaryl, and cycloheteroalkyl,

wherein the aryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, -$(C_1-C_6)$alkyl-(C=O)-$OR^8$, nitrile, sulfamoyl, -(C=O)-$OR^8$, -O-$Ar^1$, -O-$(C_1-C_4)$alkyl-$Ar^1$, $(C_1-C_6)$alkylthio, -S-$Ar^1$, -S-$(C_1-C_4)$alkyl-$Ar^1$, alkylamino, and alkylamido; or wherein the aryl is optionally substituted by two groups which are attached to adjacent carbon atoms and are combined into a saturated cyclic 5 or 6 membered ring system, optionally containing up to three heteroatoms selected from N or O, the number of N atoms being 0, 1, 2 or-3 and the number of O atoms each being 0, 1, or 2;

wherein the heteroaryl is optionally substituted with up to three substituents independently selected from the group consisting of halogen, hydroxyl, $(C_1-C_6)$alkoxy, $(C_1-C_6)$-alkyl, halogenated $(C_1-C_6)$alkyl, halogenated $(C_1-C_6)$alkoxy, -$(C_1-C_6)$alkyl-(C=O)-$OR^8$, nitrile, sulfamoyl, -(C=O)-$OR^8$, -O-$Ar^1$, -O-$(C_1-C_4)$alkyl-$Ar^1$ $(C_1-C_6)$-alkylthio, -S-$Ar^1$, -S-$(C_1-C_4)$alkyl-$Ar^1$, alkylamino, alkylamido, -$(C_1-C_4)$alkyl-$Ar^1$ and $Ar^1$; and

wherein the cycloheteroalkyl group is optionally substituted with up to three substituents independently selected from the group consisting of oxo, $(C_1-C_8)$-alkyl, $Ar^1$, -$(C_1-C_4)$-alkyl-$Ar^1$, hydroxyl, $(C_1-C_6)$alkoxy, -$(C_1-C_6)$alkyl-(C=O)-$OR^8$, nitrile, -(C=O)-$OR^8$, -O-$Ar^1$, -O-$(C_1-C_4)$alkyl-$Ar^1$, $(C_1-C_6)$alkylthio, -S-$Ar^1$, -S-$(C_1-C_4)$alky)-$Ar^1$, alkylamino and alkylamido;

(b) aryl,

which aryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, -$C_1-C_6$) alkyl-(C=O)-$OR^8$, nitro, nitrile, sulfamoyl, -(C=O)-$OR^8$, -(C=O)-$R^8$, -O-$Ar^1$, -O-$(C_1-C_4)$alkyl-$Ar^1$, $(C_1-C_6)$-alkylthio, -S-$Ar^1$, -S-$(C_1-C_4)$alkyl-$Ar^1$, $(C_1-C_6)$alkylsulfonyl, -$SO_2$-$Ar^1$, alkylamino, alkylamide, -NH-CO-$R^8$, $Ar^1$ and heteroaryl; or which aryl is optionally substituted by two groups which are attached to adjacent carbon atoms and are combined into a saturated cyclic 5 or 6 membered ring system, optionally containing up to three heteroatoms selected from N and O, the number of N atoms being 0, 1, 2 or 3 and the number of O atoms being 0, 1 or 2;

(c) heteroaryl,

which heteroaryl is optionally substituted with up to three substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$alkoxy, -$(C_1-C_6)$alkyl-(C=O)-$OR^8$, nitrile, sulfamoyl, -(C=O)-$OR^8$, -O-$Ar^1$, -O-$(C_1-C_4)$alkyl-$Ar^1$, $(C_1-C_6)$alkylthio, -S-$Ar^1$, -S-$(C_1-C_4)$alkyl-$Ar^1$, $(C_1-C_6)$alkylsulfonyl, -$SO_2$-$Ar^1$, alkylamino, alkylamido, - $(C_1-C_4)$alkyl-$Ar^1$ and $Ar^1$; or

(d) cycloheteroalkyl,

which cycloheteroalkyl group is optionally substituted with up to three substituents independently selected from the

group consisting of oxo, $(C_1-C_8)$-alkyl, $Ar^1$,-$(C_1-C_4)$alkyl-Ar1, hydroxyl, $(C_1-C_6)$alkoxy, -$(C_1-C_6)$alkyl-(C=O)-$OR^8$, nitrile, -(C=O)-$OR^8$, -O-$Ar^1$, -O-$(C_1-C_4)$alkyl-$Ar^1$, $(C_1-C_6)$alkylthio, -S-$Ar^1$, -S-$(C_1-C_4)$alkyl-$Ar^1$, alkylamino and alkylamido;

wherein

$R^7$ represents $(C_1-C_6)$alkyl, optionally substituted with up to three hydroxy groups in the alkyl chain or halogenated $(C_1-C_6)$alkyl,

$R^8$ represents hydrogen, $(C_1-C_4)$alkyl, phenyl, or $(C_1-C_4)$alkyl-phenyl, wherein the phenyl-moiety is optionally substituted with up to three substituents independently selected from the group consisting of hydroxyl, halogen, $(C_1-C_4)$alkoxy, $(C_1-C_4)$-alkyl, halogenated $(C_1-C_4)$alkyl and halogenated $(C_1-C_4)$alkoxy; and

$Ar^1$ represents phenyl or naphthyl, which are optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, or halogenated $(C_1-C_4)$-alkoxy;

or wherein the ring or ringsystem formed by $R^2$ and $R^4$ together with the nitrogen atom, to which $R^2$ and $R^4$ are attached, is selected from the group consisting of

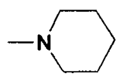 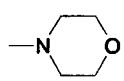 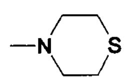 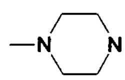 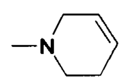

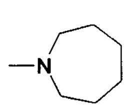 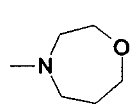 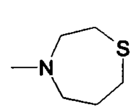 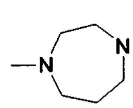

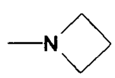 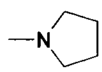 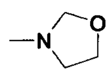 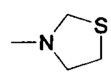 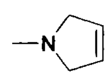

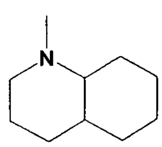 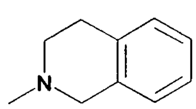 and 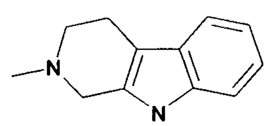

wherein the ring or the ring-system is optionally substituted

(i) with up to three substituents independently selected from the group consisting of $(C_1-C_8)$-alkyl, oxo, hydroxyl, $(C_1-C_6)$alkoxy, -$(C_1-C_6)$alkyl-(C=O)-$OR^{8'}$, nitrile, -(C=O)-$OR^{8'}$, -$OAr^2$, -O-$(C_1-C_4)$alkyl-$Ar^2$, $(C_1-C_6)$alkylthio, alkylamino, alkylamido, aryl, aryl-$(C_1-C_4)$alkyl, heteroaryl, and cycloheteroalkyl,
wherein the aryl and aryl-$(C_1-C_4)$alkyl group are optionally substituted in the aryl moiety with up to three substituents independently selected from the group consisting of hydroxyl, halogen, $(C_1-C_4)$-alkyl, $(C_1-C_4)$-alkoxy, halogenated $(C_1-C_4)$-alkyl, halogenated $(C_1-C_4)$-alkoxy and carboxyl-$(C_1-C_4)$alkyl, or wherein the aryl moiety is optionally substituted by two groups which are attached to adjacent carbon atoms and are combined into a saturated or partly unsaturated cyclic 5, 6, 7, or 8 membered ring system, optionally containing up to three heteroatoms selected from N, O and S, the number of N atoms being 0, 1, 2 or 3 and the number of O and S atoms each being 0, 1 or 2; and wherein the $(C_1-C_8)$-alkyl group is optionally substituted with up to three substituents independently selected among hydroxyl, halogen, halogenated $(C_1-C_4)$-alkoxy or $(C_1-C_4)$alkoxy,
whereby the alkyl-chain of the $(C_1-C_4)$-alkoxy moiety is optionally substituted with up to three hydroxyl;
wherein the heteroaryl is optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, halogenated $(C_1-C_4)$-alkoxy) and carboxyl-$(C_1-C_6)$alkyl; and
wherein the cycloheteroalkyl is optionally substituted with up to three substituents independently selected from the group consisting of oxo, $(C_1-C_8)$-alkyl, hydroxyl, $(C_1-C_6)$alkoxy, -(C=O)-$OR^9$, and -$(C_1-C_6)$alkyl-(C=O)-$OR^9$; or
(ii) by two groups which are attached to the same carbon atom and are combined into a saturated or partly unsaturated cyclic 4, 5, 6, 7, or 8-membered ring system, optionally containing up to three heteroatoms selected from N, O and

S, the number of N atoms being 0, 1, 2 or-3 and the number of O and S atoms each being 0, 1 or 2,
whereby the cyclic ring system is optionally substituted by up to three substitutents independently selected from oxo, $(C_1-C_6)$-alkyl, aryl and aryl-$(C_1-C_4)$-alkyl.

wherein

$Ar^2$ represents phenyl or naphthyl, which are optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, or halogenated $(C_1-C_4)$-alkoxy

$R^9$ represents hydrogen, $(C_1-C_4)$alkyl, phenyl, or $(C_1-C_4)$alkyl-phenyl; whereby the phenyl is optionally substituted with up to three substituents independently selected from the group consisting of hydroxyl, halogen, $(C_1-C_4)$ alkoxy, $(C_1-C_4)$-alkyl, halogenated $(C_1-C_4)$alkyl and halogenated $(C_1-C_4)$alkoxy.

In a further embodiment of the present invention relates to compounds of formula I, wherein $R^2$ and $R^4$ are independently selected from:

(a) an alkyl group selected from

(i) -$(C_1-C_8)$alkyl, optionally substituted with substituents independently selected from the group consisting of hydroxyl, nitrile, -O-$R^{7'}$; -O-phenyl, -O-$(C_1-C_4)$alkylphenyl, alkylamino, alkylamido, preferably carbamoyl, -S-$R^{7'}$, and -(C=O)-$OR^{8'}$, the number of substituents on said alkyl portion being up to five for hydroxyl and one, two or three for any combination of said other substituents;
(ii) -$(C_1-C_4)$alkyl, optionally substituted with one or two substituents independently selected from the group consisting of aryl, heteroaryl, and cycloheteroalkyl,
wherein the aryl is preferably selected among phenyl, naphthyl, indanyl, indenyl, and 1,2,3,4-tetrahydro-naphthalen-1-yl, more preferably the aryl is phenyl or naphthyl, and
which aryl is optionally substituted with halogen, hydroxyl, $(C_1-C_6)$alkoxy, $(C_1-C_6)$alkyl, halogenated $(C_1-C_4)$ alkyl, halogenated $(C_1-C_4)$alkoxy, sulfamoyl, or alkylamide, preferably carbamoyl, the number of substituents on said aryl portion being up to three for halogen and one or two for any combination of said other substituents; or which aryl is optionally substituted by two groups which are attached to adjacent carbon atoms and are combined into a saturated cyclic 5 or 6 membered ring system, optionally containing up to three heteroatoms selected from N and O the number of N atoms being 0, 1, 2 or 3 and the number of O atoms being 0, 1 or 2, preferably a [1,3]-dioxol group;
wherein the heteroaryl is preferably selected among pyrrolyl, thienyl, furyl, imidazolyl, thiazolyl, isothiazolyl, oxazolyl, isoxazolyl, pyrazolyl, pyridinyl, pyrimidinyl, pyrazinyl, pyridazinyl, benzothiazolyl, indolyl, quinolinyl, isoquinolinyl, benzoimidazolyl, benzofuran and benzo[b]thiophene, more preferably the heteroaryl is thienyl, furyl, imidazolyl, pyridinyl, indolyl, or benzoimidazolyl, and
which heteroaryl is optionally substituted with one or two, preferably one substituent independently selected from the group consisting of $(C_1-C_4)$alkoxy, preferably methoxy, $(C_1-C_4)$alkyl, preferably methyl, and halogenated $(C_1-C_4)$-alkyl;
wherein the cycloheteroalkyl group is preferably selected from the group consisting of pyrrolidinyl, tetrahydrofuranyl, dihydro-1H-pyrrolyl, tetrahydrothiophenyl, tetrahydropyridinyl, azetidinyl, thiazolidinyl, oxazolidinyl, piperidinyl, morpholinyl, thiomorpholinyl, piperazinyl, 1,3-dihydro-benzoimidazolyl, azepanyl, diazepanyl, oxazepanyl and thiazepanyl, preferably the cycloheteroalkyl group is piperidinyl or morpholinyl; and
which cycloheteroalkyl is optionally substituted with up to three substituents independently selected from the group consisting of oxo, hydroxyl, $(C_1-C_4)$-alkyl, phenyl, -$(C_1-C_4)$alkyl-phenyl, preferably benzyl, -(C=O)-O-$(C_1-C_4)$alkyl, and alkylamino, preferably the cycloheteroalkyl moiety is not substituted;
(iii) -cyclo$(C_3-C_8)$alkyl, optionally substituted with hydroxyl;
(iv) -$(C_1-C_4)$alkyl-cyclo$(C_3-C_8)$alkyl, optionally substituted with hydroxyl;
(v) a bicyclic ring system of 6 to 10 carbon atoms selected from the group consisting of Bicyclo[2.1.1]hexyl, Bicyclo[2.2.1]heptyl, Bicyclo[3.2.1]octyl, Bicyclo[2.2.2]octyl, Bicyclo[3.2.2]nonanyl, Bicyclo[3.3.1]nonanyl, and Bicyclo[3.3.2]decanyl; and
(vi) a fused ring system of up to 10 carbon atoms, preferably adamantyl;

(b) aryl,
wherein the aryl is preferably selected among phenyl, naphthyl, indanyl, indenyl, and 1,2,3,4-tetrahydro-naphthalen-1-yl, and
which aryl is optionally substituted with substituents independently selected from the group consisting of hydroxyl;

halogen, preferably fluorine or chlorine; $(C_1-C_6)$alkoxy, preferably $(C_1-C_2)$alkoxy; $(C_1-C_6)$alkyl, preferably $(C_1-C_4)$ alkyl; halogenated $(C_1-C_6)$alkyl, preferably halogenated $(C_1-C_4)$alkyl, more preferably trifluoromethyl; halogenated $(C_1-C_6)$alkoxy, preferably halogenated $(C_1-C_4)$alkoxy, more preferably trifluoromethoxy; $-(C_1-C_4)$alkyl-(C=O)-$OR^{8'}$; nitrile, nitro, sulfamoyl, -(C=O)-$R^{8'}$, -(C=O)-$OR^{8'}$, -NH-(C=O)-$R^{8'}$, -S-$R^{8'}$, $-SO_2-R^{8'}$, alkylamino, alkylamido, preferably carbamoyl, phenyl, and a further heteroaryl group, optionally substituted with $(C_1-C_4)$alkyl, preferably 6-methylbenzothiazolyl; the number of substituents on said aryl portion being up to three for halogen, and one or two for any combination of said other moieties; or

which aryl is optionally substituted by two groups which are attached to adjacent carbon atoms and are combined into a saturated cyclic 5 or 6-membered ring system, optionally containing up to three heteroatoms selected from N and O, the number of N atoms being 0, 1, 2 or 3 and the number of O atoms being 0, 1 or 2, preferably a [1,3]-dioxol group;

(c) heteroaryl,

wherein the heteroaryl is preferably selected among pyrrolyl, thienyl, furyl, imidazolyl, thiazolyl, isothiazolyl, oxazolyl, isoxazolyl, pyrazolyl, pyridinyl, pyrimidinyl, pyrazinyl, pyridazinyl, benzothiazolyl, indolyl, quinolinyl, isoquinolinyl, benzoimidazolyl, benzofuran and benzo[b]thiophene; more preferably heteroaryl is furyl, thiazolyl, pyrazolyl, pyridinyl, quinolinyl, or benzo[b]thiophene, and

which heteroaryl is optionally substituted with up to three, preferably up to two substituents independently selected from the group consisting of halogen, $(C_1-C_4)$-alkyl, hydroxyl, halogenated $(C_1-C_4)$alkyl, $-(C_1-C_4)$alkoxy, $-(C_1-C_4)$alkyl-(C=O)-$OR^{8'}$, - O-$Ar^{1'}$, $-SO_2-Ar^{1'}$, phenyl, $-(C_1-C_4)$alkyl-phenyl, nitrile, alkylamino, and alkylamido, preferably carbamoyl; preferably selected from the group consisting of halogen, $(C_1-C_4)$alkyl, halogenated $(C_1-C_4)$alkyl, $-(C_1-C_4)$alkyl-(C=O)-$OR^{8'}$, -O-$Ar^{1'}$, $-SO_2-Ar^{1'}$ and phenyl; or

(d) cycloheteroalkyl,

wherein the cycloheteroalkyl is preferably selected among pyrrolidinyl, tetrahydrofuranyl, dihydro-1H-pyrrolyl, tetrahydrothiophenyl, tetrahydropyridinyl, azetidinyl, thiazolidinyl, oxazolidinyl, piperidinyl, morpholinyl, thiomorpholinyl, piperazinyl, 1,3-dihydro-benzoimidazolyl, azepanyl, diazepanyl, oxazepanyl and thiazepanyl; more preferably cycloheteroalkyl is pyrrolidinyl, morpholinyl, tetrahydrofuranyl, piperidinyl or azepanyl, and

which cycloheteroalkyl is optionally substituted with up to three, preferably one or two substituents independently selected from the group consisting of oxo, $(C_1-C_4)$alkyl, phenyl, $-(C_1-C_4)$alkyl-phenyl, hydroxyl, $(C_1-C_4)$alkoxy, and $-(C_1-C_4)$alkyl-(C=O)-$OR^{8'}$; preferably selected from the group consisting of oxo, $(C_1-C_4)$alkyl, preferably methyl, and $(C_1-C_4)$alkyl-phenyl, preferably benzyl;

wherein

$R^{7'}$ represents $(C_1-C_4)$alkyl, preferably $(C_1-C_2)$alkyl, optionally substituted with one or two hydroxyl groups,
$R^{8'}$ represents hydrogen, $(C_1-C_4)$alkyl, preferably methyl, or $(C_1-C_2)$alkyl-phenyl, preferably benzyl; and
$Ar^{1'}$ represents phenyl optionally substituted with up to three halogen atoms;

or wherein the ring or ringsystem formed by $R^2$ and $R^4$ together with the nitrogen atom, to which $R^2$ and $R^4$ are attached, is selected from the group consisting of

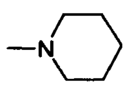 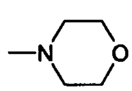 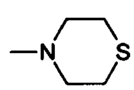 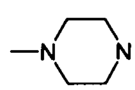

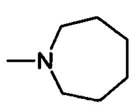 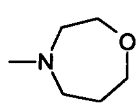 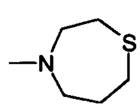 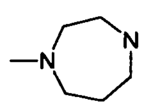

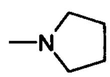 and 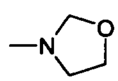

wherein the ring or the ring-system is optionally substituted

(i) with up to three substituents independently selected from the group consisting of

(a) hydroxyl,

(b) oxo,
(c) ($C_1$-$C_4$)-alkyl, optionally substituted with up to two hydroxyl and/or ($C_1$-$C_4$)-alkoxy groups, whereby the alkyl-chain of the ($C_1$-$C_4$)-alkoxy moiety may optionally be further substituted with one or two, preferably one hydroxyl group;
(d) cyclo($C_3$-$C_8$)alkyl;
(e) -(C=O)-O-($C_1$-$C_4$)-alkyl;
(f) phenyl, optionally substituted with halogen, ($C_1$-$C_4$)-alkyl, preferably methyl, ($C_1$-$C_4$)-alkoxy, or halogenated ($C_1$-$C_4$)-alkyl, preferably halogenated methyl, the number of said substituents on the phenyl moiety being up to three for halogen, and one or two for any combination of said other substituents;
(g) phenyl-($C_1$-$C_4$)alkyl, preferably benzyl, optionally substituted in the phenyl group by up three halogen, or optionally substituted in the phenyl group by two groups which are attached to adjacent carbon atoms and are combined into a saturated or partly unsaturated cyclic 5 or 6-membered ring system, optionally containing up to two O atoms;
(h) alkylamide, preferably carbamoyl;
(i) heteroaryl, wherein the heteroaryl is preferably selected from the group consisting of pyridinyl, furyl, thienyl, thiazolyl, imidazolyl, pyrazolyl, indolyl, quinolinyl, benzoimidazolyl or benzo[b]thiophene, more preferably the heteroaryl is pyridinyl; and
(j) cycloheteroalkyl, wherein the cycloheteroalkyl is preferably selected from the group consisting of pyrrolidinyl, 1,3-dihydro-benzoimidazolyl, morpholinyl, tetrahydrofuranyl, piperidinyl and azepanyl; more preferably the cycloheteroalkyl group is pyrrolidinyl or 1,3-dihydro-benzoimidazolyl,
which cycloheteroalkyl group is optionally substituted with oxo; or

(ii) by two groups which are attached to the same carbon atom and are combined into a saturated or partly unsaturated cyclic 5, 6, or 7-membered ring system, optionally containing up to three heteroatoms selected from N and O, the number of N atoms being 0, 1, 2 or 3 and the number of O atoms being 0, 1 or 2,
whereby the cyclic ring system may optionally be further substituted with up to two substituents independently selected from oxo and phenyl.

In one embodiment, the invention relates to a compound of the following formula XLII

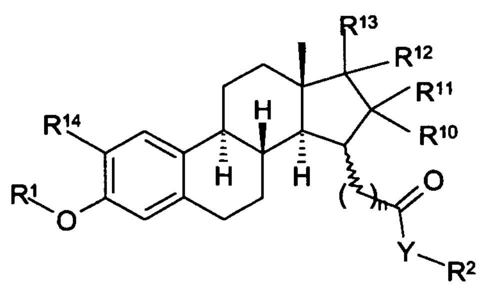
(XLII)

wherein
Y represents $-NR^4-$, -O-, a bond or $-NH-NR^4-$,
i.e. compounds of formula I, wherein -X-A-Y- together represent a group selected from

(a) $-CO-NR^4-$,
(b) -CO-O-,
(c) -CO-, and
(d) $-CO-NH-NR^4-$,

the preferred meanings of $R^1$, $R^{10}$, $R^{11}$, $R^{12}$, $R^{13}$ and $R^{14}$ are as indicated above, and n represents 0, 1, 2, 3, 4, or 5.
In one embodiment, the invention relates to a compound of the following formula VI

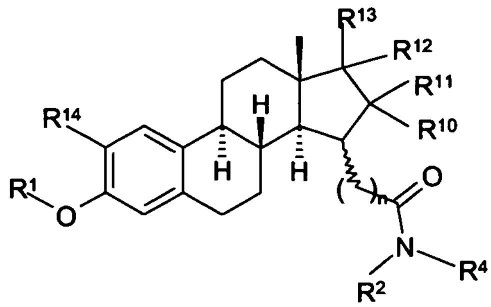

(VI)

i.e. a compound of formula I, wherein -X-A-Y- together represent -CO-$NR^4$-, and wherein the preferred meanings of $R^1$, $R^{10}$, $R^{11}$, $R^{12}$, $R^{13}$ and $R^{14}$ are as indicated above, and
n represents 0, 1, 2, 3, 4, or 5, preferably n represents 2, 3 or 4.

In this embodiment, $R^2$ preferably represents

(i) -($C_1$-$C_4$)alkyl, which is optionally substituted with one or two substituents independently selected from the group consisting of hydroxyl, halogen, and ($C_1$-$C_4$)alkoxy;
(ii) -($C_3$-$C_8$)cycloalkyl;
(iii) aryl or -($C_1$-$C_4$)alkyl-aryl, wherein the aryl is phenyl or naphthyl,
which phenyl is optionally substituted with one or two substituents independently selected from the group consisting of hydroxyl, halogen, cyano, ($C_1$-$C_4$)alkoxy and halogenated ($C_1$-$C_4$)alkoxy; or
which phenyl is optionally substituted by two groups which are attached to adjacent carbon atoms and are combined into a saturated cyclic 5 or 6-membered ring system, containing 1 or 2 O atoms; or
(iv) heteroaryl or -($C_1$-$C_4$)alkyl-heteroaryl, wherein the heteroaryl is furyl, thienyl, thiazolyl, imidazolyl, pyridinyl, indolyl, indazolyl, or benzoimidazolyl;
which heteroaryl is optionally substituted with one or two substituents independently selected from the group consisting of -($C_1$-$C_4$)alkyl and -($C_1$-$C_4$)alkyl-(C=O)-O-($C_1$-$C_4$)alkyl;

and $R^4$ is independently selected from H or -($C_1$-$C_4$)-alkyl or -($C_1$-$C_4$)-alkyl-phenyl, wherein the phenyl group is optionally substituted with one or two ($C_1$-$C_4$)alkoxy groups; or
$R^2$ and $R^4$ form together with the nitrogen atom, to which $R^2$ and $R^4$ are attached, a ring, which is selected from the group consisting of morpholine, piperidine, thiomorpholine and piperazine,
wherein the ring is optionally substituted with a -($C_1$-$C_4$)alkyl group.

In this embodiment, $R^2$ more preferably represents

(i) -($C_1$-$C_4$)alkyl, which is optionally substituted with one or two ($C_1$-$C_4$)alkoxy groups;
(ii) -($C_3$-$C_8$)cycloalkyl;
(iii) phenyl or -($C_1$-$C_4$)alkyl-phenyl,
which phenyl is optionally substituted with one or two substituents independently selected from hydroxyl, halogen, cyano and ($C_1$-$C_4$)alkoxy; or
which phenyl is optionally substituted by two groups which are attached to adjacent carbon atoms and are combined into a saturated cyclic 5 or 6-membered ring system, containing 1 or 2 O-atoms; or
(iv) heteroaryl or -($C_1$-$C_4$)alkyl-heteroaryl, wherein the heteroaryl is thiazolyl, pyridinyl, indolyl, or indazolyl;
which heteroaryl is optionally substituted with one or two -($C_1$-$C_4$)alkyl groups; and $R^4$ is independently selected from -H, -($C_1$-$C_4$)-alkyl or -($C_1$-$C_4$)-alkyl-phenyl, wherein
the phenyl group is optionally substituted with one or two ($C_1$-$C_4$)alkoxy groups; or
$R^2$ and $R^4$ form together with the nitrogen atom, to which $R^2$ and $R^4$ are attached, a ring, which is selected from the group consisting of morpholine, piperidine, and piperazine,
wherein the ring is optionally substituted with a -($C_1$-$C_4$)alkyl group.

Mostly preferred are compounds according to general formula VI, wherein
$R^2$ represents a -($C_1$-$C_4$)alkylphenyl, preferably a benzyl group, or a thiazolyl group, optionally substituted with -($C_1$-$C_4$)-alkyl, preferably methyl, and $R^4$ represents -H; or
$R^2$ and $R^4$ form together with the nitrogen atom, to which $R^2$ and $R^4$ are attached, a morpholine group, and
n represents 3.

In a further embodiment the invention relates to a compound of the following formula XL

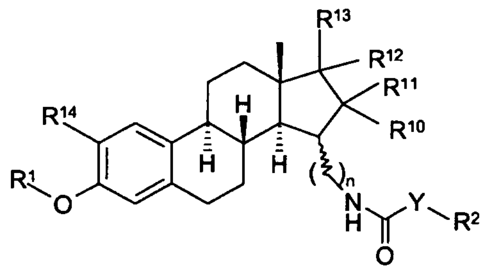

(XL)

wherein Y represents -NH-, a bond, or -O-; i.e compounds of formula I, wherein -X-A-Y-together represent -NH-CO-NH-, -NH-CO-O-, or -NH-CO-; the preferred meanings of $R^1$, $R^{10}$, $R^{11}$, $R^{12}$, $R^{13}$ and $R^{14}$ are as indicated above; and n represents 1, 2, 3, 4, 5 or 6, preferably 1, 2, 3 or 4.

A further embodiment of the invention relates to a compound of the following formula XVII,

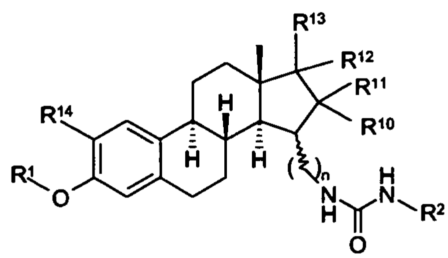

(XVII)

wherein the preferred meanings of $R^1$, $R^{10}$, $R^{11}$, $R^{12}$, $R^{13}$ and $R^{14}$ are as indicated above; and n preferably represents 1, 2, 3, or 4, even more preferably 3 or 4.

In this embodiment, $R^2$ preferably represents

(i) -($C_1$-$C_4$)alkyl,
(ii) -($C_3$-$C_8$)cycloalkyl,
(iii) -($C_1$-$C_4$)alkyl-($C_3$-$C_8$)cycloalkyl,
(iv) aryl, wherein the aryl is phenyl or naphthyl,
which phenyl is optionally substituted with one or two substituents independently selected from the group consisting of hydroxyl, halogen, -CO-O($C_1$-$C_4$)alkyl and ($C_1$-$C_4$)alkoxy; or
which phenyl is optionally substituted by two groups which are attached to adjacent carbon atoms and are combined into a saturated cyclic 5 or 6-membered ring system, containing 1 or 2 O atoms, or
(v) -($C_1$-$C_4$)alkyl-phenyl.

A further embodiment of the invention relates to a compound of the following formula XXIII,

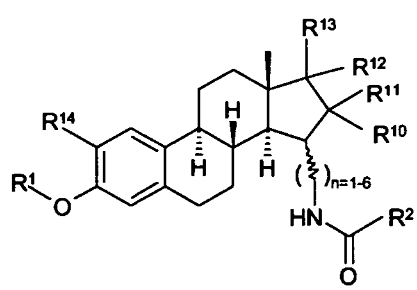

(XXIII)

wherein the preferred meanings of $R^1$, $R^{10}$, $R^{11}$, $R^{12}$, $R^{13}$ and $R^{14}$ are as indicated above; and n preferably represents 1, 2, 3, or 4.

In this embodiment, $R^2$ preferably represents

(i) -($C_1$-$C_4$)alkyl,
(ii) -($C_3$-$C_8$)cycloalkyl,
(iii) -($C_1$-$C_4$)alkyl-($C_3$-$C_8$)cyccloalkyl,

(iv) -($C_1$-$C_4$)alkyl, substituted with one or two substituents independently selected from the group consisting of -O-($C_1$-$C_4$)alkyl and -O-($C_1$-$C_4$)alkyl-phenyl,
(v) phenyl,
which phenyl is optionally substituted with one, two or three substituents independently selected from the group consisting of halogen and ($C_1$-$C_4$)alkoxy;
(vi) -($C_1$-$C_4$)alkyl-phenyl; or
(vii) adamantly.

In another embodiment, the present invention relates to compounds of formula (I), wherein - X-A-Y- together represent a group selected from -NH-$SO_2$-NH-, -NH-$SO_2$-O-, and -NH-$SO_2$-, and n represents 1, 2, 3, or 4.
A further embodiment of the invention relates to a compound of the following formula XXIV,

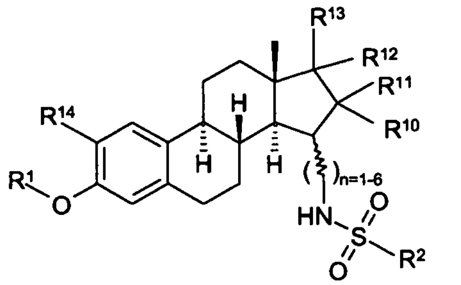

(XXIV)

wherein the preferred meanings of $R^1$, $R^{10}$, $R^{11}$, $R^{12}$, $R^{13}$ and $R^{14}$ are as indicated above; and n preferably represents 1, 2, 3, or 4.
In this embodiment, $R^2$ preferably represents

(i) aryl, wherein the aryl is selected among phenyl and naphthyl,
which aryl is optionally substituted with one or two substituents independently selected from the group consisting of halogen, nitro, ($C_1$-$C_4$)alkoxy, and -($C_1$-$C_4$)alkyl; or
(ii) heteroaryl, wherein the heteroaryl is furyl, thienyl, or thiazolyl, or indolyl,
which heteroaryl is optionally substituted with one or two substituents independently selected from the group consisting of -$SO_2$-phenyl and ($C_1$-$C_4$)alkyl.

In another embodiment, the present invention relates to compounds of formula (I), wherein - X-A-Y- together represent a group selected from -O-CO-NH-, -O-CO-, and -O-CO-NH-$SO_2$-$NR^4$-, and n represents 1, 2, 3, 4, 5 or 6.
A further embodiment of the invention relates to a compound of the following formula XXVI,

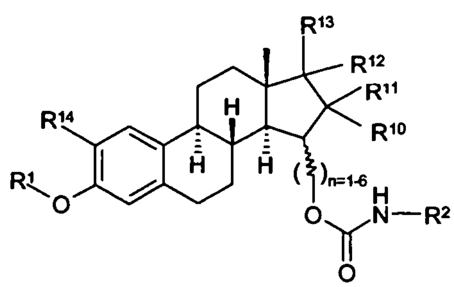

(XXVI)

wherein the preferred meanings of $R^1$, $R^{10}$, $R^{11}$, $R^{12}$, $R^{13}$ and $R^{14}$ are as indicated above; and n preferably represents 3, 4, 5 or 6.
In this embodiment, $R^2$ preferably represents phenyl or naphthyl,
which phenyl is optionally substituted with one or two substituents independently selected from the group consisting of hydroxyl, halogen, nitro, -CO-($C_1$-$C_4$)alkyl and ($C_1$-$C_4$)alkoxy and halogenated ($C_1$-$C_4$)alkyl; or
which phenyl is optionally substituted by two groups which are attached to adjacent carbon atoms and are combined into a saturated cyclic 5 or 6-membered ring system, containing 1 or 2 O atoms.
A further embodiment of the invention relates to a compound of the following formula XXVIII,

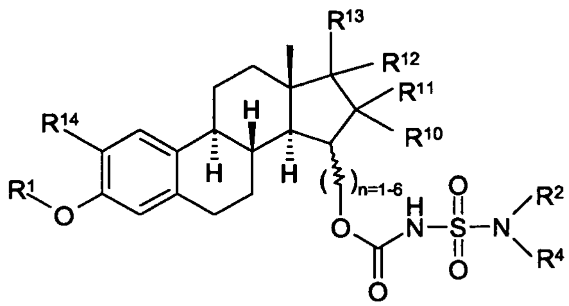

(XXVIII)

wherein the preferred meanings of $R^1$, $R^{10}$, $R^{11}$, $R^{12}$, $R^{13}$ and $R^{14}$ are as indicated above; and n preferably represents 3, 4, 5 or 6.

In this embodiment, $R^2$ preferably represents

(i) -($C_1$-$C_4$)alkyl,
(ii) -($C_3$-$C_8$)cycloalkyl,
(iii) -($C_1$-$C_4$)alkyl-phenyl,
(iv) phenyl, or
(v) heteroaryl or -($C_1$-$C_4$)alkyl-heteroaryl, wherein the heteroaryl is furyl, thienyl, thiazolyl, pyridinyl, indolyl, or benzoimidazolyl;

and preferably $R^4$ is independently selected from H, -($C_1$-$C_4$)-alkyl and -($C_1$-$C_4$)alkyl-phenyl; or $R^2$ and $R^4$ may form together with the nitrogen atom, to which $R^2$ and $R^4$ are attached, a ring, which is selected from the group consisting of morpholine, thiomorpholine and piperazyl, and which is optionally substituted with ($C_1$-$C_4$)-alkyl.

A further embodiment of the invention relates to a compound of the following formula XXXI,

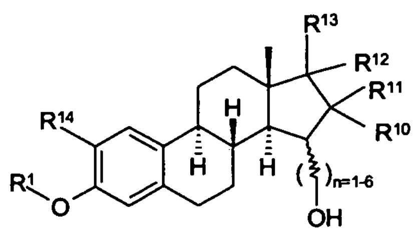

(XXXI)

wherein the preferred meanings of $R^1$, $R^{10}$, $R^{11}$, $R^{12}$, $R^{13}$ and $R^{14}$ are as indicated above; and n represents 1, 2, 3, 4, 5 or 6, preferably 3 or 4.

Preferred embodiments of the invention relate to the following compounds:

N-Benzyl-4-(2-ethyl-3-hydroxy-17-oxo-estra-1,3,5(10)-trien-15β-yl)-butyramide
N-Benzyl-4-(3-hydroxy-17-oxo-2-propyl-estra-1,3,5(10)-trien-15β-yl)-butyramide
N-Benzyl-4-(3-hydroxy-2-(2-methoxy-ethyl)-17-oxo-estra-1,3,5(10)-trien-15β-yl)-butyramide
N-Benzyl-4-(3-hydroxy-2-methoxy-17-oxo-estra-1,3,5(10)-trien-15β-yl)-butyramide
2-Ethyl-3-hydroxy-15β-(4-morpholin-4-yl-4-oxo-butyl)-estra-1,3,5(10)-trien-17-one
3-Hydroxy-15β-(4-morpholin-4-yl-4-oxo-butyl)-2-propyl-estra-1,3,5(10)-trien-17-one
3-Hydroxy-2-(2-methoxy-ethyl)-15β-(4-morpholin-4-yl-4-oxo-butyl)-estra-1,3,5(10)-trien-17-one
3-Hydroxy-2-methoxy-15β-(4-morpholin-4-yl-4-oxo-butyl)-estra-1,3,5(10)-trien-17-one
4-(2-Ethyl-3-hydroxy-17-oxo-estra-1,3,5(10)-trien-15β-yl)-N-(5-methyl-thiazol-2-yl)-butyramide
4-(3-Hydroxy-2-methoxy-17-oxo-estra-1,3,5(10)-trien-15β-yl)-N-(5-methyl-thiazol-2-yl)-butyramide
N-Benzo[1,3]dioxol-5-ylmethyl-4-(3-hydroxy-17-oxo-2-propyl-estra-1,3,5(10)-trien-15β-yl)-butyramide
4-(3-Hydroxy-17-oxo-2-propyl-estra-1,3,5(10)-trien-15β-yl)-N-pyridin-3-ylmethyl-butyramide
4-(3-Hydroxy-17-oxo-2-propyl-estra-1,3,5(10)-trien-15β-yl)-N-[2-(7-methyl-1H-indol-3-yl)-ethyl]-butyramide
3-Hydroxy-15β-(4-oxo-4-piperidin-1-yl-butyl)-2-propyl-estra-1,3,5(10)-trien-17-one
N-Benzyl-4-(3-hydroxy-17-oxo-2-propyl-estra-1,3,5(10)-trien-15β-yl)-N-methyl-butyramide
N-[2-(3,4-Dimethoxy-phenyl)-ethyl]-4-(3-hydroxy-17-oxo-2-propyl-estra-1,3,5(10)-trien-15β-yl)-N-methyl-butyramide
4-(3-Hydroxy-17-oxo-2-propyl-estra-1,3,5(10)-trien-15β-yl)-N-(1H-indazol-6-yl)-butyramide

4-(3-Hydroxy-17-oxo-2-propyl-estra-1,3,5(10)-trien-15β-yl)-N-(2-methoxy-ethyl)-butyramide
N-(2,4-Difluoro-benzyl)-4-(3-hydroxy-17-oxo-2-propyl-estra-1,3,5(10)-trien-15β-yl)-butyramide
N-Cyclohexyl-4-(2-ethoxy-3-hydroxy-17-oxo-estra-1,3,5(10)-trien-15α-yl)-butyramide
N-Benzo[1,3]dioxol-5-ylmethyl-4-(2-ethoxy-3-hydroxy-17-oxo-estra-1,3,5(10)-trien-15α-yl)-butyramide
4-(2-Ethoxy-3-hydroxy-17-oxo-estra-1,3,5(10)-trien-15a-yl)-N-[2-(7-methyl-1H-indol-3-yl)-ethyl]-butyramide
2-Ethoxy-3-hydroxy-15α-(4-oxo-4-piperidin-1-yl-butyl)-estra-1,3,5(10)-trien-17-one
4-(2-Ethoxy-3-hydroxy-17-oxo-estra-1,3,5(10)-trien-15α-yl)-N-(1H-indazo)-6-yl)-butyramide
N-Cyclohexyl-4-(3-hydroxy-2-methoxy-17-oxo-estra-1,3,5(10)-trien-15α-yl)-butyramide
N-Benzyl-4-(3-hydroxy-2-methoxy-17-oxo-estra-1,3,5(10)-trien-15α-yl)-butyramide
3-Hydroxy-2-methoxy-15α-(4-oxo-4-piperidin-1-yl-butyl)-estra-1,3,5(10)-trien-17-one
4-(3-Hydroxy-2-methoxy-17-oxo-estra-1,3,5(10)-trien-15α-yl)-N-(1H-indazol-6-yl)-butyramide
4-(17,17-Difluoro-3-hydroxy-estra-1,3,5(10)-trien-15α-yl)-1-morpholin-4-yl-butan-1-one
4-(17,17-Difluoro-3-hydroxy-estra-1,3,5(10)-trien-15β-yl)-1-morpholin-4-yl-butan-1-one
4-(17-Fluoro-3-hydroxy-estra-1,3,5(10),16-tetraen-15β-yl)-1-morpholin-4-yl-butan-1-one
3-(17,17-Difluoro-3-hydroxy-estra-1,3,5(10)-trien-15β-yl)-N-(5-methyl-thiazol-2-yl)-propionamide
4-(17-Difluoromethylene-3-hydroxy-estra-1,3,5(10)-trien-15β-yl)-1-morpholin-4-yl-butan-1-one
N-Cyclohexyl-4-(17,17-difluoro-3-hydroxy-estra-1,3,5(10)-trien-15α-yl)-butyramide
N-Benzyl-4-(17,17-difluoro-3-hydroxy-estra-1,3,5(10)-trien-15α-yl)-butyramide
4-(17,17-Difluoro-3-hydroxy-estra-1,3,5(10)-trien-15α-yl)-N-(3,4-dihydroxy-benzyl)-butyramide
4-(17,17-Difluoro-3-hydroxy-estra-1,3,5(10)-trien-15α-yl)-N-[2-(7-methyl-1H-indol-3-yl)-ethyl]-butyramide
4-(17,17-Difluoro-3-hydroxy-estra-1,3,5(10)-trien-15α-yl)-1-piperidin-1-yl-butan-1-one
4-(17,17-Difluoro-3-hydroxy-estra-1,3,5(10)-trien-15α-yl)-N-[2-(3,4-dimethoxy-phenyl)-ethyl]-N-methyl-butyramide
N-Cyclopropyl-3-(17,17-difluoro-3-hydroxy-estra-1,3,5(10)-trien-15α-yl)-propionamide
N-Cyclohexyl-3-(17,17-difluoro-3-hydroxy-estra-1,3,5(10)-trien-15β-yl)-propionamide
N-Benzo[1,3]dioxol-5-ylmethyl-3-(17,17-difluoro-3-hydroxy-estra-1,3,5(10)-trien-15β-yl)-propionamide
N-Benzyl-3-(17,17-difluoro-3-hydroxy-estra-1,3,5(10)-trien-15β-yl)-propionamide
3-(17,17-Difluoro-3-hydroxy-estra-1,3,5(10)-trien-15β-yl)-N-(3,4-dihydroxy-benzyl)-propionamide
3-(17,17-Difluoro-3-hydroxy-estra-1,3,5(10)-trien-15β-yl)-N-(3,5-dimethoxy-benzyl)-propionamide
3-(17,17-Difluoro-3-hydroxy-estra-1,3,5(10)-trien-15β-yl)-N-[2-(7-methyl-1H-indol-3-yl)-ethyl]-propionamide
3-(17,17-Difluoro-3-hydroxy-estra-1,3,5(10)-trien-15β-yl)-1-piperidin-1-yl-propan-1-one
3-(17,17-Difluoro-3-hydroxy-estra-1,3,5(10)-trien-15β-yl)-N,N-diethyl-propionamide
3-(17,17-Difluoro-3-hydroxy-estra-1,3,5(10)-trien-15β-yl)-N-[2-(3,4-dimethoxy-phenyl)-ethyl]-N-methyl-propionamide
3-Hydroxy-15β-(4-morpholin-4-yl-4-oxo-butyl)-estra-1,3,5(10)-trien-[16,17-c]-pyrazole
3-Sulphamate-15β-(4-morpholin-4-yl-4-oxo-butyl)-estra-1,3,5(10)-trien-17-one,
3-Sulphate-15β-(4-morpholin-4-yl-4-oxo-butyl)-estra-1,3,5(10)-trien-17-one,

or a physiologically acceptable salt thereof.

Pharmaceutically acceptable salts of the compounds of the invention are also within the scope of the invention.

Additionally, the invention relates to a compound of the invention for use as a medicament.

In addition, the invention relates to the use of a compound of the invention for the manufacture of a medicament for the treatment or prevention of a steroid hormone dependent disease or disorder in a mammal, in particular a human. Preferably the steroid hormone dependent disease or disorder is an estradiol dependent disease or disorder. Alternatively, the steroid dependent disease or disorder is an androgen-dependent disease or disorder.

In a further embodiment of the invention, the steroid hormone dependent disease or disorder requires the inhibition of a 17β-HSD enzyme, preferably the human 17β-HSD1 enzyme and/or the inhibition of a STS enzyme, preferably the human STS enzyme. Preferably, the steroid hormone dependent disease or disorder is mediated by the dual action of the 17β-HSD1 and the STS enzyme.

Administration of compounds of this invention in combination with other pharmaceuticals used in treatment of the listed conditions is contemplated.

The conditions to be treated include but are not limited to malign estradiol dependent disease or disorder such as breast cancer, ovarian cancer, uterine cancer, endometrial cancer, and endometrial hyperplasia. Preferably, the malign disease or disorder is characterized by a detectable level of 17β-HSD1 and/or STS expression within a cancer tissue sample. A detectable level of 17β-HSD1 and/or STS expression means that a certain level of 17β-HSD1 and/or STS mRNA or of 17β-HSD1 and/or STS protein can be detected by conventional molecular biology methods such as hybridization, PCR reactions, Northern or Western Blotting etc. An alternative detection method for 17β-HSD1 and/or STS expression is the measurement of the corresponding enzyme activity.

According to a further aspect of the invention, the estradiol dependent disease is breast cancer and the mammal

is a human post-menopausal female.

Furthermore, the conditions to be treated include but are not limited to benign estradiol dependent diseases or disorders such as endometriosis, uterine fibroids, uterine leiomyoma, adenomyosis, dysmenorrhea, menorrhagia, metrorrhagia, and urinary dysfunction.

In a further embodiment, the invention relates to use of an effective amount of a compound of the invention for the manufacturing of a medicament for the treatment or prevention of one of the aforementioned benign gynaecological diseases or disorders in a mammal whereby the mammal is a human, preferably a female and most preferably a pre- or peri-menopausal female.

According to a further aspect of the present invention, the steroid hormone dependent disease or disorder is an androgen-dependent disease or disorder. Preferably, said androgen-dependent disease or disorder is selected from the group consisting of acne, seborrhea, androgenetic alopecia, hirsutism, and prostate cancer.

According to a further aspect of the invention, the steroid hormone dependent disease or disorder to be treated is an estrogen- or androgen dependent disease or disorder requiring the lowering of the endogeneous estrogen or androgen concentration in a generalized or tissue-specific manner.

Therefore, further steroid-dependent diseases which may be treated with an effective amount of a compound of the invention are selected from the group consisting of prostadynia, benign prostatic hyperplasia, urinary dysfunction, lower urinary tract syndrome, squamous cell carcinoma, rheumatoid arthritis, type I and II diabetes, systemic lupus erythematosus, multiple sclerosis, myastenia gravis, thyroiditis, vasculitis, ulcerative colitis, Crohn's disease, psoriasis, contact dermatitis, graft versus host disease, eczema, asthma, organ rejection following transplantation, colon cancer, tissue wounds, skin wrinkles and cataracts.

According to a further embodiment, a compound of the present invention may be used for the enhancement of cognitive function, i.e. in the treatment or prevention of cognitive dysfunctions, such as senile dementia, including Alzheimer's disease, by increasing the DHEAS levels in the central nervous system.

The disclosed compounds are also useful as diagnostic agents (e.g. in diagnostic kits or for use in clinical laboratories) for screening for the presence or absence of 17β-HSD1 and/or STS enzyme activity.

SOME ADVANTAGES

One key advantage of the present invention is that the compounds of the present invention can act as selective 17β-HSD1 inhibitors and optionally additionally as STS inhibitors. Another advantage of the compounds of the present invention is that they may be potent in vivo and suited for the therapeutic use in mammals, especially humans. Some of the compounds of the present invention may be non-estrogenic compounds. Here, the term "non-estrogenic" means exhibiting no or substantially no estrogenic activity on the estrogen receptor. Another advantage is that some of the compounds may not be capable of being metabolised to compounds which display or induce hormonal activity. Some of the compounds of the present invention are also advantageous in that they may be orally active.

DETAILED DESCRIPTION OF THE INVENTION

Definitions:

The following terms are used to describe the present invention and in particular, to describe various constituents of the chemical composition useful in this invention. The terms are defined as follows:

As used herein, the terms "comprising" and "including" are used herein in their open, nonlimiting sense.

The word "compound" shall here be understood to cover any and all isomers (e. g., enantiomers, stereoisomers, diastereomers, rotomers, tautomers) or any mixture of isomers, , and any pharmaceutically acceptable salt of said compound, unless the formula depicting the compound explicitly shows a particular stereochemistry.

Where the plural form is used for compounds, salts, and the like, this is taken to mean also a single compound, salt, or the like.

The term "17β-hydroxysteroid dehydrogenase type I" or "17β-HSD1" for short is used for the enzyme EC 1.1.1.62 and reduces estrone (E1) to the biologically active estrogen, estradiol (E2).

The term "Steroid Sulphatase" or "STS" for short is used for the enzyme EC 3.1.6.2 and hydrolyses several sulphate steroids, such as estrone sulphate, dehydroepiandrosterone sulphate and cholesterol sulphate.

The terms "inhibit" and "inhibition" include the meaning of to reduce and/or eliminate and/or mask and/or prevent a certain enzyme action.

The term "17β-HSD1 inhibitor" as used herein with respect to the compound of the present invention means a compound that can inhibit 17β-HSD1 activity, such as to reduce and/or eliminate and/or mask and/or prevent the action of 17β-HSD1. The 17β-HSD1 inhibitor may act as an reversible or irreversible inhibitor of 17β-HSD1. The ability of compounds to inhibit 17β-HSD1 activity can be assessed using cell lines recombinantly expressing the human 17β-HSD1 enzyme. Details on a suitable Assay Protocol are presented in the Examples section. It is to be noted that the compound of the present invention may have other beneficial properties in addition to or in the alternative to its ability to inhibit 17β-HSD1 activity; in particular a 17β-HSD1 inhibitor may have antagonistic activity towards the nuclear estrogen receptor.

The term "STS inhibitor" as used herein with respect to the compound of the present invention means a compound that can inhibit STS activity, such as to reduce and/or eliminate and/or mask and/or prevent the action of STS. The STS inhibitor may act as an antagonist. The ability of compounds to inhibit estrone sulphate activity can be assessed using either intact MCF-7 breast cancer cells or placenta microsomes. In addition, an animal model may be used. Details on suitable Assay Protocols are presented in following sections. It is to be noted that other assays could be used to determine STS activity and thus STS inhibition. For example, reference may also be made to the teachings of international patent application WO 99/50453. Preferably, for some applications, a "STS inhibitor" is further characterized by the feature that if the sulphamate group were to be substituted by a sulphate group to form a sulphate derivative, then the sulphate derivative would be hydrolysable by an enzyme having steroid sulphatase (EC 3.1.6.2) activity, i.e. when incubated with steroid sulphatase EC 3.1.6.2 at pH 7.4 and 37˚C. In one preferred embodiment, if the sulphamate group of the compound were to be replaced with a sulphate group to form a sulphate compound then that sulphate compound would be hydrolysable by an enzyme having steroid sutphatase (EC 3.1.6.2) activity and would yield a Km value of less than 200 mM, preferably less than 150 mM, preferably less than 100 mM, preferably less than 75 mM, preferably less than 50 mM, when incubated with steroid sulphatase EC 3.1.6.2 at pH 7.4 and 37˚C. In one preferred embodiment, if the sulphamate group of the compound were to be replaced with a sulphate group to form a sulphate compound then that sulphate compound would be hydrolysable by an enzyme having steroid sutphatase (EC 3.1.6.2) activity and would yield a Km value of less than 200 μM, preferably less than 150 μM, preferably less than 100 μM, preferably less than 75 μM, preferably less than 50 μM, when incubated with steroid sulphatase EC 3.1.6.2 at pH 7.4 and 37˚C. In a preferred embodiment, the compound of the present invention is not hydrolysable by an enzyme having steroid sulphatase (EC 3.1.6.2) activity. It is to be noted that the compound of the present invention may have other beneficial properties in addition to or in the alternative to its ability to inhibit STS activity.

The terms "selective" and "selectivity" as used herein with respect to the compounds of the present invention means a compound that can inhibit 17β-HSD1 and/or STS activity, and shows a higher inhibition value for these particular targets than with regard to other enzyme targets, in particular with regard to the 17β-HSD1 enzyme, and that has weak or no affinity for nuclear receptors, in particular that has weak or no affinity for the ER. Preferably a compound of the present invention has at least about a 100fold selectivity to a desired target (e.g. 17β-HSD1 or STS), preferably at least about a 150fold selectivity to the desired target, preferably at least about a 200fold selectivity to the desired target, preferably at least about a 250fold selectivity to the desired target, preferably at least about a 300fold selectivity to the desired target, preferably at least about a 350fold selectivity to the desired target.

The term "substituted" means that the specified group or moiety bears one or more substituents. Where any group may carry multiple substituents and a variety of possible substituents is provided, the substituents are independently selected and need not be the same. The term "unsubstituted" means that the specified group bears no substituents. The term "optionally substituted" means that the specified group is unsubstituted or substituted by one or more substituents.

Any asymmetric carbon atoms may be present in the (R)-, (S)- or (R,S)-configuration preferably in the (R)- or (S)-configuration, whichever is most active, unless the stereochemistry is explicitly depicted in the corresponding compound formula. Substituents at a double bond or a ring may be present in cis- (.=Z-) or trans (=E-) form, unless the stereochemistry is explicitly depicted in the corresponding compound formula.

The compounds of formula (I) have a defined stereochemistry within the steroidal core structure according to the natural configuration for estrogenic steroids such as estradiol:

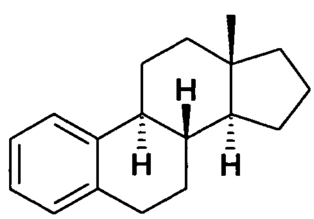

,

The stereochemistry within the steroidal core structure is always shown in the corresponding compound formula and should not vary within the scope of the present invention, whereas the stereochemistry at the carbon atoms in the steroidal core carrying additional side chains and the stereochemistry of any asymmetric carbon atom within the side chains themselves is not fixed. Therefore, the term "compounds of formula (I)" or "compouns of formula (II)" etc also

comprises the stereoisomers of the depicted compounds, unless a particular stereochemistry is explicitly shown within the formula. The stereochemistry shown in the respective formula prevails over the general term "stereoisomers".

The compounds of the formula I contain at least one additional chiral carbon atom, namely the carbon atom carrying the side chain in the 15-position of the steroide structure. The compounds can thus be present at least in two optically active stereoisomeric forms or as a racemate. The present invention includes both the racemic mixtures and the isomerically pure compounds of the formula I. The position of the substituents within the C15 position is characterized by α or β. A C15α derivative according to the present invention is represented by a compound of the following formula (II)

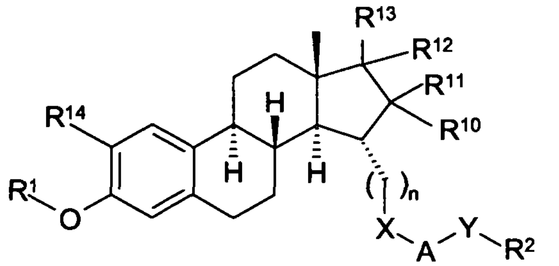

(II)

,

whereas a C15β derivative according to the present invention is represented by a compound of the following formula (III)

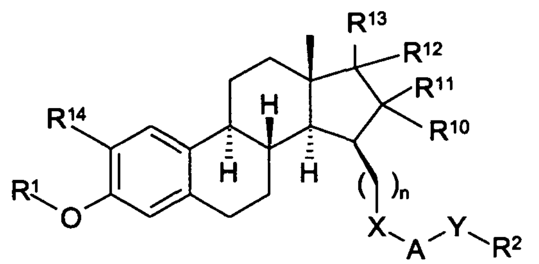

(III)

The compounds of the present invention may contain further asymmetric centers on the molecule, depending upon the nature of the various substituents. In certain instances, asymmetry may also be present due to restricted rotation about the central bond adjoining the two aromatic rings of the specified compounds. It is intended that all isomers (including enantiomers and diastereomers), either by nature of asymmetric centers or by restricted rotation as described above, as separated, pure or partially purified isomers or racemic mixtures thereof, be included within the ambit of the instant invention, unless a particular stereochemistry is explicitly depicted in the formula representing a respective compound.

The term "halogen" refers to fluorine (F, Fluoro-), bromine (Br, Bromo-), chlorine (Cl, Chloro), and iodine (J, Iodo-) atoms.

The terms "dihalogen", "trihalogen" and "perhalogen" refer to two, three and four substituents, respectively, each individually selected from the group consisting of fluorine, bromine, chlorine, and iodine atoms.

The term "hydroxyl" refers to the group -OH

The term "oxo" refers to the group =O

The term "carbamoyl" refers to the group $-CO-NH_2$

The term "thio" refers to the group =S

The term "thiol" refers to the group -SH

The term "sulfanyl" refers to the group -S-

The term "sulfoxy" or "sulfonyl" refers to the group $-SO_2-$

The term "sulfamoyl" refers to the group $-SO_2-NH_2$

The term "nitro" refers to the group $-NO_2$

The term "nitrile" or "cyano" refers to the group -CN

The term "oxime" refers to the group =N-O-Alkyl or =N-OH.

For the purpose of the present invention, the carbon content of various hydrocarbon containing moieties is indicated by a prefix designating the minimum and maximum number of carbon atoms in the moiety, i.e., the prefix $C_i$-$C_j$ defines the number of carbon atoms present from the integer "i" to the integer "j" inclusive. Thus $C_1$-$C_4$-alkyl refers to alkyl of 1-4 carbon atoms, inclusive, or methyl, ethyl, propyl, butyl and isomeric forms thereof.

The term "alkyl" stands for a hydrocarbon radical which may be linear, cyclic or branched, with single or multiple branching, whereby the alkyl group comprises 1 to 12 carbon atoms. In one embodiment, the term "alkyl" stands for a

linear or branched (with single or multiple branching) alkyl chain of 1 to 8 carbon atoms, exemplified by the term ($C_1$-$C_8$) alkyl, more preferably of 1 to 6 carbon atoms exemplified by the term ($C_1$-$C_6$)alkyl. The term ($C_1$-$C_8$)alkyl is further exemplified by such groups as methyl; ethyl; n-propyl; isopropyl; n-butyl; sec-butyl; isobutyl; tert-butyl; n-pentyl; isopentyl; neopentyl; tert-pentyl; 2- or 3-methylpentyl; n-hexyl; isohexyl, heptyl, octyl and the like. The alkyl or ($C_1$-$C_8$)alkyl group may be partially unsaturated, forming such groups as, for example, vinyl, propenyl (allyl), butenyl, pentenyl, pentinyl, hexenyl, octadienyl, and the like. The term "alkyl" further comprises cycloalkyl groups, preferably cyclo($C_3$-$C_8$)alkyl which refers to cyclopropyl, cyclobutyl, cyclopentyl, cyclohexyl, cycloheptyl, cyclooctyl and isomeric forms thereof such as methylcyclopropyl; 2- or 3-methylcyclobutyl; 2-, or 3-methylcyclopentyl, and the like. The cycloalkyl group may also be partly unsaturated, forming such groups as, for example, cyclohexenyl, cyclopentenyl, cyclooctadienyl, and the like. Furthermore, the term "alkyl" comprises a cycloalkylalkyl group comprising 4 to 12 carbon atoms, preferably "-($C_1$-$C_4$) alkyl-cyclo($C_3$-$C_8$)alkyl" which refers to a alkyl group of 1 to 4 carbon atoms as described above substituted with a cyclo ($C_3$-$C_8$)alkyl group as described above, forming such groups as for example cyclopropylmethyl, cyclohexylmethyl, cyclopentylethyl or cyclohexenylethyl. The term "alkyl" further comprises bicyclic ring systems of 6 to 10 carbon atoms, preferably Bicyclo[2.1.1]hexyl, Bicyclo[2.2.1]heptyl, Bicyclo[3.2.1]octyl, Bicyclo[2.2.2]octyl, Bicyclo[3.2.2]nonanyl, Bicyclo[3.3.1]nonanyl, Bicyclo[3.3.2]decanyl; and the like, preferably Bicyclo[2.2.1]heptyl, and fused ring systems of up to 10 carbon atoms such as adamantyl and the like.

The alkyl group may optionally be substituted by up to five, more preferably by up to three substituents independently selected from the group consisting of halogen, hydroxyl, optionally substituted aryl, optionally substituted heteroaryl, optionally substituted cycloheteroalkyl, thiol, nitro, nitrile, alkoxy, aryloxy, arylalkyloxy, amino, amido, alkylthio, arylthio, arylalkylthio, sulfamoyl, sulfonamide, acyl, carboxyl, and acylamino, as defined herein. These groups may be attached to any carbon atom of the alkyl moiety.

The alkyl group substituted with up to three independently selected aryl preferably refers to "aryl-($C_1$-$C_4$)-alkyl" or "diaryl-($C_1$-$C_4$)-alkyl", wherein the aryl is phenyl, naphthyl, indanyl, indenyl, or 1,2,3,4-tetrahydro-naphthalen-1-yl, preferably aryl is phenyl or naphthyl, forming such groups as for example benzyl, diphenylmethyl, phenethyl, phenylpropyl, diphenylpropyl, phenylbutyl, naphthylmethyl or naphthylethyl. The alkyl chain may be further substituted as defined above; for example the alkyl chain may carry an additional hydroxyl group. Furthermore, the alkyl chain may be partially unsaturated, such as a vinyl group. The aryl moiety may optionally be substituted as defined herein.

The alkyl group substituted with up to three independently selected heteroaryl group preferably refers to "heteroaryl-($C_1$-$C_4$)-alkyl", wherein the heteroaryl is pyrrolyl, thienyl, furyl, imidazolyl, thiazolyl, isothiazolyl, oxazolyl, isoxazolyl, pyrazolyl, pyridinyl, pyrimidinyl, pyrazinyl, indolyl, quinolinyl, isoquinolinyl, benzoimidazolyl, benzofuran, benzo[b] thiophene, preferably heteroaryl is furyl, indolyl, benzoimidazolyl, pyridinyl, thienyl or imidazolyl, forming such groups as for example benzoimidazolylmethyl, pyridinylmethyl, thienylmethyl, furylmethyl, indolylethyl, thienylethyl, pyridinylethyl, or imidazolylpropyl. The heteroaryl moiety may optionally be substituted as defined herein.

The alkyl group substituted with up to three independently selected cycloheteroalkyl groups preferably refers to "cycloheteroalkyl-($C_1$-$C_4$)-alkyl", wherein the cycloheteroalkyl is pyrrolidinyl, tetrahydrofuryl, tetrahydrothiophenyl, piperidinyl, morpholinyl, thiomorpholinyl, piperazinyl, azepanyl, diazepanyl, oxazepanyl or thiazepanyl, preferably cycloheteroalkyl is piperidinyl, pyrrolidinyl, or morpholinyl, forming such groups as for example morpholinylethyl, morpholinylpropyl, piperidinylethyl or pyrrolidinylethyl. The cycloheteroalkyl moiety may optionally be substituted as defined herein.

The term "alkoxy" refers to a group -OR, where R may be alkyl (wherein the alkyl chain may be optionally further substituted as defined herein). Preferably, the term "alkoxy' refers to -O-($C_1$-$C_6$)alkyl (or ($C_1$-$C_6$)alkoxy), with the ($C_1$-$C_6$)alkyl group as defined above and optionally substituted with up to three hydroxyl groups.

The term "aryloxy" refers to a group -OAr, where Ar represents aryl as defined herein, which is optionally substituted in the aryl group with up to five independently selected substituents as defined herein, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy; the number of said substituents being up to five for halogen, and up to three for any combination of said other substituents. Preferably, aryloxy refers to phenoxy, optionally substituted as defined above.

The term "arylalkyloxy" refers to a group -O-($C_1$-$C_4$)alkyl-Ar, where Ar represents aryl, which is optionally substituted in the aryl group with up to five independently selected substituents as defined herein, in particular hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy; the number of said substituents being up to five for halogen, and up to three for any combination of said other substituents. Preferably, arylalkyloxy refers to benzyloxy, optionally substituted as defined above.

The term "acyl" refers to a group -(C=O)-R, where R may be hydrogen, optionally substituted alkyl, optionally substituted aryl or aryl-($C_1$-$C_4$)-alkyl, optionally substituted heteroaryl or heteroaryl-($C_1$-$C_4$)-alkyl, as defined herein. Preferably, the term "acyl" refers to a group -(C=O)-R', where R' represents hydrogen, ($C_1$-$C_4$)alkyl, phenyl, or phenyl-($C_1$-$C_4$)alkyl, preferably benzyl, or heteroaryl-($C_1$-$C_4$)alkyl, preferably indolyl-methyl; whereby the phenyl moiety may be optionally substituted with independently selected substituents, especially hydroxyl, halogen, ($C_1$-$C_4$)alkoxy, ($C_1$-$C_4$)-alkyl or halogenated ($C_1$-$C_4$)alkyl, the number of said substituents being up to five for halogen, and up to three

for any combination of said other substituents.

The term "carbonyl" represents a preferred selection of the term "acyl" and refers to the group -CHO.

The term "alkylacyl" represents a preferred selection of the term "acyl" and refers to a group -(C=O)-alkyl, preferably -(C=O)-($C_1$-$C_4$)alkyl.

The term "carboxyl" refers to a group -(C=O)-OR, wherein R may be hydrogen, optionally substituted alkyl (preferably substituted with hydroxyl, halogen or ($C_1$-$C_4$)-alkoxy), optionally substituted aryl or aryl-($C_1$-$C_4$)-alkyl, or optionally substituted heteroaryl or heteroaryl-($C_1$-$C_4$)-alkyl, each as defined herein. Preferably, the term "carboxyl" refers to a group -(C=O)-OR', where R' represents hydrogen, ($C_1$-$C_4$)alkyl, phenyl, or phenyl-($C_1$-$C_4$)alkyl, preferably benzyl; whereby the phenyl moiety may be optionally substituted with substituents independently selected from the group consisting of hydroxyl, halogen, ($C_1$-$C_4$)alkoxy, ($C_1$-$C_4$)-alkyl, halogenated ($C_1$-$C_4$)alkyl and halogenated ($C_1$-$C_4$) alkoxy, the number of said substituents being up to five for halogen, and up to three for any combination of said other substituents.

The terms "carboxyl-($C_1$-$C_6$)alkyl" and "carboxyl-($C_1$-$C_4$)alkyl" refer to groups -($C_1$-$C_6$)alkyl-(C=O)-OR and -($C_1$-$C_4$)alkyl-(C=O)-OR, respectively, which refer to an alkyl group of 1 to 6 and 1 to 4 carbon atoms, respectively, as described above, substituted with a -(C=O)-OR group as described above. Preferably the carboxyl group refers to -(C=O)-OR', wherein R' represents hydrogen, ($C_1$-$C_4$)alkyl, phenyl, or ($C_1$-$C_4$)alkyl-phenyl, preferably benzyl. Preferred examples of such carboxyl-($C_1$-$C_6$)alkyl groups include acetic acid methyl ester, acetic acid ethyl ester, propionic acid benzyl ester, propionic acid ethyl ester, butyric acid methyl ester, and 3-methyl-butyric acid methyl ester.

The term "amino" refers to the group -NRR', where R and R' may independently be hydrogen, optionally substituted alkyl (preferred substituents comprise hydroxyl, halogen or ($C_1$-$C_4$)-alkoxy), optionally substituted aryl or aryl-($C_1$-$C_4$)-alkyl, or optionally substituted heteroaryl or heteroaryl-($C_1$-$C_4$)-alkyl, each as defined herein.

The term "alkylamino" represents a preferred selection of the term "amino" and refers to the group -NRR', where R and R' may independently be hydrogen or ($C_1$-$C_4$)alkyl.

The term "alkylthio" or "alkylsulfanyl" refers to a group -SR, where R represents optionally substituted alkyl (preferred substituents comprise hydroxyl, ($C_1$-$C_4$)-alkoxy or halogen), as defined herein; preferably R represents ($C_1$-$C_6$) alkyl, in particular ($C_1$-$C_4$)alkyl.

The term "arylthio" or "arylsulfanyl" refers to a group -S-Ar, where Ar represents optionally substituted aryl (preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), as defined herein. Preferably, arylthio refers to optionally substituted phenylsulfanyl.

The term "arylalkylthio" or "arylalkylsulfanyl" refers to a group -S-($C_1$-$C_4$)alkyl-Ar, where Ar represents optionally substituted aryl (preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), as defined herein. Preferably, arylalkylthio refers to optionally substituted benzylsulfanyl.

The term "alkylsulfonyl" refers to a group -$SO_2$-R, where R represents optionally substituted alkyl (preferred substituents comprise hydroxyl, ($C_1$-$C_4$)-alkoxy or halogen), as defined herein; preferably R represents ($C_1$-$C_6$)alkyl, in particular ($C_1$-$C_4$)alkyl.

The term "arylsulfonyl" refers to a group -$SO_2$-Ar, where Ar represents optionally substituted aryl (preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), as defined herein. Preferably, arylsulfonyl refers to optionally substituted benzenesulfonyl.

The term "arylalkylsulfonyl" refers to a group -$SO_2$-($C_1$-$C_4$)alkyl-Ar, where Ar represents optionally substituted aryl (preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), as defined herein. Preferably, arylalkylsulfonyl refers to optionally substituted benzylsulfonyl.

The term "amido" refers to the group -(C=O)-NRR', where R and R' may independently be hydrogen, optionally substituted alkyl (preferred substituents comprise hydroxyl, halogen or ($C_1$-$C_4$)-alkoxy), optionally substituted aryl or aryl-($C_1$-$C_4$)-alkyl ((preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), or optionally substituted heteroaryl or heteroaryl-($C_1$-$C_4$)-alkyl, as defined herein.

The term "alkylamido" represents a preferred selection of the term "amido" and refers to the group -(C=O)-NRR', where R and R' may be independently selected from hydrogen or ($C_1$-$C_4$)alkyl.

The term "acylamino" refers to the group -NR-CO-R', where R and R' may independently be hydrogen, optionally substituted alkyl (preferred substituents comprise hydroxyl, halogen or ($C_1$-$C_4$)-alkoxy), optionally substituted aryl or aryl-($C_1$-$C_4$)-alkyl (preferred substituents comprise hydroxyl, halogen, ($C_1$-$C_4$)-alkyl, ($C_1$-$C_4$)-alkoxy, halogenated ($C_1$-$C_4$)-alkyl, or halogenated ($C_1$-$C_4$)-alkoxy), optionally substituted heteroaryl or heteroaryl-($C_1$-$C_4$)-alkyl, as defined herein. Preferably, acylamino refers to -NH-CO-($C_1$-$C_4$)-alkyl.

The term "carbonylamino" represents a preferred selection of the term "acylamino" and refers to the group -NR-CO-$CH_2$-R', where R and R' may be independently selected from hydrogen or ($C_1$-$C_4$)alkyl.

The term "sulfonamide" refers to the group -$SO_2$-NRR', wherein R and R' may independently be selected from

hydrogen or $(C_1-C_4)$alkyl.

Halogenated alkyl, halogenated alkoxy and halogenated alkylthio are substituents in which the alkyl moieties (preferably $(C_1-C_6)$alkyl, more preferred $(C_1-C_4)$alkyl, and most preferred methyl) are substituted either partially or in full with halogens, generally with chlorine and/or fluorine. Preferred examples of such substituents are trifluoromethyl, trifluoromethoxy, trifluoromethylthio, dichloromethyl, pentafluoroethyl, dichloropropyl, fluoromethyl and difluoromethyl.

The term "cycloheteroalkyl" refers to a four- to eight-membered heterocyclic ring containing at least one heteroatom, such as N, O or S, the number of N atoms being 0-3 and the number of O and S atoms each being 0-1, which system may be saturated, partly unsaturated or hydroaromatic, and which ring can be part of a multiple condensed ring-system in which some rings may be aromatic. Examples of such cycloheteroalkyls include pyrrolidinyl, tetrahydrofuryl, tetrahydrothiophenyl, tetrahydropyridinyl, azetidinyl, thiazolidinyl, oxazolidinyl, piperidinyl, morpholinyl, thiomorpholinyl, piperazinyl, azepanyl, diazepanyl, oxazepanyl, thiazepanyl, dihydro-1H-pyrrolyl, 3,6-dihydro-2H-pyridinyl, 1,3-dihydrobenzoimidazolyl and the like. Preferred examples of such cycloheteroalkyl groups are pyrrolidinyl, morpholinyl, tetrahydrofuryl, piperidinyl or azepanyl.

The cycloheteroalkyl group may optionally be substituted by up to three substituents, independently selected from the group consisting of oxo, alkyl, optionally substituted aryl or aryl-$(C_1-C_4)$-alkyl, hydroxyl, $(C_1-C_6)$alkoxy, halogenated $(C_1-C_6)$alkyl, halogenated $(C_1-C_6)$alkoxy, carboxyl-$(C_1-C_6)$alkyl, thiol, nitrile, sulfamoyl, sulfonamide, carboxyl, aryloxy or arylalkyloxy, $(C_1-C_6)$alkylthio, arylthio or arylalkylthio, amino, amido, acyl, and acylamino, as defined herein. The substituents of the cycloheteroalkyl groups may be attached to any carbon atom of the cycloheteroalkyl moiety. Substituted cycloheteroalkyl is preferably substituted with oxo, $(C_1-C_4)$alkyl, preferably methyl, phenyl and/or phenyl-$(C_1-C_4)$alkyl, in particular benzyl.

The terms "aryl" or "Ar" refer to an aromatic carbocyclic group comprising 6 to 14, more preferably 6 to 10, carbon atoms and having at least one aromatic ring or multiple condensed rings in which at least one ring is aromatic. Preferably, aryl is phenyl, naphthyl, indanyl, indenyl, or 1,2,3,4-tetrahydro-naphthalen-1-yl.

The term "heteroaryl" refers to an aromatic carbocyclic group of having a single 4 to 8 membered ring or multiple condensed rings comprising 6 to 14, more preferably 6 to 10, ring atoms and containing at least one heteroatom, such as N, O or S, within at least one ring, the number of N atoms being 0-3 and the number of O and S atoms each being 0-1; in which group at least one heterocyclic ring is aromatic. Examples of such groups include pyrrolyl, thienyl, furyl, imidazolyl, thiazolyl, isothiazolyl, oxazolyl, isoxazolyl, pyrazolyl, pyridinyl, pyrimidinyl, pyrazinyl, pyridazinyl, indolyl, quinolinyl, isoquinolinyl, benzothiazolyl, benzoimidazolyl, 1,3-dihydro-benzoimidazolyl, benzofuran, benzo[b]thiophene and the like. Preferably, heteroaryl is quinolinyl, furyl, benzoimidazolyl, pyridinyl, thienyl, indolyl, benzo[b]thiophene, pyridinyl, imidazolyl, pyrazolyl or thiazolyl.

The aryl and the heteroaryl group may optionally be substituted by substituents independently selected from the group consisting of halogen, hydroxyl, $(C_1-C_6)$alkoxy, $(C_1-C_6)$alkyl, halogenated $(C_1-C_6)$alkyl, halogenated $(C_1-C_6)$ alkoxy, carboxyl-$(C_1-C_6)$alkyl, oxo, thiol, nitro, nitrile, sulfamoyl, sulfonamide, carboxyl, aryloxy or arylalkyloxy, $(C_1-C_6)$ alkylthio, arylthio or arylalkylthio, alkylsulfonyl, arylsulfonyl, amino, amido, acyl, and acylamino, as defined herein, the number of said substituents being up to five for halogen, and up to three for any combination of said other substituents; whereby the aryloxy, arylalkyloxy, arylthio or arylalkylthio group may be further optionally substituted in the aryl moiety with independently selected substituents as defined herein. The heteroaryl group may further be optionally substituted with an aryl group, which may be optionally substituted in the aryl moiety with independently selected substituents as defined herein. The aryl group may further be optionally substituted with a heteroaryl group or a second aryl group.

The aryl may be further substituted by two groups which are attached to adjacent carbon atoms and are combined into a saturated or partly unsaturated cyclic 5, 6, 7, or 8 membered ring system, optionally containing up to three heteroatoms, such as N, O or S, the number of N atoms being 0-3 and the number of O and S atoms each being 0-2. Preferably, the two groups which are attached to adjacent carbon atoms, are combined into a saturated cyclic 5 or 6 membered ring system, optionally containing up to three heteroatoms, such as N or O, the number of N atoms being 0-3 and the number of O atoms each being 0-2. This cyclic ring system may optionally be further substituted by an oxo group. Preferred examples of such a substituted aryl groups are benzo[1,3]dioxol and 1,3-dihydro-benzoimidazol-2-one.

The statement is made that when two side chains are found on a single N, they can be combined, including the N to which they are attached, into a heterocyclic ring of 4-, 5-, 6-, 7- or 8 atoms, which can be saturated, partly unsaturated or aromatic, which can optionally contain up to three additional heteroatoms selected from N, O or S, the number of N atoms being 0-3 and the number of O and S atoms each being 0-2; and which ring can be part of a multiple condensed ring-system, in which some rings may be aromatic. Preferred examples of such heterocyclic ring systems, including the N, to which the respective side chains are attached, comprise:

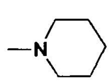 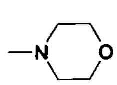 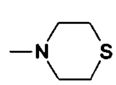 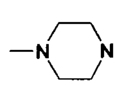 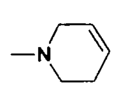

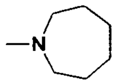 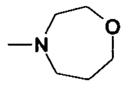 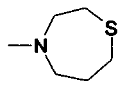 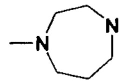

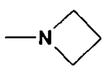 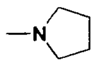 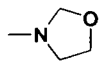 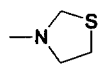 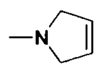

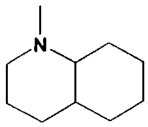 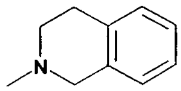 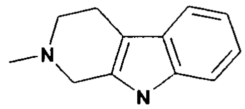

The aforementioned heterocyclic ring system can be optionally substituted by up to three substituents, which can be attached to any carbon or nitrogen atom of the heterocyclic ring system. Preferred examples of substituted heterocyclic ring systems are:

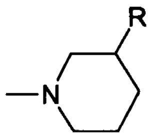 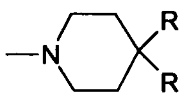 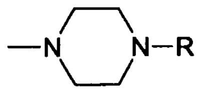

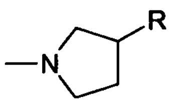 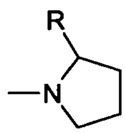 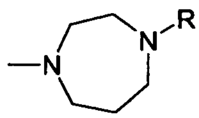

The optional up to three independently selected substituents for the heterocyclic ring system may be chosen among optionally substituted alkyl, halogen, hydroxyl, oxo, thiol, nitro, nitrile, ($C_1$-$C_6$)-alkoxy, aryl, heteroaryl, optionally substituted cycloheteroalkyl, aryloxy, arylalkyloxy, amino, amido, alkylthio, arylthio, arylalkylthio, sulfamoyl, sulfonamide, acyl, carboxyl, and acylamino, as defined herein, whereby all aryl or heteroaryl moieties may be optionally substituted with up to five, preferably up to three independently selected substituents as defined herein.

Furthermore, the aforementioned heterocyclic ring system may be substituted by two groups which are attached to the same carbon atom and are combined into a saturated or partly unsaturated cyclic 4, 5, 6, 7, or 8 membered ring system, optionally containing up to three heteroatoms, such as N, O or S, the number of N atoms being 0-3 and the number of O and S atoms each being 0-2. This cyclic ring system may optionally be further substituted by up to three substitutents independently selected from oxo, ($C_1$-$C_6$)-alkyl, aryl, preferably phenyl, and aryl-($C_1$-$C_4$)-alkyl, preferably benzyl. Preferred examples of such substituted heterocyclic ring systems are 1,4-dioxa-8-aza-spiro[4.5]decane, 1,3,8-triaza-spiro[4.5]decane, 1,3,8-triaza-spiro[4.5]decan-4-one, 1-Phenyl-1,3,8-triaza-spiro[4.5]decane, and 1-Phenyl-1,3,8-triaza-spiro[4.5]decan-4-one.

The term "sulphamate group" as used herein, refers to a group -O-$SO_2$-$NR^3R^{3'}$, and includes a steroidal ester of sulphamic acid or a steroidal ester of an N-substituted derivative of sulphamic acid, or a salt thereof. If -O-$R^1$ is a sulphamate group then the compound of the present invention is referred to as a sulphamate compound.

The term "carbamate group" as used herein, refers to a group -O-CO-$NR^3R^{3'}$, and includes a steroidal ester of carbamic acid or a steroidal ester of an N-substituted derivative of carbamic acid, or a salt thereof. If -O-$R^1$ is a carbamate group then the compound of the present invention is referred to as a carbamate compound.

The term "phosphonate group" as used herein, refers to a group -O-PO($OR^{16}$)-$R^3$, and includes a steroidal ester of phosphonic acid or a steroidal ester of an O-substituted derivative of phosphonic acid, or a salt thereof. If -O-$R^1$ is a phosphonate group then the compound of the present invention is referred to as a phosphonate compound.

The term "thiophosphonate group" as used herein, refers to a group -O-PS($OR^{16}$)-$R^3$, and includes a steroidal ester of thiophosphonic acid or a steroidal ester of an O-substituted derivative of thiophosphonic acid, or a salt thereof. If -O-$R^1$ is a thiophosphonate group then the compound of the present invention is referred to as a thiophosphonate compound.

The term "phosphate group" as used herein, refers to a group -O-PO($OR^{16}$)-$OR^3$, and includes a steroidal ester of phosphoric acid or a steroidal ester of an O-substituted derivative of phosphoric acid, or a salt thereof. If -O-$R^1$

is a phosphate group then the compound of the present invention is referred to as a phosphate compound.

The term "sulphonate group" as used herein, refers to a group $-O-SO_2-R^3$, and includes a steroidal ester of sulphonic acid, or a salt thereof. If $-O-R^1$ is a sulphonate group then the compound of the present invention is referred to as a sulphonate compound.

The term "sulphate group" as used herein, refers to a group $-O-SO_2-OR^3$, and includes a steroidal ester of sulphuric acid, or a salt thereof. If $-O-R^1$ is a sulphate group then the compound of the present invention is referred to as a sulphate compound.

For all above-mentioned sulphamate-, carbamate-, phosponate-, thiophosphonate-, phosphate-, sulphonate-, and sulphate- groups, the substituents $R^3$ and $R^{3,}$, if present, are independently selected from H, alkyl, aryl and arylalkyl, as defined herein, or form together with the nitrogen atom, to which $R^3$ and $R^{3'}$ are attached, a heterocyclic 4-, 5-, 6-, 7- or 8-memberred ring, which is optionally saturated, partly unsaturated, or aromatic; which optionally contains up to three additional heteroatoms selected from N, O or S, the number of additional N atoms being 0, 1, 2 or 3 and the number of O and S atoms each being 0, 1 or 2. Preferably, at least one of R9 and R10 is H, and even more preferred, each of $R^9$ and $R^{10}$ is H. If the substituent $R^{16}$ is present in one of the aforementioned groups, then represents -H, alkyl, or arylalky, as defined herein above. Preferably, $R^{16}$ represents -H.

The term "pharmaceutically acceptable salts" refers to salt forms that are pharmacologically acceptable and substantially non-toxic to the subject being administered the compounds of the invention. Pharmaceutically acceptable salts of compounds of formula I include conventional and stoichiometrical acid-addition salts or base-addition salts formed from suitable non-toxic organic or inorganic acids or inorganic bases. Acid addition salts, for example, from compounds of formula I with a basic nitrogen atom are formed preferably with organic or inorganic acids. Suitable inorganic acids are, for example, halogenic acids such as hydrochloric acid, sulfuric acid, or phosphoric acid. Suitable organic acids are, for example, carboxylic, phosphonic, or sulfonic acids, for example acetic acid, propionic acid, glycolic acid, lactic acid, hydroxybutyric acid, malic acid, malenic acid, malonic acid, salicylic acid, fumaric acid, succinic acid, adipic acid, tartaric acid, citric acid, glutaric acid, 2- or 3-glycerophosphoric acid and other mineral and carboxylic acids well known to those skilled in the art. The salts are prepared by contacting the free base forms with a sufficient amount of the desired acid to produce a salt in the conventional manner. Compounds containing acidic substituents may also form salts with inorganic or organic bases. Examples of suitable bases for salt formation include, but are not limited to, inorganic bases such as alkali or alkaline earth-metal (e.g., sodium, potassium, lithium, calcium, or magnesium) hydroxides, and those derived from ammonium hydroxides (e.g., a quaternary ammonium hydroxide such as tetramethylammonium hydroxide). Also contemplated are salts formed with pharmaceutical acceptable amines such as ammonia, alkyl amines, hydroxyalkylamines, N-methylglucamine, benzylamines, piperidines, and pyrrolidines and the like. Certain compounds will be acidic in nature, e. g. those compounds which possess a carboxyl or phenolic hydroxyl group. Salts of phenols can be made by heating acidic compounds with any of the above mentioned bases according to procedures well known to those skilled in the art.

As used herein, the term "composition" is intended to encompass a product comprising the specified ingredients in the specified amounts, as well as any product which results, directly or indirectly, from combination of the specified ingredients in the specified amounts.

The phrase "effective amount" as used herein, means an amount of a compound or composition which is sufficient enough to significantly and positively modify the symptoms and/or conditions to be treated (e. g., provide a positive clinical response). The effective amount of an active ingredient for use in a pharmaceutical composition will vary with the particular condition being treated, the severity of the condition, the duration of the treatment, the nature of concurrent therapy, the particular active ingredient(s) being employed, the particular pharmaceutically acceptable excipient(s)/carrier(s) utilized, and like factors within the knowledge and expertise of the attending physician.

## Administration forms

The compounds of the invention are primarily intended for treatment in a mammal, preferably in humans and other primates, of steroid hormone dependent diseases or disorders, in particular estradiol dependent diseases or disorders, wherein the steroid hormone dependent disease or disorder preferably requires the inhibition of a 17β-HSD enzyme, preferably the 17β-HSD1 enzyme.

The compounds may be administered orally, dermally, parenterally, by injection, by pulmonal or nasal delivery, or sublingually, rectally or vaginally in dosage unit formulations. The term "administered by injection" includes intravenous, intraarticular, intramuscular (e.g. by depot injection where the active compounds are released slowly into the blood from the depot and carried from there to the target organs), intraperitoneal, intradermal, subcutaneous, and intrathecal injections, as well as use of infusion techniques. Dermal administration may include topical application or transdermal administration. One or more compounds may be present in association with one or more non-toxic pharmaceutically acceptable auxiliaries such as excipients, adjuvants (e.g. buffers), carriers, inert solid diluents, suspensing agents, preservatives, fillers, stabilizers, antioxidants, food additives, bioavailability enhancers, coating materials, granulating

and disintegrating agents, binding agents etc., and, if desired, other active ingredients.

The pharmaceutical composition may be formulated for example as immediate release, sustained release, pulsatile release, two or more step release, depot or other kind of release formulations.

Commonly known and used pharmaceutically acceptable auxiliaries as well as further suitable diluents, flavorings, sweetening agents, coloring agents etc. may be used, depending on the intended mode of administration as well as particular characteristics of the active compound to be used, such as solubility, bioavailability etc. Suitable auxiliaries and further ingredients may be such as recommended for pharmacy, cosmetics and related fields and which preferably are listed in the European Pharmacopoeia, FDA approved or cited in the "GRAS" list (FDA List of food additives that are 'generally recognized as safe' (GRAS)).

One mode of application of the compounds of general formula (I) or of pharmaceutical compositions comprising one or more of said compounds is oral application, e. g., by tablets, pills, dragees, hard and soft gel capsules, granules, pellets, aqueous, lipid, oily or other solutions, emulsions such as oil-in-water emulsions, liposomes, aqueous or oily suspensions, syrups, elixiers, solid emulsions, solid dispersions or dispersible powders. For the preparation of pharmaceutical compositions for oral administration, the compounds suitable for the purposes of the present invention as defined above can be admixed with commonly known and used adjuvants and excipients such as for example, gum arabic, talcum, starch, sugars (such as, e. g., mannitose, methyl cellulose, lactose), gelatine, surface-active agents, magnesium stearate, aqueous or nonaqueous solvents, paraffin derivatives, cross-linking agents, dispersants, emulsifiers, lubricants, conserving agents, flavoring agents (e. g., ethereal oils), solubility enhancers (e. g., benzyl benzoate or benzyl alcohol) or bioavailability enhancers (e.g. Gelucire™). In the pharmaceutical composition, the active ingredients may also be dispersed in a microparticle, e. g. a nanoparticulate, composition.

For parenteral administration, the active agents can be dissolved or suspended in a physiologically acceptable diluent, such as, e. g., water, buffer, oils with or without solubilizers, surface-active agents, dispersants or emulsifiers. As oils for example and without limitation, olive oil, peanut oil, cottonseed oil, soybean oil, castor oil and sesame oil may be used. More generally spoken, for parenteral administration the active agent can be in the form of an aqueous, lipid, oily or other kind of solution or suspension or even administered in the form of liposomes or nano-suspensions.

Transdermal application can be accomplished by suitable patches, as generally known in the art, specifically designed for the transdermal delivery of active agents, optionally in the presence of specific permeability enhancers. Furthermore, also emulsions, ointments, pastes, creams or gels may be used for transdermal delivery.

Another suitable mode of administration is via intravaginal devices (e. g. vaginal rings) or intrauterine systems (IUS) containing reservoirs for controlled release of active agents over extended periods of time. For rectal or vaginal administration of the drug the compounds may also

Another suitable mode of administration is via intravaginal devices (e. g. vaginal rings) or intrauterine systems (IUS) containing reservoirs for controlled release of active agents over extended periods of time. For rectal or vaginal administration of the drug the compounds may also be administered in the form of suppositories. These compositions can be prepared by mixing the drug with a suitable non-irritating excipient which is solid at ordinary temperatures but liquid at the rectal or vaginal temperature and will therefore melt in the rectum or vagina to release the drug.

Another mode of application is by implantation of a depot implant comprising an inert carrier material, such as biologically degradable polymers or synthetic silicones such as e. g. silicone rubber. Such implants are designed to release the active agent in a controlled manner over an extended period of time (e. g., 3 to 5 years).

It will be appreciated by those skilled in the art that the particular method of administration will depend on a variety of factors, all of which are considered routinely when administering therapeutics. It will also be understood, however, that the actual dosages of the agents of this invention for any given patient will depend upon a variety of factors, including, but not limited to the activity of the specific compound employed, the particular composition formulated, the mode of administration, time of administration, route of administration and the particular site, host, and disease being treated, and furthermore the age of the patient, the body weight of the patient, the general health of the patient, the gender of the patient, the diet of the patient, rate of excretion, drug combinations, and the severity of the condition undergoing therapy. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests. Optimal dosages for a given set of conditions may be ascertained by those skilled in the art using conventional dosage-determination tests in view of the experimental data for a given compound. For oral administration, an exemplary daily dose generally employed will be from about 0.01 μg/kg to about 100 mg/kg of total body weight, whereby courses of treatment may be repeated at appropriate time intervals. The daily dosage for parenteral administration will generally be from about 0.01 μg/kg to about 100 mg/kg of total body weight. A daily rectal dosage regimen will generally be from about 0.01 μg/kg to about 200 mg/kg of total body weight. A daily vaginal dosage regimen will generally be from about 0.01 μg/kg to about 100 mg/kg of total body weight. The daily topical dosage regimen will generally be from about 0.1 μg to about 100 mg administered between one to four times daily. The transdermal concentration will generally be that required to maintain a daily dose of from 0.01 μg/kg to 100 mg/kg of total body weight.

## Abbreviations and Acronyms

As employed herein, the following terms have the indicated meanings.

| | | | |
|---|---|---|---|
| ACN | acetonitrile | Hünig base | N-Ethyldiisopropylamine = N(iPr)2Et = EDIPA): |
| Aq | aqueous | | |
| Bn | benzyl | *m*-CPBA | m-chloroperoxybenzoic acid |
| BOC | tert-butoxycarbonyl | MeOH | methanol |
| conc. | concentrated | min | minute(s) |
| d | day(s) | MOM | methoxy methyl |
| DAST | N,N-diethylaminosulfur trifluoride40 | NAD(P)[H] | nicotinamide-adenine-dinucleotide (phosphate) [reduced NAD(P)] |
| DCM | dichloromethane = $CH_2Cl_2$ | | |
| DHP | 3,4-dihydro-[2H]-pyran | NMM | N-methylmorpholine |
| DIAD | diisopropyl azodicarboxylate | NMO | N-methylmorpholine N-oxide |
| DIBAH | Diisobutyl aluminiumhydrid | NMR | nuclear magnetic resonance |
| DIPEA | N,N-diisopropylethylamine | PG | protection group |
| DME | dimethyl ethylene glycol = 1,2-dimethoxyethane | pTosOH | para-toluene sulphonic acid |
| | | Rt | Retention time |
| DMF | N,N-dimethylformamide | RT | room temperature |
| DMSO | dimethylsulfoxide | sat | saturated |
| E1 | estron | STS | steroid sulphatase |
| E2 | estradiol | T3P | propylphosphonic acid anhydride |
| EDCI | 1-(3-dimethylaminopropyl)-3-ethylcarbodiimide | TBAF | Tetrabutylammonium-fluorid-Lösung |
| EDCI•HCl | 1-(3-dimethylaminopropyl)-3-ethylcarbodiimide hydrochloride | TBDMS | tert-butyl dimethyl siloxy |
| | | TBME | tert-butyl methyl ether |
| ER | estrogen receptor | TEA | triethylamine |
| EtOAc | ethyl acetate | TEOF | Triethylorthoformat ($CH(OEt)_3$) |
| h | hour(s) | THF | tetrahydrofuran |
| HMPA | hexamethylphosphoramide | THP | tetrahydropyran |
| HOBT | 1-Hydroxybenzotriazole Hydrate | TLC | thin-layer chromatography |
| HPLC | High Performance Liquid Chromatography | TMSCI | trimethylsilylchloride / $Me_3SiCl$ |
| | | TPAP | tetrapropylammonium perruthenate |
| HSD | hydroxysteroid dehydrogenase | | |

## Numbering of compound formulas and intermediates

The general structure formulas are typically designated with a number in roman format, followed by α or β indicating the stereochemistry at the C15 atom of the estron core if necessary. If the number of methylen groups attached at the C15 position is specified (i.e. the value of "n"), the roman number is followed by a hyphen and a number indicating the amount of methylen groups. Finally, a letter a, b or c is attached after the number "n", indicating the nature of the substituent R1 at the O-atom in C3 position of the estron core (a = hydrogen, b = methyl, and c = benzyl). The prefix C in front of the number indicates that the compound may be substituted in C2 by a residue $R^{14}$. The prefix D in front of the number that the compound may be substituted in C2 by a residue $R^{14}$ and may be additionally modified within the C16-C17 position.

For example, compound IV is the general acid building block:

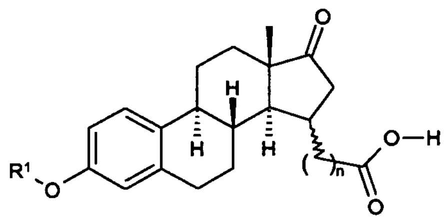

(IV)

Therefore, a compound IVβ-3a would represent a derivative of IV with β stereochemistry at C15, three methylen groups and a hydroxy group in C3 position, i.e.:

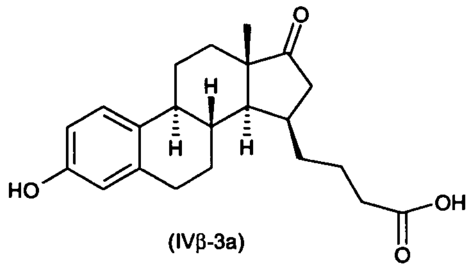

(IVβ-3a)

If particular structures of synthesized examples falling under a general formula are presented, then the designation of the general formula is followed by the particular number of this example, i.e. Example No. 652 of formula (XXXIIIα-1a)-652

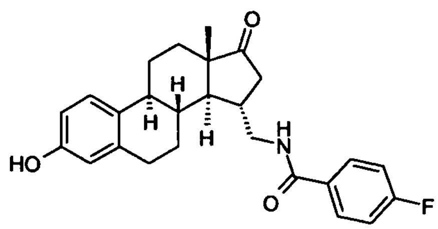

(XXXIIIα-1a)-652

This example 652 is a particular compound of the general formula XXXIIIα-1a, wherein R2 is a 4-fluoro-phenyl residue.

## General Preparative Methods

The compounds of the present invention may be prepared by use of known chemical reactions and procedures. Nevertheless, the following general preparative methods are presented to aid the reader in synthesizing the 17β-HSD1 and/or STS inhibitors, with specific details provided below in the experimental section to illustrate working examples.

All variable groups of these methods are as described in the generic description if they are not specifically defined below.

It is recognized that compounds of the invention with each claimed optional functional group may not be prepared by each of the below-listed methods. Within the scope of each method, optional substituents may appear on reagents or intermediates which may act as protecting or otherwise non-participating groups. Utilizing methods well known to those skilled in the art, these groups are introduced and/or removed during the course of the synthetic schemes which provide the compounds of the present invention.

## Flow Diagrams

The synthesis of 3, 15 substituted estrone derivatives bearing a side chain of the amide, ester, carbonyl, hydrazone, alcohol, ether, urea, carbamate, "retro"-amide, sulfonyl urea, sulfamide, sulfamate, "retro"-sulfonamide, "retro"-carbamate, "retro"-ester or sulfonylcarbamate type in position C15 is extensively described within the international application WO 2005/047303, which is hereby incorportated by reference in its entity.

The additional modifications of the steroidal core at positions C2, C3, C16 and or C17, which are disclosed in the present invention, may be introduced in the following order of general chemical modifications (General Synthesis Scheme). The introduction of the $R^{14}$ substituent in C2 position - if present in the final compound - has to take place first, starting from the 17β-estradiol using methods well known in the art (Steps A). In parallel, the C17-OH function is oxidized to the corresponding keto function. Depending on the desired nature of R1, a suitable group also functioning as protecting group may be introduced at this point. Then, the estron derivative of formula (V) is converted into the central intermediate, the 15, 16-unsaturated estrone of formula X (Steps B), which is further derivated in the C15 position by introduction of the basic side chain ("so called building blocks"). These building blocks are reacted with the appropriate compounds carrying the $R^2/R^4$ substitutents to lead to the desired C15 substituted compound (Steps C). The obtained educt may be further modified within the C16 and C17 position by introducing appropriate substituents $R^{10}$, $R^{11}$, $R^{12}$ and $R^{13}$ or by introducing a heterocyclic ring structure (Steps D). Finally, if necessary, the protection group in C1 position may be separated to deliver the C3-OH derivative or may be further substituted with an alternative $R^1$ side chain or may be derivated to the corresponding sulphamate, phosponate, carbamate, thiophosponate, sulphonate, sulphate or phosphate compounds (Steps E).

**GENERAL SYNTHESIS SCHEME:**

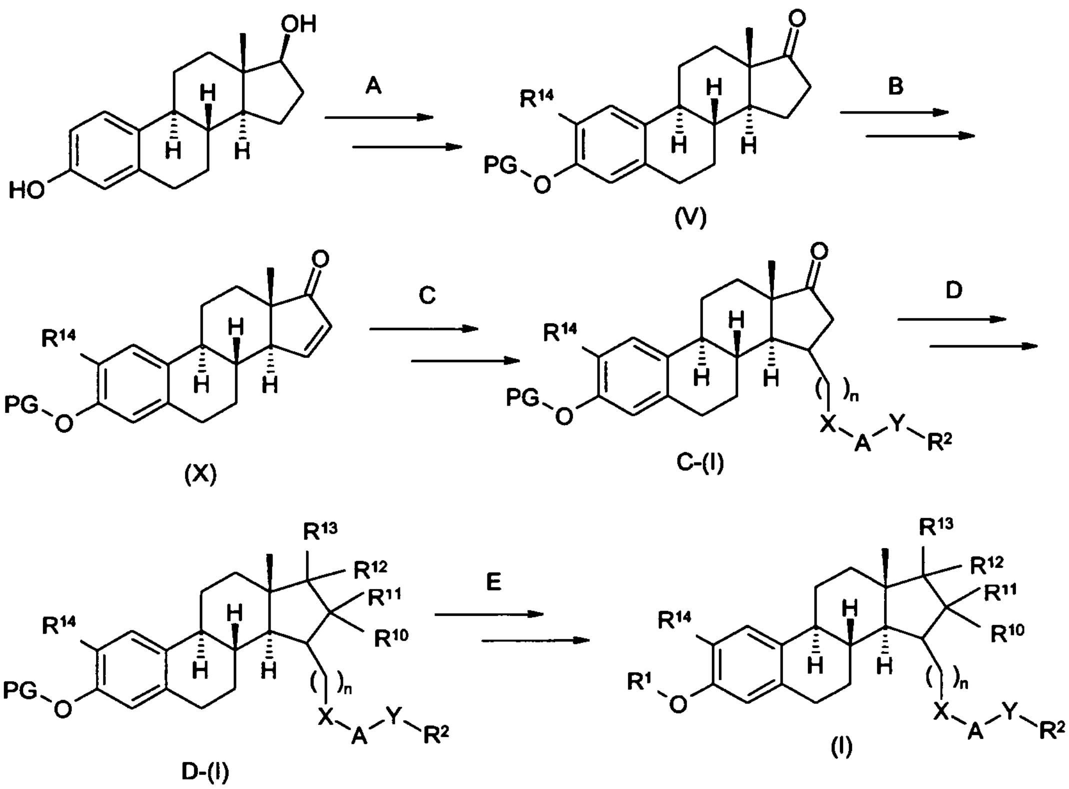


**Step A - Introduction of a $R^{14}$ side chain in C2 position of 17β-Estradiol or Estron**

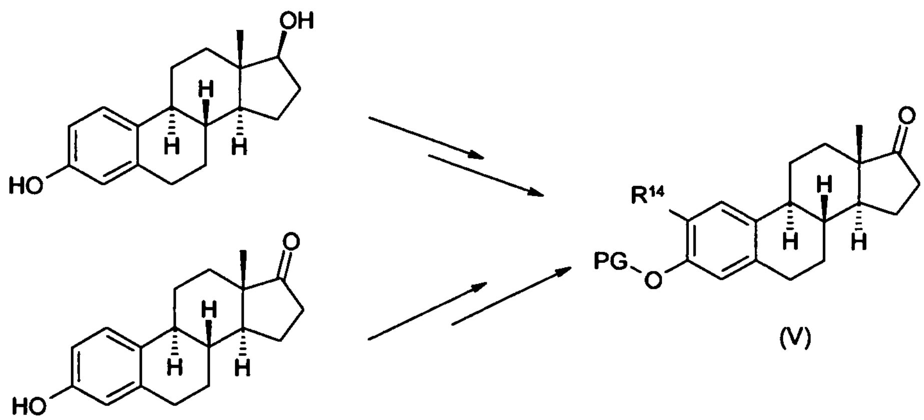

(V)

The introduction of various side chains in the estron core is known from the literature, e.g. Rao et al (2002) describe the synthesis of 2-methoxyestradiol, and the synthesis of 2-ethoxy-estradiol was disclosed by Verdier-Pinard et al (2000). 2-Ethyl-estron may be prepared from estrone by Friedel-Crafts acetylation of estrone-3-O-methyl ether and catalytic hydrogenation, followed by demethylation, which produced the desired product. Alternatively, the introduction of substituents on the 2-position may be obtained by using a Fries-rearrangement starting with estradiol and the reagent $(RCO)_2O$ with R = lower alkyl, as described by Rao et al. (2002): After acylation, the compounds should be converted into the R-CO-substituted derivatives in C2 position. Reduction of the acyl function may be achieved by reduction with Pd/C and $H_2$ [Gonzalez et al (1982)]. Alternatively, the acetoxy-group in C2-position could be oxidized with $PhI(CF_3CO_2)_2$ according to [Yoshikawa et al. (2002)]. The newly introduced hydroxy group may be further alkylated, followed by reduction of the ketone, resulting in an alkoxy-alkyl substituted estradiol derivative. An alternative strategy to introduce an alkoxy-alkyl group is exemplified for the methoxy-ethyl group: After MOM-protection of the 17β-estradiol, the MOM-protected estradiol is iodinated [Mohanakrishnan & Cushman (1999)]. Then, the MOM-group is replaced with a TBDMS group. Negishi coupling with allylbromide gives the 2-allyl substituted estrone derivative, which can be oxidised and methylated (including some protective group manipulations). Further synthetic ways to 2-alkyl-substituted estron or estradiol derivatives have been displayed previously [see e.g. Mohanakrishnan & Cushman (1999); Day et al. (2003); Cushman et al (1995), and Lunn & Farkas (1968)] The synthesis of further estron derivatives with various substituents in 2-position was disclosed by Cushman et al (2002).

During the introduction of the C2 side chain, the 3-hydroxy function of the steroidal core is typically protected with a methyl or benzyl group (exemplified by PG). For example, the methyl derivative can be prepared using MeJ and acetone, whereas the corresponding Benzyl-derivative may be prepared using Benzylbromid, DIPEA and acetone. Enone intermediates with other substituents in $R^1$ (= PG), in particular optionally substituted $C_1$-$C_4$-alkyl, can be prepared accordingly by using the appropriate optionally substituted $C_1$-$C_4$-alkyl-bromide or $C_1$-$C_4$-alkyl-iodide.

**Step B - Synthesis of the 15, 16-unsaturated Estrone of formula X (Intermediate I)**

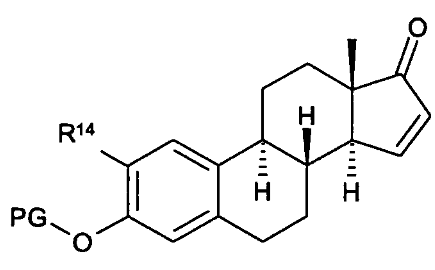

(X)

The ketal of the formula (IX) can be prepared according to Nambara from the corresponding 2-substituted estron of formula V [Nambara et al. (1976)] as depicted within the following scheme 1. If not yet protected, the introduction of PG groups in C3 position can be achieved according to a procedure described by Labaree (2003).

## SCHEME 1

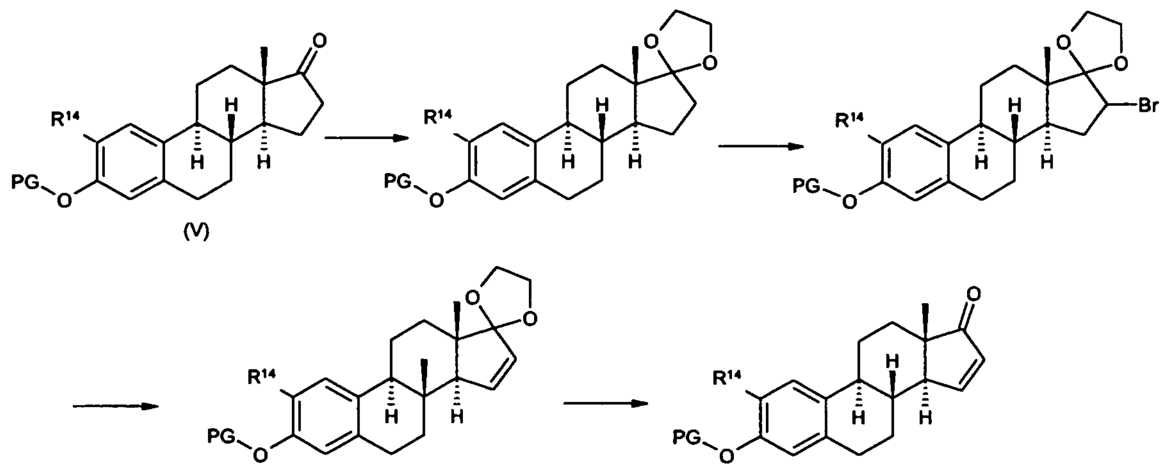

(V)

(IX)

(X)

The C17 keto function of the C2 substituted and protected estron derivative of formula (V) is protected as acetal, followed by bromination. The elimination of the bromide yielded the desired 15, 16-unsaturated estron. Finally, the ketal derivative is hydrolysed to give the appropriate enone-derivative X.

Alternatively, the enone intermediate of formula X can be prepared from the corresponding estrone derivative according to a procedure described by Poirier et al. (1991).

**Step C - Introduction of the side chain in C15 position**

The "**Step C**" modification -the introduction of the side chain in C15 position - is carried out in two major steps: In a first step the 15, 16-unsaturated Estrone of formula X is converted into a so-called building block carrying an alkyl side chain in C15 position with a terminal amino, carboxy, or alcohol function. The synthesis of some exemplary building blocks is depicted in the Experimental Section "Intermediates", and was fully disclosed in international patent application WO 2005/047303.

The second step of the "**Step C**" modification - the conversion of the building blocks into the desired derivatives carrying the complete side chain in C15 position - is exemplified below by using one of the following synthetic schemes as shown in Flow Diagrams I to XV.

Certain formula I compounds, in which X represents a bond, A represents CO, Y represents NH or $NR^4$ and n represents an integer from 0 to 5, may be prepared by a reaction as shown in **Flow Diagram Ia.**

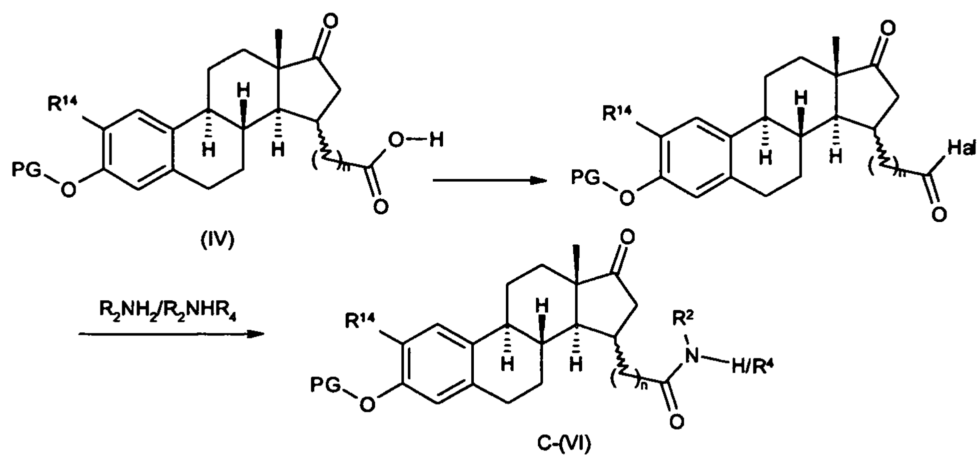


(IV)

C-(VI)

The free acid (IV) may be converted to the reactive acyl halide, in particular the acid chloride, by reaction with $SOCl_2$, $COCl_2$, $PCl_5$ or $PBr_3$ or the like. The amide derivatives C-(VI) may be prepared by a base catalyzed addition-elimination reaction, where the halogen residue is substituted with the appropriate amine $R^2NH_2$ or $R^2NHR^4$ in the presence of a base, for example DIPEA. Alternatively, especially suited for derivatives with n > 2, the amide derivatives

may be prepared directly from the free acids by nucleophilic substitution with the appropriate amine. Alternatively, the amide derivatives may be prepared directly from the free acids by nucleophilic substitution with the appropriate amine as shown in **Flow Diagram Ib**:

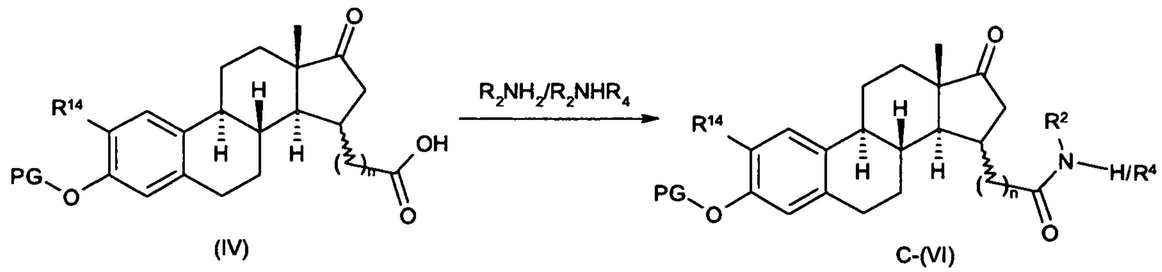


(IV) C-(VI)

Certain formula I compounds, in which X represents a bond, A represents CO, Y represents O, and n represents an integer from 0 to 5, may be prepared by a reaction as shown in **Flow Diagram II:**

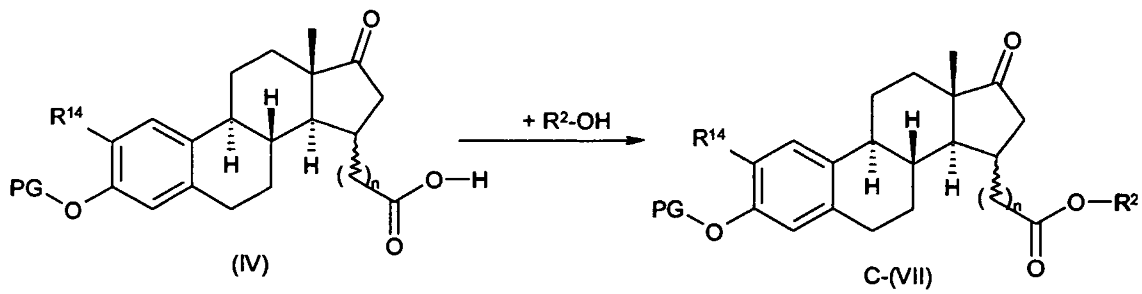


(IV) C-(VII)

The ester derivatives C-(VII) may be prepared from the free acid (IV) by esterification with the appropriate alcohol $R^2$-OH.

Certain formula I compounds, in which X represents a bond, A represents CO, Y represents a bond, and n represents an integer from 0 to 5, may be prepared by a reaction as shown in **Flow Diagram III**:

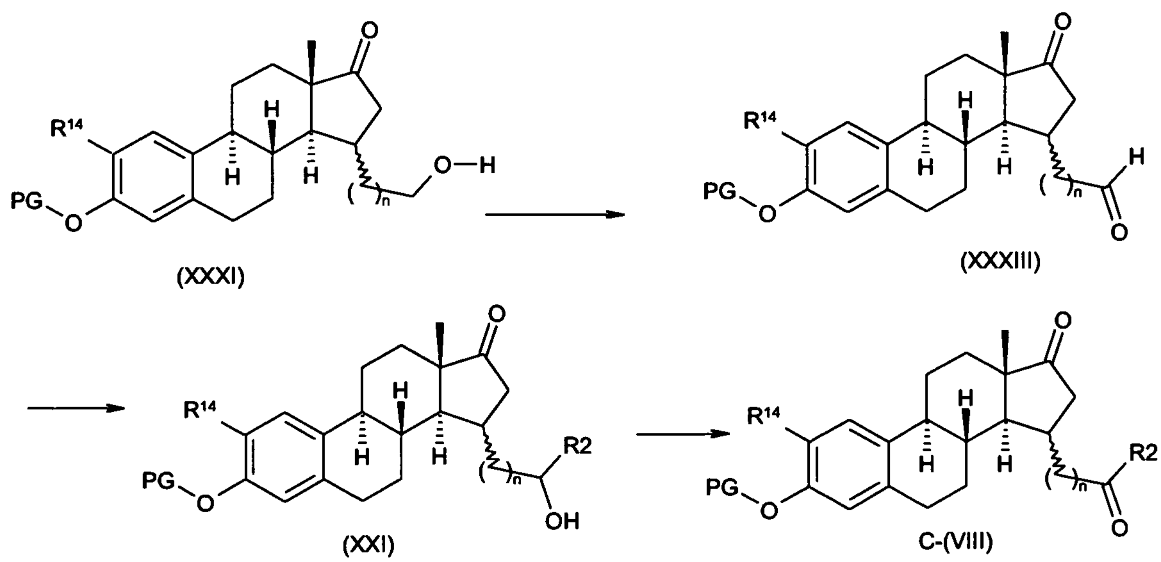

(XXXI) (XXXIII) (XXI) C-(VIII)

The alcohol (XXXI) may be converted to the corresponding aldehyde (XXXIII) via Dess-Martin Oxidation. Subsequently the aldehyde may be converted by a nucleophilic addition-elimiation reaction with a Grignard or other organometallic reagent, substituted with the appropriate R2 residue to the corresponding secondary alcohol (XXI), which thereafter can be oxidized again to the desired ketone C-(VIII).

Certain formula I compounds, in which X represents a bond, A represents CO, Y represents NH-NR$^4$ or NH-NH, and n represents an integer from 0 to 5, may be prepared by a reaction as shown in **Flow Diagram IVa**.

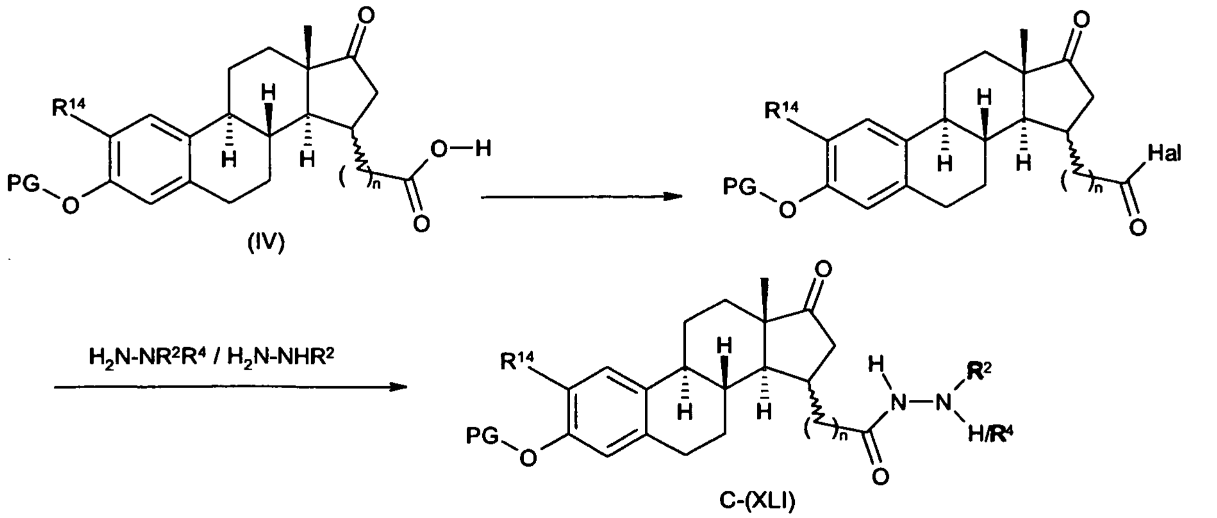


(IV)

C-(XLI)

The free acid (IV) may be converted to the reactive acyl halide, in particular the acid chloride, by reaction with $SOCl_2$, $COCl_2$, $PCl_5$ or $PBr_3$ or the like. The hydrazide derivatives C-(XLI) may be prepared by a base catalysed addition-elimination reaction, where the halogen residue is substituted with the appropriate hydrazine $H_2N$-$NHR^2$ or $H_2N$-$NR^2R^4$ in the presence of a base, for example DIPEA. Alternatively, especially suited for derivatives with $n > 2$, the hydrazide derivatives may be prepared directly from the free acids by nucleophilic substitution with the appropriate hydrazine using e.g. polymer bound carbodiimid, HOBT and DCM, as shown in **Flow Diagram IVb:**

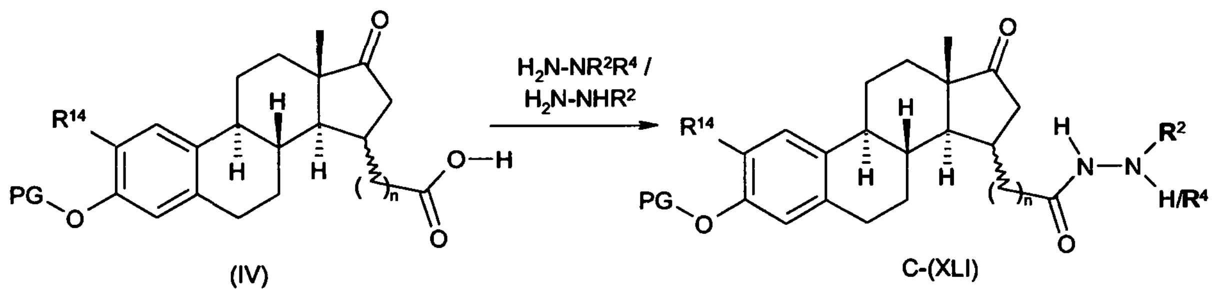


(IV)

C-(XLI)

Certain formula I compounds, in which X represents a NH, A represents CO, Y represents NH, and n represents an integer from 1 to 6, may be prepared by a reaction as shown in **Flow Diagram Va**:

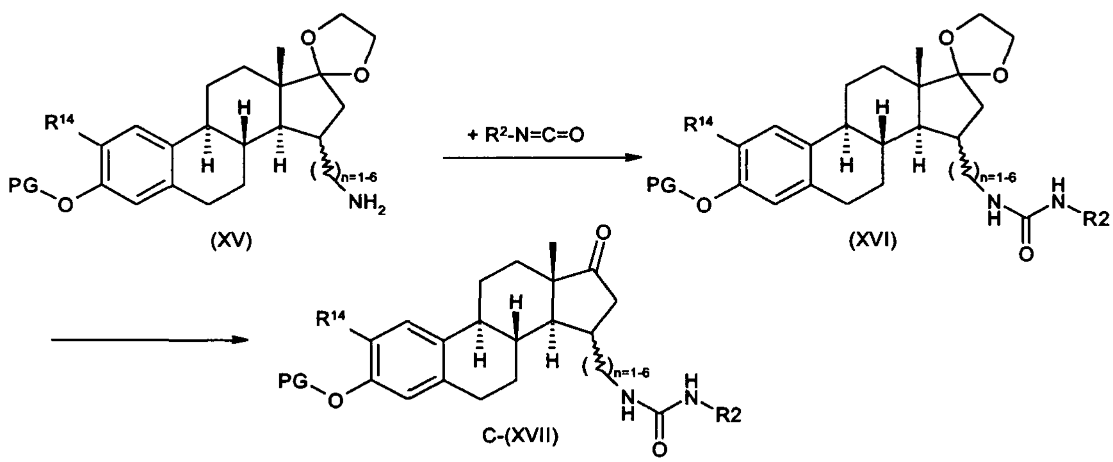


(XV)

(XVI)

C-(XVII)

The urea derivatives of the general formula C-(XVII) may be prepared by the reaction of the amine building block (XV) with an appropriately substituted Isocyanate ($R^2$-N=C=O). After the addition, the ketal function is converted into the keto function. Alternatively, the amine may be first reacted with carbodiimidazol or triphosghen to form a reactive

carbamoyl compound, which than can react further with a suitable amine $R^2R^4$-NH. A further synthesis variant may use the unprotected amine (XXIX) as starting material for the reaction with an appropriately substituted Isocyanate ($R^2$-N=C=O) as shown in **Flow Diagram Vb**

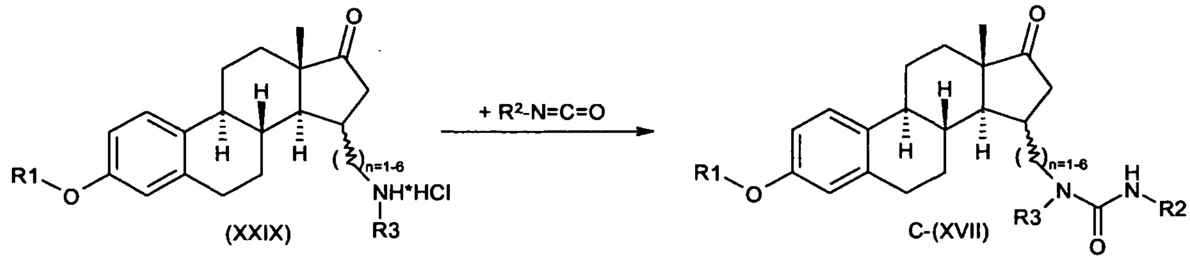


(XXIX) C-(XVII)

Certain formula I compounds, in which X represents a -NH-, A represents $SO_2$, Y represents NH, and n represents an integer from 1 to 6, may be prepared by a reaction as shown in **Flow Diagram VI**

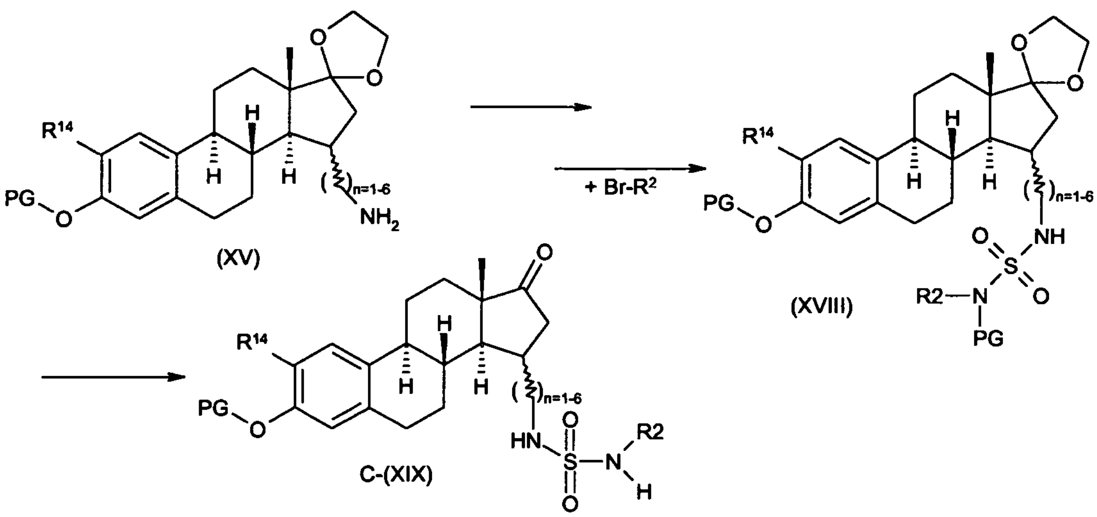


(XV) (XVIII) C-(XIX)

In a first step, the amine building block (XV) may be converted into a protected, for example Boc-protected, sulfamide compound by a reaction with the appropriately protected chlorosulfonyl isocyanate. In a second step, the protected sulfamide compound is allowed to react with the appropriate Bromo-reagent ($R^2$-Br) to provide the still protected, substituted sulfamide derivative of the formula (XVIII). After deprotection, the desired N-substituted sulfamide derivative of formula C-(XIX) is obtained.

Certain formula I compounds, in which X represents a NH, A represents CO, Y represents O, and n represents an integer from 1 to 6, may be prepared by a reaction as shown in **Flow Diagram VII**:

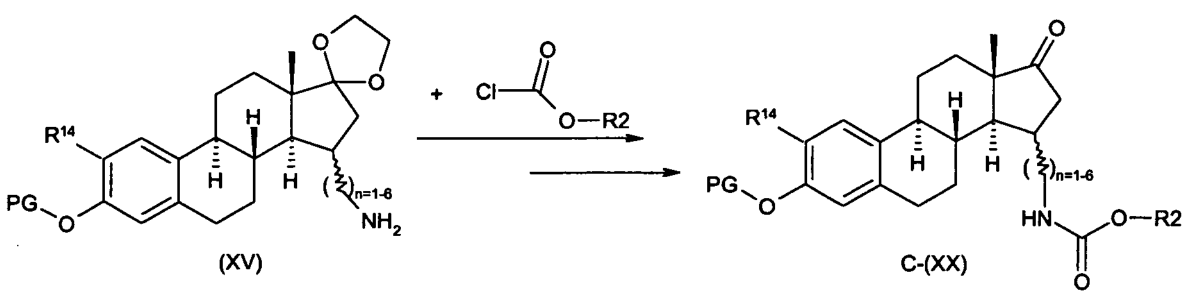

(XV) C-(XX)

The carbamate derivatives of the general formula C-(XX) may be prepared by the reaction of the amine building block (XV) with an appropriate chloroformic acid ester ($R^2$-O-CO-Cl). After the addition-elimination reaction, in a second step the ketal function is converted into the keto function.

Certain formula I compounds, in which X represents a NH, A represents $SO_2$, Y represents O, and n represents

an integer from 1 to 6, may be prepared by a reaction as shown in **Flow Diagram VIII**:

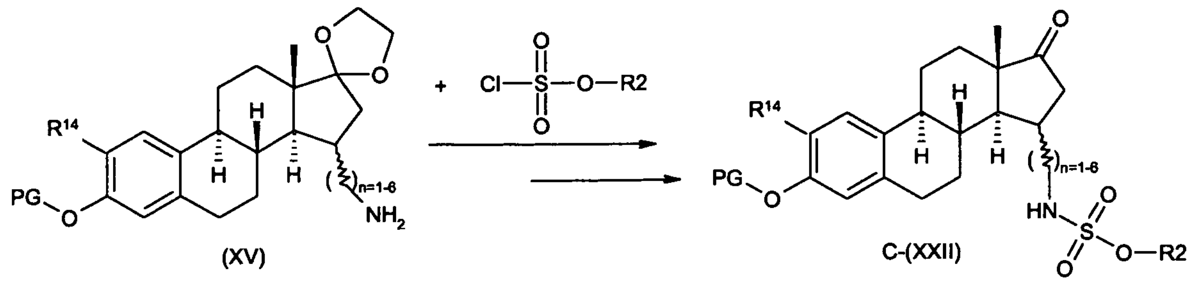

(XV) C-(XXII)

The sulfamate derivatives of the general formula C-(XXII) may be prepared by the reaction of the amine building block (XV) with an appropriate chlorosulfonic acid ester ($R^2$-O-$SO_2$-Cl). After the addition-elimination reaction, in a second step the ketal function is converted into the keto function.

Certain formula I compounds, in which X represents a NH, A represents CO, Y represents a bond, and n represents an integer from 1 to 6, may be prepared by a reaction as shown in **Flow Diagram IXa**:

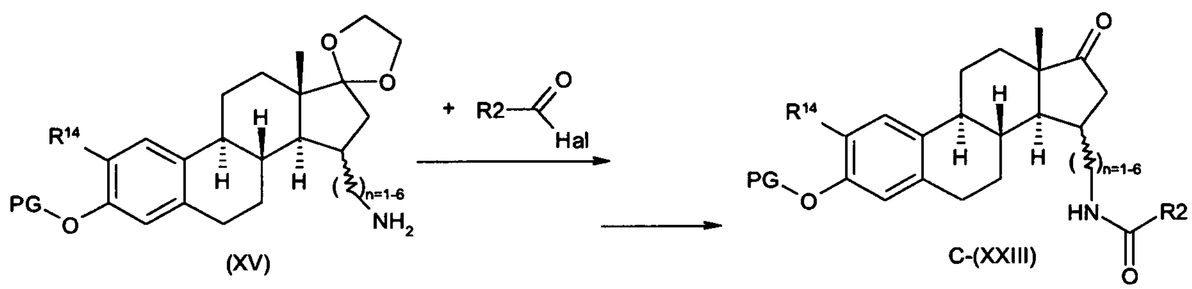

(XV) C-(XXIII)

The "retro"-amide derivatives of the general formula C-(XXIII) may be prepared by the reaction of the amine building block (XV) with an appropriate acid halide, e.g. an acid chloride ($R^2$-CO-Cl). After the addition-elimination reaction, in a second step the ketal function is converted into the keto function. Alternatively, the reaction with an appropriate acid halide, e.g. an acid chloride ($R^2$-CO-Cl), can be performed using the amino-hydrochloride salt of the estrone (XXIX) as starting material as shown in the following **Flow Diagram IXb**:

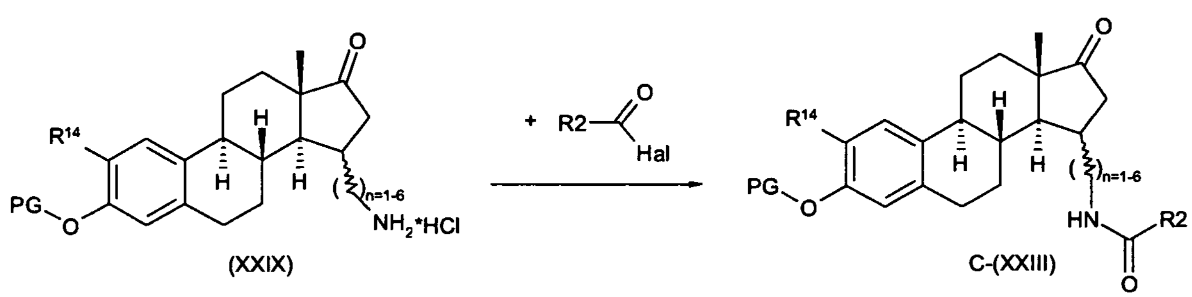

(XXIX) C-(XXIII)

Certain formula I compounds, in which X represents a NH, A represents $SO_2$, Y represents a bond, and n represents an integer from 1 to 6, may be prepared by a reaction as shown in **Flow Diagram Xa**:

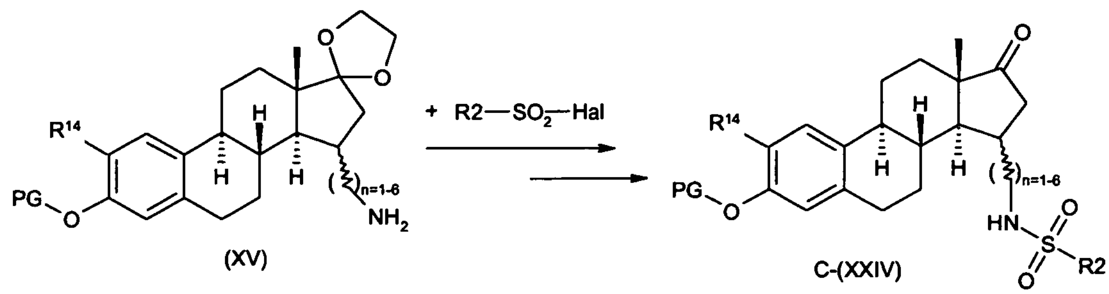


(XV) C-(XXIV)

The sulfonamide derivatives of the general formula C-(XXIV) may be prepared by the reaction of the amine building block (XV) with an appropriate sulfonic acid halide, e.g. a sulfonic acid chloride (R2-$SO_2$-Cl). After the addition-elimination reaction, in a second step the ketal function is converted into the keto function. Alternatively, the reaction with an appropriate sulfonic acid halide, e.g. sulfonic acid chloride ($R^2$-$SO_2$-Cl), can be performed using the amino-hydrochloride salt of the estrone (XXIX) as starting material as shown in the following **Flow Diagram Xb**:

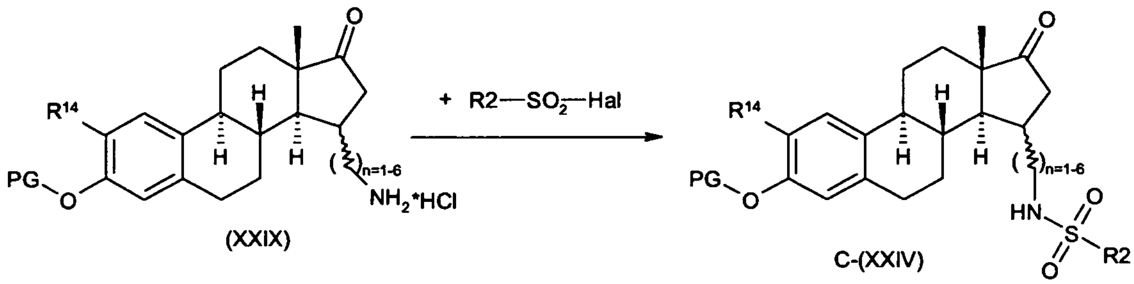


(XXIX) C-(XXIV)

Certain formula I compounds, in which X represents a NH, A represents CO, Y represents NH-$SO_2$, and n represents an integer from 1 to 6, may be prepared by a reaction as shown in **Flow Diagram XI**:

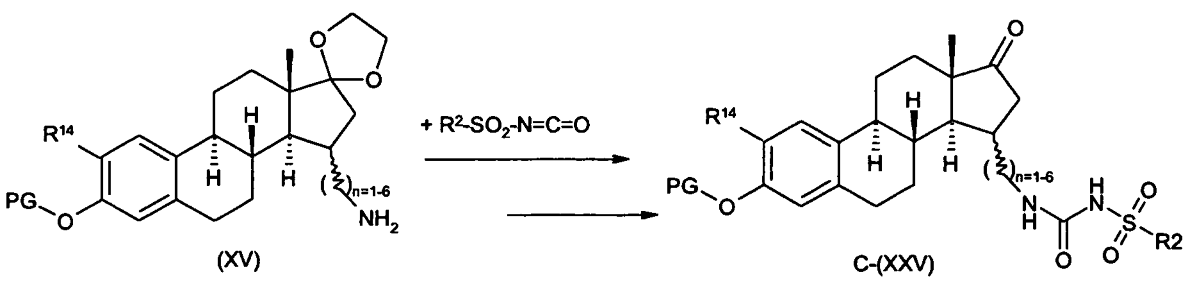


(XV) C-(XXV)

The sulfonyl urea derivatives of the general formula C-(XXV) may be prepared by the reaction of the amine building block (XV) with an appropriately substituted sulfonyl isocyanate ($R^2$-$SO_2$-N=C=O). After the addition, the ketal function is converted into the keto function.

Certain formula I compounds, in which X represents an O, A represents CO, Y represents $NR^4$, and n represents an integer from 1 to 6, may be prepared by a reaction as shown in **Flow Diagram XII**:

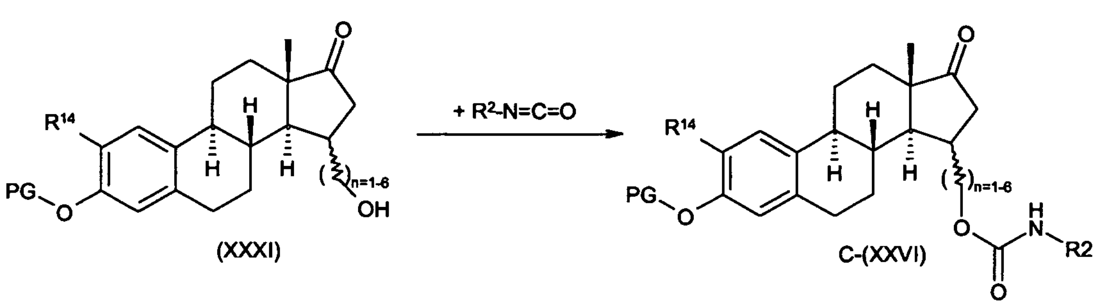


(XXXI) C-(XXVI)

The "retro"-carbamate derivatives of the general formula C-(XXVI) may be prepared by the reaction of the estrone alcohol building block (XXXI) with an appropriately substituted isocyanate ($R^2$-N=C=O) and subsequent purification.

Certain formula I compounds, in which X represents a O, A represents CO, Y represents a bond, and n represents an integer from 1 to 6, may be prepared by a reaction as shown in **Flow Diagram XIII**:

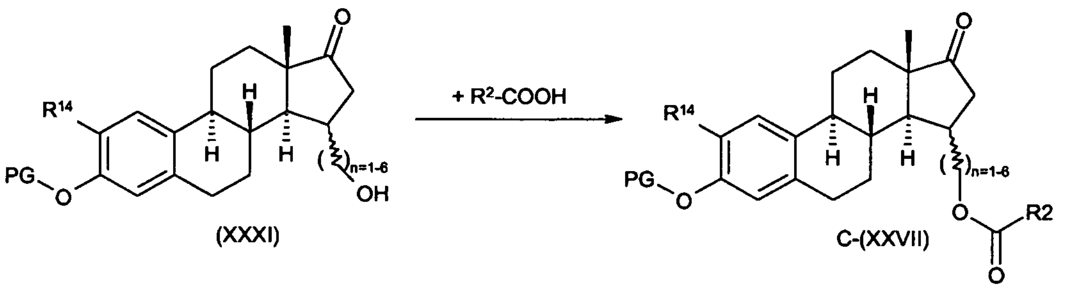


(XXXI) C-(XXVII)

The "retro"-ester derivatives of the general formula C-(XXVII) may be prepared by the esterification of the estrone alcohol building block (XXXI) with the appropriate carboxylic acid $R^2$-COOH and subsequent purification.

Certain formula I compounds, in which X represents a O, A represents CO, Y represents NH-$SO_2$-$NR^4$, and n represents an integer from 1 to 6, may be prepared by a reaction as shown in **Flow Diagram XIV**:

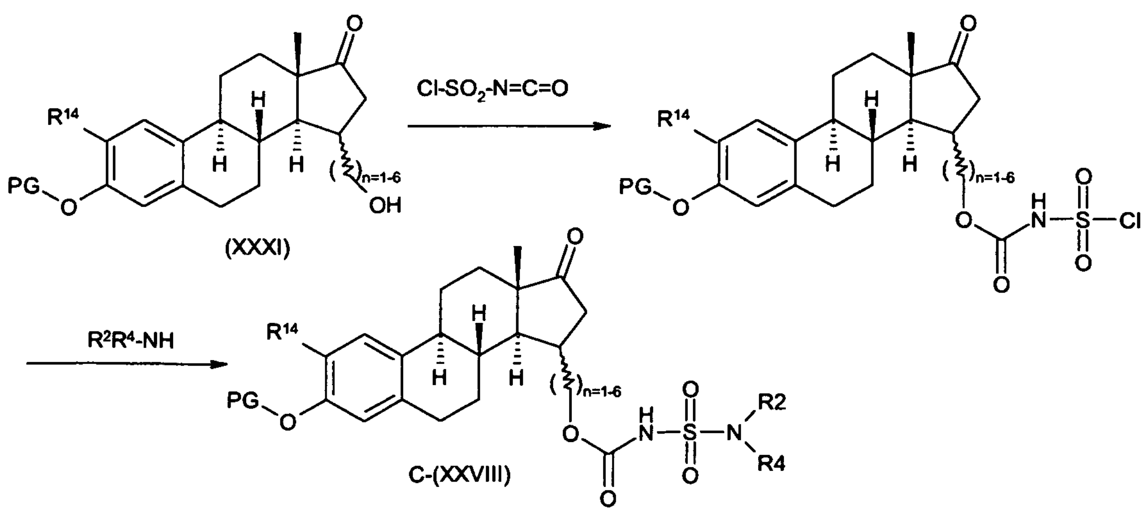


(XXXI) C-(XXVIII)

The sulfonylcarbamate derivatives of the general formula C-(XXVIII) may be prepared by a two-step synthesis: In a first step, the estrone alcohol building block (XXXI) is converted to the chlorosulfonylcarbamate intermediate by reaction with chlorosulfonyl isocyanate. Subsequently, the intermediate is allowed to react with the appropriate primary or secondary amine $HNR^2R^4$ in in order to give the desired sulfonylcarbamate derivative.

Certain formula I compounds, in which X-A-Y represents O, and R2 is different from H may be prepared by a reaction as shown in **Flow Diagram XV:**

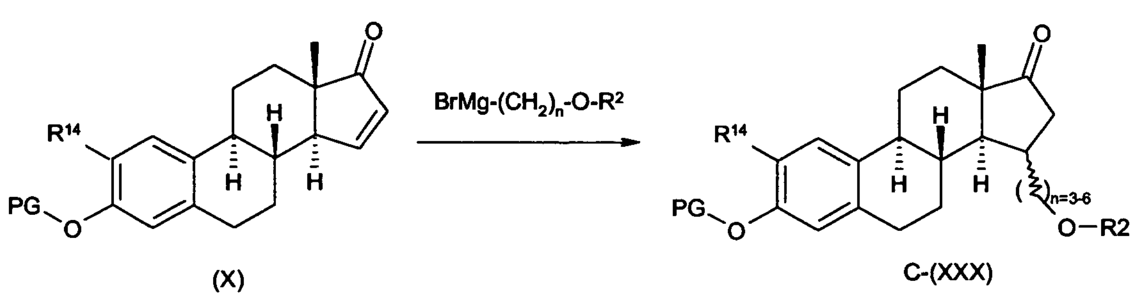


(X) C-(XXX)

The ether derivatives of the general formula C-(XXX) may be prepared the reaction of an appropriate Grignard reagent BrMg-$(CH_2)_n$-O-$R^2$ (for n = 3-6) with the 15,16-unsaturated estrone derivative of formula X. Alternatively, ether derivatives may be prepared by derivatisation of the corresponding alcohol of the general formula (XXXI).

The synthesis of certain formula I compounds, in which X-A-Y represents O, R2 represents H, and n represents an integer from 1 to 6, according to general formula C-(XXXI) is decribed within the section "Intermediates".

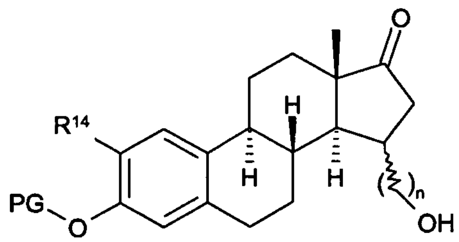

C-(XXXI)

**Step D - Modification of the C17-keto function or introduction of a heterocyclic ring system in C16-C17**

Since the C15-side chain as well as the C2 side chain were already introduced, it is clear for the skilled artisan, that, where necessary, functional groups in the alcohol D(I)-OH may be protected in known manner and the protecting group or groups removed at the end of the reaction.

**Step D - 1**, for compounds when the substituents $R^{10}$, $R^{11}$, $R^{12}$ and $R^{13}$ together with the carbon atoms, to which they are attached, form a structure -$CR^{13}R^{12}$-$CR^{11}R^{10}$-, which is selected from the group of

(a)

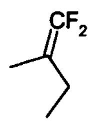

forming a compound of general formula D-(I)-(=$CF_2$)

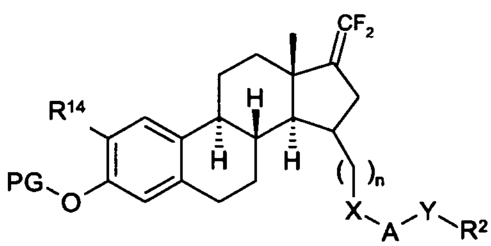

D-(I)-(=$CF_2$)

,

(b)

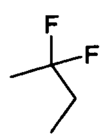

,

forming a compound of general formula D-(I)-$F_2$

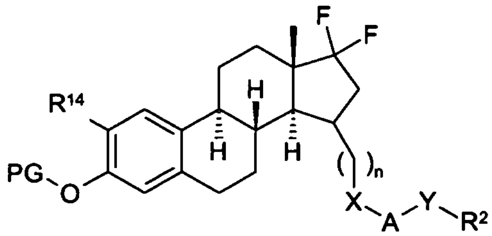

D-(I)-F$_2$

,

(c)

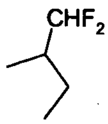

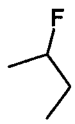

and

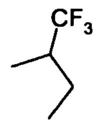

,

forming a compound of general formula D-(I)-(c)

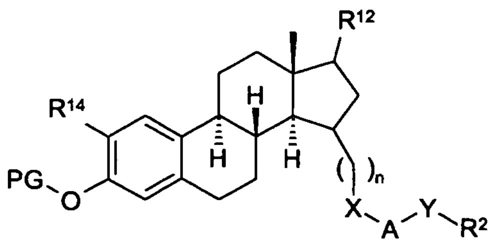

D-(I)-(c)

wherein $R^{12}$ represents -F, $-CF_3$, or $-CF_2H$; and

(d)

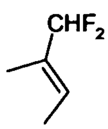

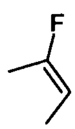

and

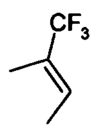

,

forming a compound of general formula D-(I)-(d)

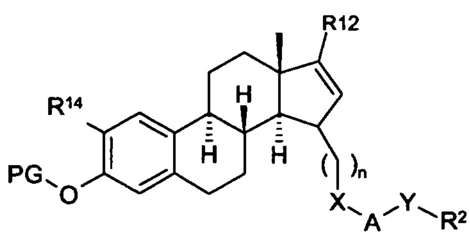

D-(I)-(d)

,

or

wherein $R^{12}$ represents -F, $-CF_3$, or $-CF_2H$.

For synthesis of some D-(I) compounds and in order to enable library synthesis, it might be necessary that some of the reaction steps explained under "STEPS C - the introduction of the C15 side chain" have to be carried out

after having introduced the respective fluoro group. A typical scenario might be that after optional introduction of the $R^{14}$ residue in C2 position, the 15,16-unsaturated intermediate (X) is prepared. This is further derivatized to the appropriate acid, alcohol, amid or alkenyl intermediate ("building block" - see section "Intermediates"). Then, the fluoro group is introduced into C17 position of the steroidal core using a synthesis scheme as described in more detail below. The so-obtained intermediate is then used for optional further modification of the C15 side chain and introduction of the R2/R4 substituents. Finally any protection groups in C3 position might be cleaved off.

D-(I)-(a): Synthesis of compounds, wherein $R^{10}$ and $R^{11}$ both represent -H and $R^{12}$ and $R^{13}$ together represent $=CF_2$

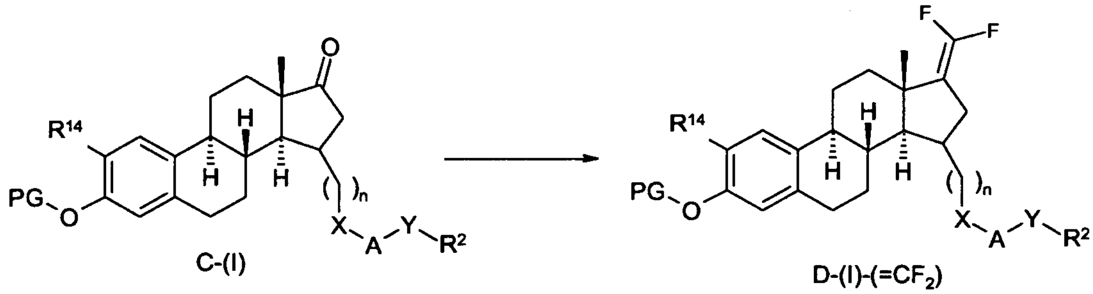

C-(I) D-(I)-(=$CF_2$)

The introduction of a $=CF_2$ group in C17 position of the estron core is a reaction well known in the art, see e.g. Edwards et al (1990) using $F_2CP(O(Ph)_2$ as fluorinating reagent, or by using the Homer reaction with $F_2CP(O)(OEt)_2$ as fluorinating reagent Schwarz et al (2001). In addition, the reaction can be carried out according to procedures described within international patent application WO 96/28462. Subsequent deprotection of the C3-hydroxy function may be obtained using standard techniques.

D-(I)-(b): Synthesis of compounds, wherein $R^{10}$ and $R^{11}$ both represent -H and $R^{12}$ and $R^{13}$ both individually represent -F

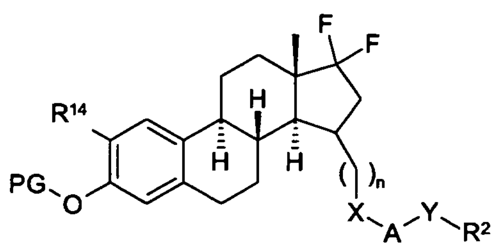

D-(I)-$F_2$

**Reaction Scheme:**

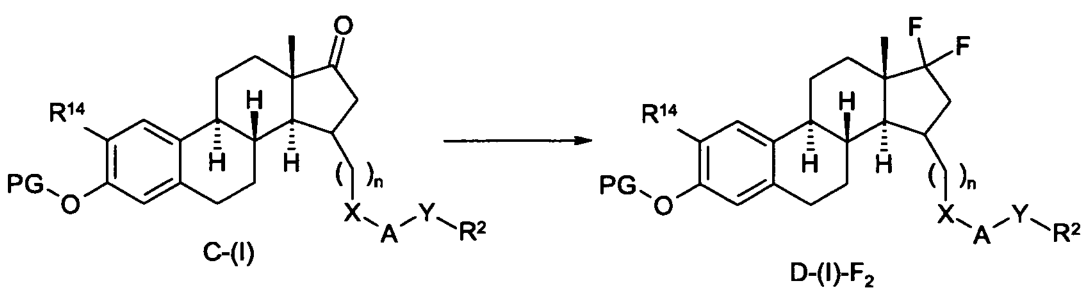

C-(I) D-(I)-$F_2$

The difluorination of the C17 atom of the estron core is a reaction well known in the art and was already disclosed in US patents US 3,413,321 and US 3,347,878. Furthermore, the difluorination of the C17 atom of the estron core may be achieved using the DAST (N,N-diethylaminosulfur trifluoride) reagent [Liu et al (1992)].

D-(I)-(c): Synthesis of compounds, wherein $R^{10}$, $R^{11}$ and $R^{13}$ all represent -H and $R^{12}$ is selected from **-F,** $-CF_3$, and $-CF_2H$

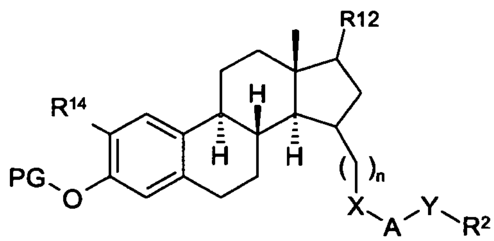

D-(I)-(c)

1. $R^{12}$ represents -F

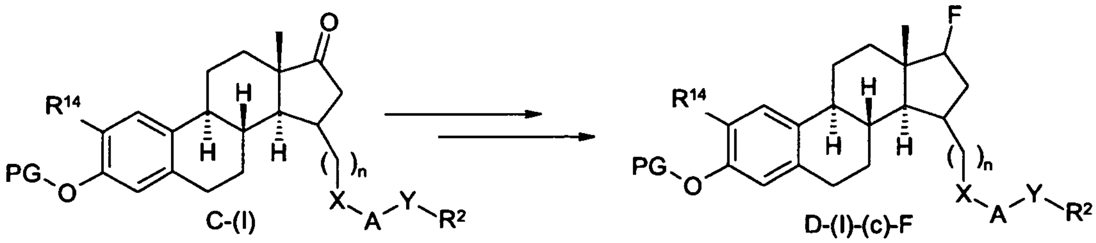

The mono-fluorination of the C17 atom of the estron core is a reaction well known in the art and may be performed according to the disclosure of US patent US 3,275,623.

2. $R^{12}$ represents $-CF_2H$

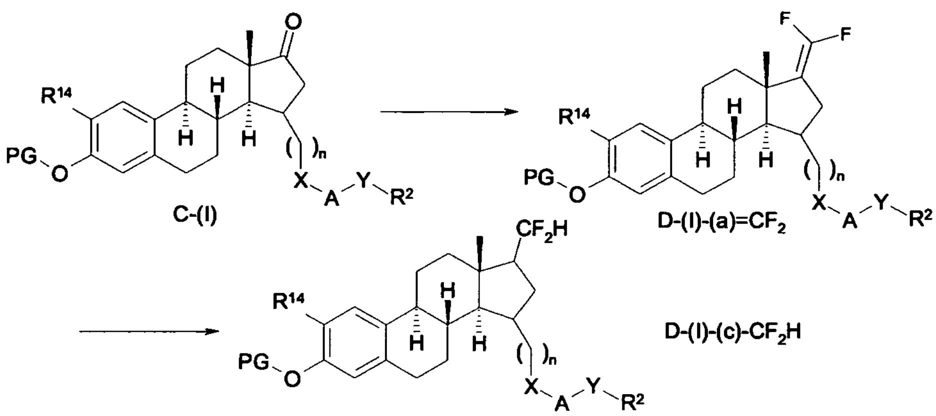

The desired compound of the general formula D-(I)-(c)-$CF_2H$ may be obtained by hydrogenation of the corresponding 17-difluoromethylene substituted derivative, the synthesis of which is described above. If desired, the protection group is subsequently removed.

3. $R^{12}$ represents $-CF_3$

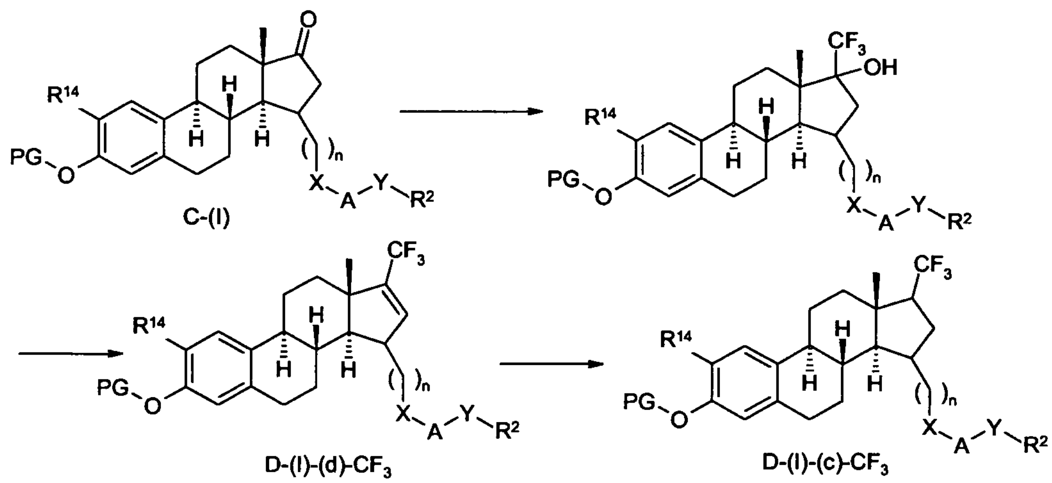

C-(I)

D-(I)-(d)-$CF_3$ D-(I)-(c)-$CF_3$

The introduction of the $-CF_3$ group in C17 position of the estron core may be performed according to Wang & Ruan (1994). Then, the double bond in C16, C17 position is introduced by acidic elimination to deliver a compound of general formula D-(I)-(d)-$CF_3$. The unsaturated derivative may be converted into the corresponding saturated compound by hydrogenation. If desired, the protection group is subsequently removed to deliberate the 3-hydroxy function.

D-(I)-(d): Synthesis of compounds, wherein $R^{10}$ represents -H, $R^{11}$ together with $R^{13}$ forms a bond, and $R^{12}$ is selected from -F, $-CF_3$. and $-CF_2H$

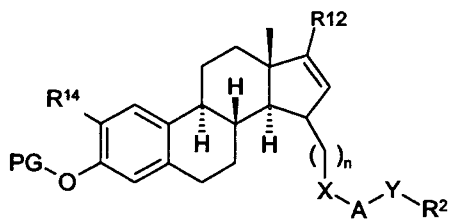

D-(I)-(d)

1. $R^{12}$ represents -F

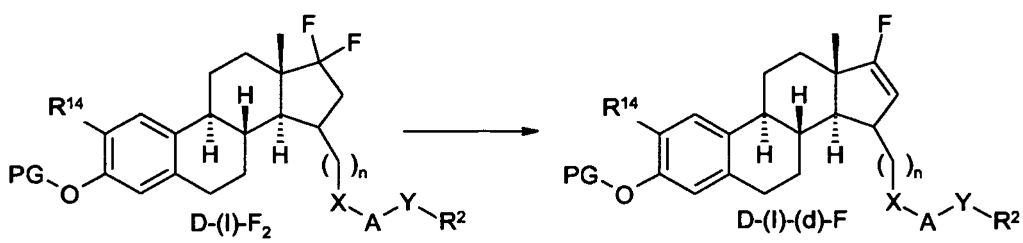

D-(I)-$F_2$ D-(I)-(d)-F

The 17-monofluorinated, 16,17-unsaturated estron derivative may be obtained starting from the corresponding 17-difluorinated compound, the synthesis of which was explained above, according to the procedure described by Liu et al. (1992). If desired, the protection group can be subsequently removed.

2. $R^{12}$ represents $-CF_2H$

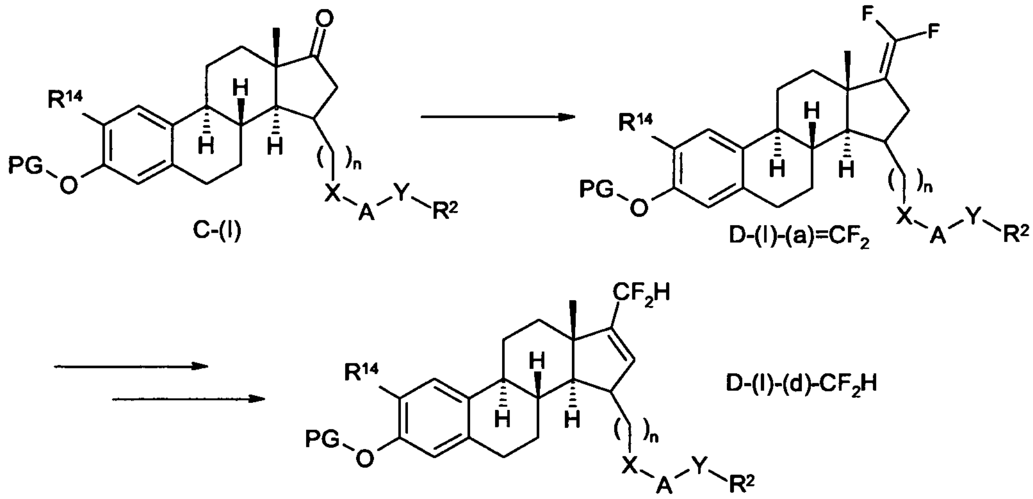

C-(I)

D-(I)-(a)=$CF_2$

D-(I)-(d)-$CF_2H$

The desired compound of the general formula D-(I)-(d)-$CF_2H$ may be obtained by Pd-catalyzed isomerization of the double bond of the corresponding 17-difluoromethylene substituted derivative, the synthesis of which has been described above. If desired, the protection group can be subsequently removed.

3. $\underline{R^{12}}$ <u>represents -$CF_3$</u>

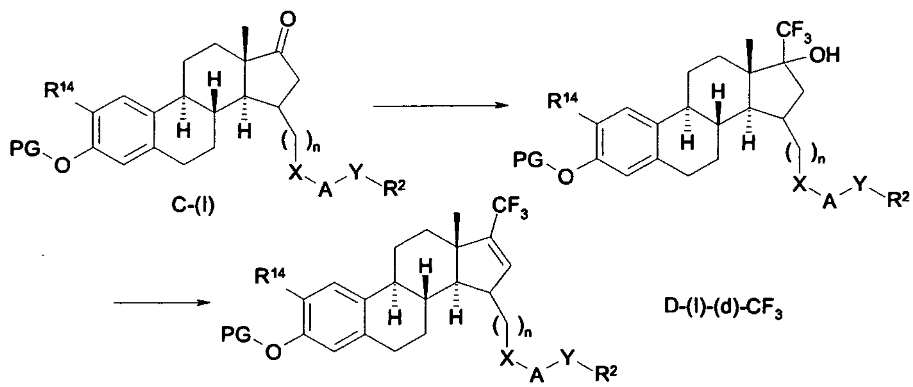

C-(I)

D-(I)-(d)-$CF_3$

The introduction of the -$CF_3$ group in C17 position of the estron core may be performed according to Wang & Ruan (1994). Then, the double bond in C16, C17 position is introduced by acidic elimination to deliver the 16, 17 unsaturated estron derivative of general formula D-(I)-(d)-$CF_3$. If desired, the protection group is subsequently removed to deliberate the 3-hydroxy function.

**Step D - 2,** for compounds when the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring, which is partly unsaturated or aromatic, which contains one, two or three heteroatoms independently selected from N, O and S, the number of N atoms being 0, 1, 2 or 3 and the number of O and S atoms each being 0, 1 or 2, wherein one heteroatom is directly attached to the C17 C-atom of the steroidal core; and which ring is optionally substituted with an alkyl group.

The synthesis of estron derivatives carrying an additional heterocyclic ring in C16-C17 position of the steroidal core has already been disclosed within international patent application WO 2004/085457; the synthesis schemes depicted there can also be applied to the intermediates of the present invention in order to receive the compounds of the present invention. Some reactions are exemplified in more detail below.

Preferably, the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring to provide a compound of one of the following formulas

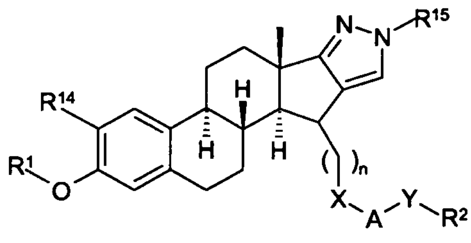,

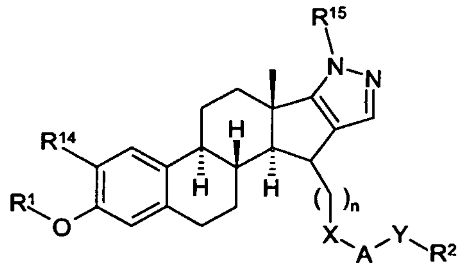,

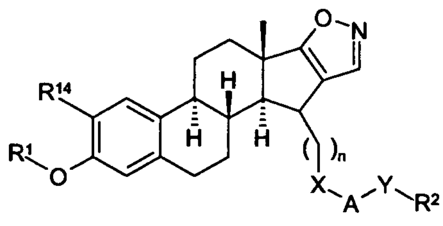,

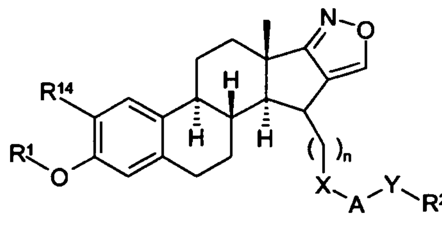,

or

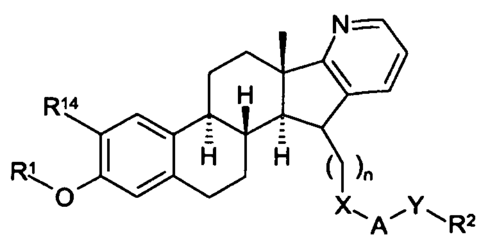

wherein $R^{15}$ represents -H or $-(C_1-C_4)$alkyl.

For synthesis of D-(II) compounds it might be necessary that some of the reaction steps explained under "STEPS C - the introduction of the C15 side chain" have to be carried out after having introduced the heterocyclic ring system. A typical scenario might be, that after optional introduction of the $R^{14}$ residue in C2 position, the 15,16-unsaturated intermediate (X) is prepared. This is further derivatized to the appropriate acid, alcohol, amid or alkenyl intermediate ("building block"). Then, the heterocyclic ring system is introduced including the C16-C17 carbon atoms attached to the D-ring using a synthesis scheme according to WO 2004/085457 or as described below. The so-obtained intermediate is then used for further modification of the C15 side chain and introduction of the R2/R4 substituents. Finally any protection groups in C3 position might be cleaved off.

<u>D-(II)-(a) and D-(II)-(b): Synthesis of compounds of formula D-(II)-(a) and D-(II)-(b)</u>

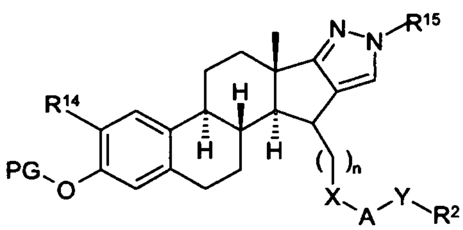

and

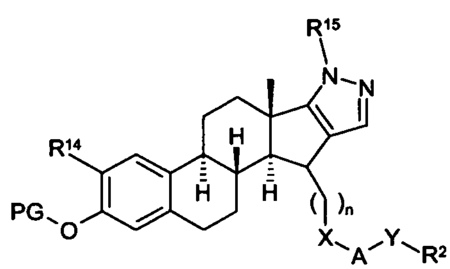

The pyrazole-unit is known in steroid-chemistry and is constructed in 3 steps as depicted in the following scheme for D-(II)-(a):

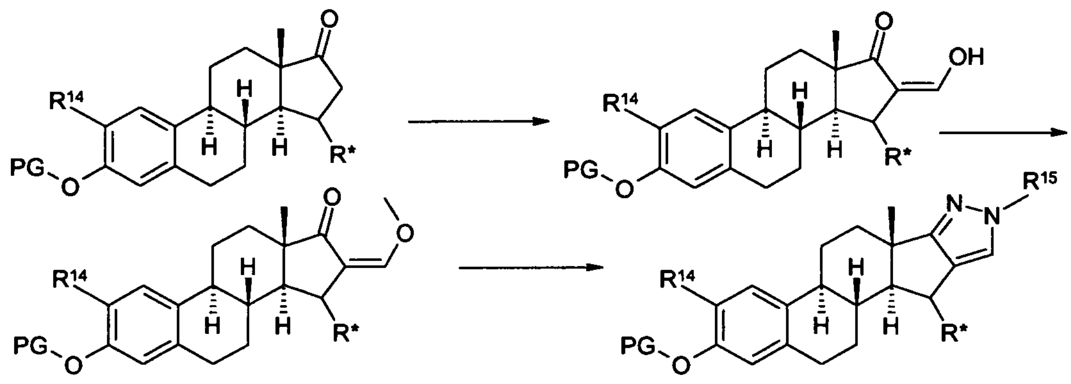

The R* residue may represent the completely introduced C15 side chain -(CH2)$_n$-X-A-Y-$R_2$, or may represent an intermediate side chain such as $-CH_2-CH=CH_2$, or $-CH_2-CH_2-CH_2-CH=CH_2$ (see also SCHEMES 7B and 7C for introduction and further modification of this alkenyl side chain).

First a α-hydroxymethylene moiety is introduced with NaOMe (or NaH) and ethylformate [Wölfling et al (2003), Oda et al (1989), Schneider et al (1983)]. After methylation with $K_2CO_3$ and MeI (WO 2004/85457) or MeOH and $CeCl_3$ [Akanni & Marples (1993)], the ring is closed with the appropriate hydrazine or alkylhydrazine, e.g. methylhydrazine [Xenos & Catsoulacos (1985)]. Alternatively, the methylpyrazine is constructed from the methoxymethylene compound with hydrazine, followed by alkylation with MeI.

D-(II)-(c) and D-(II)-(d): Synthesis of compounds of formula D-(II)-(c) and D-(II)-(c)

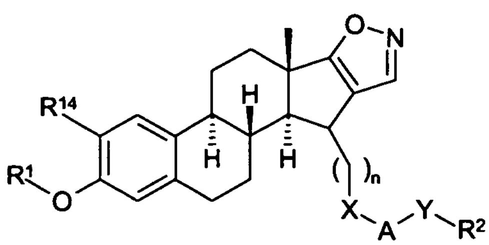

and

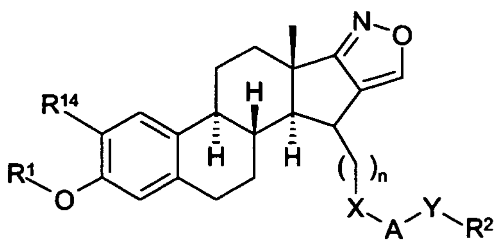

The introduction of the isooxazole group as attached heterocycle to the D-ring of the steroidal core may be achieved according to the synthesis of the corresponding pyrazole derivative and is constructed in 3 steps as depicted in the following scheme for D-(II)-(c/d):

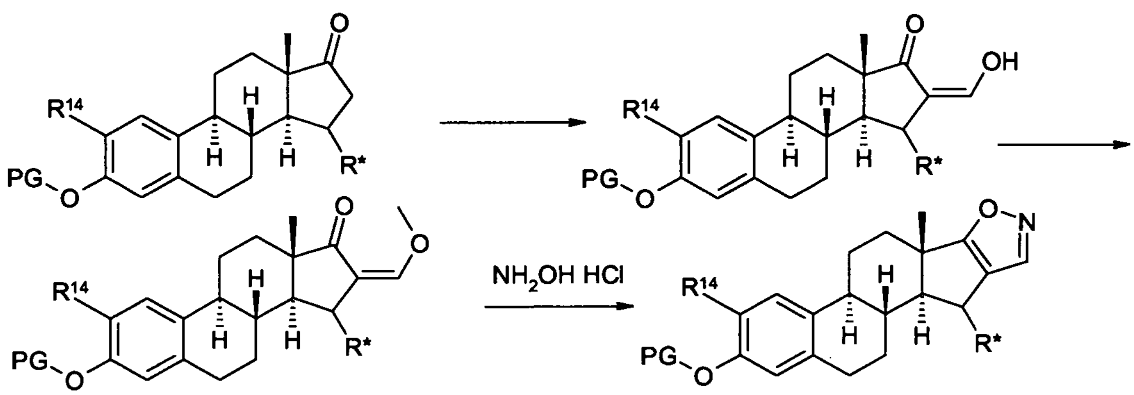


The R* residue may represent the completely introduced C15 side chain $-(CH_2)_n$-X-A-Y-$R_2$, or may represent an intermediate side chain such as $-CH_2-CH=CH_2$, or $-CH_2-CH_2-CH_2-CH=CH_2$ (see also SCHEMES 7B and 7C for introduction and further modification of this alkenyl side chain).

First a $\alpha$-hydroxymethylene moiety is introduced with NaOMe (or NaH) and ethylformate [Wölfling et al (2003), Oda et al (1989), Schneider et al (1983)]. After methylation with $K_2CO_3$ and MeI (WO 2004/85457 A2) or MeOH and $CeCl_3$ [Akanni & Marples (1993)], the ring is closed with the appropriate hydroxylamine.

D-(II)-(e): Synthesis of compounds of formula D-(II)-(e)

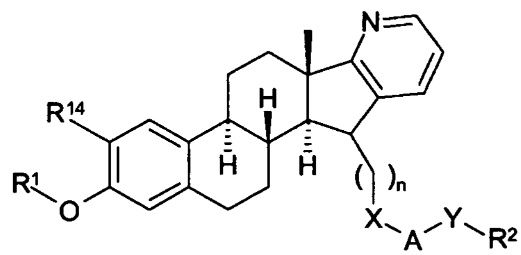

The synthesis of the C15 estrone derivatives with an attached pyridin ring to the D-ring of the steroidal core is fully disclosed in internation patent application WO 2004/085457.

**Step E - Modification of the R1 residue**

In case that R1 represents -H, or optionally substituted -(C1-C6)alkyl, phenyl or -(C1-C6)alkylphenyl, then the substituent may already have been introduced during synthesis of the Intermediates as explained for R1 = H, R1 = methyl and R1 = benzyl. In case of further modification of the 3-OH function to a sulphamate, carbamate, phosphonate, thiophosphonate, sulphonate, phosphate or sulphate group, this may be obtained by one of the following reactions:

SULPHAMATE COMPOUND PREPARATION

The sulphamate compounds of the present invention may be prepared by reacting the correspondingly substituted estron derivative of the general formula D-(I) with a free 3-OH group with a suitable sulfamoyl chloride of the general formula $R^3R^{3'}NSO_2Cl$.

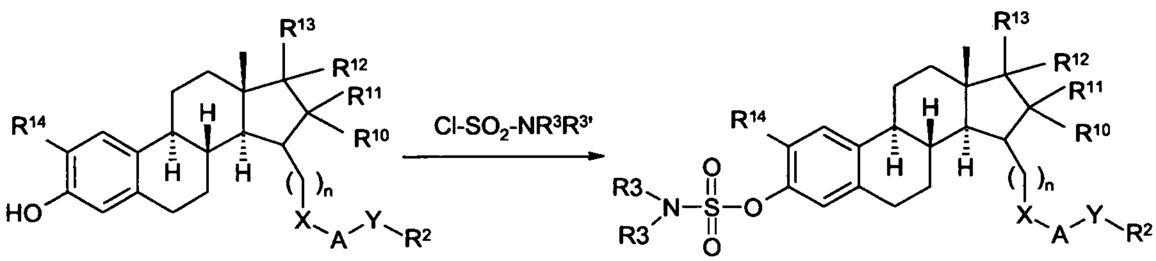


D-(I)-OH

(I)-SO2-NR3R3'

Typical conditions for carrying out the reaction are as follows: Sodium hydride and a sulfamoyl chloride are added to a stirred solution of the alcohol D(I)-OH in anhydrous dimethyl formamide at 0˚C. Subsequently, the reaction is allowed to warm to RT whereupon stirring is continued for a further 24 h. The reaction mixture is poured onto a cold saturated solution of sodium bicarbonate and the resulting aqueous phase is extracted with DCMe. The combined organic extracts are dried over anhydrous $MgS0_4$. Filtration followed by solvent evaporation in vacuo and co-evaporated with toluene affords a crude residue which is further purified by flash chromatography.

Alternatively, sulfamoyl chloride (1 mmol) was added to a stirred solution of the alcohol D(I)-OH (0.5 mmol) in anhydrous N,N-dimethylacetamide (0.75 ml) at 0˚C. The mixture was stirred at RT for 3 h and then poured into cold brine (10 ml). The resulting mixture was extracted with EtOAc (3x10 ml), the combined organic layers were washed with brine (10 ml), dried ($MgSO_4$), and concentrated under reduced pressure. The product was purified by flash chromatography on silica gel.

Where necessary, functional groups in the alcohol D(I)-OH may be protected in known manner and the protecting group or groups removed at the end of the reaction. Preferably, the sulphate compounds are prepared according to the teachings of Page et a/ (1990).

CARBAMATE COMPOUND PREPARATION

The carbamate compounds of the present invention may be prepared by derivatisation of the correspondingly substituted estron derivative of the general formula D-(I) with a free 3-OH group.

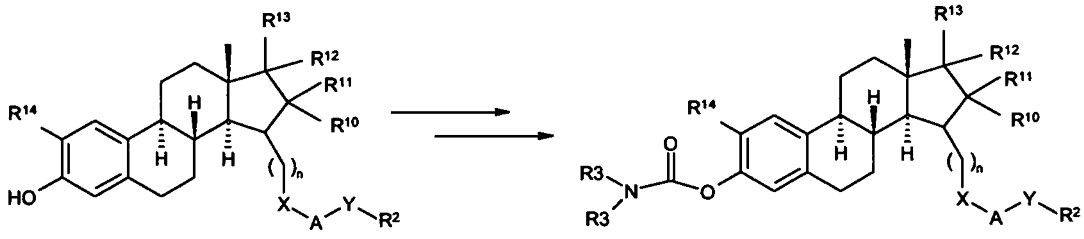

D-(I)-OH (I)-CO-NR$^3$R$^{3'}$

Typical conditions for carrying out the reaction are as follows: 1 eq Estrone derivative D-(I)-OH, 3 eq N-methylmorpholine and 1/3 eq Triphosgen were dissolved in DCM and stirred for 30 min at 0°C. Then, 1 eq of the desired amine was added and the reaction mixture stirred for 12 h at RT. Thereafter the reaction mixture was quenched by adding 1M $NaHCO_3$. The organic layer was separated and extracted with 1 M $KHSO_4$ and 1 M NaCl. After drying over $Na_2SO_4$ the solution was evaporated to dryness and purified by column chromatography. Where necessary, functional groups in the alcohol D(I)-OH may be protected in known manner and the protecting group or groups removed at the end of the reaction.

SULPHONATE COMPOUND PREPARATION

The sulphonate compounds of the present invention may be prepared starting from the correspondingly substituted estron derivative and by suitably combining the teachings of Page et a/ (1990) and published international patent application WO 93/05063.

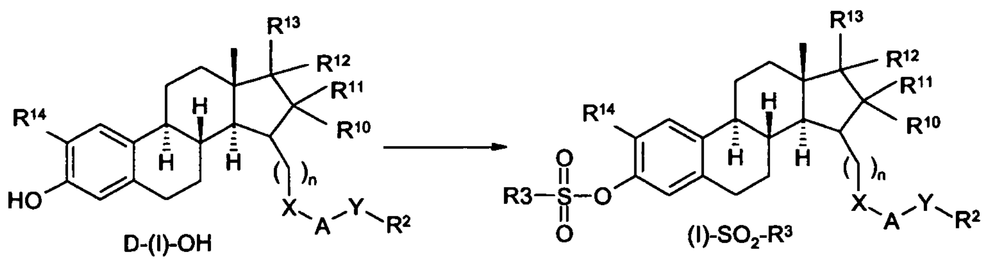

D-(I)-OH (I)-$SO_2$-R$^3$

PHOSPHONATE COMPOUND PREPARATION

The phosphonate compounds of the present invention may be prepared starting from the correspondingly substituted estron derivative and by suitably combining the teachings of Page et a/ (1990) and published international patent application WO 93/05063.

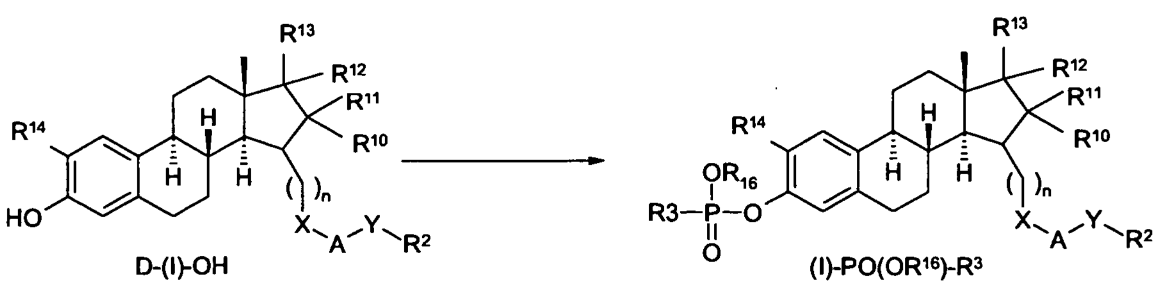

D-(I)-OH (I)-PO(OR$^{16}$)-R$^3$

THIOPHOSPHONATE COMPOUND PREPARATION

The thiophosphonate compounds of the present invention may be prepared starting from the correspondingly substituted estron derivative and by suitably combining the teachings of Page et a/ (1990) and published international

patent application WO 93/05063.

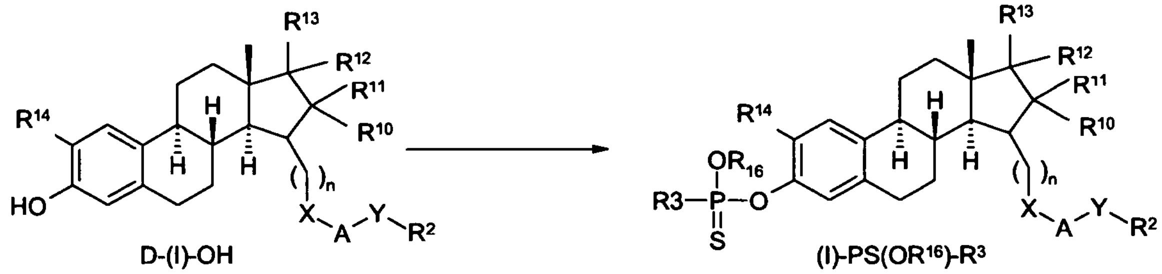

D-(I)-OH

(I)-PS($OR^{16}$)-$R^3$

SULPHATE COMPOUND PREPARATION

The sulphate compounds of the present invention may be prepared starting from the correspondingly substituted estron derivative using known sulfating reagents, such as complexes of sulfur trioxide with Lewis bases such as trialkylamines (e.g. $SO_3$*$Et_3N$), DMF, or pyridine.

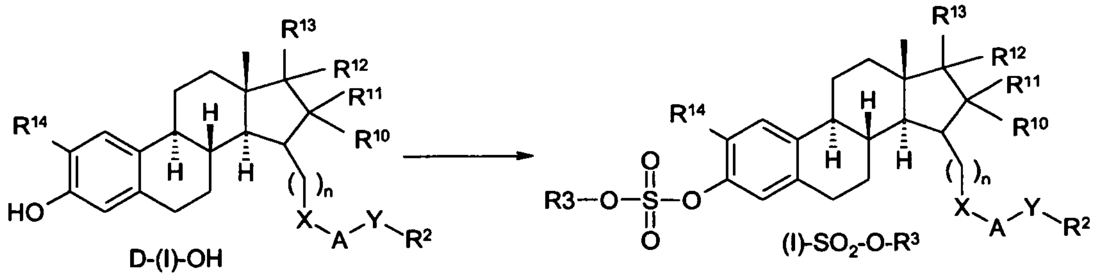

D-(I)-OH

(I)-$SO_2$-O-$R^3$

PHOSPHATE COMPOUND PREPARATION

The phosphate compounds of the present invention may be prepared starting from the correspondingly substituted estron derivative by phosphorylation using e.g. phosphoramidite chemistry or treatment with pyrophosphoric tetrachloride.

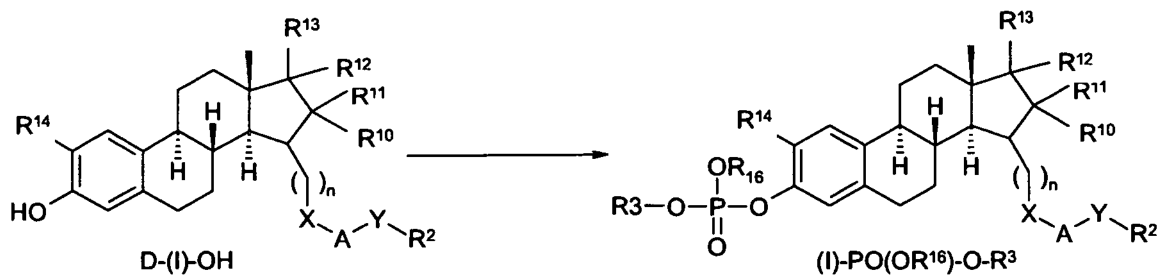

D-(I)-OH

(I)-PO($OR^{16}$)-O-$R^3$

## Experimental

Examples of preparations of compounds of the invention are provided in the following detailed synthetic procedures. In the tables of compounds to follow, the synthesis of each compound is referenced back to these exemplary preparative steps.

In single compound synthesis as well as in combinatorial synthesis all reactions were stirred magnetically or shaked with an orbital shaker unless otherwise indicated. Sensitive liquids and solutions were transferred via syringe or cannula, and introduced into reaction vessels through rubber septa, in these cases the reaction were carried out under a positive pressure of dry argon or dry nitrogen. Commercial grade reagents and solvents were used without further purification.

Unless otherwise stated, the term "concentration under reduced pressure" refers to use of a Buchi or Heidolph rotary evaporator ("Rotavapor") or vaccum centrifuges ("GeneVac" or "Christ alpha RVC") at approximately 15 mm of Hg. All temperatures are reported uncorrected in degrees Celsius (˚C). Unless otherwise indicated, all parts and percentages are by volume.

Thin-layer chromatography (TLC) was performed on Merck® pre-coated glass-backed silica gel or aluminium sheets 60A F-254 250 μm plates. Visualization of plates was effected by one or more of the following techniques: (a) ultraviolet illumination (254 nm or 266 nm), (b) exposure to iodine vapor, (c) spraying of the plate with Schlittler's reagent solution followed by heating, (d) spraying of the plate with anisaldehyde solution followed by heating, and/or (e) spraying of the plate with Rauxz reagent solution followed by heating. Column chromatography (flash chromatography) was performed using 230-630 mesh ICN, SiliTech 60A silica gel.

Melting points (mp) were determined using a Reichert Thermovar melting point apparatus or a Mettler DSC822 automated melting point apparatus and are uncorrected.

Fourier transform infrared spectra were obtained using a Perkin Elmer spectrophotometer.

Proton ($^1H$) nuclear magnetic resonance (NMR) spectra were measured with a Bruker ARX (400 MHz) or Bruker ADVANCE (500 MHz) spectrometer with either $Me_4Si$ (δ 0.00) or residual protonated solvent ($CHCl_3$ δ 7.26; $CHD_2OD$ δ 3.30; DMSO-$d_5$ δ 2.50) as standard. Carbon ($^{13}C$) NMR spectra were measured with a Bruker ARX (100 MHz) or Bruker ADVANCE (126 MHz) spectrometer with either $Me_4Si$ (δ 0.00) or solvent ($CDCl_3$ 8 77.05; $CD_3OD$ δ 49.0; DMSO-$d_6$ δ 39.45) as standard.

HPLC electrospray mass spectra (HPLC ES-MS) were obtained using the following method and equipment: Samples were separated by reversed phase high pressure liquid chromatography (RP-HPLC) coupled to a quadrupol MS. HPLC was performed at a flow of 1000 μl/min using Xter-raMS C18 columns (i.d. 4.6 mm, length 50 mm, particle size 2.5 μm) or Phenomenex Luna C18(2) 30*4.6mm columns. For most samples, a gradient from 0% eluent B to 95% B was run in 10 min, with eluent A consisting of water, 10 mM ammonium-acetate at pH 5 + 5% acetonitrile and eluent B consisting of acetonitrile. Two different setups were used: 1. Waters Alliance 2795 coupled to a Waters ZQ MS, a Waters 2996 diode array detector (DAD) and an evaporative light scattering detector (ELSD, EL-ELS1000, PolymerLabs). Ionization: electrospray positive and negative mode ES +/-; or 2. LC200 pump (PE) coupled to an API100 MS (Applied Biosystems Sciex), a variable wavelength detector Waters 2487 set to 225 nm, and an ELSD (Sedex 75), ES+. In both setup versions spectra were scanned with a scan range of m/z 100 to 800 or 100 to 900.

Gas chromatography - mass spectra (GC-MS) analyses were performed with an Agilent 6890 gas chromatograph equipped with an DB-5MS column (0.25 i.d., length 30 m) and an Agilent 5973 MSD quadrupol detector (ionization with electron impact (EI) at 70eV; source temperature 230˚C).

Elemental analyses were conducted by a VarioEL elemental analyzer (Elementar Analysen-systeme) for determination of C, H, and N. Acetanilide was used for conditioning and calibration.

NMR spectra, LRMS, elemental analyses and HRMS of the compounds were consistent with the assigned structures.

I**ntermediates**

I. Estron derivatives substituted in C2 position of the steroidal core of formula (V) (Step A)

**3-Benryloxy-estra-1,3,5(10)-trien-2,17β-diol (V- C2-A)**

3-Benzyloxy-estra-1,3,5(10)-trien-2,17-diol was prepared starting from estradiol by introduction of the hydroxy side chain in C2 position as described by Rao et al. (2002) in which a Fries rearrangement and a Baeyer Villiger reaction is used.

Detailed Synthesis

Estra-1,3,5(10)-triene-3,17β-diol diacetate (C2-2):

Under an $N_2$-atmosphere, $Ac_2O$ (375 ml, 3.993 mol) was added dropwise during 20 min to a solution of estradiol (150 g, 0.551 mol) in pyridine (1500 ml). The clear colourless solution obtained was stirred at RT overnight. The reaction mixture was then cooled to 0˚C and MeOH (375 ml) was added dropwise during 25 min. The reaction mixture was stirred at 0˚C for 2 h, then allowed to warm to RT and concentrated *in vacuo.* The residue was recrystallized from hot MeOH to yield (C2-2) (176 g, 90%) as white crystals.

2-Acetyl-estra-1,3,5(10)-triene-3,17β-diol 17-acetate (C2-3):

Under an $N_2$-atmosphere, $ZrCl_4$ (530 g, 2.27 mol) was added in one portion to a solution of (C2-2) (176 g, 0.493 mol) in DCM (13 l). The turbid yellow mixture obtained was stirred at RT for 48h. The reaction mixture was cooled to 0˚C, ice water (3 l) was added and the mixture was allowed to warm to RT overnight. The mixture was washed with $H_2O$, sat $NaHCO_3$ (aq), brine, dried over $Na_2SO_4$, filtered, and concentrated *in vacuo* to yield (C2-3) (167 g, 95%) as a

yellow powder.

2-Acetyl-3-benzyloxy-estra-1,3,5(10)-triene-170-ol 17-acetate (C2-4):

Under an $N_2$-atmosphere, $K_2CO_3$ (97 g, 0.702 mol) was added in one portion to a solution of (C2-3) (167 g, 0.468 mol), benzyl bromide (61.6 ml, 0.515 mol) and 18-crown-6 (4.7 g, 0.018 mol) in acetone (1 l). After 108 h at $T_{intem}$=56 °C, the reaction mixture was allowed to cool to RT, poured into $H_2O$, stirred for 1 h after which the turbid mixture was filtered over a glass fritted filter. The residue was washed with $H_2O$, and dried *in vacuo* to yield (C2-4) (209 g, 100%) as a brown solid.

3-Benzyloxy-estra-1,3,5(10)-triene-2,17β-diol diacetate (C2-5):

Under $N_2$ atmosphere, $NaH_2PO_4$ (2.496 mol) was added to a solution of C2-4 (167 g, 0.468 mol) in DCM (7 l). Then *m*-CPBA (75% with $H_2O$, 0.889 mol) was added portionwise during 10 min. The turbid mixture obtained was stirred at RT for overnight. The reaction mixture was poured into $H_2O$, and the mixture obtained was stirred for 1 h. The organic layer was isolated and the aqueous layer was extracted with DCM. The combined organic layers were washed with $H_2O$, 10% $Na_2SO_3$ (aq), half-sat. $NaHCO_3$ (aq), brine, dried over $Na_2SO_4$, filtered, and concentrated *in vacuo* to yield C2-5 (247 g, quant.) as a clear yellow powder.

3-Benzyloxy-estra-1,3,5(10)triene-2,17β-diol (V-C2-A):

A solution of KOH (4.46 mol) in $H_2O$ (5 l) was added to a solution of (C2-5) (511 g, 1.181 mol) in THF (5 l) and MeOH (5 l). The mixture obtained was stirred at $T_{intem}$=65 °C overnight, and then allowed to cool to RT. The reaction mixture was acidified with conc. HOAc to pH 4 and diluted with $H_2O$and EtOAc (1:3). The organic layer was isolated and the aqueous layer was extracted with EtOAc. The combined organic layers were washed with brine, dried over $Na_2SO_4$, filtered, and concentrated *in vacuo* to yield 358 g brown solid. The solid was triturated with TBME (2 L) for 2 h, filtered over a glass fritted filter (P2) and the residue was washed with TBME, then with acetone, and dried *in vacuo* to yield (C2-A) (256 g) as an off-white solid. The combined filtrates were concentrated *in vacuo* to yield 125 g brown resin. The resin was dissolved in DCM, applied to $SiO_2$ and eluted with DCM:$NH_3$ 7N in MeOH = 97.5 : 2.5 to yield 76 g yellow solid ($R_f$ = 0.39). The solid was triturated with TBME (250 ml), filtered over a glass fritted filter (P4). The residue was washed with DCM and dried *in vacuo* to yield (V-C2-A) (15.4 g) as an off- white solid. Total yield: 61 %.

**3-Benzyloxy-2-methoxy-estra-1,3,5(10)-triene-17one (V-C2-B):**

3-Benzyloxy-2-methoxy-estra-1,3,5(10)-triene-17 onewas prepared starting from (V-C2-A) according to the procedure described by Rao et al. (2002) and within US 6,043,236.

Detailed Synthesis

3-Benzyloxy-2-methoxy-estra-1,3,5(10)-triene-17β-ol (C2-6):

Under an $N_2$-atmosphere, $LiOH{\cdot}H_2O$ (16.2 g, 0.386 mol) was added to a solution of (V-C2-A) (118 g, 0.312 mol mol) in THF (1.5 l). After adding $Me_2SO_4$ (0.350 mol) the mixture obtained was stirred at 55°C for 3h. The mixture was allowed to cool to RT overnight, concentrated *in vacuo,* and the residue was dissolved in DCM. The organic layer was washed with $H_2O$, brine, dried over $Na_2SO_4$, filtered, and concentrated *in vacuo* to yield (C2-6) (115 g, 94%) as orange resin.

3-Benzyloxy-2-methoxy-estra-1,3,5(10)-triene-17one (V-C2-B):

To a mixture of (C2-6) (118 g, 0.301 mol) and TPAP (0.014 mol) in acetone (2 l), NMO (0.448 mol) was added portion-wise. The mixture obtained was stirred at RT overnight, and optionally filtered over Celite. The filtrate / reaction mixture was concentrated *in vacuo* to yield 128 g black solid. The solid was purified by column chromatography ($SiO_2$ with DCM) to yield (V-C2-B) (97 g, 83%) as a pale yellow solid ($R_f$ = 0.78).

**3-Benzyloxy-2-ethyl-estra-1,3,5(10)-triene-17one (V-C2-C):**

3-Benzyloxy-2-ethyl-estra-1,3,5(10)-triene-17one was prepared starting from (C2-4) by performing a Wolff-Kishner reduction to obtain the ethyl side chain. The oxidation of the C17 hydroxyl function was achieved by TPAP

oxidation using the procedures of Ley et al (1994). Alternatively, 3-Benzyloxy-2-ethyl-estra-1,3,5(10)triene-17-one was prepared starting from (C2-3) by reduction of the acyl function which was achieved by reaction with Pd/C and $H_2$ [Gonzalez et al (1982)], subsequent benzylation of the 3-hydroxy function, deprotection of the C17 hydroxy function and TPAP oxidation.

Detailed Synthesis

3-Benzyloxy-2-ethyl-estra-1,3,5(10)-triene-17β-ol (C2-7):

To benzyl protected acyl ketone (C2-4) (765 g, 1.71 mol) was added diethylene glycol (1900 ml), KOH (5.14 mol) and $H_2NNH_2$*$H_2O$. The mixture was heated to 120-140˚C overnight. A Dean-Stark trap was placed and water and $H_2NNH_2$*$H_2O$ were removed by distillation by heating the reaction mixture to 190˚C. After NMR analysis revealed complete conversion, the mixture was cooled to 50˚C and water (3 l) was added. The mixture became very thick and unstirrable. The dissolved part was poured in a mixture of water (15 l) and EtOAc (5 l) and the sticky oil was first dissolved in EtOAc (5 l) and then added to the mixture. The layers were separated and the organic layer was washed with water and concentrated to give (C2-7) (543 g, 81%) as orange / yellow oil which solidified upon standing.

3-Benzyloxy-2-ethyl-estra-1,3,5(10)-triene-17one (V-C2-C).

Alcohol (C2-7) (433 g, 1.39 mol) and powdered 4A molsieves (695 g, 500 mg/ mmol) in DCM (2.7 l) were cooled with an ice bath and TPAP (19.5 g, 55.6 mmol, 4 mol%) was added. NMO (2.09 mol) was added under ice/water cooling. After 3 h the reaction mixture was filtered over $SiO_2$ (DCM) and all fractions before the black fraction (TPAP) were collected. The DCM was concentrated to give ketone (V-C2-C) (465 g, 86%) as a yellow solid.

**3-Benzyloxy-2-ethoxy-estra-1,3,5(10)-triene-17one (V-C2-D)**

**3-Benzyloxy-2-(2-methoxy-ethoxy)-estra-1,3,5(10)-triene-17one (V-C2-E)**

In the first step, the 2-hydroxy function of 3-Benzyloxy-estra-1,3,5(10)-triene-2,17β-diol (C2-A) was alkylated using ethylsulfate and LiOH or methoxyethanol under Mitsunobu conditions. Subsequently, the alcohol was oxidated with TPAP and NMO to the corresponding estron derivative.

Detailed Synthesis

3-Benzyloxy-2-ethoxy-estra-1,3,5(10)-triene-17β-ol (C2-8)

Intermediate (V-C2-A) (15.0 g, 39.68 mmol) was dissolved in THF (250 ml), under $N_2$ atmosphere. LiOH (47.62 mmol) and $Et_2SO_4$ (43.65 mmol) were added. The mixture was heated at 55˚C for 5h, then cooled to RT and stirred for 48h. The mixture was concentrated in *vacuo.* DCM was added and the organic layer was washed with water and brine, dried over $Na_2SO_4$ and concentrated in *vacuo* yielding 21.7 g of a greenish semi-solid. The mixture was dissolved in THF under $N_2$ atmosphere. LiOH (0.8 g) and $Et_2SO_4$ (2.0 ml) were added. The mixture was heated to reflux and refluxed over the weekend. The mixture was concentrated in *vacuo.* DCM was added and the organic layer was washed with water and brine, dried over $Na_2SO_4$ and concentrated in vacuo to afford 16.7 g (41.07 mmol, quant.) of brown oil. Pentane was added and the formed solid was filtered yielding 13.5 g (C2-8) (84%) as a white solid.

3-Benzyloy-2-(2-methoxyethoxy)-estra-1,3,5(10)-triene-17β-ol (C2-9)

Intermediate (V-C2-A) (15.0 g, 39.68 mmol), $PPh_3$ (79.37 mmol) and methoxyethanol (79.37 mmol) were suspended in DCM (500 ml) and cooled in an ice / water bath, under $N_2$ atmosphere. DIAD (79.37 mmol) was added drop wise in 1h at below 5˚C. After addition a clear solution was formed which was warmed to RT overnight. The solution was concentrated in vacuo yielding 58.8 g thick brown oil. Purification *via* column chromatography ($SiO_2$, eluens DCM to 1% MeOH in DCM) yielded 34 g of thick oil. A second purification *via* column chromatography was done ($SiO_2$, eluens 10% EtOAc to 50% EtOAc in heptan). Two fractions were collected, 7.61 g (44%) of pure product and 5.3 g which was purified *via* column chromatography yielding 3.1 g (18%). Both fractions were mixed yielding 10.8 g (C2-9) (62%) as white powder.

3-Benzyloxy-2-ethoxy-estra-1,3,5(10)-triene-17one (V-C2-D)

Compound (V-C2-D) (11.8 g, 29.6 mmol, 89%) was obtained as yellow solid from alcohol (C2-8) (13.5 g, 33.25 mmol) according to the procedure described for (V-C2-B).

3-Benzyloxy-2-(2-methoxy-ethoxy)-estra-1,3,5(10)-triene-17one (V-C2-E)

Compound (V-C2-E) (11.6 g, 26.8 mmol) as white solid was obtained from alcohol (C2-9) (10.8 g, 24.54 mmol) according to the procedure described for (V-C2-B).

**3-Benzyloxy-2-(2-methoxy-ethyl)-estra-1,3,5(10)-triene-17one (V-C2-F)**

Building Block V-C2-F was prepared starting from intermediate 2-Acetyl-3-benzyloxy-estra-1,3,5(10)-triene-17β-ol 17-acetate (C2-4).

2-Acetyl-3-benzyloxy-estra-1,3,5(10)-triene-17β-ol (C2-10)

Compound (C2-4) (119 g, 266 mmol) was dissolved in a mixture of THF (500 ml) and MeOH (500 ml) under $N_2$ atmosphere. A solution of KOH (1.06 mol) in water (1 l) was added. The reaction mixture was stirred at 75˚C for 16h. After cooling to RT the pH of the mixture was adjusted to 4 using HAc. After dilution with water, the aqueous layer was separated. The organic layer was washed with brine, dried over $Na_2SO_4$, concentrated in vacuo, stripped with toluene and again dried *in vacuo* yielding compound (C2-10) (70.0 g, 173.0 mmol, 65 %) as a yellow syrup.

3-Benzyloxy-2-(2-methoxy-1-oxo-ethyl)-estra-1,3,5(10)-triene-17β-ol (C2-11)

Compound C2-10 (70 g, 173 mmol) was suspended in diethyl ether (2 l) and bromine (363 mmol) was added dropwise at 0˚C under $N_2$ atmosphere. The reaction mixture was stirred at ambient temperature for 14h. The solvent was removed *in vacuo* and the residue suspended in MeOH. Sodium methoxide (173 mmol) was added and the reaction mixture stirred at RT for 72h. This was poured into water, acidified with conc. aq. HCl and the water layer extracted with DCM. The organic layer was washed with brine, dried over $Na_2SO_4$ and concentrated in vacuo. Via column chromatography ($SiO_2$, heptane/EtOAc 2/1 to 0/100 stepwise) compound C2-11 (14.3 g, 32.9 mmol, 19 %) was isolated.

3-Hydroxy-2-(2-methoxy-ethyl)-estra-1,3,5(10)-triene-17β-ol (C2-12)

Palladium on charcoal (10%, 15 g) was suspended in water (175 ml) under $N_2$ atmosphere and added to a solution of compound C2-11 (14.3 g, 32.9 mmol) in THF (175 ml) and t-butanol (175 ml). $H_2$ at ambient pressure was applied and the reaction mixture was stirred at ambient temperature for 80h. The reaction mixture was filtered over Celite and the filter cake was washed with ethanol. The filtrate was concentrated *in vacuo* yielding crude compound C2-12 (8.3 g, 25.1 mmol, 77 %). After purification by column chromatography ($SiO_2$, heptane/EtOAc = 3/1 to 1/2 stepwise) pure C2-12 (3.6 g, 10.89 mmol, 33 %) was isolated.

3-Benzyloxy-2-(2-methoxy-ethyl)-estra-1,3,5(10)-triene-17β-ol (C2-13)

Compound C2-12 (3.6 g, 10.89 mmol) was dissolved in acetone (30 ml) under $N_2$ atmosphere. Subsequently, benzylbromide (21.78 mol), anhydrous $K_2CO_3$ (21.78 mmol) and 18-crown-6 (290 mg, 1.09 mmol) were added. The reaction mixture was refluxed for 24h (65˚C external) and allowed to cool to RT. The mixture was poured into water (150 ml) and stirred for 1h. The water layer was separated and extracted with toluene. The combined organic layers were washed with brine, dried over $Na_2SO_4$ and evaporated *in vacuo* leaving compound C2-13 (4.92 g, max. 10.89 mmol, quant.) as a yellowish solid.

3-Benzyloxy-2-(2-methoxy-ethyl)-estra-1,3,5(10)-triene-17-one (V-C2-F)

Compound V-C2-F (3.43 g, 8.19 mmol, 70 %) was obtained as a pale solid from alcohol C2-13 (4.92 g, max. 10.89 mmol) according to the procedure described for (V-C2-B).

**3-Benzyloxy-2-propyl-estra-1,3,5(10)-triene-17one (V-C2-G):**

3-Benzyloxy-2-propyl-estra-1,3,5(10)-triene-17one was prepared starting from estradiol by introduction of the

propionate side chain in C2 position as described by Rao et al. (2002) using a Fries rearrangement. Then the keto function is reduced to obtain the propyl side chain by reaction with Pd/C and $H_2$ [Gonzalez et al (1982)]. The subsequent oxidation of the C17 hydroxyl function was achieved by TPAP oxidation using the procedures of Ley et al. (1994).

Detailed Synthesis

Estra-1,3,5(10)-triene-3,17β-diol di propionic acid ester (C2-14):

Estradiol (200 g, 0.734 mol) was dissolved in pyridine (2 l) under $N_2$ atmosphere. Propionic anhydride (344 g, 2.64 mol) was added. The reaction mixture was stirred at ambient temperature until the reaction was completed. The reaction mixture was cooled on an ice-water bath, quenched with MeOH (250 ml) and stirred at ambient temperature for 1 ½ h. The mixture was concentrated *in vacuo* and the residue was dissolved in toluene. The organic layer was separated, washed with water, 10% aqueous citric acid, sat. aq. $NaHCO_3$ and dried over $Na_2SO_4$. The organic layer was concentrated *in vacuo* and the residue was stripped with toluene, yielding compound C2-14 (272 g, 0.708 mol, 96 %) as a white solid.

Propionic acid 3-hydroxy-2-propionyl-estra-1,3,5(10)-triene-17-yl ester (C2-15):

Compound C2-14 (272 g, 0.708 mol) was dissolved in DCM (10 L) under $N_2$ atmosphere. Zirconium chloride (758 g, 3.25 mol) was added, which resulted in a yellow suspension. The mixture was stirred at ambient temperature until the conversion was completed. The reaction mixture was cooled to 3°C before 200 g ice was added in batches. Water (2 L) was added and the mixture was stirred at 4°C for 1 hr. Then an additional amount of water (5 L) was added. The aqueous layer was separated and extracted with DCM. The combined organic layers were filtered over $Na_2SO_4$ and concentrated *in vacuo.* The residue was stripped with toluene leaving a green residue. The residue was dissolved in DCM and filtered over $SiO_2$ leaving compound C2-15 (255 g, 0.663 mol, 94 %) as an orange solid.

Propionic acid 3-hydroxy-2-propyl-estra-1,3,5(10)-triene-17-yl ester (C2-16):

Pd (10%) on charcoal (120 g) was suspended in water (800 ml) under $N_2$ atmosphere. *t*-Butanol (800 ml) and a solution of compound C2-15 (115 g, 0.299 mol) in THF (800 ml) were added. The mixture was applied to $H_2$ and stirred at ambient temperature until the reaction was completed. The mixture was filtered over Celite (2x) and the filter cake was washed with THF. The filtrate was concentrated *in vacuo* yielding compound C2-16 (107 g, 0.289 mol, 97%) as gray solid.

Propionic acid 3-benzyloxy-2-propyl-estra-1,3,5(10)-triene-17-yl ester (C2-17):

Compound C2-16 (238 g, 0.642 mol) was dissolved in acetone (1.5 l) under $N_2$ atmosphere. Subsequently, benzylbromide (0.7 mol), anhydrous $K_2CO_3$ (0.723 mol) and 18-crown-6 (0.038 mol) were added. The reaction mixture was refluxed overnight. Additional amounts of $K_2CO_3$ (0.181 mol + 0.362 mol) and benzylbromide (0.084 mol) were added. After refluxing the mixture for additional 64 h, the mixture was allowed to cool to 30°C and poured into water. The mixture was extracted with toluene. The organic layers were combined and concentrated *in vacuo.* The residue was stripped with toluene leaving compound C2-17 (335 g, max. 0.642 mol) as a wax like solid.

3-Benzyloxy-2-propyl-estra-1,3,5(10)-triene-17β-ol (C2-18):

Compound C2-17 (69.9 g, max. 134 mmol) was dissolved in a mixture of THF (600 ml) and MeOH (600 ml) under $N_2$ atmosphere. A solution of KOH (34.4 g, 613 mmol) in water (600 ml) was added. The reaction mixture was stirred at 55°C for 3h. MeOH was removed *in* vacuo from the mixture. DCM (400 ml) was added and the pH of the mixture was adjusted to 1 using 3M HCl. The aqueous layer was separated and extracted with DCM (2x 200 ml). The organic layers were combined, washed with sat aq $NaHCO_3$ (200 ml) and dried over $Na_2SO_4$. The organic layer was concentrated in vacuo yielding compound C2-18 (61.6 g, 152 mmol, 88 %) as a yellow syrup after column chromatography ($SiO_2$, DCM/heptane = 85/15).

3-Benzyloxy-2-propyl-estra-1,3,5(10)-triene-17-one (V-C2-G):

Compound V-C2-G (57.6 g, 143 mmol, 94 %) was obtained as a pale solid from alcohol C2-18 (61.6 g, 152 mmol) according to the procedure described for (V-C2-B).

3-Hydroxy-2-propyl-estra-1,3,5(10)-triene-17-one (V-C2-G-a):

Compound V-C2-G (1.10 g, 2.73 mmol) was dissolved in THF (15 ml) under $N_2$ atmosphere. A suspension of palladium on charcoal (10 %, 130 mg) in THF (10 ml) was added. $H_2$ was applied at ambient pressure and the reaction mixture was stirred at RT for 3 d. The reaction mixture was filtered over Celite and the filter cake was washed with THF (20 ml). The filtrate was concentrated *in vacuo* yielding compound (V-C2-G-a) (240 mg, 0.768 mmol, 28 %) after column chromatography ($SiO_2$, DCM).

II. 15, 16-unsaturated and C2 substituted Estrone derivatives of formula (X) (Step B)

The estrone of general formula V was converted into the corresponding 15, 16 unsaturated derivative by the 4-step reaction as depicted in SCHEME 1 according to Nambara 1976: After protection of the C17 keto function as acetal (ethylene glycol, TEOF and p-TosOH in toluene, work-up with water and TEA), the acetal was brominated (with pyridinium perbromate and ethylene glycol in DME, work-up with $Na_2S_2O_3$). Subsequently, HBr was eliminated by reaction with K-O-tert-butyl in DMSO. Finally, the deprotection of the acetal was achieved with p-TosOH in DME and water.
The following intermediates were prepared according to this procedure:

3-Benzyloxy-2-methoxy-estra-1,3,5(10),15-tetraene-17-one (X-C2-B)
3-Benzyloxy-2-ethyl-estra-1,3,5(10),15-tetraene-17-one (X-C2-C)
3-Benzyloxy-2-ethoxy-estra-1,3,5(10),15-tetraene-17-one (X-C2-D)
3-Benzyloxy-2-methoxy-ethoxy-estra-1,3,5(10),15-tetraene-17-one (X-C2-E)
3-Benzyloxy-2-methoxy-ethyl-estra-1,3,5(10),15-tetraene-17-one (X-C2-F)
3-Benzyloxy-2-propyl-ethoxy-estra-1,3,5(10),15-tetraene-17-one (X-C2-G)

III. Introduction of the basic side chain in C15 position

The detailed synthesis of the following intermediates, wherein $R^{14}$ represents H, is fully disclosed within international patent application WO 2005/47303, which is incorporated by reference herein. For intermediates with $R^{14}$ is different from H, detailed synthesis is given for exemplary compounds.

IIIa. The optionally 2-substituted ketal derivative of the Estron-15α-yl-carbaldehyde of formula XIII-0

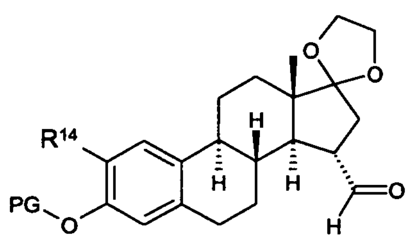

(XIII-0)

The protected aldehyde intermediate of formula XIII-0 with PG = $CH_3$ (XIIIb) or PG = Benzyl (XIIIc) can be prepared according to a procedure depicted within the following scheme 2:

SCHEME 2

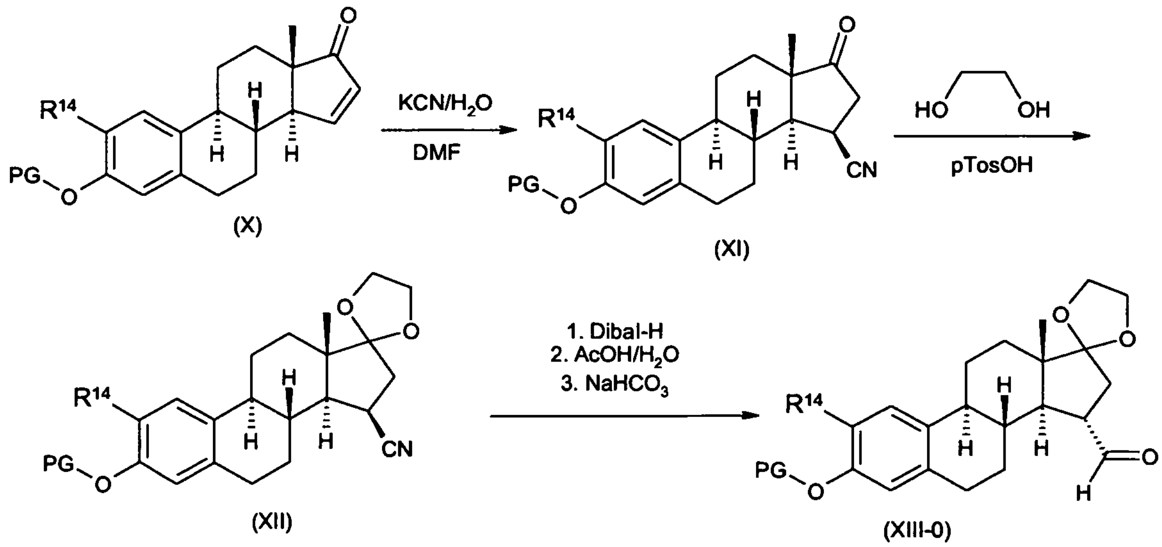


(X) (XI) (XII) (XIII-0)

The optionally 2-substituted 15,16-unsaturated estrone of formula (X) was converted into the corresponding cyano-estrone (XI) by a cyanide Michael addition at the D-ring. The nitrile was introduced in the beta configuration as was proven by 2D-NMR. Epimerization of this stereocenter had been accomplished in a following step. First the ketone functionality was protected as the acetal (XII), followed by conversion of the nitrile to the corresponding aldehyde (XIII-0) by the addition of DIBAH to the nitrile and the consecutive hydrolysis of the imine product. At this stage the epimerization took place for about 90% (2D-NMR). Consecutive washing of the mixture with aqueous bicarbonate gave the $\alpha$-isomer with a d.e $\leq$ 98%.

IIIb. Optionally 2-substituted compounds of formula IV: Estron-15-yl-$C_0$-$C_5$-alkyl-carboxylic acid

Acid building block IV-0: (n = 0)

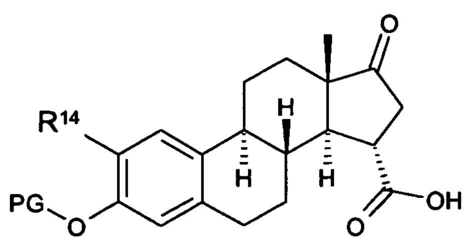

(IV-0)

The individual steps in the synthesis of acid building block of the formula IV-0B are depicted in the following scheme 3.

SCHEME 3

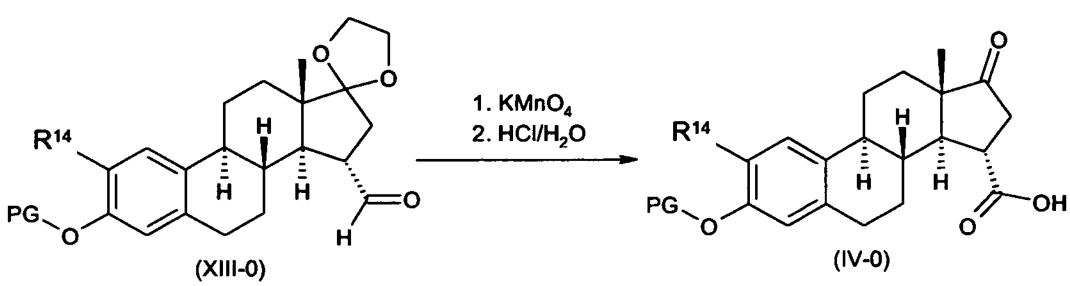


(XIII-0) (IV-0)

The ketal derivative of the optionally 2-substituted 17-oxo-estra-1,3,5(10)-trien-15α-yl-carbaldehyde of formula XIII-0 is oxidized to the corresponding carboxylic acid and converted into the unprotected 15a-substituted estrone derivative of formula IV-0.

Acid building block IV-1: (n = 1):

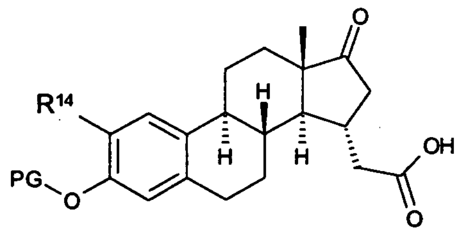

(IV-1)

The acid building block IV-1 may be synthesized via two different routes. The individual steps of the first synthesis route of acid building block IV-1 are depicted in the following scheme 4. The same kind of procedure can be applied for n = 2 and for other side chains within the PG position.

## SCHEME 4

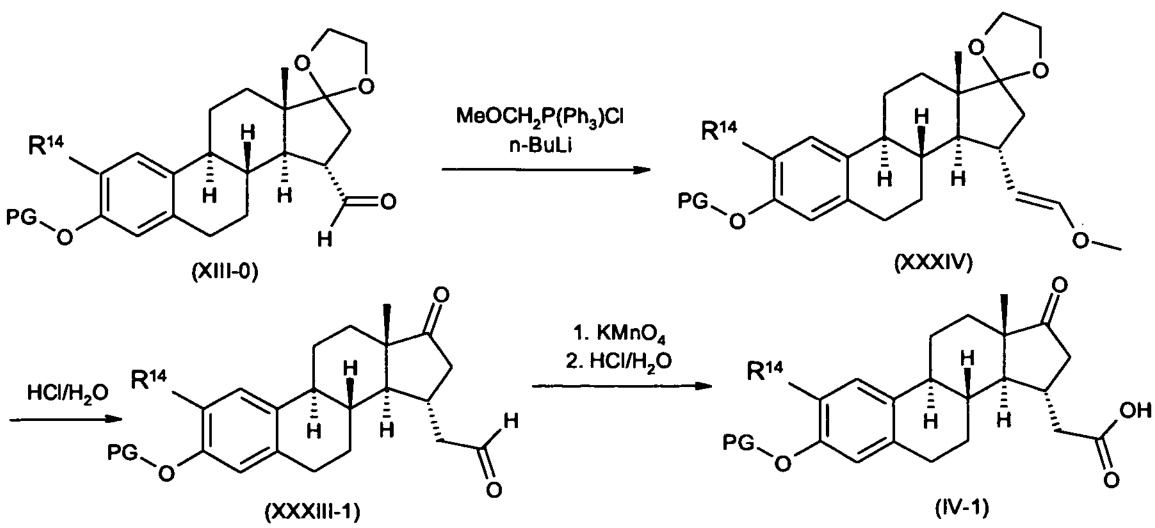


(XIII-0) (XXXIV) (XXXIII-1) (IV-1)

The ketal derivative of the 17-oxo-estra-1,3,5(10)-trien-15α-yl-carbaldehyde of formula XIII-0 is converted into the methyl enol ether of the formula XXXIV via a Wittig reaction with $MeOCH_2LiP(Ph)_3$. Hydrolysis with $HCl_{(aq)}$ delivered the unprotected acetaldehyde derivative XXXIII-1. The acetaldehyde derivative is then further oxidized to the corresponding carboxylic acid IV-1.

Alternative synthesis route for the acid building block IV-1: (n = 1):

IV-1b: (n = 1 and PG = $CH_3$): 3-Methoxy-17-oxo-estra-1,3,5(10)-trien-15α-yl-acetic acid

Alternatively, compound IV-1b can be prepared directly from the enone derivative of formula X according to the following synthesis scheme 5:

## SCHEME 5

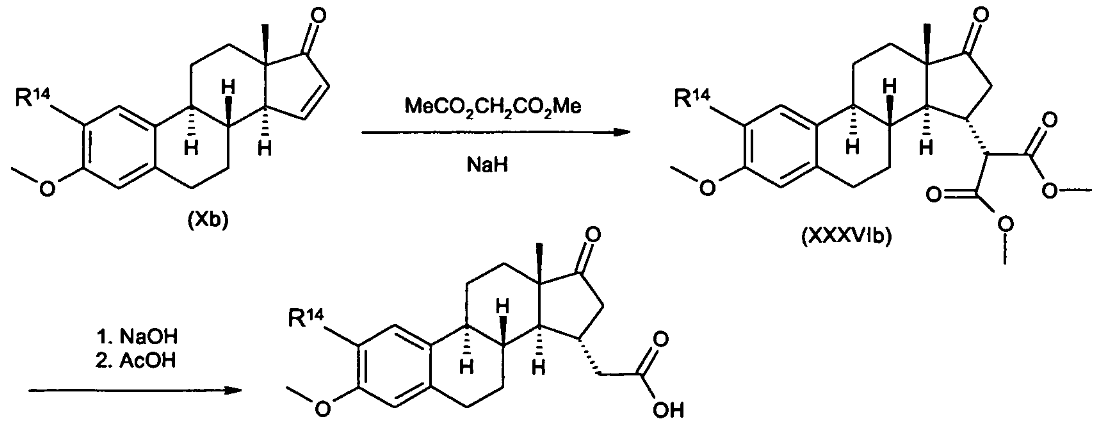


(Xb)

(XXXVIb)

(IV-1b)

A Michael addition of the dimethylmalonate-anion to the enone derivative delivered the diester XXXVIb, which was converted into the acid building block of formula IV-b by alkaline ester hydrolysis and decarboxylation in refluxing acetic acid.

Optionally 2-substituted acid building blocks IVβ-2, IVβ-3, IVB-4, IV5-5, IVβ-6 (n = 2, 3, 4, 5, 6): IVβ-3b (n = 3 and PG = $CH_3$): 4-(3-Methoxy-17-oxo-estra-1,3,5(10)-trien-15β-yl)-butyric acid

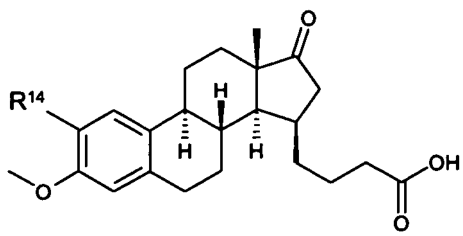

(IVβ-3b)

The individual steps in the synthesis of acid building block of the formula IVP-βb are depicted in the following scheme 6. The same kind of procedure can be applied for n = 4, 5, or 6 and for other alkyl side chains within the $R^1$ position using the appropriate BrMg-$C_5$-$C_7$-alkoxy-THP as Grignard Reagent. Furthermore, this reaction scheme also delivers the estrone-alcohol building block in form of the intermediate of formula XXXIβ-4b.

SCHEME 6

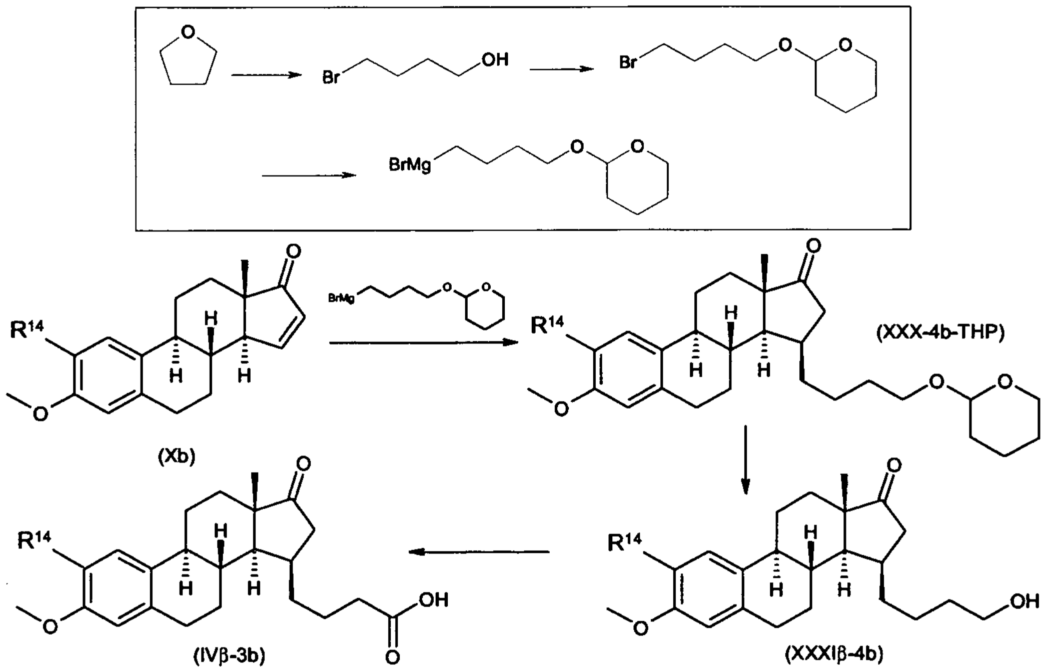

(Xb)

(XXX-4b-THP)

(IVβ-3b)

(XXXIβ-4b)

4-Bromo-butanol-THP ether was prepared by adding HBr solution to refluxing THF. The resulting bromide was dissolved in DCM, p-TosOH and DHP were added at 0°C to give the protected alcohol. This was filtered over $SiO_2$ and further purified by column chromatography, yielding 9.3% over 2 steps. The protected alcohol was dissolved in THF and added to activated magnesium, and the resulting Grignard reagent added to $CuI_2$ in HMPA. The 15, 16-unsaturated Estrone derivative of formula Xb, dissolved in dry THF and TMSCI, was added at -40±5°C. Subsequently, after hydrolysis of the silyl ether, the resulting compound XXX-4b-THP was deprotected with p-TosOH/MeOH to give the alcohol derivative XXXI-4b, which was converted, without purification, into the free acid IV-3b by a Jones oxidation. The oil was purified by column chromatography, yielding the free acid of formula IV-3b in 30% yield over three steps.

Acid building blocks IVβ-2 (n = 2 and PG = H, $CH_3$ or benzyl): optionally 2-substituted 3-(3-Benzyloxy/Methoxy/Hydroxy-17-oxo-estra-1,3,5(10)-trien-15β-yl-propanoic acid

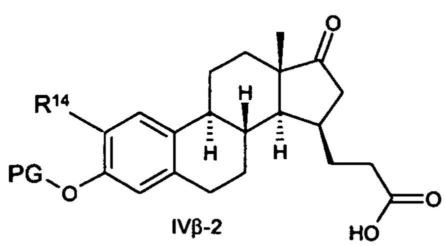

IVβ-2

The optionally 2-substituted carboxylic acid IVβ-2 can be prepared by oxidation of the alcohol derivative of formula XXXIβ-3b or XXXIβ-3c according to the preparation of the carboxylic acid IVβ-3b (see section for the preparation of the alcohol derivatives below for synthesis of XXXIβ-3b and XXXIβ-3c) and optionally subsequent debenzylation of the C3 hydroxy function.

Acid building block IVβ-3c: Optionally 2-substituted 4-(3-Benzyloxy-17-oxo-estra-1,3,5(10)-trien-15β-yl)-butyric acid

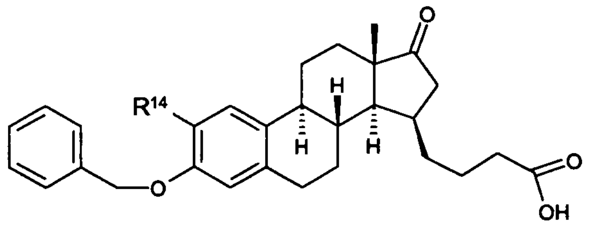

(IVβ-3c)

The synthesis of acid building block of the formula IVβ-3c is performed according to any of the procedures depicted in the following schemes 7A, 7B and 7C. Furthermore, reaction scheme 7A also delivers the estrone-alcohol building block in form of the intermediate of formula XXXIβ-4c. The same kind of procedure can be applied for n = 4, 5, or 6 and for other alkylaryl substitutents within the $R^1$ position using the appropriate BrMg-$C_5$-$C_7$-alkoxy-THP as Grignard Reagent.

## SCHEME 7A

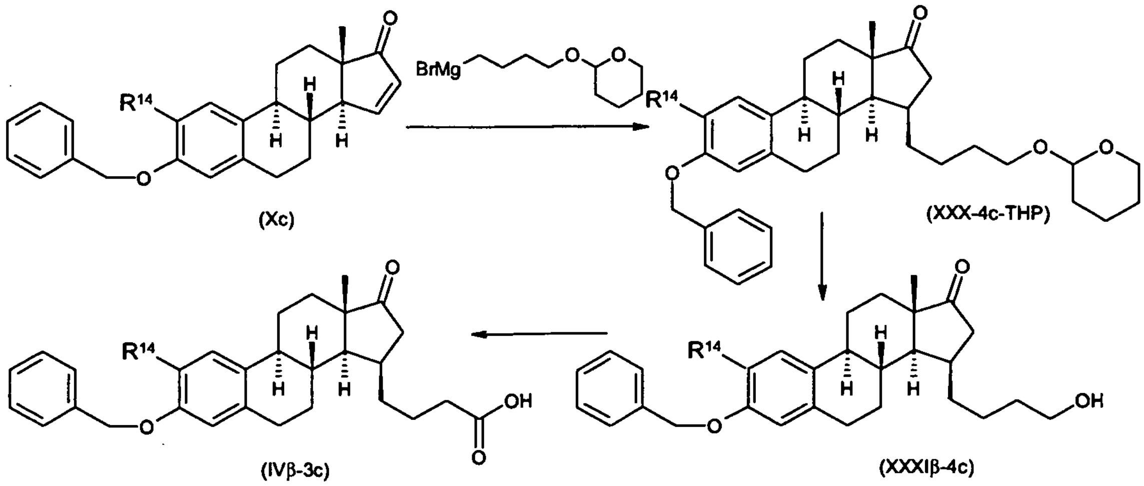

(Xc)

(XXX-4c-THP)

(IVβ-3c)

(XXXIβ-4c)

4-Bromo-butanol-THP ether was prepared by adding HBr solution to refluxing THF. The resulting bromide was dissolved in DCM, p-TosOH and DHP were added at 0°C to give the protected alcohol. This was filtered over $SiO_2$ and further purified by column chromatography, yielding 9.3% over 2 steps. The protected alcohol was dissolved in THF and added to the activated magnesium, and the resulting Grignard reagent added to $CuI_2$ in HMPA. The 15, 16-unsaturated Estrone derivative of formula Xc (preferably with $R^{14}$ = H), dissolved in dry THF and TMSCI, was added at - 40±5°C. Subsequently, the resulting compound XXX-4c-THP was deprotected with p-TosOH/MeOH to give XXXIβ-4c in 47% over 2 steps, which was converted, without purification, into the free acid IVβ-3c by a Jones oxidation in a yield of 96%.

## SCHEME 7B

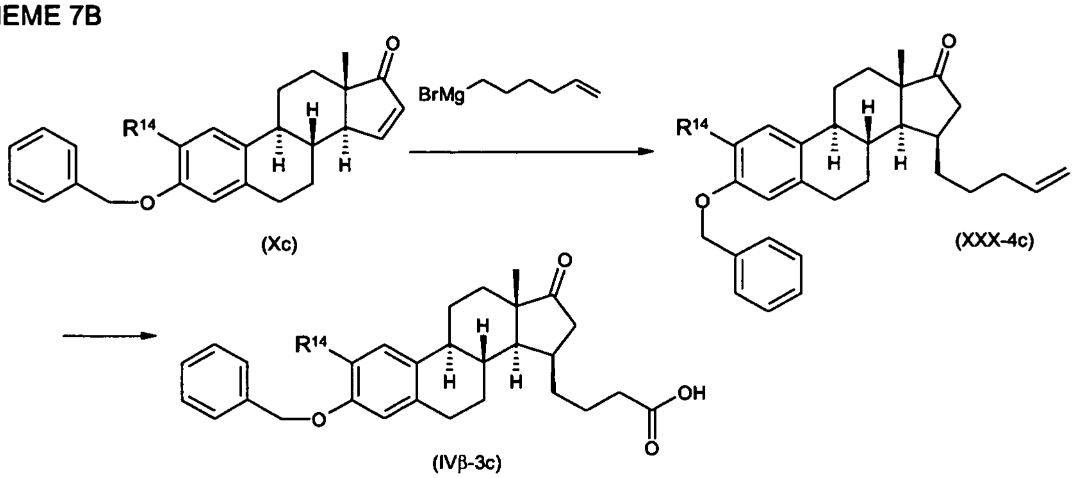

(Xc)

(XXX-4c)

(IVβ-3c)

The 15, 16-unsaturated Estrone derivative of formula Xc (preferably with $R^{14}$ = Ethyl, n-Propyl or Methoxyethyl) was subjected to a 1,4 addition using a freshly prepared Gringard Reagent. Subsequently, the resulting compound XXX-4c was oxidized to the free acid IVβ-3c (see also the reaction SCHEME 12).

SCHEME 7C

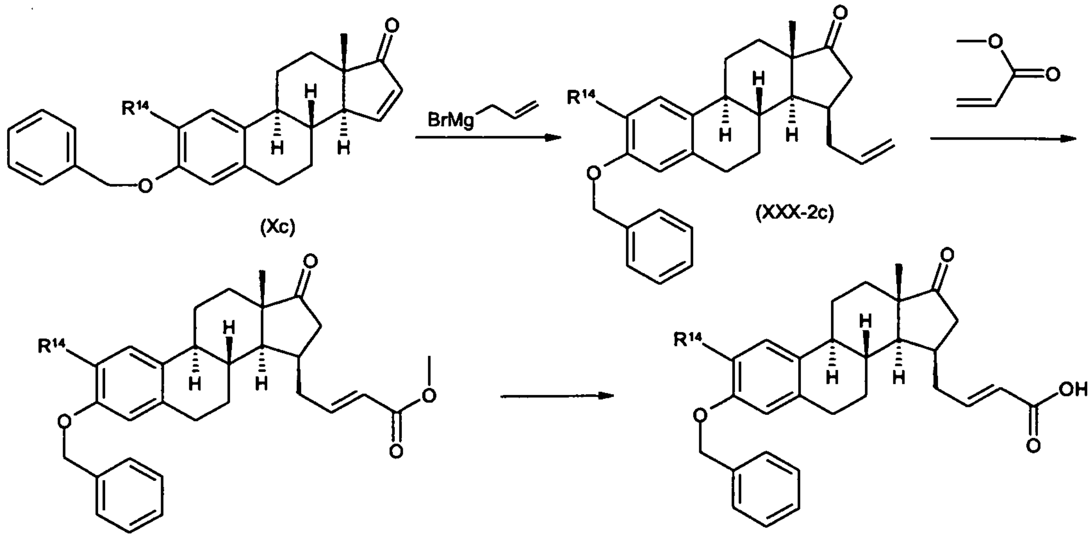


(Xc)

(XXX-2c)

SCHEME 7C, continued

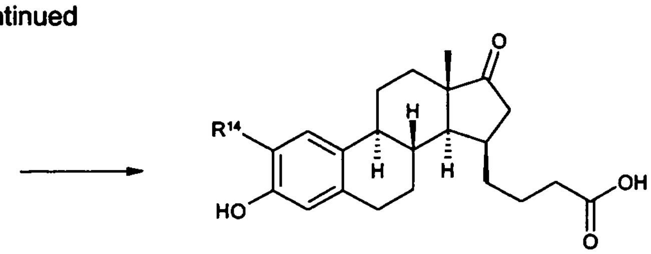

(IVb-3a)

The 15, 16-unsaturated Estrone derivative of formula Xc (preferably with $R^{14}$ = Methoxy) was subjected to a 1,4 addition using a freshly prepared Gringard Reagent. Subsequently, the resulting compound XXX-2c was reacted with with acrylic acid methyl ester using a Grubb II catalyst, known as olefin metathesis. After removal of the methyl group, the free acid (IVb-3a) is obtained by hydrogenation and deprotection. Alternatively, the last two steps may be performed in reversed order.

<u>Detailed Synthesis for exemplary compounds</u>

**4-(3-Hydroxy-2-methoxy-17-oxo-estra-1,3,5(10)-trien-15β-yl)-butyric acid (IVβ-(C2-B)-3a)**

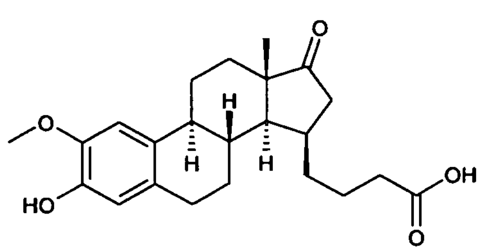

(IVb-(C2-B)-3a)

Compound (IVβ-(C2-B)-3a) was prepared according to general procedure depicted in SCHEME 7C starting from compound (X-C2-B).

15β-Allyl-3-benzyloxy-2-methoxy-estra-1,3,5(10)-trien-17-one (XXXβ-(C2-B)-2c):

Under $N_2$-atmosphere, LiCl (247 mmol) and CuI (247 mmol) were dissolved in THF (500 ml). The solution obtained was cooled to $T_{intem}$= -78˚C, and allyl-MgBr (1 M in $Et_2O$, 246 mmol) was added dropwise during 1.5 h. After stirring for 0.5 h, TMSCl (171 mmol) was added and the reaction mixture was further stirred at $T_{intem}$= -78˚C for 0.5 h. Then, a solution of (X-C2-B) (26.5 g, 68.2 mmol) in THF (250 ml) was added dropwise during 1.5 h. The reaction mixture was stirred at $Ti_{ntem}$= -78˚C for 1.5 h, after which it was allowed to warm to RT, quenched with sat. $NH_4Cl$ (aq) (600 ml), and stirred at RT overnight. The reaction mixture was filtered over celite and the residue was washed with EtOAc (200 ml). The organic layer was isolated from the combined filtrates, and the aqueous layer was extracted with EtOAc. The combined organic layers were washed with 1 N HCl, 1 N $NH_4OH$ (aq), brine, dried over $Na_2SO_4$, filtered, and concentrated *in vacuo* to yield (XXX-(C2-B)-2c) (34.8 g, 98%).

4-(3-Benzyloxy-2-methoxy-17-oxo-estra-1,3,5(10)-trien-158-yl)-but-2-enoic acid methyl ester:

Under $N_2$-Atmosphere, in oven-dried glassware, Grubbs II catalyst (2.36 mmol) was added to a solution of (XXX-(C2-B)-2c) (60.4 mmol) and methyl acrylate (150 mmol) in DCM (500 ml). The mixture obtained was stirred at RT overnight, heated at $T_{intem}$=39 ˚C for 8 h, after which it was allowed to cool to RT. The reaction mixture was evaporated to dryness to furnish 30.8 g resin, which was applied to $SiO_2$ (1500 ml) with DCM and eluted with a DCM:EtOAc gradient (99:1 to 90:10) to yield the desired compound (18.5 g, 63%) ($R_f$=0.1 (DCM)). This was dissolved in THF (250 ml) and heated at reflux with activated charcoal (1 g) for 20 min. The mixture obtained was allowed to cool to RT, filtered and the filtrate was concentrated *in vacuo.*

4-(3-Hydroxy-2-methoxy-17-oxo-estra-1,3,5(10)-trien-15β-yl)-butyric acid methyl ester VIIβ-(C2-B)-3a-1)

A solution of 4-(3-Benzyloxy-2-methoxy-17-oxo-estra-1,3,5(10)-trien-15β-yl)-but-2-enoic acid methyl ester (17.3 g, 2.36 mmol) in THF (440 ml) and MeOH (440 ml) was purged with $H_2$ (balloon). Then Pd (10% on carbon, 50% $H_2O$) (1.80 g) was added, and the mixture obtained was stirred under $H_2$ pressure for over night. The reaction mixture was filtered over two filter papers and concentrated *in vacuo* to yield the desired compound (VIIβ-(C2-B)-3a-1) (13.8 g, 97%), which represents a compound falling under the scope of the present invention.

4-(3-Hydroxy-2-methoxy-17-oxo-estra-1,3,5(10)-trien-15β-yl)-butyric acid (IVβ-(C2-B)-3a)

A solution of $LiOH{\cdot}H_2O$ (197 mmol) in $H_2O$ (450 ml) was added to a solution of (VIIβ-(C2-B)-3a-1) (34.5 mmol) in THF (450 ml) and the mixture obtained was stirred at RT overnight. The reaction mixture was concentrated *in vacuo* to remove THF and diluted with $H_2O$. The mixture was washed with DCM, acidified with 1N HCl to pH 1 and extracted with DCM. The combined extracts were washed with brine, dried over $Na_2SO_4$, filtered and concentrated *in vacuo* to yield (IVβ-(C2-B)-3a) (11.7 g, 88%).

**4-(3-Hydroxy-2-(2-methoxy-ethyl)-17-oxo-estra-1,3,5(10)-trien-15β-yl)-butyric acid**

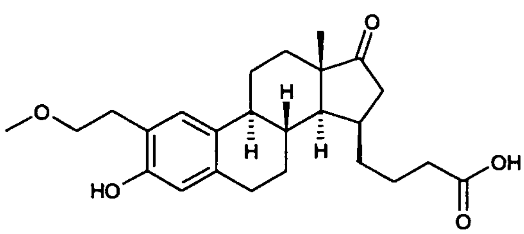

(IVb-(C2-F)-3a)

Compound (IVβ-(C2-F)-3a) was prepared according to general procedure depicted in SCHEME 7C starting from compound (X-C2-F).

15-Allyl-3-(benzyloxy)-2-(2-methoxy-ethyl)-estra-1,3,5(10)trtien-17-one (XXXβ-(C2-F)-2c):

A flame dried flask was charged with CuI (1.38 mmol) and LiCl (1.38 mmol) under $N_2$ atmosphere. THF (5 ml) was added and stirred at ambient temperature until a clear green solution was obtained. After cooling the solution to

-78˚C, allylmagnesium bromide in $EtO_2$ (1.38 mmol) was added dropwise and stirred at -78˚C for 1h. Then TMSCl (1.38 mmol) was added in a single batch. A solution of compound X-C2-F (192 mg, 0.46 mmol) in THF (5 ml) was added dropwise at -78˚C. The reaction mixture was stirred at -78˚C for 2h and the mixture was allowed to reach RT overnight. The mixture was quenched with sat aq $NH_4Cl$ (50 ml). The organic layer was separated and washed with aq. 1 M HCl (25 ml), aq. 1 M $NH_4OH$ (25 ml) and brine (25 ml). The combined organic layers were dried over $NaSO_4$ and concentrated *in vacuo* yielding compound (XXXβ-(C2-F)-2c) (220 mg, max. 0.46 mmol). Purification via $SiO_2$ (DMC/methanol = 100/0 to 98/2) delivered pure (XXXβ(C2-F)-2c) (56 mg, 0.122 mmol, 22 %).

4-(3-Benzyloxy-2-(2-methoxy-ethyl)-17-oxo-estra-1,3,5(10)-trien-15β-yl)-but-2-enoic acid ethyl ester:

Compound (XXXβ-(C2-F)-2c) (46 mg, 0.10 mmol) was dissolved in DCM (5 ml) under $N_2$ atmosphere. Ethyl acrylate (0.135 mmol) and Grubbs II catalyst (0.01 mmol) were added. The reaction mixture was stirred at ambient temperature for 18h. The reaction mixture was filtered over celite and concentrated *in vacuo* yielding the title compound (46 mg, 0.086 mmol, 85 %) after purification over $SiO_2$ (DCM/MeOH = 100/0 to 98/2).

4-(3-Hydroxy-2-(2-methoxy-ethyl)-17-oxo-estra-1,3,5(10)-trien-15β-yl)-butyric acid ethyl ester (VIIβ-(C2-F)-3a-1)

Palladium on charcoal (10%, 15 mg) was suspended in methanol (5 ml) under $N_2$ atmosphere. A solution of the previous compound (45 g, 0.085 mmol) in THF (5 ml) was added carefully. $H_2$ at ambient pressure was applied and the reaction mixture was stirred at ambient temperature over the weekend. The reaction mixture was filtered over Celite and the filter cake was washed with THF (10 ml). The filtrate was concentrated *in vacuo* yielding compound (VIIβ-(C2-F)-3a-1) (40 mg, max. 0.085 mmol), which also represents a compound falling under the scope of the present invention.

4-(3-Hydroxy-2-(2-methoxy-ethyl)-17-oxo-estra-1,3,5(10)-trien-15β-yl)-butvic acid (IVβ-(C2-F)-3a)

The previous compound (40 mg, max. 0.085 mmol) was dissolved in a mixture of THF (2 ml), water (2 ml) and $LiOH \cdot H_2O$ (0.45 mmol). The mixture was stirred at ambient temperature for 5h. THF was removed *in vacuo* and the residue diluted with water (5 ml). The alkaline layer was washed with DCM (1x10 ml) and the organic layer discarded. The water layer was acidified till pH 3 using aq. 1M HCl and extracted with DCM (4x 25 ml). The combined organic layers were washed with brine (25 ml), dried over $Na_2SO_4$ and concentrated *in vacuo* yielding compound (IVβ-(C2-F)-3a) (26 mg, 0.063 mmol, 74 %) as pale solid.

The following further building blocks were prepared according to this procedure:

4-(3-Hydroxy-2-ethyl-17-oxo-estra-1,3,5(10)-trien-158-yl)-butyric acid (IVβ-(C2-C)-3a)
4-(3-Hydroxy-2-propyl-17-oxo-estra-1,3,5(10)-trien-15B-yl)-butyric acid (IVβ-(C2-G)-3a)

Optionally 2-substituted acid building block with a stereochemistry at C15: IVα-3a (n = 3 and PG = H): 4-(3-Hydroxy-17-oxo-estra-1,3,5(10)-trien-15α-yl)-butyric acid

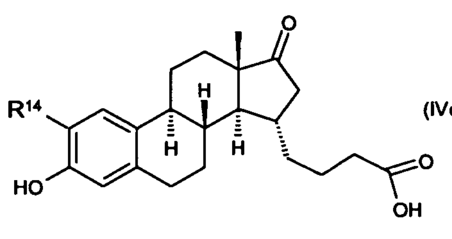

(IVα-3a)

The individual steps in the synthesis of the optionally 2-substituted acid building block of the formula IVα-3a are performed according to any of the procedures depicted in schemes 8A and 8B. Furthermore, reaction scheme 8A also delivers the still ketal-protected estrone-alcohol building block in form of the intermediate of formula XLIVα-1c. Debenzylation and deprotection delivers the estrone-alcohol XXXIα-1a.

## SCHEME 8A

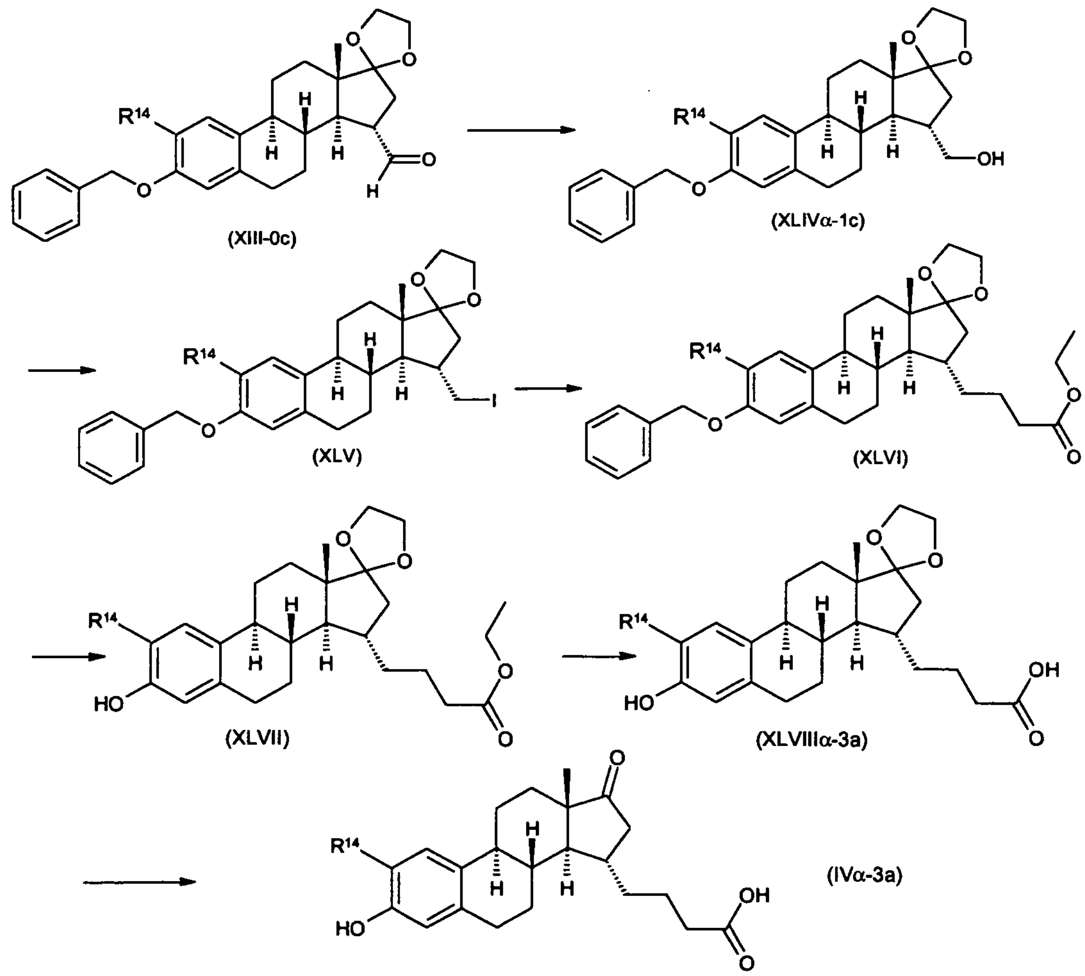

(XIII-0c) (XLIVα-1c) (XLV) (XLVI) (XLVII) (XLVIIIα-3a) (IVα-3a)

Reduction of the aldehyde XIII-0c with $NaBH_4$ (EtOH, 2h, RT) gave the alcohol XLIVα-1c, which was further treated with iodine, triphenylphosphine and imidazole to give the iodide XLV. Subsequently, ethylacrylate was coupled to iodine XLV and gave compound XLVI after purification by column chromatography. Reduction of compound XLVI was performed under $H_2$ atmosphere to give compound XLVII, which was transformed into the protected carboxylic acid building block XLVIIIα-3a by saponification. The carboxylic acid IVα-3a was obtained by deprotection.

**SCHEME 8B**

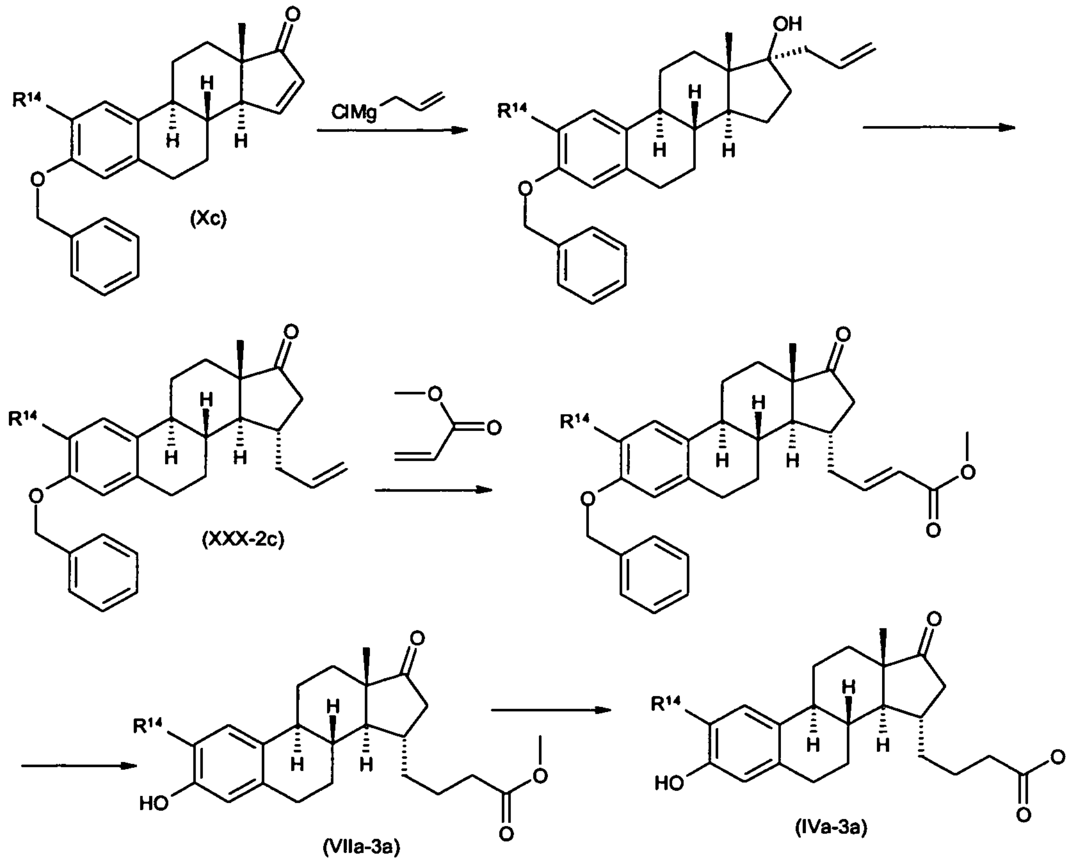


The allyl group was introduced into the optionally C2 substituted, 15, 16-unsaturated Estrone derivative of formula Xc by reaction with allylmagnesium chloride or bromide, followed by an oxy-cope rearrangement catalysed by KH and 18-Crown-6. Subsequently, the resulting compound XXX-2c was reacted with acrylic acid methyl ester using a Grubb II catalyst, known as olefin metathesis. The free acid (IVa-3a) is obtained by hydrogenation, deprotection, and, in the last step, hydrolysation of the methyl ester with LiOH.

Detailed Synthesis for 4-(2-Ethoxy-3-hydroxy-17-oxo-estra-1,3,5(10)-trien-15α-yl)-butyric acid

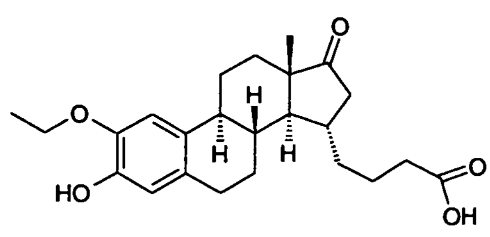

**(IVα-(C2-D)-3a)**

17-Allyl-3-(benzyloxy)-2-ethoxy-estra-1,3,5(10)trien-17-ol

Allylmagnesiumchloride (1.7 M in THF, 48.39 mmol) was added dropwise to a solution of ketone X-C2-D (6.5 g, 16.1 mmol) in THF (200 ml) under $N_2$ atmosphere at 0˚C. After stirring for 4h at 0-5˚C, the solution was poured into sat aq $NH_4Cl$. The water layer was extracted with DCM. The combined organic layers were washed with sat $NaHCO_3$, dried over $Na_2SO_4$ and concentrated in vacuo yielding 7.8 g (100%) as thick yellow oil.

15α-Allyl-3-benzyloxy-2-ethoxy-estra-1,3,5(10)-trien-17-one (XXX-(C2-D)-2c)

KH 30% in oil (89.98 mmol) was suspended in THF (50 ml), under $N_2$ atmosphere. 17-Allyl-3-(benzyloxy)-2-ethoxy-estra-1,3,5(10)-trien-17-ol (7.7 g, 17.30 mmol) and 18-Crown-6 (88.25 mmol) were dissolved in THF (200 ml) and added dropwise in 40 min. The mixture was stirred for 3 h at RT. The mixture was carefully poured into IPA *via* canula. The mixture was poured into aq. sat. $NH_4Cl$. The water layer was extracted with EtOAc. The combined organic layers were washed with brine, dried over $Na_2SO_4$ and concentrated in *vacuo*. Purification *via* filtration over Silica (eluens heptan to 40% DCM in heptan) provided 4.2 g (9.51 mmol, 55%) of (XXX-(C2-D)-2c).

4-(3-Benzyloxy-2-ethoxy-17-oxo-estra-1,3,5(10)-trien-15α-yl) but-2-enoic acid methyl ester

Substrate (XXX-(C2-D)-2c) (4.2 g, 9.51 mmol) was dissolved in DCM (100 ml) under $N_2$ atmosphere. Methylacrylate (23.76 mmol) and Grubbs (II) catalyst (0.380 mmol) were added. The mixture was refluxed overnight. The mixture was concentrated in *vacuo* to afford 4.7 g of dark brown solid. Purification *via* column chromatography yielded 3.17 g (66%) of a brown semi-solid.

4-(3-Hydroxy-2-ethoxy-17-oxo-estra-1,3,5(10)-trien-15α-yl) butyric acid methyl ester (VIIa-(C2-D)-3a)

4-(3-Benzyloxy-2-ethoxy-17-oxo-estra-1,3,5(10)-trien-15α-yl) but-2-enoic acid methyl ester (3.17 g, 6.30 mmol) was dissolved in THF (80 ml) and MeOH (80 ml). Pd/C 10% (50% in water, 0.3 g) was added. The mixture was stirred at 1 atm $H_2$ (balloon) at RT for 48 h. The mixture was filtered over Celite. The filter cake was rinsed with MeOH and the filtrate was concentrated in *vacuo* to provide 2.8 g (6.72 mmol, 100%) of a greenish solid.

4-(2-Ethoxy-3-hydroxy-17-oxo-estra-1,3,6(10)-trien-15α-yl)-butyric acid (IVa-(C2-D)-3a)

Methylester (VIIa-(C2-D)-3a) (2.8 g, 6.72 mmol) was dissolved in THF (80 ml). LiOH (39.86 mmol) was dissolved in water (80 ml) and added in one portion. The solution was stirred at RT overnight. The solution was concentrated in *vacuo* and water (350 ml) was added. The water layer was washed with DCM (3 x 200 ml) and acidified to pH 1 with 2N HCl and extracted DCM. The combined organic layers were washed with brine, dried over $Na_2SO_4$ and concentrated in vacuo to afford 1.84 g (68%) of white foam, LC-MS purity of 97-100%.

$^1$H-NMR ($CDCl_3$, 300 MHz): δ 0.95 (s,3H), 1.25 (m, 2H), 1.4 (t, 3H), 1.5 (m, 3H), 1.60-2.1 (m, 6H), 2.1-2.45 (m, 6H), 2.80 (m, 3H), 4.1 (q, 2H), 6.65 (s, 1H), 6.80 (s, 1 H)

4-(3-Hydroxy-2-methoxy-17-oxo-estra-1,3,5(10)-trien-15α-yl)-butyric acid (IVa-(C2-B)-3a) and 4-(3-Hydroxy-2-(2-methoxy-ethoxy)-17-oxo-estra-1,3,5(10)-trien-15α-yl)-butyric acid (IVa-(C2-E)-3a)

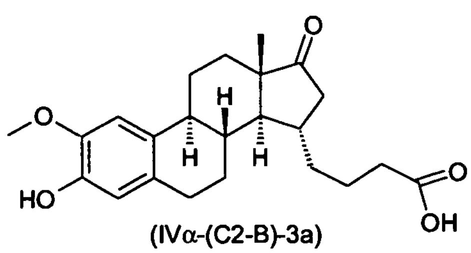

(IVα-(C2-B)-3a)

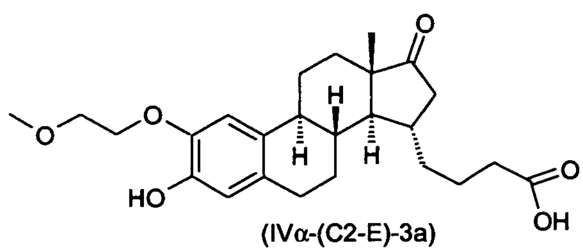

(IVα-(C2-E)-3a)

These two intermediates were synthesized accordingly from ketones X-C2-B and X-C2-E, respectively.

4-(3-Hydroxy-2-(2-methoxy-ethoxy)-17-oxo-estra-1,3,5(10)-trien-15α-yl)-butyric acid (IVa-(C2-E)-3a)

$^1$H-NMR ($CDCl_3$, 300 MHz): δ0.95 (s, 3H), 1.3 (t, 2H), 1.5 (m, 3H), 1.6-2.1 (m, 6H), 2.1-2.4 (m, 6H), 2.8 (m, 3H), 3.4 (s, 3H), 3.7 (t, 2H), 4.1 (m, 2H), 6.62 (s, 1H), 6.9 (s, 1 H)

4-(3-Hydroxy-2-methoxy-17-oxo-estra-1,3,5(10)-trien-15α-yl)-butvric acid (IVa-(C2-B)-3a)

$^1$H-NMR (shifts in ppm): 0.96-1.04 (s, 3H), 1.20-2.48 (m, 16H), 2.70-2.94 (m, 3H), 3.80-3.92 (s, 3H), 6.60-6.68 (s, 1H), 6.76-6.80 (s, 1H).

IIIc. Compounds of formula XXXI (alcohol derivatives): Optionally 2-substituted 15-hydroxy-$C_1$-$C_6$-alkyl-Estron

Alcohol building block XXXIα-1 (n = 1)

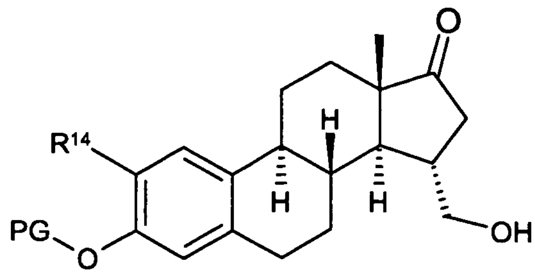

(XXXIα-1)

The synthesis of the alcohol derivatives XXXIa-1a (PG = $R^1$ = H), XXXIa-1b (PG = $R^1$ = $CH_3$), and XXXIα-1c (PG = $R^1$ = benzyl) is depicted in the following scheme 9:

**SCHEME 9**

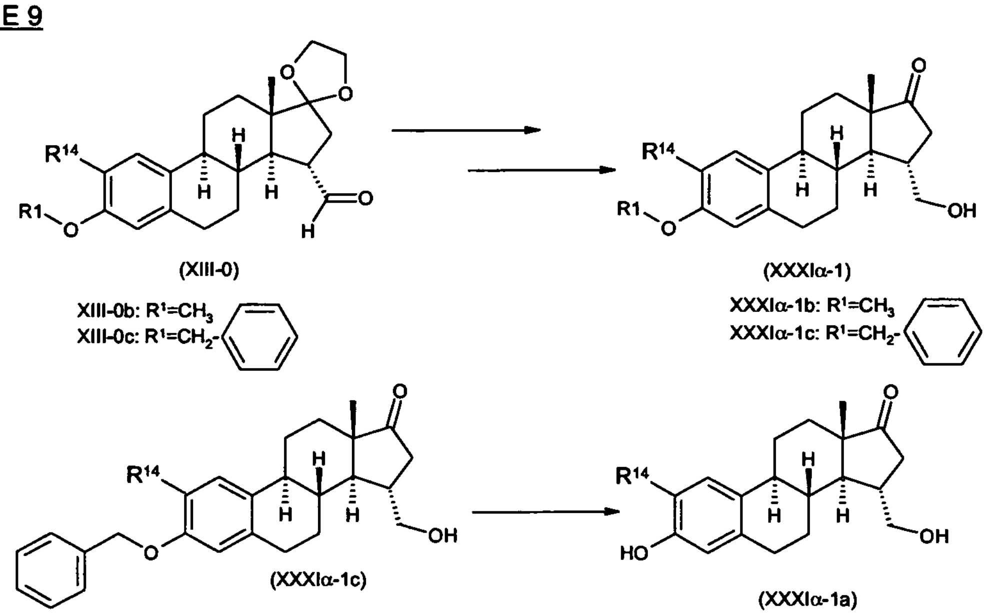

(XIII-0)

XIII-0b: $R^1$=$CH_3$

XIII-0c: $R^1$=$CH_2$-

(XXXIα-1)

XXXIα-1b: $R^1$=$CH_3$

XXXIα-1c: $R^1$=$CH_2$-

(XXXIα-1c)

(XXXIα-1a)

Reduction of the aldehydes XIII-0b or XIII-0c using $NaBH_4$ followed by ketal hydrolysis gave the corresponding alcohols XXXIα-1b and XXXIα-1c. The alcohol XXXIα-1c was debenzylated to give XXXIα-1 a using Pd/C and a 5 bar hydrogen atmosphere.

Alcohol building blocks XXXI-3c and XXXI-3a (n = 3) and XXXI-5c and XXXI-5a (n = 5) with PG=$R^1$=benzyl or PG=$R^1$=H):

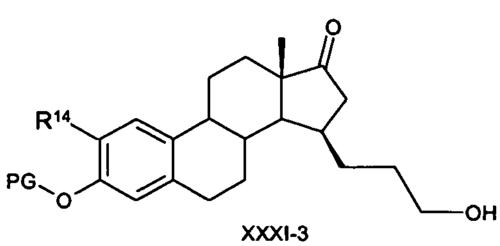

XXXI-3

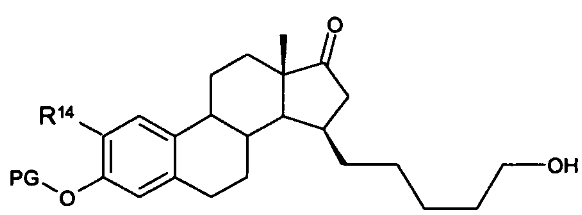

XXXI-5

The synthesis of the optionally 2-substituted alcohol building blocks of formula XXXI-3 and XXXI-5 is depicted

in the following scheme 10.

**SCHEME 10**

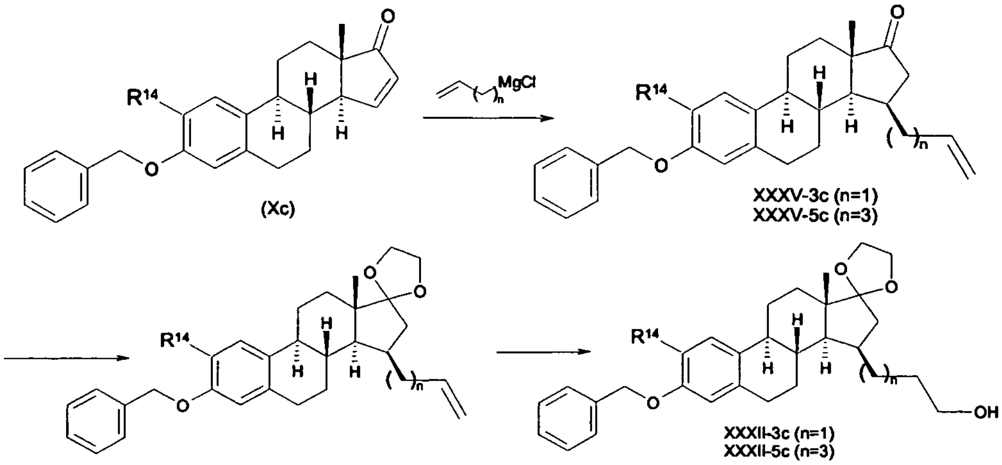

(Xc)
XXXV-3c (n=1)
XXXV-5c (n=3)
XXXII-3c (n=1)
XXXII-5c (n=3)

**SCHEME 10, continued**

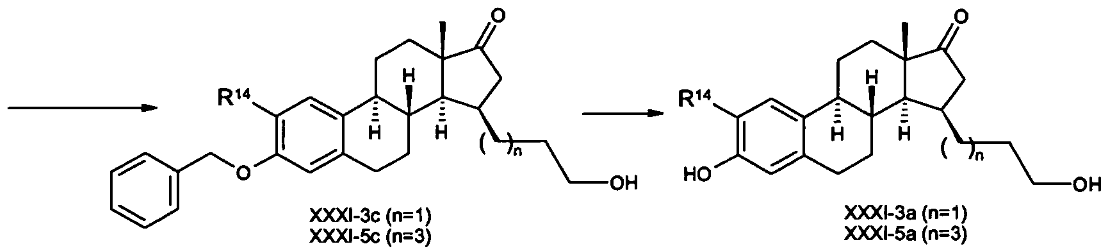

XXXI-3c (n=1)
XXXI-5c (n=3)

XXXI-3a (n=1)
XXXI-5a (n=3)

The 15, 16-unsaturated Estrone derivative of formula Xc (preferably with $R^{14}$ = H, Ethyl, n-Propyl or Methoxyethyl) was subjected to a 1,4 addition using a freshly prepared Gringard Reagent (allylmagnesiumchloride or pentenylmagnesiumchloride) delivering compound XXXV. After protection of the ketone functionality in C17 as ketal, the alkenyl side chain was hydroborated and subsequently oxidized to provide compound XXXII. After deprotection of the C17 keto function by treatment with pTosOH, the benzyl function is optionally cleaved off to deliver the alcohol building blocks XXXI- 3 and XXXI-5, respectively.

Alcohol building blocks XXXI-4b, XXXI-5b, XXXI-6b (n = 4, 5, 6):

15β-(4-Hydroxy-$C_4$-$C_6$-alkyl)- 3-methoxy- estra- 1,3,5 (10)-trien- 17-one:

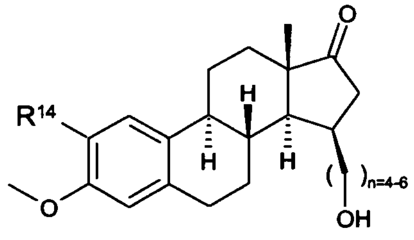

(XXXI-4b)
(XXXI-5b)
(XXXI-6b)

The general synthesis of the alcohol building block of formula XXXI-4/5/6b is depicted in the following scheme 11.

SCHEME 11

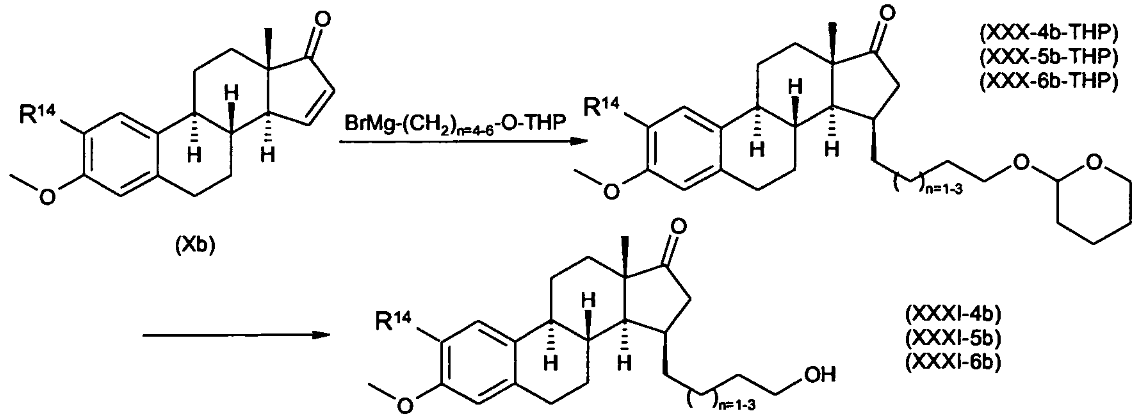


(Xb)

(XXX-4b-THP)
(XXX-5b-THP)
(XXX-6b-THP)

(XXXI-4b)
(XXXI-5b)
(XXXI-6b)

General Procedure

Magnesium (3-10eq) is added in a dry three-neck flask under $N_2$ atmosphere and activated by iodine. The bromo compound (2-6.5 eq) dissolved in dry THF is added dropwise to the magnesium. The reaction mixture is allowed to react for 1-2 h at RT or reflux. The solution is transferred to dry three-neck flask containing CuI (0.06-0.7 eq) and DMPU or HMPA (2-7 eq) in dry THF cooled to -40 °C. The resulting mixture is stirred for 30 min at -40 °C after which a mixture of 15,16-unsaturated estron derivative of formula X (1 eq) and TMSCl (2-2.5 eq) dissolved in THF is added dropwise. After complete addition the mixture is allowed to reach RT. Then $NH_4Cl$-solution is added, the layers are separated and the aqueous phase is extracted with EtOAc (3x). The combined organic phases are dried over $Na_2SO_4$ and the solvent is evaporated. The crude product is dissolved in methanol and $K_2CO_3$ (1eq) is added to hydrolyse the silyl ether. After complete hydrolysis water is added and most of the methanol is evaporated. The mixture is diluted with EtOAc, the layers are separated and the water layer is extracted with EtOAc. The combined organic layers are dried over $Na_2SO_4$ and the solvent is evaporated. The resulting product of general formula XXX is then further worked-up to give the alcohol of general formula XXXI.

Alcohol building block XXXI-4 (n = 4) with PG = Benzyl or H

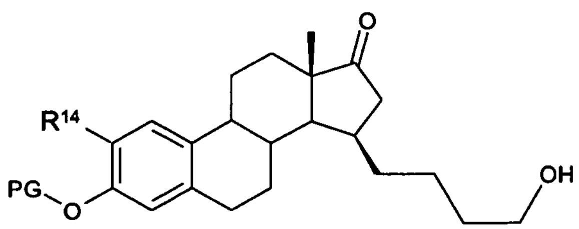

XXXI-4

The detailed synthesis of these compounds is already displayed within the section for the synthesis of acid building block of the formula IV-3c above. The 3-hydroxy-derivative can be obtained by debenzylation of the XXXI-4c compound.

Alcohol building blocks XXXI-6c and XXXI-6a (n = 6):

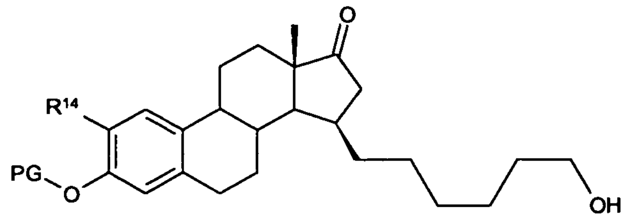

(XXXI-6)

The detailed synthesis of these compounds is performed according to the general procedure displayed in SCHEME 11 starting with the 15,16 unsaturated estron derivative Xc as educt. The 3-hydroxy-derivative can be obtained by hydrolysis of the XXXI-6c compound.

IIId. Optionally 2-substituted compounds of formula IV with substitution of the C17 keto function

3-(17,17-Difluoro-3-hydroxy-estra-1,3,5(10)-trien-15β-yl)-propanoic acid (IVβ-2a-D1F2)

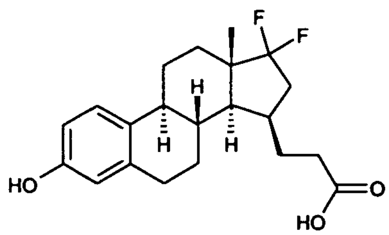

IVβ-2a-D1F2

The individual steps in the synthesis of the acid building block of the formula IVβ-2a-D1F2 are depicted in the following scheme 12.

SCHEME 12

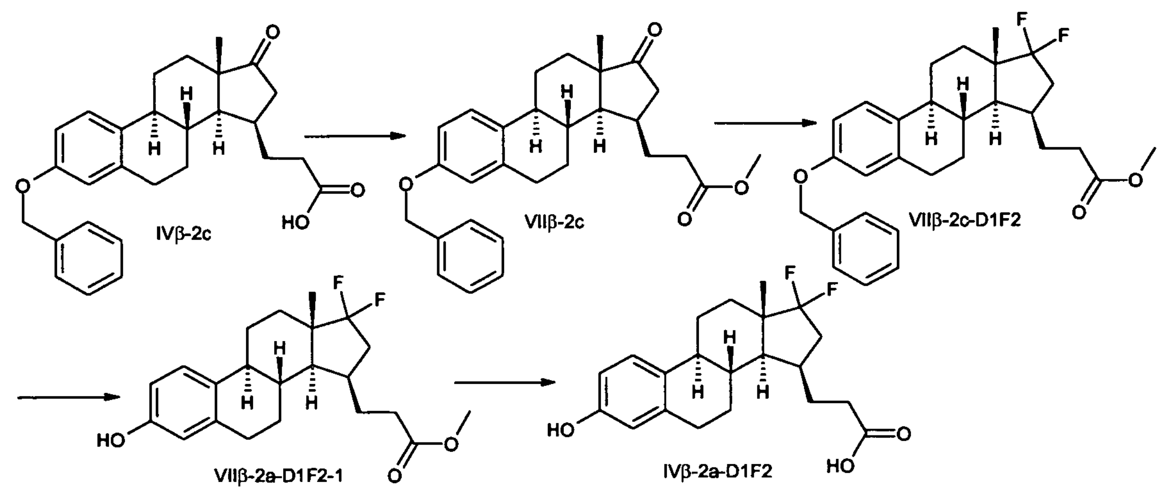

IVβ-2c

VIIβ-2c

VIIβ-2c-D1F2

VIIβ-2a-D1F2-1

IVβ-2a-D1F2

3-(3-Benzyloxy-17-oxo-estra-1,3,5(10)-trien-15β-yl)-propanoic acid of formula IVβ-2c is transformed in the corresponding methyl ester by an esterification reaction as depicted in general flow diagram II using an EDCI coupling. Fluorination of the obtained methyl ester with deoxofluor gave compound VIIβ-2c-D1F2. Subsequent debenzylation, followed by saponification with LiOH afforded the desired building block IVβ-2a-D1F2.

Detailed Synthesis

3-(3-Benzyloxy-17-oxo-estra-1,3,5(10)-trien-15β-yl)-propionic acid methyl ester (VIIβ-2c)

A mixture of compound IVb-2c (38 mmol), $Et_3N$ (117 mmol), MeOH (44 mmol), HOBt (44 mmol) and EDCI (49

mmol) in DCM (650 ml) was stirred overnight. The reaction mixture was washed with 1N HCl and $H_2O$. The organic layer was dried over $Na_2SO_4$ and concentrated *in vacuo* to yield VIIβ-2c (38 mmol, 99%) as orange oil.

3-(3-Benzyloxy-17,17-difluoro-estra-1,3,5(10)-trien-15β-yl)-propionic acid methyl ester

Deoxofluor (50% in toluene, 247 ml, 670 mmol) was added to a solution of VIIβ-2c (27.2 g, 60.9 mmol) in toluene (130 ml). The mixture was stirred for 5 d, during which two times 10 drops of EtOH were added. DCM (200 ml) was added and the mixture was cooled on ice. Saturated $Na\text{-}HCO_3$ (300 mL) was added. The layers were separated and the aqueous layer was extracted with DCM. The combined organic layers were washed with brine, dried over $Na_2SO_4$ and concentrated *in vacuo* to give crude VIIβ-2c-D1F2 (26.5 g). Purification by column chromatography ($SiO_2$, DCM-heptan 2:1 to DCM) gave VIIβ-2c-D1F2 (2.94 g, 6.3 mmol, 10%) as yellow oil.

3-(17,17-Difluoro-3-hydroxy-estra-1,3,5(10)-trien-15β-yl)-propionic acid methyl ester

A mixture of VIIβ-2c-D1 F2 (2.94 g, 6.3 mmol), Pd/C (10%, 440 mg), MeOH (75 mL) and EtOAc (32 mL) was stirred for 2 d under 1 bar $H_2$. After 1 day another portion of Pd/C (484 mg) was added. The mixture was filtered over Celite and the filter cake was washed with MeOH and EtOAc. The filtrate was concentrated *in vacuo* to give 2.1 g of crude VIIβ-2a-D1 F2. Purification by column chromatography ($SiO_2$, DCM) gave VIIβ-2a-D1F2 (1.46 g, 3.9 mmol, 61%) as yellow oil.

3-(17,17-Difluoro-3-hydroxy-estra-1,3,5(10)-trien-15β-yl)-propionic acid (IVβ-2a-D1F2)

A solution of $LiOH{*}H_2O$ (934 mg, 22 mmol) in water (60 mL) was added to a solution of VIIβ-2a-D1 F2 (1.46 g, 3.9 mmol) in THF (60 mL). The mixture was stirred overnight and concentrated in vacuo. Water (250 ml) was added and the mixture was washed with DCM and the pH was adjusted to 1. The aq. layer was extracted with DCM. The combined organic layers were dried over $Na_2SO_4$ and concentrated *in vacuo* to yield IVβ-2a-D1 F2 (1.2 g, 3.3 mmol, 85%) as yellow foam.

$^1$H-NMR-listing: 1.027-1.34 (s, 3H), 1.408-2.421 (m, 15H), 2.837-2.960 (m, 2H), 6.573-6.651 (m, 2H), 7.121-7.257 (d, 1H).

$^{19}$F-NMR-listing: -104- -106 (d, 1F), -115 - -117 (d, 1 F).

4-(17,17-Difluoro-3-hydroxy-estra-1,3,5(10)-trien-15α-yl)-butanoic acid (IVα-3a-D1 F2)

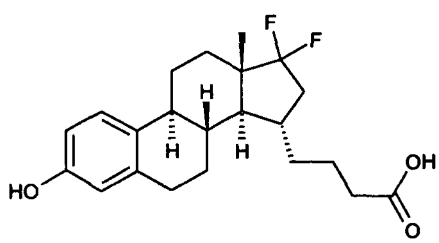

IVα-3a-D1F2

The acid building block of formula IVα-3a-D1F2 was synthesized starting from intermediate compound Xc and using the reaction steps as depicted in SCHEME 8B: The allyl group was introduced into the 15, 16-unsaturated Estrone derivative of formula Xc by reaction with allylmagnesium chloride, followed by an oxy-cope rearrangement catalysed by KH and 18-Crown-6. Subsequently, the resulting compound XXX-2c was reacted with acrylic acid methyl ester using a Grubb II catalyst (olefin metathesis). Then, deviating from SCHEME 8B, the 17-keto function of the resulting 4-(3-Benzyloxy-17-oxo-estra-1,3,5(10)-trien-15a-yl) but-2-enoic acid methyl ester was converted to a bisfluoro group using deoxofluor as described for VIIβ-2c-D1F2. Subsequently, the well known hydrogenation step was performed to obtain the butanoic acid ester side chain, and finally the ester was hydrolysed with LiOH to give the target compound.

$^1$H-NMR-listing: 0.94 (s, 3H), 1.10-2.06 (m, 4H), 2.18-2.55 (m, 14H), 2.74-2.92 (m, 2H), 6.52 (d, 1H), 6.64 (dd, 1H), 7.15 (d, 1H)

$^{19}$F-NMR-listing: -104.5 (dd, 1F), -117.0 (d, 1F).

4-(3-Benzyloxy-17-difluoromethylene-estra-1,3,5(10)-trien-15-yl)butan-1-ol

4-(17-Difluoromethylene-3-hydroxy-estra-1,3,5(10)-trien-15-yl)-butyryl bromide

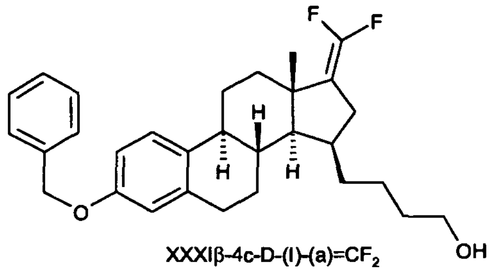
XXXIβ-4c-D-(I)-(a)=$CF_2$

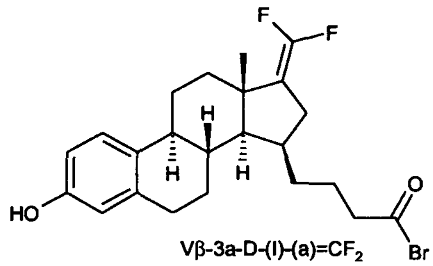
Vβ-3a-D-(I)-(a)=$CF_2$

Synthesis of XXXIβ-4c-D-(I)-(a)=$CF_2$ was achieved in a 3-step reaction starting from intermediate compound XXXIβ-4c using the Horner reaction as described for general synthesis step D-(I)-(a). Subsequently, the fluorinated alcohol derivative XXXI-4c-D-(I)-(a)=$CF_2$ was converted into the free acid by a Jones oxidation, followed by a debenzylation step using $BBr_3$ to deliver the desired intermediate Vβ-3a-D-(I)-(a)=CF2.

Detailed Synthesis:

3-Benzyloxy-15-[4-(*tert*-butyldimethylsilanyloxy)-butyl]-estra-1,3,5(10)-trien-17-one

To a solution of intermediate XXXIβ-4c (108 mg; 0.25 mmol) and imidazole (41.0 mg; 0.60 mmol) in DMF (1 ml) *ter*t-Butyldimethylsilylchloride (0.30 ml; 0.30 mmol; 1M in THF) was added dropwise. After stirring for 16 h at RT, the reaction mixture was poured into $H_2O$ and extracted with DCM. The organic phases were dried over $MgSO_4$. After removal of the solvent, the oily residue was purified by column chromatography ($SiO_2$, DCM) to yield 3-Benzyloxy-15-[4-(*tert*-butyldimethylsilanyloxy)-butyl]-estra-1,3,5(10)-trien-17-one (126 mg, 92%) as colourless oil.

[4-(3-Benzvloxy-17-difluoromethylene-estra-1,3,5(10)-trien-15-yl)-butoxy]-*tert*-butyldimethylsilane

Lithium diisopropylamide (0.60 ml; 1.08 mmol; 1.8 M in THF/Heptane/Ethylbenzene) is added dropwise to a solution of Difluoromethyl-phosphonic acid diethylester (204 mg; 1.08 mmol) in THF (3 ml) at -78˚C and stirred for 30 min. Subsequently, a solution of the ketone obtained in the previous reaction step (148 mg; 0.27 mmol) in THF (4 ml) is added and the mixture is stirred for 30 min and further until the mixture was warmed to RT. The mixture is heated for 5 h under reflux and allowed to cool to RT. After addition of water, the solution is extracted with DCM. The combined organic phases are dried over $MgSO_4$. After removal of the solvent, the residue is purified by column chromatography ($SiO_2$, DCM/Hexane 1:2) to yield [4-(3-Benzyloxy-17-difluoromethylene-estra-1,3,5(10)-trien-15-yl)-butoxy]-tert-butyldimethylsilane (107 mg, 68 %) as colorless oil.

4-(3-Benzyloxy-17-difluoromethylene-estra-1,3,5(10)-trien-15-yl)-butan-1-ol XXXIβ-4c-D-(I)-(a)=$CF_2$

A solution of the obtained TBDMS-Ether (97.0 mg; 167 μmol) in TBAF (1 ml; 1 mmol; 1 M in THF) was stirred for 4 h at RT. The reaction mixture is poured into $H_2O$ and extracted with DCM. The combined organic phases are dried over $MgSO_4$. After removal of the solvent, the residue is purified by column chromatography ($SiO_2$, DCM) to yield XXXIβ-4c-D-(I)-(a)=$CF_2$ (76.0 mg, 98 %) as yellow solid.

4-(3-Benzyloxy-17-difluoromethylene-estra-1,3,5(10)-trien-15-yl)-butyric acid IVβ-3c-D-(I)-(a)=$CF_2$

After dissolving the alcohol XXXIβ-4c-D-(I)-(a)=CF2 in 10 ml acetone, Jones reagent (1g $CrO_3$, 7 ml $H_2O$, 3 ml 100% $H_2SO_4$) was added at 0˚C up to the point the solution remained reddish. After stirring for 10 min, any excess of Jones reagent was destroyed by adding isopropanol. After filtration over silicagel, the filtrate was evaporated to dryness. The residue was diluted with DCM and washed several times with water, dried over $Na_2SO_4$ and again evaporated to dryness. The crude product was used further without any purification.

4-(17-Difluoromethylene-3-hydroxy-estra-1,3,5(10)-trien-15-yl)-butyryl bromide Vβ-3a-D-(I)-(a)=$CF_2$

The crude benzylated estrone acid derivative IVβ-3c-D-(I)-(a)=$CF_2$ was dissolved in 10 ml dry DCM and few drops $BBr_3$ were added at ambient temperature. The reaction mixture was stirred for 1 h and directly used in further reaction steps without any work-up.

4-(3-Benzyloxy-17-trifluoromethyl-estra-1,3,5(10), 16-tetraen-15-yl)-butan-1-ol

4-(3-Hydroxy-17-trifluoromethyl-estra-1,3,5(10),16-tetraen-15-yl)- butyryl bromide

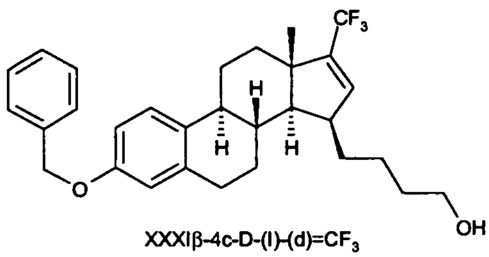

XXXIβ-4c-D-(I)-(d)=$CF_3$

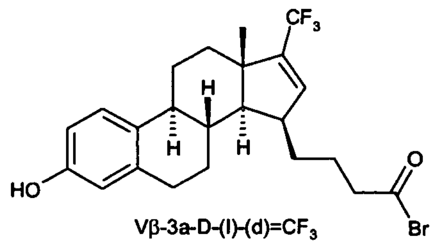

Vβ-3a-D-(I)-(d)=$CF_3$

Synthesis of XXXIβ-4c-D-(I)-(d)-$CF_3$ was achieved in a 4-step reaction starting from intermediate compound XXXIβ-4c as described for general synthesis step D-(I)-(d). Subsequently, the fluorinated alcohol derivative XXXI-4c-D-(I)-(d)=$CF_3$ was converted into the free acid by a Jones oxidation, followed by a debenzylation step using $BBr_3$ to deliver the desired intermediate Vβ-3a-D-(I)-(d)-$CF_3$.

3-Benzyloxy-15-(4-hydroxybutyl)-17-trifluoromethyl-estra-1,3.5(10)-trien-17-ol

(Trifluoromethyl)trimethylsilan (7.60 ml; 15.0 mmol; 2M in THF) was added to a solution of intermediate XXXIβ-4c (1.08 g; 2.50 mmol) in THF precooled to 0˚C. After addition of TBAF (60.0 mg; 0.63 mmol), the reaction mixture was stirred for 0.5 h at 0˚C and for 16 h at RT. Water was added and the resulting the solution was extracted with ether. The combined organic phases were dried over $MgSO_4$. After removal of the solvent, the residue was dissolved in TBAF solution (10.0 ml; 10.0 mmol; 1 M in THF) and the resulting mixture was stirred for 4 h at RT. Water was added and the resulting the solution was extracted with ether. The combined organic phases were dried over $MgSO_4$. The remaining oil was purified by column chromatography ($SiO_2$; Ether/DCM 1:1) yielding the title compound (942 mg, 75%) as colourless oil.

2,2-Dimethylpropionic acid 4-(3-benzyloxy-17-hydroxy-17-trifluoromethyl-estra-1,3,5(10-trien-15-yl)-butylester

Pivaloylchloride (0.50 ml; 4.10 mmol) was added dropwise to a solution of 3-Benzyloxy-15-(4-hydroxybutyl)-17-trifluoromethyl-estra-1,3,5(10)-trien-17-ol (1.65 g; 3.28 mmol) in pyridine (15 ml) at 0˚C. After stirring for 2 h, the reaction mixture was poured into ice water and stirred for another h. After extraction with DCM, the combined organic phases were dried over $MgSO_4$. After evaporation of the solvent, the residue was purified by column chromatography ($SiO_2$, DCM) yielding the title compound (1.85 g, 96%) as colourless oil.

2,2-Dimethylpropionic acid 4-(3-benzyloxy-17-trifluoromethyl-estra-1,3,5(10),16-tetraen-15-yl)-butylester

Phosphorylchloride (0.25 ml; 200 μmol) was added to a solution of 2,2-Dimethylpropionic acid 4-(3-benzyloxy-17-hydroxy-17-trifluoromethyl-estra-1,3,5(10)-trien-15-yl)-butylester (80.0 mg; 136 μmol) in pyridine (2.50 ml) and the resulting mixture is heated under reflux for 24h. The mixture was allowed to cool to RT, diluted with ice water and extracted with ether. The combined organic phases were dried over $MgSO_4$ and the solvent was evaporated. The residue was purified by column chromatography ($SiO_2$, DCM) yielding the title compound (60.0 mg, 78%) as colourless oil.

4-(3-Benzyloxy-17-trifluoromethyl-estra-1,3,5(10),16-tetraen-15-yl)-butan-1-ol XXXIβ-4c-D-(I)-d-$CF_3$,

DIBAH (1.00 ml; 1.00 mmol; 1M in THF) was added dorpwise to a solution of the pivaloate (60.0 mg; 106 μmol) in DCM (5 ml) at -78˚C. After stirring for 6h, 1 N HCl (20 ml) was added and the reaction mixture was extracted with

DCM. The combined organic phases were dried over $MgSO_4$ and the solvent was evaporated. The residue was purified by column chromatography ($SiO_2$, DCM) yielding XXXIβ-4c-D-(I)-d-$CF_3$ (46.0 mg, 90) as colourless oil.

4-(3-Benzyloxy-17-trifluoromethyl-estra-1,3,5(10),16-tetraen-15-yl)-butyric acid IVβ-3c-D(I)-(d)-$CF_3$

After dissolving 180 mg of the alcohol XXXIβ-4c-D-(I)-(d)-$CF_3$ in 10 ml acetone, Jones reagent (1g $CrO_3$, 7 ml $H_2O$, 3 ml 100% $H_2SO_4$) was added at 0°C up to the point the solution remained reddish. After stirring for 10 min, any excess of Jones reagent was destroyed by adding isopropanol. After filtration over silicagel, the filtrate was evaporated to dryness. The residue was diluted with DCM and washed several times with water, dried over $Na_2SO_4$ and again evaporated to dryness. The crude product was used further without any purification.

4-(3-Hydroxy-17-trifluoromethyl-estra-1,3,5(10)-trien-15-yl)-butyryl bromide Vβ-3a-D-(I)-(d)-$CF_3$

The crude benzylated estrone acid derivative IVβ-3c-D-(I)-(d)-$CF_3$ was dissolved in 10 ml dry DCM and few drops BBr3 were added at ambient temperature. The reaction mixture was stirred for 1 h and directly used in further reaction steps without any work-up.

IIIe. Optionally 2-substituted compounds of formula XV (protected amine building block) (n = 1 - 6)

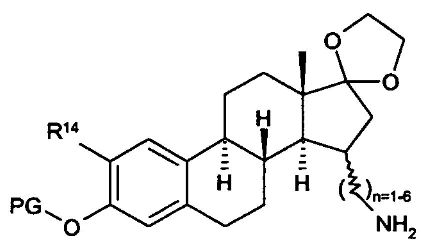

(XV)

Protected amine building block XV-1 (n = 1):

The individual steps in the synthesis of amine building block of the formula XV-1 are depicted in the following scheme 13.

SCHEME 13

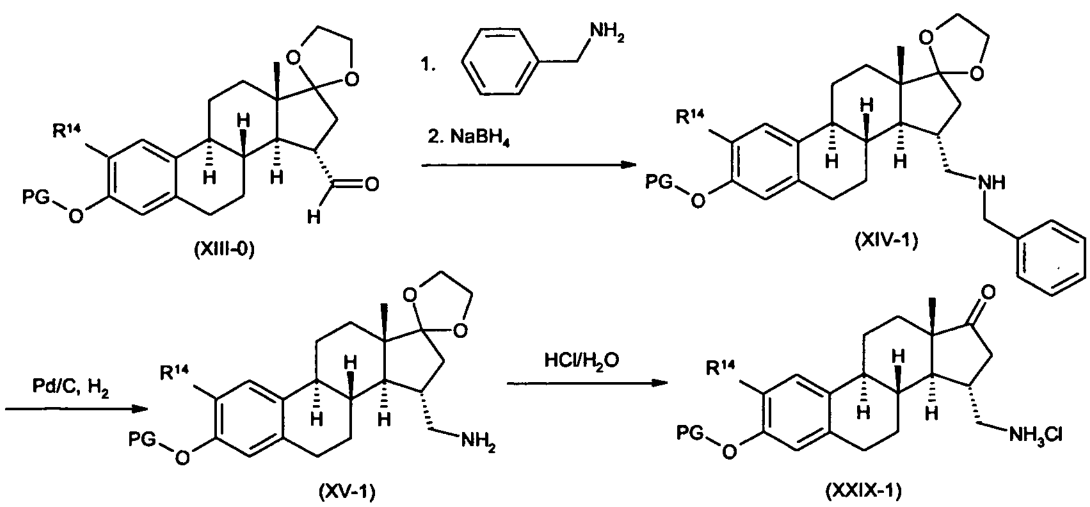


Dissolving aldehydes XIII-0b (PG=$CH_3$) or XIII-0c (PG=benzyl) in benzylamine and reduction of the residual imine in THF gave benzylamine XIV-1b (PG=$CH_3$) and XIV-1c (PG=benzyl), which were debenzylated to XV-1b (PG=$CH_3$) and XV-1a (PG=H), using Pd/C and $H_2$ at 5 bar, and dissolved in dilute HCl to give the respective ammonium chlorides XXIX-1b (PG=$CH_3$) and XXIX-1 a (PG=H). Standard purification methods failed due to what seems to be

instability of these ammonium salts. For these amines it was known that these should be treated as HCl salts since the free amine is not stable (ene-amines), but even the salts seem to be at least heat-sensitive. The crude reaction mixture has a purity of ~90% (HPLC-MS).

Amine building block XVα-3: (n = 3):

The individual steps in the synthesis of amine building block of the formula XVα-3 are depicted in the following scheme 16.

**SCHEME 16:**

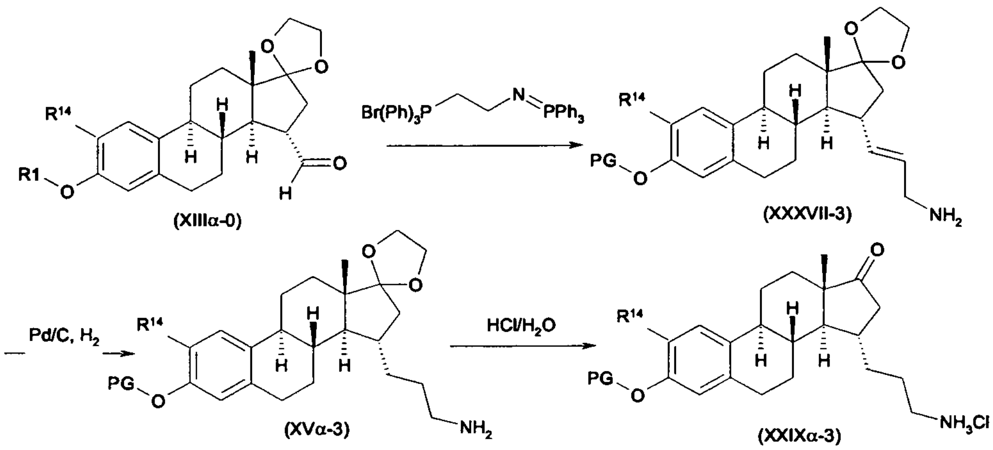


(XIIIα-0) (XXXVII-3) (XVα-3) (XXIXα-3)

The protected aldehyde derivative of formula (XIIIα-0) is converted into the corresponding aminopropenyl by a Wittig reaction (see also SCHEME 4). The aminopropenyl (XXXVII-3) is subsequently reduced to the 15-aminopropyl derivative of formula XVα-3. The protecting ketal group is converted into the 17-oxo group via acid hydrolysis.

The same kind of procedure can be applied using different Wittig reagents of the general formula Hal $(Ph)_3P-(CH_2)_{n=3-5}-R^*$ in order to obtain amine building blocks with longer side chains (i.e. n = 4, 5, or 6), wherein R* for example represents $-N=P(Ph)_3$, $-N_3$, or $-NH-CO-O-CH_3$.

Amine building block XVα-4: (n = 4):

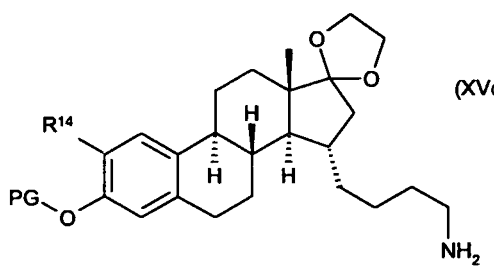

(XVα-4)

Furthermore, the amine building block XVα-4b was synthesized corresponding to SCHEME 16 using Hal $Ph_3P-CH_2-CH_2-CH_2-N_3$ as Wittig reagent. (LC-MS (ES+): rt 4.57 min, m/z (rel. Intens) 386 [(M+H)+, 100%])

Amine building block XVβ-4: (n = 4):

The individual steps in the synthesis of amine building block of the formula XVβ-4 with β configuration at the C15 atom of the steroidal core are depicted in the following scheme 17.

## SCHEME 17

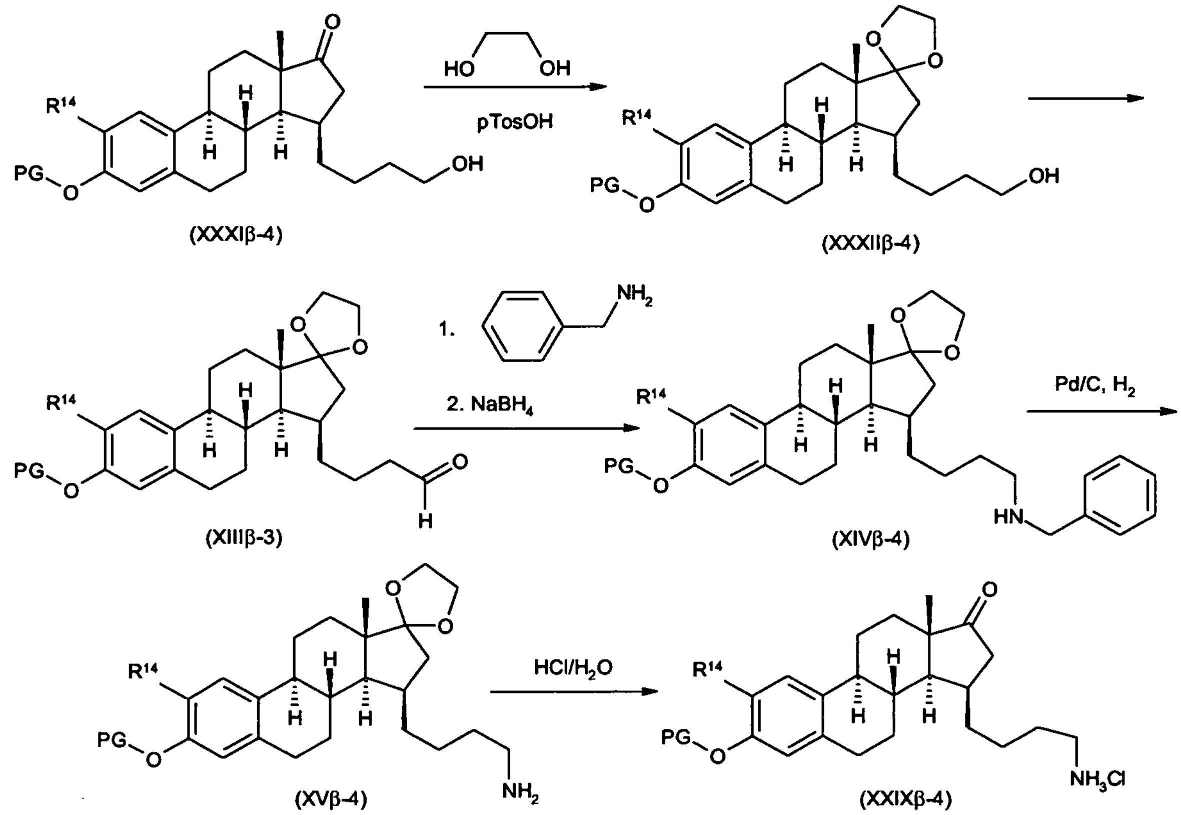


(XXXIβ-4) (XXXIIβ-4) (XIIIβ-3) (XIVβ-4) (XVβ-4) (XXIXβ-4)

In a first step, the 17 oxo function of the butanol derivative of the formula XXXIβ-4 (for synthesis of XXXIβ-4 see above) is converted into the ketal group (compound of formula XXXIIβ-4). Then, the alcohol function is selectively reduced to the aldehyde giving compound of the formula XIIβ-3. The protected aldehyde derivative of the formula XIIIβ-3 is converted into a secondary amine by addition of Benzylamine and subsequent reduction (reductive amination). Further reduction of the secondary amine delivers the desired, still protected amine building block of the formula XVβ-4. The protecting ketal group can be converted into the 17-oxo group via acid hydrolysis.

The same kind of procedure can be applied for n = 5 or 6 and for other substituents within the $R^1$ position.

Amine building block (n = 1 - 6) of general formula XXIX

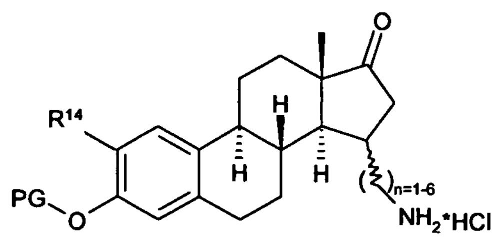

(XXIX)

Alternatively, the synthesis of the amine building blocks of general formula XXIX can also be performed starting with an activated alcohol function and a subsequence substitution reaction, and does not need any protection of the Estron-C17 keto function according to the following general scheme 18.

## SCHEME 18

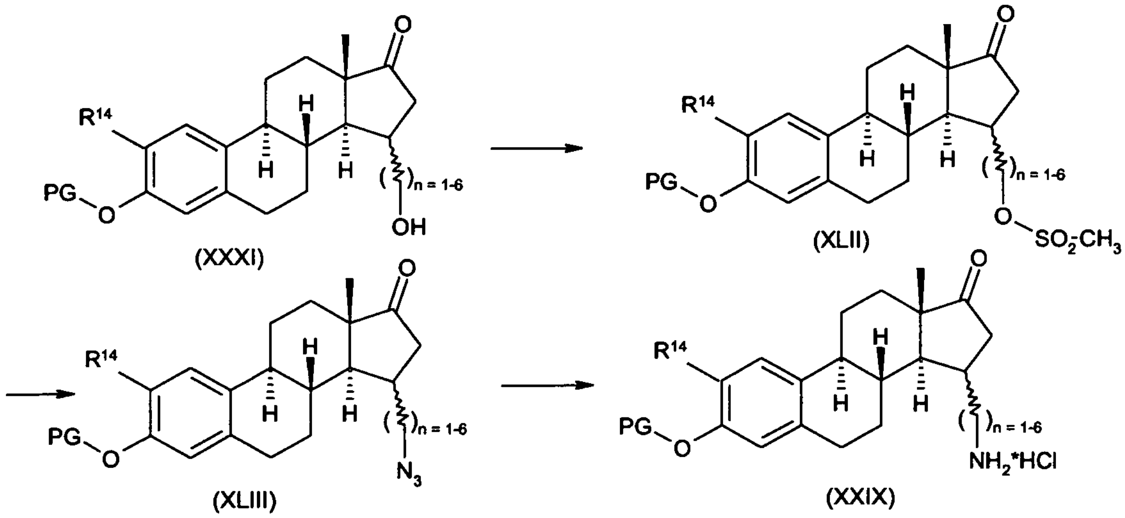
(XXXI)
(XLII)
(XLIII)
(XXIX)

Step C - Synthesis of Intermediates of general formula C-(I) with $R^{14}$ = H

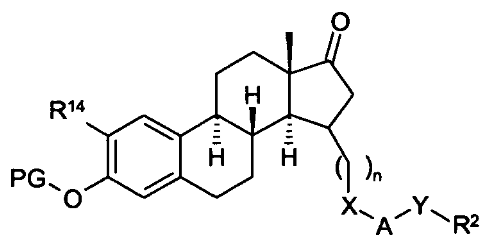
C-(I)

The synthesis of the intermediates falling under the general formula C-(I), wherein $R^{14}$ represents H, is fully disclosed in international patent application WO 2005/047303, and was performed according to the reaction schemes depicted in general flow diagrams I to XV herewithin. The following table gives an overview of exemplary intermediates prepared. The number given in the first column corresponds to the compound number as disclosed in international patent application WO 2005/047303.

Table 1: Exemplary intermediates of the general formula VI (amide derivatives)

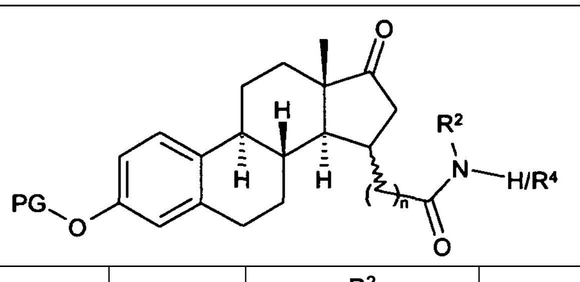
C-(VI)

| No. | n | C15 stereo | PG or $R^1$ | $R^2$ | $R^4$ | MS m/z | HPLC Rt [min] |
|---|---|---|---|---|---|---|---|
| | | | | 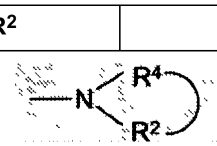 | | | |
| **1** | 3 | beta | -H | morpholin-4-yl | | | |
| **2** | 3 | beta | $-CH_3$ | morpholin-4-yl | | | |

(continued)

| No. | n | C15 stereo | PG or $R^1$ | $R^2$ | $R^4$ | MS m/z | HPLC Rt [min] |
|---|---|---|---|---|---|---|---|
| | | | | 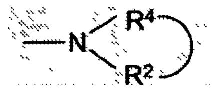 | | | |
| **4B** | 3 | beta | $-CH_3$ | methyl | H | | |
| **4C** | 3 | beta | benzyl | methyl | H | | |
| **5** | 3 | beta | $-CH_3$ | Cyclopropyl | H | 409.3 | 5.89 |
| **7** | 3 | beta | $-CH_3$ | Furan-2-yl | H | 449.3 | 5.74 |
| **9** | 3 | beta | $-CH_3$ | 2-Morpholin-4-yl-ethyl | H | 482.2 | 5.05 |
| **12** | 3 | beta | $-CH_3$ | 1-Benzyl-piperidin-4-yl | H | 542.3 | 5.24 |
| **13** | 3 | beta | $-CH_3$ | Quinolin-3-yl | H | 496.3 | 5.98 |
| **15** | 3 | beta | $-CH_3$ | 3,4-Dichloro-benzyl | H | 527.2 | 6.35 |
| **16** | 3 | beta | $-CH_3$ | 3,4-Dimethoxy-benzyl | H | 519.3 | 5.66 |
| **17** | 3 | beta | $-CH_3$ | 2-Hydroxy-2-phenyl-ethyl | H | 489.3 | 5.60 |
| **18** | 3 | beta | $-CH_3$ | 2-Dimethylamino-ethyl | H | 440.3 | 4.71 |
| **19** | 3 | beta | $-CH_3$ | 2-(2-Hydroxy-ethoxy)-ethyl | H | 457.3 | 5.04 |
| **21** | 3 | beta | $-CH_3$ | 2-(3,4-Dimethoxy-phenyl)-ethyl | H | 533.3 | 5.79 |
| **23** | 3 | beta | $-CH_3$ | 3-Imidazol-1-yl-propyl | H | 477.3 | 4.95 |
| **24** | 3 | beta | $-CH_3$ | 1H-Benzoimidazol-2-yl-methyl | H | 499.3 | 5.39 |
| **25** | 3 | beta | $-CH_3$ | 4-Hydroxy-3-methoxy-benzyl | H | 505.3 | 5.49 |
| **26** | 3 | beta | $-CH_3$ | Carbamoyl-methyl | H | 426.2 | 6.30 |
| **28** | 3 | beta | $-CH_3$ | 2-(4-Sulfamoyl-phenyl)-ethyl | H | 552.3 | 5.37 |
| **30** | 3 | beta | $-CH_3$ | 4-Trifluoromethoxy-benzyl | H | 543.3 | 6.35 |
| **32** | 3 | beta | $-CH_3$ | 4-Fluoro-3-trifluoromethyl-benzyl | H | 545.3 | 6.30 |
| **33** | 3 | beta | $-CH_3$ | 2-Oxo-tetrahydro-furan-3-yl | H | 453.3 | 5.32 |
| **34** | 3 | beta | $-CH_3$ | 2-Oxo-azepan-3-yl | H | 480.3 | 5.33 |

(continued)

| No. | n | C15 stereo | PG or $R^1$ | $R^2$ | $R^4$ | MS m/z | HPLC Rt [min] |
|---|---|---|---|---|---|---|---|
| | | | | 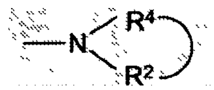 | | | |
| **35** | 3 | beta | $-CH_3$ | 4-Hydroxy-cyclohexyl | H | 467.3 | 5.18 |
| **36** | 3 | beta | H | 2-(7-Methyl-1H-indol-3-yl)-ethyl | H | | |
| **37** | 3 | beta | H | 2,4-Difluoro-benzyl | H | | |
| **38** | 3 | beta | H | Benzyl | methyl | | |
| **39** | 3 | alpha | H | Benzyl | H | | |
| **40** | 3 | alpha | H | Morpholin-4-yl | | | |
| **42** | 0 | alpha | $-CH_3$ | 2-(1H-Indol-3-yl)-ethyl | H | 470.3 | 1.92 |
| **44** | 0 | alpha | $-CH_3$ | 1-Benzyl-pyrrolidin-3-yl | H | 486.3 | 1.48 |
| **47** | 0 | alpha | $-CH_3$ | Phenethyl | H | 431.2 | 1.95 |
| **49** | 0 | alpha | $-CH_3$ | Cyclopropylmethyl | H | 381.2 | 1.83 |
| **50** | 0 | alpha | $-CH_3$ | Cyclohexylmethyl | H | 423.3 | 2.06 |
| **51** | 0 | alpha | $-CH_3$ | 2,2-Diphenyl-ethyl | H | 507.3 | 2.12 |
| **54** | 0 | alpha | $-CH_3$ | 3,3-Diphenyl-propyl | H | 521.3 | 2.14 |
| **56** | 0 | alpha | $-CH_3$ | 2-Pyridin-2-yl-ethyl | H | 432.2 | 1.46 |
| **60** | 0 | alpha | $-CH_3$ | 4-Methoxy-benzyl | H | 447.2 | 1.89 |
| **63** | 0 | alpha | $-CH_3$ | sec-butyl | H | 383.2 | 1.87 |
| **66** | 0 | alpha | $-CH_3$ | Bicyclo[2.2.1]hept-2-yl | H | 421.3 | 2.02 |
| **71** | 0 | alpha | $-CH_3$ | Indan-2-yl | H | 443.2 | 1.97 |
| **73** | 0 | alpha | $-CH_3$ | 2-Hydroxy-ethyl | H | 371.2 | 1.55 |
| **77** | 0 | alpha | $-CH_3$ | 3-Morpholin-4-yl-propyl | H | 454.3 | 1.37 |
| **79** | 0 | alpha | $-CH_3$ | 4-Phenyl-butyl | H | 459.3 | 2.08 |
| **80** | 0 | alpha | $-CH_3$ | $-(CH_2)_3-CO-O-CH_3$ / (butyric acid methyl ester)-4-yl | H | 427.2 | 1.74 |
| **81** | 0 | alpha | $-CH_3$ | 1-Oxo-1-benzoxy-propan-2-yl / (Propionic acid benzyl ester)-2-yl | H | 489.3 | 1.99 |
| **83** | 0 | alpha | $-CH_3$ | 1,2,3,4-Tetrahydro-naphthalen-1-yl | H | 457.3 | 2.08 |
| **84** | 0 | alpha | $-CH_3$ | 2-Fluoro-benzyl | H | 435.2 | 1.92 |

(continued)

| No. | n | C15 stereo | PG or $R^1$ | $R^2$ | $R^4$ | MS m/z | HPLC Rt [min] |
|---|---|---|---|---|---|---|---|
| | | | | 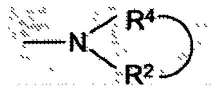 | | | |
| **85** | 0 | alpha | $-CH_3$ | 3-Hydroxy-propyl | H | 385.2 | 1.58 |
| **89** | 0 | alpha | $-CH_3$ | 2-Phenyl-propyl | H | 445.3 | 2.02 |
| **97** | 0 | alpha | $-CH_3$ | Thiophen-2-yl-methyl | H | 423.2 | 1.91 |
| **103** | 0 | alpha | $-CH_3$ | 1-Hydroxymethyl-cyclopentyl | H | 425.3 | 1.81 |
| **105** | 0 | alpha | $-CH_3$ | 5-Methyl-thiazol-2-yl | H | 424.2 | 1.95 |
| **107** | 0 | alpha | $-CH_3$ | 4-Benzyl-piperidin-1-yl | | 485.3 | 2.22 |
| **109** | 0 | alpha | $-CH_3$ | 3,4-Dihydro-1H-isoquinolin-2-yl | | 443.2 | 2.05 |
| **111** | 0 | alpha | $-CH_3$ | 4-Pyridin-2-yl-piperazin-1-yl | | 473.3 | 1.63 |
| **117** | 0 | alpha | $-CH_3$ | 4-(4-Chloro-benzyl)-piperazin-1-yl | | 520.2 | 1.67 |
| **118** | 0 | alpha | $-CH_3$ | 4-(3-Chloro-phenyl)-piperazin-1-yl | | 506.2 | 2.16 |
| **120** | 0 | alpha | $-CH_3$ | 4-[2-(2-Hydroxy-ethoxy)-ethyl]-piperazin-1-yl | | 484.3 | 1.39 |
| **121** | 0 | alpha | $-CH_3$ | 4-(4-Chloro-phenyl)-piperazin-1-yl | | 506.2 | 2.16 |
| **124** | 0 | alpha | $-CH_3$ | 1,3,4,9-Tetrahydro-beta-carbolin-2-yl | | 482.3 | 2.04 |
| **125** | 0 | alpha | $-CH_3$ | 4-Hydroxy-4-phenyl-piperidin-1-yl | | 487.3 | 1.90 |
| **126** | 0 | alpha | $-CH_3$ | 4-(2-Chloro-phenyl)-piperazin-1-yl | | 506.2 | 2.20 |
| **127** | 0 | alpha | $-CH_3$ | 4-(4-Methoxy-phenyl)-pipernzin-1-yl | | 502.3 | 2.01 |
| **128** | 0 | alpha | $-CH_3$ | 1-Piperidine-3-carboxylic acid amide / 3-(carboxylic acid amide)-piperidin-1-yl | | 438.3 | 1.63 |
| **130** | 0 | alpha | $-CH_3$ | 4-Methyl-piperazin-1-yl | | 410.3 | 1.40 |
| **132** | 0 | alpha | $-CH_3$ | 2-Methoxymethyl-pyrrolidin-1-yl | | 425.3 | 1.93 |
| **133** | 0 | alpha | $-CH_3$ | 4-(2-Fluoro-phenyl)-piperazin-1-yl | | 490.3 | 2.10 |
| **138** | 0 | alpha | $-CH_3$ | 3,6-Dihydro-2H-pyridin-1-yl | | 393.2 | 1.92 |
| **140** | 0 | alpha | $-CH_3$ | 1-Pyrrolidine 2-carboxylic acid amide / 2-(carboxylic acid amide)-pyrrolidin-1-yl | | 424.2 | 1.60 |
| **145** | 0 | alpha | $-CH_3$ | 4-Pyrrolidin-1-yl-piperidin-1-yl | | 464.3 | 1.43 |
| **147** | 0 | alpha | $-CH_3$ | Azetidin-1-yl | | 367.2 | 1.74 |
| **150** | 0 | alpha | $-CH_3$ | Propyl | cyclopropylmethyl | 423.3 | 2.13 |
| **151** | 0 | alpha | $-CH_3$ | 2-Cyano-ethyl | pyridin-3-ylmethyl | 471.3 | 1.67 |
| **154** | 0 | alpha | $-CH_3$ | Benzyl | 2-dimethylnmino-ethyl | 488.3 | 1.56 |
| **156** | 0 | alpha | $-CH_3$ | 2-Methoxy-ethyl | 2-Methoxy-ethyl | 443.3 | 1.90 |
| **157** | 0 | alpha | $-CH_3$ | Methyl | 1-methyl-piperidin-4-yl | 438.3 | 1.43 |
| **161** | 0 | alpha | $-CH_3$ | Propyl | propyl | 411.3 | 2.11 |

(continued)

| No. | n | C15 stereo | PG or $R^1$ | $R^2$ | $R^4$ | MS m/z | HPLC Rt [min] |
|---|---|---|---|---|---|---|---|
| | | | | 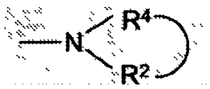 | | | |
| 162 | 0 | alpha | $-CH_3$ | Methyl | 2-dimethylamino-ethyl | 412.3 | 1.42 |
| 163 | 0 | alpha | $-CH_3$ | Methyl | phenethyl | 445.3 | 2.08 |
| 164 | 0 | alpha | $-CH_3$ | Methyl | allyl | 381.2 | 1.92 |
| 165 | 0 | alpha | $-CH_3$ | Ethyl | pyridin-4-yl-methyl | 446.3 | 1.61 |
| 166 | 0 | alpha | $-CH_3$ | Methyl | methyl | 355.2 | 1.78 |
| 168 | 1 | alpha | $-CH_3$ | Diphenyl-methyl | H | 507.3 | 2.14 |
| 171 | 1 | alpha | $-CH_3$ | Naphthalen-1-ylmethyl | H | 481.3 | 2.10 |
| 183 | 1 | alpha | $-CH_3$ | 2-Piperidin-1-yl-ethyl | H | 452.3 | 1.47 |
| 189 | 1 | alpha | $-CH_3$ | Cyclopentyl | H | 409.3 | 1.95 |
| 191 | 1 | alpha | $-CH_3$ | 3-Phenyl-propyl | H | 459.3 | 2.05 |
| 195 | 1 | alpha | $-CH_3$ | 1-Ethyl-propyl | H | 411.3 | 1.99 |
| 197 | 1 | alpha | $-CH_3$ | 2-Methoxy-ethyl | H | 399.2 | 1.74 |
| 198 | 1 | alpha | $-CH_3$ | 2-Pyrrolidin-1-yl-ethyl | H | 438.3 | 1.46 |
| 199 | 1 | alpha | $-CH_3$ | 2-(5-Methoxy-1H-indol-3-yl)-ethyl | H | 514.3 | 1.91 |
| 203 | 1 | alpha | $-CH_3$ | 1-Phenyl-ethyl | H | 445.3 | 2.02 |
| 204 | 1 | alpha | $-CH_3$ | 1,2-Diphenyl-ethyl | H | 521.3 | 2.17 |
| 206 | 1 | alpha | $-CH_3$ | 2,6-Dimethoxy-benzyl | H | 491.3 | 2.00 |
| 207 | 1 | alpha | $-CH_3$ | 4-Fluoro-benzyl | H | 449.2 | 1.98 |
| 208 | 1 | alpha | $-CH_3$ | 3,5-Dimethoxy-benzyl | H | 491.3 | 1.95 |
| 209 | 1 | alpha | $-CH_3$ | 2-Phenoxy-ethyl | H | 461.3 | 1.99 |
| 211 | 1 | alpha | $-CH_3$ | 1-Naphthalen-1-yl-ethyl | H | 495.3 | 2.13 |
| 219 | 1 | alpha | $-CH_3$ | 2,4-Difluoro-benzyl | H | 467.2 | 2.00 |
| 222 | 1 | alpha | $-CH_3$ | Isobutyl | H | 397.3 | 1.94 |
| 224 | 1 | alpha | $-CH_3$ | 2-Cyclohex-1-enyl-ethyl | H | 449.3 | 2.18 |
| 225 | 1 | alpha | $-CH_3$ | 2-Hydroxy-1-methyl-ethyl | H | 399.2 | 1.65 |
| 226 | 1 | alpha | $-CH_3$ | 2-Methylsulfanyl-ethyl | H | 415.2 | 1.85 |

(continued)

| No. | n | C15 stereo | PG or $R^1$ | $R^2$ | $R^4$ | MS m/z | HPLC Rt [min] |
|---|---|---|---|---|---|---|---|
| | | | | 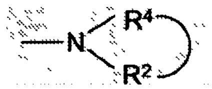 | | | |
| **227** | 1 | alpha | $-CH_3$ | 3,4,5-Trimethoxy-benzyl | H | 521.3 | 1.90 |
| **229** | 1 | alpha | $-CH_3$ | 2-Hydroxy-cyclohexyl | H | 439.3 | 1.76 |
| **233** | 1 | alpha | $-CH_3$ | 3-Diethylamino-propyl | H | 454.3 | 1.49 |
| **234** | 1 | alpha | $-CH_3$ | Hexyl | H | 425.3 | 2.11 |
| **235** | 1 | alpha | $-CH_3$ | 3,4-Difluoro-benzyl | H | 467.2 | 1.99 |
| **236** | 1 | alpha | $-CH_3$ | 2-Trifluoromethyl-benzyl | H | 499.2 | 2.05 |
| **238** | 1 | alpha | $-CH_3$ | (3-Methyl-butyric acid methyl ester)-2-yl / 2-(3-methyl)-butyric acid methyl ester | H | 455.3 | 1.92 |
| **239** | 1 | alpha | $-CH_3$ | 5-Methyl-thiazol-2-yl | H | 438.2 | 1.95 |
| **240** | 1 | alpha | $-CH_3$ | Cyclobutyl | H | 395.2 | 1.85 |
| **241** | 1 | alpha | $-CH_3$ | 4-Benzyl-piperazin-1-yl | | 500.3 | 1.56 |
| **243** | 1 | alpha | $-CH_3$ | 4-Benzo[1,3]dioxol-5-ylmethyl-piperazin-1-yl | | 544.3 | 1.54 |
| **244** | 1 | alpha | $-CH_3$ | 4-(2-oxo-1,3-dihydro-benzoimidazol-1-yl)-piperidin-1-yl | | 541.3 | 1.83 |
| **246** | 1 | alpha | $-CH_3$ | 2,5-Dihydro-pyrrol-1-yl | | 393.2 | 1.89 |
| **247** | 1 | alpha | $-CH_3$ | 4-Phenyl-piperazin-1-yl | | 486.3 | 2.11 |
| **249** | 1 | alpha | $-CH_3$ | Pyrrolidin-1-yl | | 395.2 | 1.89 |
| **250** | 1 | alpha | $-CH_3$ | 4-(4-Fluoro-phenyl)-piperazin-1-yl | | 504.3 | 2.10 |
| **251** | 1 | alpha | $-CH_3$ | 4-(2-Methoxy-phenyl)-piperazin-1-yl | | 516.3 | 2.08 |
| **252** | 1 | alpha | $-CH_3$ | 4-(4-Chloro-phenyl)-4-hydroxy-piperidin-1-yl | | 535.2 | 2.04 |
| **253** | 1 | alpha | $-CH_3$ | 4-(4-trifluoromethyl-phenyl)-piperazin-1-yl | | 554.3 | 2.23 |
| **256** | 1 | alpha | $-CH_3$ | 4-Methyl-[1,4]diazepan-1-yl | | 438.3 | 1.43 |
| **259** | 1 | alpha | $-CH_3$ | 1,4-Dioxa-8-aza-spiro[4.5]decan-8-yl | | 467.3 | 1.89 |
| **260** | 1 | alpha | $-CH_3$ | 1-piperidine-4-carboxylic acid ethyl ester / 4-(carboxylic acid ethyl ester)-piperidin-1-yl | | 481.3 | 1.98 |
| **266** | 1 | alpha | $-CH_3$ | Azepan-1-yl | | 423.3 | 2.08 |
| **268** | 1 | alpha | $-CH_3$ | 4-(3-Trifluoromethyl-phenyl)-piperazin-1-yl | | 554.3 | 2.23 |

(continued)

| No. | n | C15 stereo | PG or $R^1$ | $R^2$ | $R^4$ | MS m/z | HPLC Rt [min] |
|---|---|---|---|---|---|---|---|
| | | | | 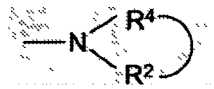 | | | |
| **269** | 1 | alpha | $-CH_3$ | 3-Hydroxy-pyrrolidin-1-yl | | 411.2 | 1.62 |
| **271** | 1 | alpha | $-CH_3$ | 4-Oxo-1-phenyl-1,3,8-triaza-spiro[4.5] decan-8-yl | | 555.3 | 1.87 |
| **273** | 1 | alpha | $-CH_3$ | 4-pyridin-4-yl-piperazin-1-yl | | 487.3 | 1.46 |
| **274** | 1 | alpha | $-CH_3$ | 4-Hydroxy-piperidin-1-yl | | 425.3 | 1.64 |
| **275** | 1 | alpha | $-CH_3$ | octahydro-quinolin-1-yl | | 463.3 | 2.29 |
| **276** | 1 | alpha | $-CH_3$ | 3-Hydroxy-piperidin-1-yl | | 425.3 | 1.70 |
| **279** | 1 | alpha | $-CH_3$ | 1-pyrrolidine-2-carboxylic acid methyl ester / 2-(carboxylic acid methyl ester)-pyrrolidin-1-yl | | 453.3 | 1.86 |
| **281** | 1 | alpha | $-CH_3$ | 2-Hydroxymethyl-pyrrolidin-1-yl | | 425.3 | 1.73 |
| **282** | 1 | alpha | $-CH_3$ | 4-o-tolyl-piperazin-1-yl | | 500.3 | 2.23 |
| **283** | 1 | alpha | $-CH_3$ | 4-(2-Ethoxy-phenyl)-piperazin-1-yl | | 530.3 | 2.14 |
| **284** | 1 | alpha | $-CH_3$ | 4-Cyclohexyl-piperazin-1-yl | | 492.3 | 1.48 |
| **286** | 1 | alpha | $-CH_3$ | thiazolidin-3-yl | | 413.2 | 1.89 |
| **288** | 1 | alpha | $-CH_3$ | methyl | 2-pyridin-2-yl-ethyl | 460.3 | 1.66 |
| **289** | 1 | alpha | $-CH_3$ | Methyl | 2,3,4,5,6-pentahydroxy-hexyl | 519.3 | 1.49 |
| **293** | 1 | alpha | $-CH_3$ | methyl | naphthalen-1-ylmethyl | 495.3 | 2.20 |
| **296** | 1 | alpha | $-CH_3$ | benzyl | ethyl | 459.3 | 2.15 |
| **297** | 1 | alpha | $-CH_3$ | benzyl | phenethyl | 535.3 | 2.31 |
| **299** | 1 | alpha | $-CH_3$ | methyl | Butyl | 411.3 | 2.06 |
| **302** | 1 | alpha | $-CH_3$ | Benzyl | 2-cyano-ethyl | 484.3 | 2.01 |
| **303** | 1 | alpha | $-CH_3$ | propyl | methyl | 397.3 | 1.97 |
| **306** | 1 | alpha | $-CH_3$ | phenethyl | methyl | 459.3 | 2.08 |
| **308** | 1 | alpha | $-CH_3$ | methyl | pyridin-4-ylmethyl | 460.3 | 1.61 |
| **312** | 2 | beta | $-CH_3$ | Furan-2-ylmethyl | H | 435.24 | 5.93 |
| **313** | 2 | beta | $-CH_3$ | Methyl | Cyclohexyl | 451.31 | 6.86 |
| **316** | 2 | beta | $-CH_3$ | morpholin-4-yl | | 425.57 | |
| **318** | 2 | beta | $-CH_3$ | pyridin-3-ylmethyl | H | 446.26 | 5.33 |
| **320** | 2 | beta | $-CH_3$ | benzyl | H | | |
| **323** | 2 | beta | $-CH_3$ | 4-Chloro-benzyl | H | 479.22 | 6.45 |
| **326** | 2 | beta | $-CH_3$ | Butyl | H | 411.28 | 6.1 |
| **329** | 2 | beta | $-CH_3$ | 5-methyl-thiazol-2-yl | H | 452.21 | 6.26 |

(continued)

| No. | n | C15 stereo | PG or $R^1$ | $R^2$ | $R^4$ | MS m/z | HPLC Rt [min] |
|---|---|---|---|---|---|---|---|
| | | | | 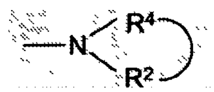 | | | |
| **329A** | 2 | beta | -H | 5-methyl-thiazol-2-yl | H | 452.21 | 6.26 |
| **332** | 4 | beta | $-CH_3$ | Cyclooctyl | H | 493.36 | 5.05 |
| **334** | 4 | beta | $-CH_3$ | 2-thiazol-4-yl-acetic acid ethyl ester / 4-(acetic acid ethyl ester)-thiazol-2-yl | H | 552.27 | 4.6 |
| **335** | 4 | beta | $-CH_3$ | Benzo[1,3]dioxol-5-ylmethyl | H | 517.28 | 4.41 |
| **336** | 4 | beta | $-CH_3$ | morpholin-4-yl | | 453.29 | 4.2 |
| **339** | 4 | beta | $-CH_3$ | pyridin-4-ylmethyl | H | 1474.29 | 3.93 |
| **341** | 4 | beta | $-CH_3$ | 2-Methoxy-benzyl | H | 503.3 | 4.56 |
| **342** | 4 | beta | $-CH_3$ | 3-Fluoro-benzyl | H | 491.28 | 4.54 |
| **347** | 4 | beta | $-CH_3$ | 2-(7-methyl-1H-indol-3-yl)-ethyl | H | 540.34 | 4.58 |
| **348** | 5 | beta | $-CH_3$ | Cyclohexyl | H | 479.34 | 7 |
| **354** | 5 | beta | $-CH_3$ | morpholin-4-yl | | 467.65 | |
| **355** | 5 | beta | $-CH_3$ | thiomorpholin-4-yl | | 483.28 | 6.86 |
| **358** | 5 | beta | $-CH_3$ | Phenyl | H | 473.29 | 7.00 |
| **363A** | 5 | beta | -H | 2-(4-Hydroxy-phenyl)-ethyl | H | | |
| **364** | 5 | beta | $-CH_3$ | Methyl | benzyl | 501.32 | 7.25 |
| **366** | 5 | beta | $-CH_3$ | 2-Thiophen-2-yl-ethyl | H | 507.28 | 6.81 |
| **368** | 5 | beta | $-CH_3$ | 5-methyl-thiazol-2-yl | H | 494.26 | 6.87 |

Table 2: Intermediates of the general formula VII (ester derivatives)

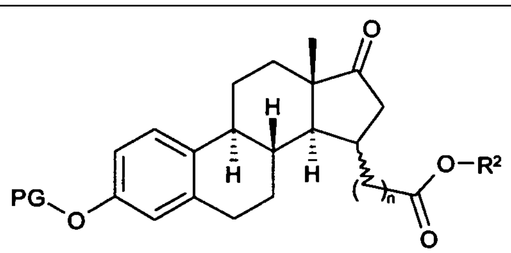

C-(VII)

| No. | n | C15 stereo | PG or $R^1$ | $R^2$ | MS m/z | HPLC Rt [min] |
|---|---|---|---|---|---|---|
| **369** | 5 | beta | $-CH_3$ | isopropyl | | |

Table 3: Intermediates of the general formula VIII (ketone derivatives)

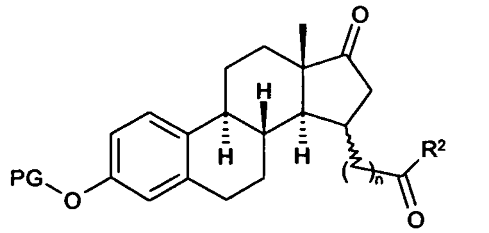

C-(VIII)

| No | n | C15 stereo | PG or $R^1$ | $R^2$ | MS m/z | HPLC Rt [min] |
|---|---|---|---|---|---|---|
| 370 | 2 | beta | $-CH_3$ | ethyl | | |

Table 4: Intermediates of the general formula XLI (Hydrazide derivatives)

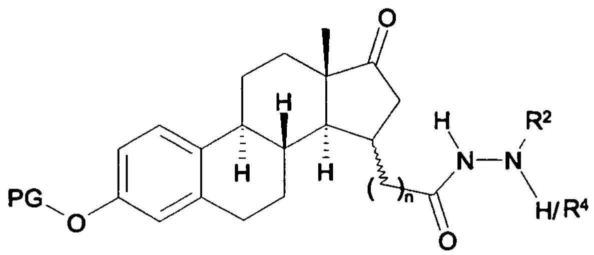

C-(XLI)

| No. | n | C15 stereo | PG or $R^1$ | $R^2$ | $R^4$ | MS m/z | HPL C Rt [min] |
|---|---|---|---|---|---|---|---|
| | | | | 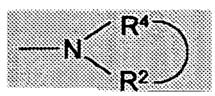 | | | |
| **371** | 2 | beta | $-CH_3$ | morpholin-4-yl | | 440.27 | 3.62 |
| **372** | 2 | beta | $-CH_3$ | 7-chloro-quinolin-4-yl | H | 531.23 | 3.9 |
| **374** | 2 | beta | $-CH_3$ | $-CO-CH_3$ / acetyl | H | 412.24 | 3.43 |
| **376** | 2 | beta | $-CH_3$ | $-CH_2-CO-O-CH_2-CH_3$ | H | 456.26 | 3.82 |
| **377** | 2 | beta | $-CH_3$ | 2-Fluoro-phenyl | H | 464.25 | 4.21 |
| **381** | 3 | beta | $-CH_3$ | Azepan-1-yl | | 466.32 | 4.45 |
| **382** | 3 | beta | $-CH_3$ | 2-(1H-indol-3-yl)-acetyl | H | 541.29 | 3.93 |
| **386** | 3 | beta | $-CH_3$ | -CO- phenyl | H | 488.27 | 3.93 |
| **388** | 3 | beta | $-CH_3$ | methyl | phenyl | 474.29 | 4.37 |
| **390** | 3 | beta | $-CH_3$ | 3,5-dichloro-phenyl | H | 528.19 | 4.66 |
| **391** | 3 | beta | $-CH_3$ | -CO-(3,4,5-trimethoxy)-phenyl | H | 578.3 | 3.92 |
| **393** | 3 | beta | $-CH_3$ | 3-methoxy-phenyl | H | 490.28 | 4.19 |
| **394** | 3 | beta | $-CH_3$ | 6-chloro-pyridazin-3-yl | H | 496.22 | 4.47 |
| **395** | 3 | beta | $-CH_3$ | 2-Methoxymethyl-pyrrolidin-1yl | | 482.31 | 4.03 |
| **401** | 4 | beta | $-CH_3$ | methyl | Methyl | 426.29 | 3.94 |

(continued)

| No. | n | C15 stereo | PG or R1 | R2 | R4 | MS m/z | HPL C Rt [min] |
|---|---|---|---|---|---|---|---|
| | | | | 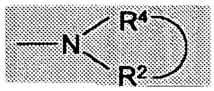 | | | |
| **405** | 4 | beta | $-CH_3$ | benzothiazol-2-yl | H | 531.26 | 5.19 |
| **406** | 4 | beta | $-CH_3$ | 4-methyl-piperazin-1-yl | | 481.33 | 3.46 |
| **408** | 5 | beta | $-CH_3$ | piperidin-1-yl | | 480.34 | 4.58 |
| **409** | 5 | beta | $-CH_3$ | 4-methanesulfonyl-phenyl | H | 566.28 | 4.15 |
| **410** | 5 | beta | $-CH_3$ | -CO-(3- Methoxy-) phenyl | H | 546.31 | 4.26 |
| **411** | 5 | beta | $-CH_3$ | acetyl | H | 454.28 | 3.8 |
| **413** | 5 | beta | $-CH_3$ | benzyl | H | 502.32 | 4.78 |
| **416** | 5 | beta | $-CH_3$ | 3,4-dichloro-phenyl | H | 556.23 | 4.9 |

Table 5: Intermediates of the generated formula XVII (Urea derivatives)

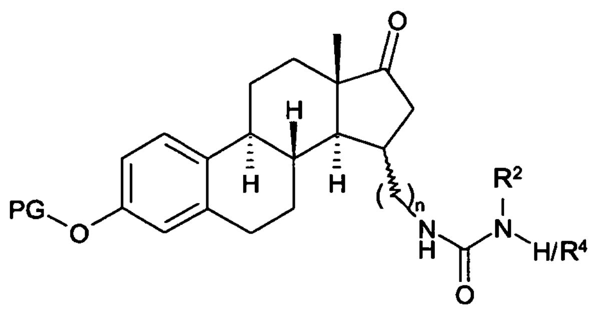

C-(XVII)

| No. | n | C15 stereo | PG or R1 | R2 | R4 | MS m/z | HPLC Rt [min] |
|---|---|---|---|---|---|---|---|
| | | | | 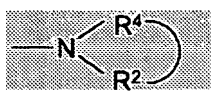 | | | |
| **420** | 1 | alpha | -H | 3-nitro-phenyl | -H | 436.22 | 3.74 |
| **421** | 1 | alpha | -H | 3,4-Dichloro-benzyl | -H | 486.21 | 3.98 |
| **423** | 1 | alpha | -H | Benzyl | -H | 490.25 | 3.8 |
| **427** | 1 | alpha | -H | 4-methoxy-phenyl | -H | 463.21 | 3.76 |
| **428** | 1 | alpha | -H | 3-Cyano-phenyl | -H | 432.24 | 3.56 |
| **433** | 1 | alpha | -H | isopropyl | -H | 454.32 | 4.17 |
| **434** | 1 | alpha | -H | octyl | -H | 442.25 | 3.34 |
| **443** | 3 | beta | $-CH_3$ | 3-Methoxy-phenyl | -H | 490.28 | 4.33 |
| **444** | 3 | beta | $-CH_3$ | 3-trifluoromethyl-phenyl | -H | 528.26 | 4.69 |
| **445** | 3 | beta | $-CH_3$ | 4-Fluoro-phenyl | -H | 478.26 | 4.36 |

(continued)

| No. | n | C15 stereo | PG or $R^1$ | $R^2$ | $R^4$ | MS m/z | HPLC Rt [min] |
|---|---|---|---|---|---|---|---|
| | | | | 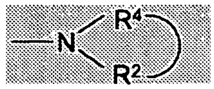 | | | |
| **448** | 3 | beta | $-CH_3$ | 4-trifluoromethyl-phenyl | -H | 528.26 | 4.7 |
| **451** | 3 | beta | $-CH_3$ | naphthalen-1-yl | -H | 510.29 | 4.5 |
| **453** | 3 | beta | $-CH_3$ | 2-benzoic acid methyl ester / 2-(carboxylic acid methyl ester)-phenyl | -H | 518.28 | 4.76 |
| **455** | 3 | beta | $-CH_3$ | 3-Acetyl-phenyl | -H | 502.28 | 4.23 |
| **458** | 3 | beta | $-CH_3$ | Biphenyl-2-yl | -H | 536.3 | 4.79 |
| **462** | 3 | beta | $-CH_3$ | 4-(6-methyl-benzothinzol-2-yl)-phenyl | -H | | |
| **466** | 4 | beta | $-CH_3$ | 2,4-Dichloro-phenyl | -H | 542.21 | 5.19 |
| **467** | 4 | beta | $-CH_3$ | 3-Fluoro-phenyl | -H | 492.28 | 4.63 |
| **475** | 4 | beta | $-CH_3$ | Cyclohexyl | -H | 480.34 | 4.56 |
| **481** | 4 | beta | $-CH_3$ | 4-Acetyl-phenyl | -H | 516.3 | 4.37 |
| **482** | 4 | beta | $-CH_3$ | 4-trifluoromethoxy-phenyl | -H | 558.27 | 4.89 |
| **485** | 4 | beta | $-CH_3$ | naphthalen-2-yl | -H | 524.3 | 4.82 |
| **486** | 4 | beta | $-CH_3$ | 3-propionic acid ethyl ester / 1-ethoxy-1-oxo-propan-3-yl | -H | 498.31 | 4.18 |
| **488** | 4 | beta | $-CH_3$ | 3,4-Dimethoxy-phenyl | -H | 534.31 | 4.28 |
| **489** | 4 | beta | $-CH_3$ | Benzo[1,3]dioxol-5-yl | -H | 518.28 | 4.42 |
| **490** | 4 | alpha | -H | 4-benzoic acid ethyl ester / 4-(carboxylic acid ethyl ester)-phenyl | -H | 532 | 5.91 |
| **491** | 4 | alpha | -H | Cyclohexylmethyl | -H | 480 | 6.42 |
| **492** | 4 | alpha | -H | Phenyl | -H | 460 | 5.65 |

Table 6: Intermediates of the general formula XIX (sulfamide derivatives)

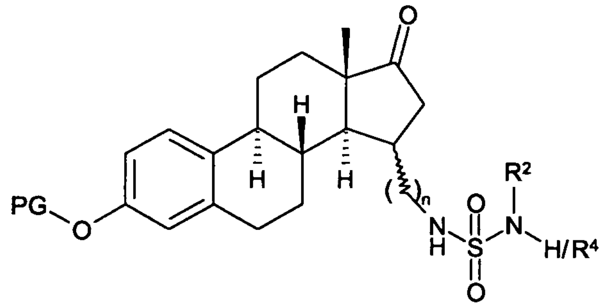

C-(XIX)

| No. | n | C15 stereo | PG or $R^1$ | $R^2$ | $R^4$ |
|---|---|---|---|---|---|
| | | | | 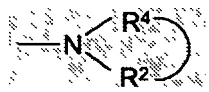 | |
| 493 | 2 | alpha | $-CH_3$ | 2-phenyl-ethyl | -H |
| 494 | 2 | alpha | $-CH_3$ | 2-naphthalen-1-yl-ethyl | -H |
| 495 | 2 | alpha | $-CH_3$ | 3,3-Diphenyl-propyl | -H |
| 496 | 2 | alpha | $-CH_3$ | 3-Methyl-butyl | -H |
| 497 | 2 | alpha | $-CH_3$ | 2-((Phenylsulfonyl)methyl)-benzyl | -H |
| 498 | 2 | alpha | $-CH_3$ | Naphthalen-2-yl-methyl | -H |
| 499 | 2 | alpha | $-CH_3$ | 2-(Difluoromethoxy)benzyl | -H |
| 500 | 2 | alpha | $-CH_3$ | 2-[N,N-(2-Hydroxy-ethyl)-phenyl-amino]-ethyl | -H |
| 501 | 2 | alpha | $-CH_3$ | 2,5-Bis(trifluoromethyl)-benzyl | -H |
| 502 | 2 | alpha | $-CH_3$ | acetic acid 2-ethyl ester / $-CH_2-CH_3-O-CO-CH_3$ | -H |
| 503 | 2 | alpha | $-CH_3$ | Naphthaten-1-yl-carbamic acid 2-ethyl ester | -H |
| 504 | 2 | alpha | $-CH_3$ | 2,3-bichlorophenyl-carbamic acid 2-ethyl ester | -H |
| 505 | 2 | alpha | $-CH_3$ | 2,3-Dihydro-benzo[1,4]dioxin-2-ylmethyl | -H |
| 506 | 2 | alpha | $-CH_3$ | 4-Fluoro-2,3-dihydro-benzofuran-2-yl-methyl | -H |
| 507 | 2 | alpha | $-CH_3$ | 2-Phenyl-benzyl | -H |
| 508 | 2 | alpha | $-CH_3$ | 2-Indol-3-yl-ethyl | -H |
| 509 | 2 | alpha | $-CH_3$ | $-CH_2$-(3- benzoic acid methyl ester) / (3-carboxylic acid methyl ester)-benzyl | -H |
| 511 | 2 | alpha | $-CH_3$ | 3,5-Bis(trifluoromethyl)benzyl | -H |
| 512 | 2 | alpha | $-CH_3$ | 3-Benzoyl-benzyl | -H |
| 513 | 2 | alpha | $-CH_3$ | 3,2-Dihydroxy-propyl | -H |
| 514 | 2 | alpha | $-CH_3$ | 2-(4-Chlorobenzoyl)-benzofuran-3-yl-methyl | -H |

(continued)

| No. | n | C15 stereo | PG or $R^1$ | $R^2$ | $R^4$ |
|---|---|---|---|---|---|
| | | | | 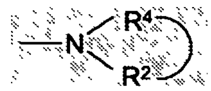 | |
| 515 | 2 | alpha | $-CH_3$ | 3-propionic acid ethyl ester / 1-ethoxy-1-oxo-propan-3-yl | -H |
| 516 | 2 | alpha | $-CH_3$ | 3-Phenoxy-propyl | -H |
| 517 | 2 | alpha | $-CH_3$ | 2-(4-Acetophenone)-ethyl | -H |
| 518 | 2 | alpha | $-CH_3$ | 1,2,3-Thiodiazol-4-yl-benzyl | -H |
| 519 | 2 | alpha | $-CH_3$ | $-CH_2$-(4- benzoic acid methyl ester) / (4-carboxylic acid methyl ester)-benzyl | -H |
| 520 | 2 | alpha | $-CH_3$ | $-CH_2$-(4-phenyl-acetic acid phenacyl ester) / $-CH_2$-(4-phenyl-$CH_2$-CO-O-$CH_2$-CO- phenyl) | -H |
| 521 | 2 | alpha | $-CH_3$ | 4-(Tert-butyl)benzyl | -H |
| 523 | 2 | alpha | $-CH_3$ | 7-Methoxy-coumarin-4-yl-methyl | -H |
| 524 | 2 | alpha | $-CH_3$ | 4-Methylbenzyl | -H |
| 525 | 2 | alpha | $-CH_3$ | 4-Methylsulfonylbenzyl | -H |
| 526 | 2 | alpha | $-CH_3$ | 4-Phenoxy-butyl | -H |
| 527 | 2 | alpha | $-CH_3$ | Benzofurazan-5-yl-methyl | -H |
| 528 | 2 | alpha | $-CH_3$ | 2-(6-Amino-9H-purin-9-yl)-ethyl | -H |
| 529 | 2 | alpha | $-CH_3$ | 3-Cyano-benzyl | -H |
| 530 | 2 | alpha | $-CH_3$ | 2-Cyano-benzyl | -H |
| 531 | 2 | alpha | $-CH_3$ | 4-Cyano-benzyl | -H |
| 532 | 2 | alpha | $-CH_3$ | Benzoic acid 2-ethyl ester / $-CH_2$-$CH_3$-O- CO- phenyl | -H |
| 534 | 2 | alpha | $-CH_3$ | Cyclopropylmethyl | -H |
| 536 | 2 | alpha | $-CH_3$ | 4-(1,3-Dioxo-1,3-dihydro-isoindol-2-yl)-butyl | -H |

Table 7: Intermediates of the general formula XX (carbamate derivatives)

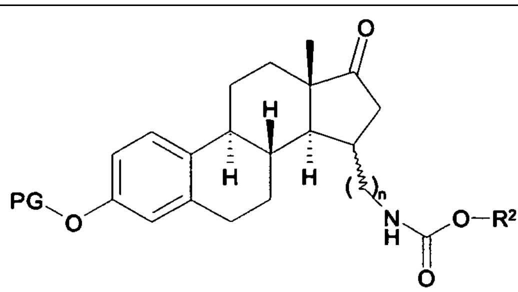

C-(XX)

| No. | n | C15 stereo | PG or $R^1$ | $R^2$ | MS m/z | HPLC Rt [min] |
|---|---|---|---|---|---|---|
| 538 | 1 | alpha | -H | Isobutyl | 400 | 5.58 |
| 539 | 1 | alpha | -H | 4-Nitro-benzyl | 478 | 5.62 |

Table 8: Intermediates of the general formula XXII (sulfamate derivatives)

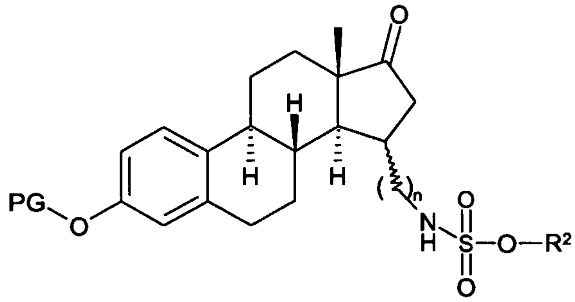

C-(XXII)

| No. | n | C15 stereo | PG or $R^1$ | $R^2$ | MS m/z | HPLC Rt [min] |
|---|---|---|---|---|---|---|
| **540** | **1** | alpha | $-CH_3$ | Ethyl | | |
| **541** | **1** | alpha | $-CH_3$ | Butyl | | |
| **542** | **1** | alpha | $-CH_3$ | Benzyl | | |
| **543** | **1** | alpha | $-CH_3$ | Phenyl | | |

Table 9: Intermediates of the general formula XXIII ("retro"-amide derivatives)

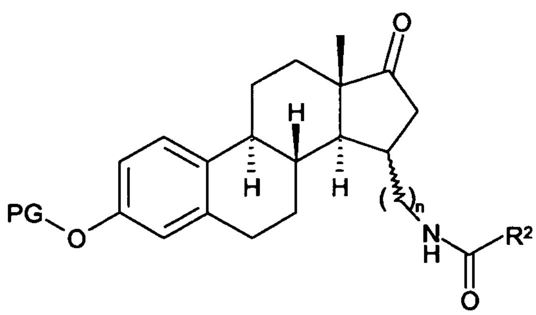

C-(XXIII)

| No. | n | C15 stereo | PG or $R^1$ | $R^2$ | MS m/z | HPLC Rt [min] |
|---|---|---|---|---|---|---|
| **544** | 1 | alpha | $-CH_3$ | 3,5-Bis-trifluoromethyl-pheny | 553.21 | 4.73 |
| **548** | 1 | alpha | $-CH_3$ | 3-Methoxy-phenyl | 447.24 | 4.27 |
| **551** | 1 | alpha | $-CH_3$ | 4-Hexyloxy-phenyl | 517.32 | 5.25 |
| **552** | 1 | alpha | $-CH_3$ | 4-Trifluoromethyl-phenyl | 485.22 | 4.59 |
| **554** | 1 | alpha | $-CH_3$ | Phenoxy-methyl | 447.24 | 4.31 |
| **557** | 1 | alpha | $-CH_3$ | Ethyl | 369.23 | 3.81 |
| **559** | 1 | alpha | $-CH_3$ | 2-Cyclopentyl-ethyl | 437.29 | 4.56 |
| **561** | 1 | alpha | $-CH_3$ | Furan-2-yl | 407.21 | 4.03 |
| **562** | 1 | alpha | $-CH_3$ | Thiophen-2-yl-methyl | 437.2 | 4.15 |
| **565** | 1 | alpha | $-CH_3$ | Acetic acid methyl ester / *-CH2-CO- O- CH3* | 413.22 | 3.79 |
| **567** | 1 | alpha | $-CH_3$ | 2,4,5-Trifluorophenyl | 471.2 | 4.51 |
| **569** | 1 | alpha | $-CH_3$ | 1-Phenyl-5-trifluoromethyl-1H-pyrazol-4-yl | 551.24 | 4.53 |
| **570** | 1 | alpha | $-CH_3$ | Adamantan-1-yl | 475.31 | 4.89 |

(continued)

| No. | n | C15 stereo | PG or $R^1$ | $R^2$ | MS m/z | HPLC Rt [min] |
|---|---|---|---|---|---|---|
| **574** | 4 | alpha | -H | 2-methoxy-phenyl | 475.27 | 5.77 |
| **577** | 4 | alpha | -H | 4-Chloro-phenyl | 479.22 | 5.95 |
| **580** | 4 | alpha | -H | methoxymethyl | 413.26 | 5.01 |
| **584** | 4 | alpha | -H | 1-ethoxy-1-oxo-propan-3-yl | 469.28 | 5.25 |
| **588** | 4 | alpha | -H | naphthalen-2-yl | 495.28 | 6.04 |
| **593** | 4 | alpha | -H | Benzo[b]thiophene-2-yl | 501.23 | 6.09 |
| **601** | 3 | alpha | -H | benzyl | 445.26 | 3.69 |
| **602** | 3 | alpha | -H | Phenethyl | 459.28 | 3.78 |
| **606** | 3 | alpha | -H | 3-Cyano-phenyl | 456.24 | 3.72 |
| **609** | 3 | beta | $-CH_3$ | 2,4-Dichloro-phenyl | 513.18 | 6.74 |
| **622** | 3 | beta | $-CH_3$ | 4-Cyano-phenyl | 470.26 | 6.24 |
| **625** | 3 | beta | $-CH_3$ | 3,5-Dichloro-phenyl | 513.18 | 7.16 |
| **627** | 3 | beta | $-CH_3$ | Benzyloxy-methyl | 489.29 | 6.53 |
| **629** | 4 | beta | $-CH_3$ | 3,4-Difluoro-phenyl | 495.26 | 6.84 |
| **635** | 4 | beta | $-CH_3$ | 2-bromo-phenyl | 537.19 | 6.7 |
| **637** | 4 | beta | $-CH_3$ | 3-Chloro-phenyl | 493.24 | 6.97 |
| **641** | 4 | beta | $-CH_3$ | 4-methoxy-phenyl | 489.29 | 6.54 |
| **645** | 4 | beta | $-CH_3$ | 2,2-dimethyl-propyl | 453.32 | 6.72 |
| **648** | 4 | beta | $-CH_3$ | cyclohexyl | 465.32 | 6.82 |
| **650** | 4 | beta | $-CH_3$ | naphthalen-1-yl | 509.29 | 6.93 |
| **653** | 3 | alpha | -H | 3,4-Dichlorophenyl | 500 | 4.17 |
| **655** | 4 | alpha | -H | 4-Fluorobenzyl | 478 | 5.72 |
| **657** | 4 | alpha | -H | 2,4-Difluorophenyl | 482 | 5.86 |
| **658** | 5 | beta | $-CH_3$ | Phenyl | 474 | 6.80 |
| **660** | 5 | beta | $-CH_3$ | 4-Fluorophenyl | 492 | 6.68 |

Table 10: Intermediates of the general formula XXIV ("retro"-sulfonamide derivatives)

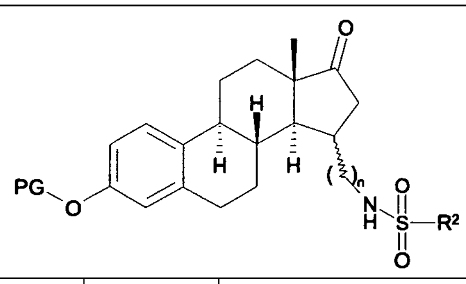

C-(XXIV)

| NO | n | C15 stereo | PG or $R^1$ | $R^2$ | MS m/z | HPLC Rt [min] |
|---|---|---|---|---|---|---|
| **661** | 1 | alpha | -H | Naphthalene-2-yl | 489.2 | 5.75 |
| **663** | 1 | alpha | -H | Quinoline-8-yl | 490.19 | 5.41 |

(continued)

| NO | n | C15 stereo | PG or $R^1$ | $R^2$ | MS m/z | HPLC Rt [min] |
|---|---|---|---|---|---|---|
| **666** | 1 | alpha | -H | 4-(N-acetyl)-amino-phenyl | 496.2 | 4.83 |
| **668** | 1 | alpha | -H | 4-methoxy-phenyl | 469.19 | 5.35 |
| **673** | 1 | alpha | -H | 3.4-Dichloro-phenyl | 507.1 | 5.98 |
| **676** | 1 | alpha | -H | 3-Chloro-phenyl | 473.14 | 5.67 |
| **704** | 3 | beta | $-CH_3$ | 4-nitro-phenyl | 526.21 | 4.54 |
| **706** | 3 | beta | $-CH_3$ | benzyl | 495.24 | 4.51 |
| **707** | 3 | beta | $-CH_3$ | propyl | 447.24 | 4.34 |
| **713** | 3 | beta | $-CH_3$ | 2.5-Dichloro-thiophene-3-yl | 555.11 | 5.04 |
| **715** | 3 | beta | $-CH_3$ | 3-methyl-phenyl | 495.24 | 4.67 |
| **716** | 3 | beta | $-CH_3$ | 3.4-dimethoxy-phenyl | 541.25 | 4.37 |
| **717** | 3 | beta | $-CH_3$ | 4-Benzenesulfonyl-thiophene-2-yl | 627.18 | 4.59 |
| **720** | 4 | beta | $-CH_3$ | Thiophene-2-yl | 501.2 | 4.65 |
| **723** | 4 | beta | $-CH_3$ | 4-Fluoro-phenyl | 513.23 | 4.74 |
| **727** | 4 | beta | $-CH_3$ | 3-trifluoromethyl-phenyl | 563.23 | 4.97 |
| **728** | 4 | beta | $-CH_3$ | 3.5-Bis-trifluoromethyl-phenyl | 631.22 | 5.23 |
| **729** | 4 | beta | $-CH_3$ | 2.5-dimethoxy-phenyl | 555.27 | 4.72 |
| **731** | 4 | beta | $-CH_3$ | 4-trifluoromethoxy-phenyl | 579.23 | 5.03 |
| **739** | 5 | beta | $-CH_3$ | 4-Methyl-phenyl | 524 | 6.91 |

Table 11: Intermediates of the general formula XXV (sulfonylurea derivatives)

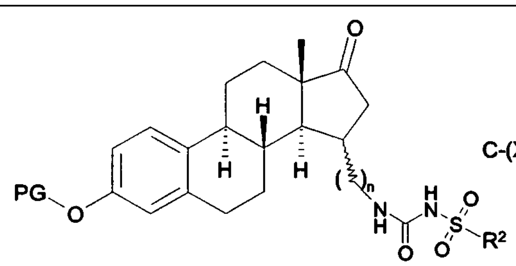

C-(XXV)

| No. | n | C15 stereo | PG or $R^1$ | $R^2$ | MS m/z | HPLC Rt [min] |
|---|---|---|---|---|---|---|
| **740** | 2 | alpha | $-CH_3$ | Phenyl | | |
| **741** | 2 | alpha | $-CH_3$ | 4-Chloro-phenyl | | |
| **742** | 2 | alpha | $-CH_3$ | 4-Methyl-phenyl | | |
| **743** | 2 | alpha | $-CH_3$ | 2-Methyl-phenyl | | |

Table 12: Intermediates of the general formula XXVI ("retro"-carbamate derivatives)

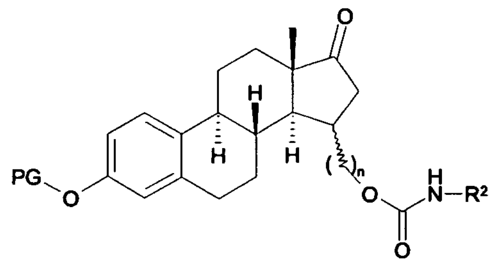

C-(XXVI)

| No. | n | C15 stereo | PG or $R^1$ | $R^2$ | MS m/z | HPLC Rt [min] |
|---|---|---|---|---|---|---|
| **744** | 3 | beta | $-CH_3$ | 2,4-Dichloro-phenyl | 529 | 5.52 |
| **745** | 3 | beta | $-CH_3$ | 4-Trifluoromethyl-phenyl | 529 | 5.18 |
| **747** | 3 | beta | $-CH_3$ | 3-Cyano-phenyl | 486 | 4.74 |
| **748** | 3 | beta | $-CH_3$ | Benzo[1,3]dioxol-5-yl- | 505 | 4.68 |
| **751** | 4 | beta | $-CH_3$ | 3-Fluoro-phenyl | 493 | 5.1 |
| **755** | 4 | beta | $-CH_3$ | 2-benzoic acid methyl ester | 533 | 5.67 |
| **761** | 5 | beta | $-CH_3$ | 3-Nitro-phenyl | 534 | 5.21 |
| **764** | 5 | beta | $-CH_3$ | 3.4-Dichloro-benzyl | 571 | 5.48 |
| **767** | 6 | beta | $-CH_3$ | 4-benzoic acid ethyl ester | 553 | 5.56 |
| **769** | 6 | beta | $-CH_3$ | Naphthalen-1-yl | 561 | 6.12 |
| **773** | 6 | beta | $-CH_3$ | 3,4-Dichloro-phenyl | 553 | 5.56 |

Table 13: Intermediates of the general formula XXVII ("retro"-ester derivatives)

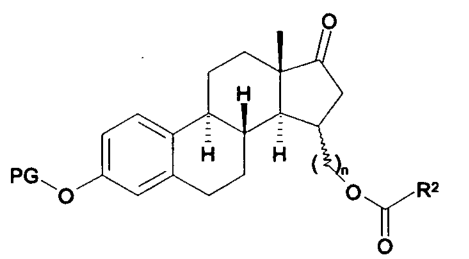

C-(XXVII)

| No | n | C15 stereo | PG or $R^1$ | $R^2$ | MS m/z | HPLC Rt [min] |
|---|---|---|---|---|---|---|
| **774** | 4 | beta | $-CH_3$ | Tert-Butyl | 441 | 7.85 |
| **775** | 5 | beta | $-CH_3$ | Tert-Butyl | 455 | 8.07 |

Table 14: Intermediates of the general formula XXVIII (sulfonylcarbamate derivatives)

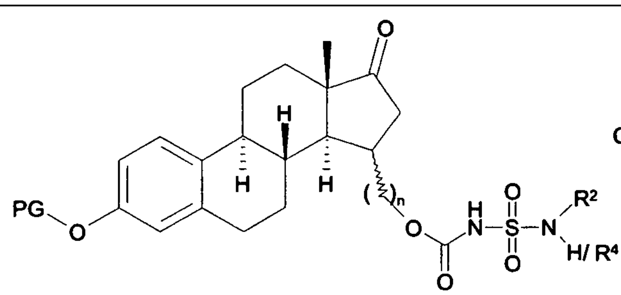

C-(XXVIII)

(continued)

| No. | n | C15 stereo | PG or $R^1$ | $R^2$ | $R^4$ | MS m/z | HPLC Rt [min] |
|---|---|---|---|---|---|---|---|
| | | | | 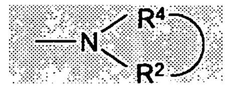 | | | |
| **790** | 3 | beta | -$CH_3$ | 2-(1H-indol-3-yl)-ethyl | H | 607 | 4.36 |
| **792** | 3 | beta | -$CH_3$ | cyclohexyl | H | 546 | 4.59 |
| **795** | 3 | beta | -$CH_3$ | morpholine-4-yl | | 534 | 3.96 |
| **797** | 4 | beta | -$CH_3$ | 4-methyl-piperazine-1-yl | | 561 | 3.57 |
| **802** | 4 | beta | -$CH_3$ | methyl | benzyl | 582 | 4.84 |
| **805** | 5 | beta | -$CH_3$ | benzyl | H | 582 | 4.64 |
| **811** | 5 | beta | -$CH_3$ | methyl | butyl | 562 | 5.09 |
| **817** | 6 | beta | -$CH_3$ | butyl | H | 562 | 4.9 |
| **818** | 6 | beta | -$CH_3$ | phenyl | H | 582 | 4.46 |

INTERMEDIATES 820 to 834 - alcohols

The synthesis of the following estrone-alcohol derivatives of general formula XXXI is described in the section "Intermediates, Chapter IV - Compounds of formula XXXI".

Intermediate No. 820: 15a-Hydroxymethyl-3-hydroxy-estra-1,3,5(10)-trien-17-one (XXXIα-1a)
Intermediate No. 821: 15α-Hydroxymethyl-3-methoxy-estra-1,3,5(10)-trien-17-one (XXXIα-1b)
Intermediate No. 822: 3-Benzyloxy-15α-hydroxymethyl-estra-1,3,5(10)-trien-17-one (XXXIα-1c)
Intermediate No. 823: 3-Hydroxy-15β-(3-Hydroxypropyl)-estra-1,3,5(10)-trien-17-one (XXXIβ-3a)
Intermediate No. 824: 15β-(3-Hydroxypropyl)-3-methoxyestra-1,3,5(10)-trien-17-one (XXXIβ-3b)
Intermediate No. 825: 3-Benzyloxy-15β-(3-hydroxypropyl)-estra-1,3,5(10)-trien-17-one (XXXIβ-3c)
Intermediate No. 826: 3-Hydroxy-15β-(4-hydroxybutyl)-estra-1,3,5(10)-trien-17-one (XXXIβ-4a)
Intermediate No. 827: 15β-(4-Hydroxybutyl)-3-methoxy-estra-1,3,5(10)-trien-17-one (XXXIβ-4b)
Intermediate No. 828: 3-Benzyloxy-15β-(4-hydroxybutyl)-estra-1,3,5(10)-trien-17-one (XXXIβ-4c)
Intermediate No. 829: 3-Hydroxy-15β-(5-Hydroxypentyl)-estra-1,3,5(10)-trien-17-one (XXXIβ-5a)
Intermediate No. 830:15β-(5-Hydroxypentyl)-3-methoxy-estra-1,3,5(10)-trien-17-one (XXXIβ-5b)
Intermediate No. 831: 3-Benzyloxy-15β-(5-hydroxypentyl)-estra-1,3,5(10)-trien-17-one (XXXIβ-5c)
Intermediate No. 832: 3-Hydroxy-15β-(6-hydroxyhexyl)-3-estra-1,3,5(10)-trien-17-one (XXXIβ-6a)
Intermediate No. 833: 15β-(6-Hydroxyhexyl)-3-methoxy-estra-1,3,5(10)-trien-17-one (XXXIβ-6b)
Intermediate No. 834: 3-Benzyloxy-15β-(6-hydroxyhexyl)-estra-1,3,5(10)-trien-17-one (XXXIβ-6c)

Table 15: Intermediates of the general formula XXX (ether derivatives)

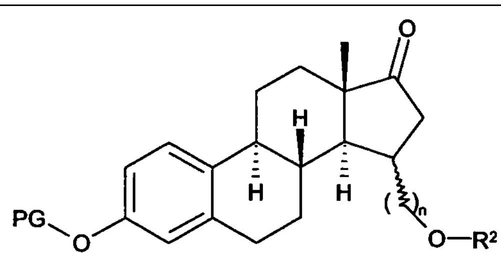

C-(XXX)

| No. | n | C15 stereo | PG or $R^1$ | $R^2$ | MS m/z | HPLC Rt [min] |
|---|---|---|---|---|---|---|
| **835** | 3 | beta | -$CH_3$ | Methyl | 374 | 6.8 |

(continued)

| No. | n | C15 stereo | PG or $R^1$ | $R^2$ | MS m/z | HPLC Rt [min] |
|---|---|---|---|---|---|---|
| **836** | 4 | beta | -$CH_3$ | Phenyl | 450 | 7.77 |

Examples

In order to more fully illustrate the nature of the invention and the manner of practicing the same, the following examples are presented, but they should not be taken as limiting.

1. Compounds carrying an additional substituent in C2 position of the steroidal core

EXAMPLE 1: N-Benzyl-4-(2-ethyl-3-hydroxy-17-oxo-estra-1,3,5(10)-trien-15β-yl)-butyramide

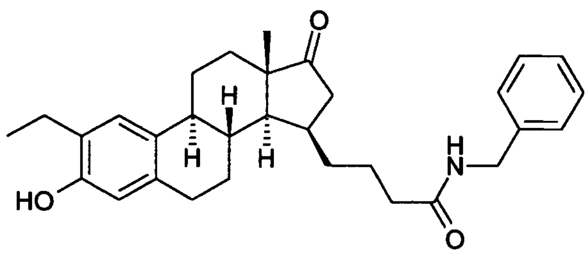

Example 1 was obtained from intermediate compound (IVb-(C2-C)-3a) by amid coupling according to Flow Diagram Ib: Compound (IVb-(C2-C)-3a) (100 mg, 0.26 mmol) was dissolved in a mixture of EtOAc (35 ml), benzyl amine (0.26 mmol), TEA (0.52 mmol) and propylphosphonic acid anhydride in EtOAc (T3P) (50 w/w %, 0.52 mmol) under $N_2$ atmosphere at 0˚C. After stirring for 2h at ambient temperature, the reaction mixture was poured into water (50 ml) and diluted with EtOAc (25 ml). The aqueous layer was neutralized to pH 8 with aq $NaHCO_3$, separated and extracted with EtOAc. The combined organic layers were washed with water and dried over $Na_2SO_4$. The organic layer was concentrated *in vacuo* yielding Compound No. 1 (56 mg, 0.12 mmol, 46 %) after column chromatography ($SiO_2$, DCM/MeOH = 100/0 to 95/5).

$^1H$-NMR (300 MHz, $CDCl_3$): δ 0.99 (*s*, 3H, Steroid-$CH_3$), 1.22 (*t, J* = 7.4 Hz, 3H, Ethyl), 1.3-1.8 (*m*, 11 H, Steroid), 2.2-2.42 (*m*, 8H, Steroid), 2.60 (*q, J* = 7.4 Hz, 2H, Ethyl), 2.8-2.88 (*m*, 2H, Steroid), 4.44 (*d, J* = 5.8 Hz, 2H, $CH_2$-Ph), 4.64 (bs, 1H, OH), 5.67 (bs, 1H, NH), 6.53 (s, 1H, Steroid-Ar-H), 7.03 (*s*, 1H, Steroid-Ar-H), 7.23-7.34 (*m*, 5H, Bn) ppm.

EXAMPLE 2: N-Benzyl-4-(3-hydroxy-17-oxo-2-propyl-estra-1,3,5(10)-trien-15β-yl)-butyramide

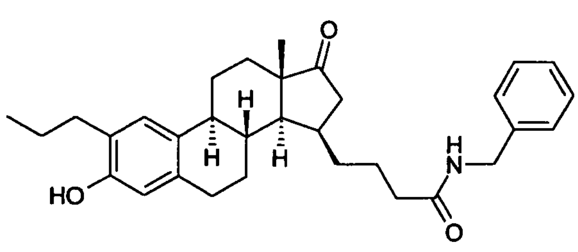

Example 2 was obtained from intermediate compound (IVb-(C2-G)3a) by amid coupling according to Flow Diagram Ib: Compound (IVb-(C2-G)-3a) (500 mg, 1.25 mmol) was dissolved in a mixture of EtOAc (25 ml), benzyl amine (2.50 mmol), TEA (3.75 mmol) and propylphosphonic acid anhydride in EtOAc (T3P) (50 w/w %, 1.50 mmol) under $N_2$ atmosphere. After the reaction mixture had been stirred at ambient temperature for 1h, it was stirred at 45 ˚C for 16h. The reaction mixture was allowed to reach ambient temperature, poured into water (50 ml) and diluted with EtOAc (25 ml). The aqueous layer was separated and extracted with EtOAc. The combined organic layers were, washed with aq.

1 M HCl, washed with brine and dried over $Na_2SO_4$. The organic layer was concentrated *in vacuo* yielding **Compound No. 2** (370 mg, 0.756 mmol, 60%) after column chromatography ($SiO_2$, DCM/MeOH = 97.5/2.5).

$^1$H-NMR (300 MHz, $CDCl_3$): δ 0.96 (*t*, 3H), 0.98 (s, 3H), 1.3-1.6 (*m*, 4H), 1.6-1.8 (*m*, 7H), 1.90 (*d*, 1 H), 2.00 (*d*, 1H, broad), 2.2-2.5 (*m*, 7H), 2.58 (*t*, 2H), 2.7-2.9 (*m*, 2H), 4.23 (*d*, 2H), 4.97 (*s*, 1H, broad), 5.72 (*t*, 1H, broad), 6.50 (*s*, 1H), 7.00 (*s*, 1 H), 7.2-7.3 (*m*, 5H) ppm.

EXAMPLE 3:

N-Benzyl-4-(3-hydroxy-2-(2-methoxy-ethyl)-17-oxo-estra-1,3,5(10)-trien-15β-yl)-butyramide

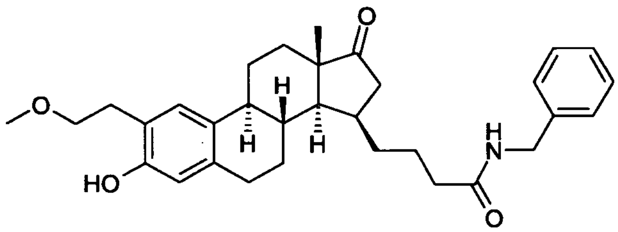

Example 3 was obtained from intermediate compound (IVb-(C2-F)-3a) by amid coupling according to **Flow Diagram Ib** using the procedure as described for Example 1.

$^1$H-NMR (300 MHz, $CDCl_3$): δ 0.99 (s, 3H, Steroid-$CH_3$), 1.2-2.0 (*m*, 11H, Steroid), 2.2-2.42 (*m*, 7H, Steroid), 2.84 (*m*, 4H, Steroid), 3.41 (*s*, 3H, OMe), 3.67 (*m*, 2H, $OCH_2$), 4.44 (*d, J* = 5.5 Hz, 2H, $CH_2$-Ph), 5.71 (b*m*, 1H, OH or NH), 6.67 (*s*, 1H, Steroid-Ar-H), 6.94 (s, 1H, Steroid-Ar-H), 7.25-7.34 (*m*, 5H, Bn), 7.96 (s, 1H, OH or NH) ppm.

EXAMPLE 4: N-Benzyl-4-(3-hydroxy-2-methoxy-17-oxo-estra-1,3,5(10)-trien-15β-yl)-butyramide

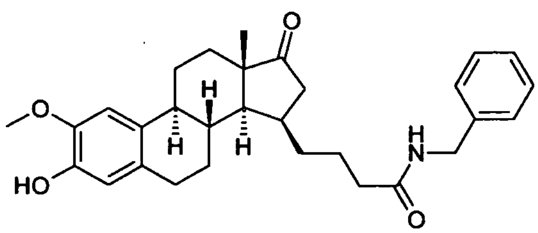

Example 4 was obtained starting from intermediate compound (IVb-(C2-B)-3a) by amide coupling according to **Flow Diagram Ib.** A solution of the 0.07 mmol **(IVb-(C2-B)-3a),** 0.077 mmol HOBT, 0.231 mmol NMM and 0.154 mmol EDCI in 5 ml DCM were added to 0.07 mmol of benzyl amine. The reaction mixture was stirred for 24 h at ambient temperature. The solvent was removed in vacuo at 40˚C. Than 4 ml EtOAc and 4 ml water were added. After vigorous stirring for 2 min, the organic phase was separated, dried with $Na_2SO_4$ and evaporated in vacuo at 40˚C. The crude product was treated with 2 ml THF, 10 mg LiOH and 0.5 ml water. After evaporation and further extraction (EtOAc and 0.1 M $KHSO_4$), 50 mg trisaminoeethlyamine polymer bound were added. After filtration and evaporating to dryness the compound No. 4 was obtained (HPLC Rt = 3.79).

EXAMPLE 5: 2-Ethyl-3-hydroxy-15β-(4-morpholin-4-yl-4-oxo-butyl)-estra-1,3,5(10)-trien-17-one

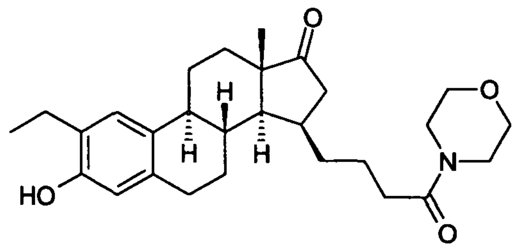

Example 5 was prepared starting from intermediate compound (IVb-(C2-C)-3a), which was converted into the desired amid by amid coupling with morpholine according to **Flow Diagram Ib** and as described above for Example 2 (Compound **(IVb-(C2-C)3a)** (110 mg, 0.28 mmol), EtOAc (30 ml), morpholine (0.28 mmol), TEA (0.57 mmol), T3P (0.34 mmol). Compound No. 5 (68 mg, 0.15 mmol, 54 %) obtained after column chromatography ($SiO_2$, DCM/MeOH = 100/0 to 95/5).

$^1$H-NMR (300 MHz, $CDCl_3$): δ 1.01 (s, 3H, Steroid-$CH_3$), 1.22 (*t, J* = 7.5 Hz, 3H, Ethyl), 1.3-2.0 (*m*, 11H, Steroid), 2.2-2.42 (*m*, 7H, Steroid/Morpholine), 2.60 (*q*, J = 7.7 Hz, 2H, ethyl), 2.82-2.90 (*m*, 2H, Steroid), 3.44-3.50 (*m*, 2H), 3.6-3.70 (*m*, 6H, Morpholine), 4.61 (s, 1H, OH), 6.53 (s, 1H, Steroid-Ar-H), 7.04 (s, 1H, Steroid-Ar-H) ppm.

EXAMPLE 6: 3-Hydroxy-15β-(4-morpholin-4-yl-4-oxo-butyl)-2-propyl-estra-1,3,5(10)-trien-17-one

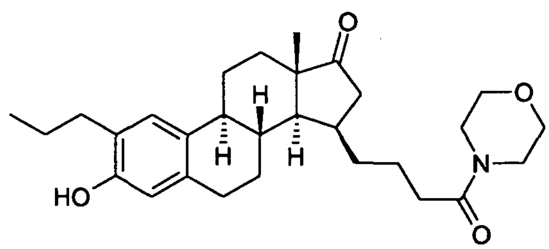

Example 6 was prepared as described above for Example 2 starting from intermediate compound (IVb-(C2-G)-3a), which was converted into the desired amid by amid coupling with morpholine according to **Flow Diagram Ib** (Compound (IVb-(C2-G)-3a) (500 mg, 1.25 mmol), EtOAc (25 ml), morpholine (2.5 mmol), TEA (3.75 mmol), T3P (1.5 mmol). Compound No. 6 (225 mg, 0.481 mmol, 38 %) was obtained after column chromatography ($SiO_2$, DCM/MeOH = 97.5/2.5).

$^1$H-NMR (300 MHz, $CDCl_3$): δ 0.98 (*t*, 3H), 1.01 (*s*, 3H), 1.3-1.8 (*m*, 11 H), 1.90 (*d*, 1H), 2.00 (*d*, 1H, broad), 2.2-2.5 (*m*, 7H), 2.35 (*t*, 2H), 2.8-3.0 (*m*, 2H), 3.45 (t, 2H), 3.6-3.70 (*m*, 6H), 5.30 (*s*, 1H), 6.54 (*s*, 1H), 7.00 (*s*, 1 H) ppm.

EXAMPLE 7:

3-Hydroxy-2-(2-methoxy-ethyl)-15β-(4-morpholin-4-yl-4-oxo-butyl)-estra-1,3,5(10)trien-17-one

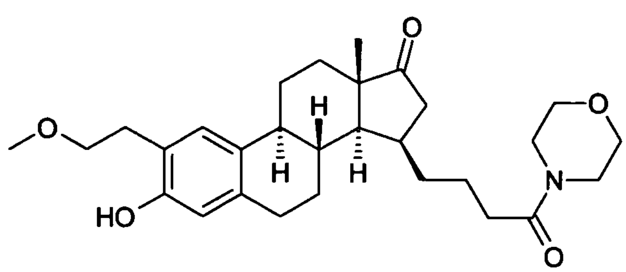

Example 7 was prepared from intermediate compound (IVb-(C2-F)-3a) by amid coupling according to **Flow Diagram Ib**: Compound (IVb-(C2-F)-3a) (85 mg, 0.221 mmol) was dissolved in a mixture of EtOAc (40 ml), morpholine (0.24 mmol), TEA (0.44 mmol) and T3P in in EtOAc (50 w/w %, 0.26 mmol) under $N_2$ atmosphere at 0 ˚C. After stirring

at ambient temperature for 16h, the reaction mixture was poured into water (100 ml) and diluted with EtOAc (50 ml). The aqueous layer was neutralized to pH 8 with aq. $NaHCO_3$, separated and extracted with EtOAc (3x 50 ml). The combined organic layers were washed with water (50 ml) and dried over $Na_2SO_4$. The organic layer was concentrated *in vacuo* yielding Compound No. 7 (43 mg, 0.088 mmol, 40 %) after column chromatography ($SiO_2$, DCM/MeOH = 99/1 to 94/6).

$^1$H-NMR (300 MHz, $CDCl_3$): δ 1.00 (*s*, 3H, Steroid-$CH_3$), 1.2-2.05 (*m*, 9H, Steroid), 2.2-2.44 (*m*, 8H, Steroid + morpholine), 2.81 (*m*, 4H, Steroid), 3.40 (s, 3H, OMe), 3.45 (*t*, *J* = 4.7 Hz, 2H, $OCH_2$), 3.57-3.72 (*m*, 8H, morpholine), 6.67 (s, 1H, Steroid-Ar-H), 6.94 (s, 1H, Steroid-Ar-H), 7.97 (*s*, 1 H, OH or NH) ppm.

EXAMPLE 8: 3-Hydroxy-2-methoxy-15β-(4-morpholin-4-yl-4-oxo-butyl)-estra-1,3,5(10)-trien-17-one

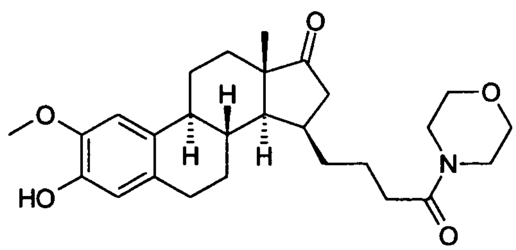

Example 8 was prepared starting from intermediate compound (IVb-(C2-B)-3a), which was converted into the desired amid by amid coupling with morpholine according to **Flow Diagram Ib** and as described above for Example 4 using 0.07 mmol of (IVb-(C2-B)-3a) and 0.07 mmol morpholine, yielding Compound No. 4.

$^{13}$C NMR (126 MHz, CHLOROFORM-*d*): δ ppm 17.7 (q, 1 C) 25.1 (t, 1 C) 25.8 (t, 1 C) 26.8 (t, 1 C) 28.7 (t, 1 C) 30.9 (t, 1 C) 32.8 (t, 1 C) 33.9 (t, 1 C) 34.4 (d, 1 C) 36.0 (d, 1 C) 41.9 (t, 1 C) 42.7 (t, 1 C) 44.8 (d, 1 C) 45.9 (t, 1 C) 47.1 (s, 1 C) 52.8 (d, 1 C) 56.1 (d, 1 C) 66.6 (t, 1 C) 66.9 (t, 1 C) 107.8 (d, 1 C) 114.6 (d, 1 C) 129.2 (s, 1 C) 131.4 (s, 1 C) 143.7 (s, 1 C) 144.7 (s, 1 C) 171.2 (s, 1 C) 220.9 (s, 1 C)

$^1$H NMR (501 MHz, CHLOROFORM-*d*): δ ppm 1.02 (s, 3 H) 1.32 - 1.85 (m, 7 H) 1.87 - 1.95 (m, 1 H) 1.98 - 2.06 (m, 1 H) 2.25 - 2.51 (m, 5 H) 2.75 - 2.92 (m, 2 H) 3.42 - 3.51 (m, 2 H) 3.57 - 3.72 (m, 6 H) 3.86 (s, 3 H) 5.52 (s, 1 H) 6.67 (s, 1 H) 6.78 (s, 1 H)

EXAMPLE 9:

4-(2-Ethyl-3-hydroxy-17-oxo-estra-1,3,5(10)-trien-15β-yl)-N-(5-methyl-thiazol-2-yl)-butyramide

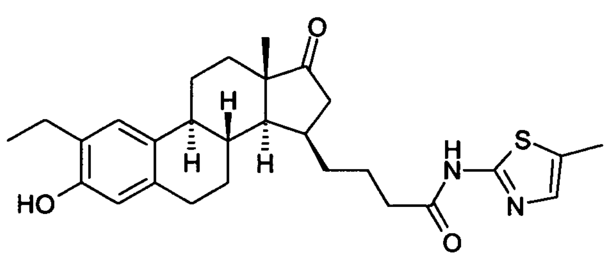

Example 9 was prepared as described above for Example 2 starting from intermediate compound (IVb-(C2-C)-3a), which was converted into the desired amid by amid coupling with 2-amino-5-methylthiazole according to **Flow Diagram Ib:** (Compound (IVb-(C2-C)-3a) (730 mg, 1.89 mmol), EtOAc (150 ml), 2-amino-5-methylthiazole (1.89 mmol), TEA (3.79 mmol), T3P (2.27 mmol). Compound No. 9 (280 mg, 0.58 mmol, 31 %) was obtained after recrystallization from DCM.

$^1$H-NMR (300 MHz, d-DMSO): δ 0.90 (s, 3H, Steroid-$CH_3$), 1.05 (*t, J* = 7.4 Hz, 3H, Ethyl), 1.2-1.4 (*m*, 4H, Steroid), 1.44-1.74 (*m*, 6H, Steroid), 1.82-1.93 (*m*, 1H, Steroid), 2.1-2.7 (*m*, 14H, Steroid), 6.43 (*s*, 1 H, Steroid-Ar-H), 6.89 (*s*, 1 H, Steroid-Ar-H), 7.08 (*d, J* = 1.0 Hz, 1H, Thiazol-H), 8.82 (b*s*, 1 H, NH or OH), 11.82 (b*s*, 1 H, NH or OH) ppm.

EXAMPLE 10:

4-(3-Hydroxy-2-methoxy-17-oxo-estra-1,3,5(10)-trien-15β-yl)-N-(5-methyl-thiazol-2-yl)-butyramide

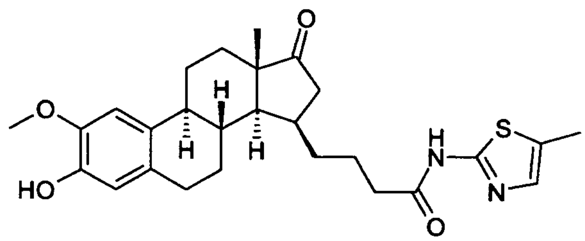

Example 10 was prepared starting from intermediate compound (IVb-(C2-B)-3a), which was converted into the desired amid by amide coupling according to **Flow Diagram Ib** and as described above for Example 4 using 0.07 mmol of (IVb-(C2-B)-3a) and 0.07 mmol 2-amino-5-methylthiazole, yielding Compound No. 10 (33.7mg, MS 484, Rt 3.86).

FURTHER COMPOUNDS

A variety of compounds numbered 11 to 28 and falling under the scope of general formula (I), in which X-A-Y represents -CO-NR$^4$, R$^1$ represents H, R$^{14}$ represents -O-CH$_3$ ,C15 is substituted in the β position and n is 3, were prepared by parallel chemistry using a reaction according to general flow diagram **Ib** and as described in EXAMPLE 4.

TABLE 16

| 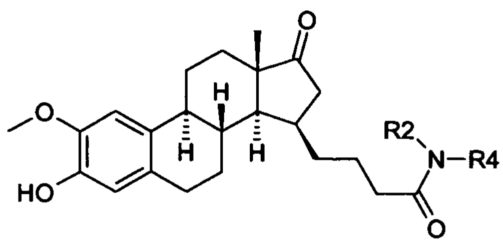 | | | | |
|---|---|---|---|---|
| **No.** | **R$^2$** | **R$^4$** | **MW** | **HPLC Rt [min]** |
| | 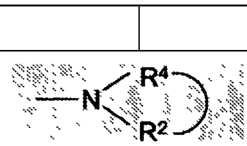 | | | |
| 11 | Cyclopropyl | H | 425.6 | 3.48 |
| 12 | Cyclohexyl | H | 467.6 | 3.90 |
| 13 | Benzo[1,3]dioxol-5-ylmethyl | H | 519.6 | 3.73 |
| 14 | 2-Pyridin-2-yl-ethyl | H | 490.6 | 3.43 |
| 15 | Pyridin-3-yl-methyl | H | 476.6 | 3.36 |
| 16 | 2-Methoxy-ethyl | H | 443.6 | 3.41 |
| 17 | 2,4-bifluorobenzyl | H | 511.6 | 3.88 |
| 18 | 3,5-bimethoxy-benzyl | H | 535.7 | 3.78 |
| 19 | 2-(7-Methyl-1H-indol-3-yl)-ethyl | H | 542.7 | 3.92 |
| 20 | 1-Methyl-1H-imidnzol-4-ylmethyl | H | 479.6 | 3.18 |
| 21 | Piperidin-1-yl | | 453.6 | 3.90 |
| 22 | Methyl | Benzyl | 489.7 | 4.07 |

(continued)

| No. | $R^2$ | $R^4$ | MW | HPLC Rt [min] |
|---|---|---|---|---|
| | 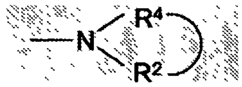 | | | |
| 23 | Ethyl | Ethyl | 441.6 | 3.84 |
| 24 | Methyl | 2-(3,4-Dimethoxy-phenyl)-ethyl | 563.7 | 3.87 |
| 25 | 4-Isopropyl-piperazin-1-yl | | 496.7 | 3.19 |
| 26 | 3,4-Difluoro-phenyl | H | 497.6 | 4.15 |
| 27 | 3-Chloro-phenyl | H | 496.0 | 4.24 |
| 28 | 3-Trifluoromethoxy-phenyl | H | 545.6 | 4.40 |

A variety of compounds numbered 29 to 89 and falling under the scope of general formula (I), in which X-A-Y represents -CO-NR$^4$, R$^1$ represents H, and n is 3, were prepared by parallel chemistry using a reaction according to general flow diagram Ib.

Synthesis Protocol: 0.07 mmol of the individual amine was weight out into a reaction flask. A solution of 0.07 mmol of the respective steroidal building block (IVb-(C2-G)-3a), (IVa-(C2-D)-3a), and (IVa-(C2-B)-3a) in 5 ml DCM were added. Then, 0.077 mmol polymer bound HOBT, 0.231 mmol polymer bound NMM and 0.154 mmol polymer bound EDCI were added. The reaction mixture was stirred for 24 h at ambient temperature. Afterwards, the reaction mixture was filtrated, washed twice with 1 ml DCM and evaporated to dryness. The crude product was treated with 2 ml THF, 10 mg LiOH and 0.5 ml water. After evaporation and further extraction (EtOAc and 0.1 M $KHSO_4$) approx. 50 mg trisaminoeethlyamine polymer bound were added yielding the desired product after filtration and evaporating to dryness.

TABLE 17

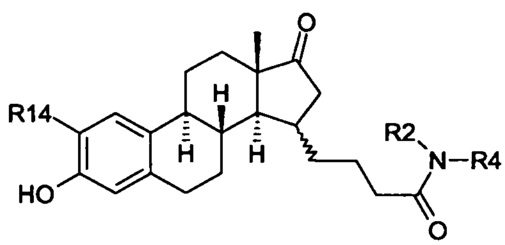

| No. | $R^{14}$ | C15 stereo | $R^2$ | $R^4$ | MW | HPLC Rt [min] |
|---|---|---|---|---|---|---|
| | | | 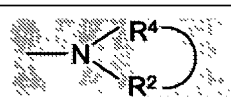 | | | |
| 29 | propyl | beta | cyclohexyl | H | 479.7 | 6.09 |
| 30 | propyl | beta | Benzo[1,3]dioxol-5-ylmethyl | H | 531.7 | 5.90 |
| 31 | propyl | beta | 2-pyridin-2-yl-ethyl | H | 502.7 | 5.46 |
| 32 | propyl | beta | pyridin-3-ylmethyl | H | 488.7 | 5.36 |
| 33 | propyl | beta | 3,5-Dimethoxy-benzyl | H | 547.7 | 5.96 |
| 34 | propyl | beta | 2-(7-methyl-1H-indol-3-yl)-ethyl | H | 554.8 | 6.12 |
| 35 | propyl | beta | 1-methyl-1H-imidazol-4-ylmethyl | H | 491.7 | 5.12 |
| 36 | propyl | beta | piperidin-1-yl | | 465.7 | 6.05 |
| 37 | propyl | beta | methyl | benzyl | 501.7 | 6.27 |
| 38 | propyl | beta | 2-(3,4-Dimethoxy-phenyl)-ethyl | H | 575.8 | 6.01 |

(continued)

| No. | $R^{14}$ | C15 stereo | $R^2$ | $R^4$ | MW | HPLC Rt [min] |
|---|---|---|---|---|---|---|
| | | | 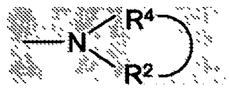 | | | |
| 39 | propyl | beta | 4-isopropyl-piperazin-1-yl | | 508.7 | 5.10 |
| 40 | propyl | beta | 1H-indazol-6-yl | H | 513.7 | 6.26 |
| 41 | propyl | beta | 2-methoxy-ethyl | H | 455.3 | 5.78 |
| 42 | propyl | beta | 2,4-difluorobenzyl | H | 523.3 | 6.50 |
| 43 | ethoxy | alpha | cyclopropyl | H | 439.6 | 5.06 |
| 44 | ethoxy | alpha | cyclohexyl | H | 481.7 | 5.67 |
| 45 | ethoxy | alpha | Benzo[1,3]dioxol-5-ylmethyl | H | 533.7 | 5.49 |
| 46 | ethoxy | alpha | 2-pyridin-2-yl-ethyl | H | 504.7 | 4.98 |
| 47 | ethoxy | alpha | pyridin-3-ylmethyl | H | 490.6 | 4.89 |
| 48 | ethoxy | alpha | benzyl | H | 489.7 | 5.57 |
| 49 | ethoxy | alpha | 2-methoxy-ethyl | H | 457.6 | 4.93 |
| 50 | ethoxy | alpha | 2,4-difluorobenzyl | H | 525.6 | 5.70 |
| 51 | ethoxy | alpha | 3,5-Dimethoxy-benzyl | H | 549.7 | 5.55 |
| 52 | ethoxy | alpha | 7-methyl-1H-indol-3-yl | H | 556.7 | 5.70 |
| 53 | ethoxy | alpha | 1-methyl-1H-imidazol-4-ylmethyl | H | 493.6 | 4.58 |
| 54 | ethoxy | alpha | morpholin-4-yl | | 469.6 | 5.07 |
| 55 | ethoxy | alpha | piperidin-1-yl | | 467.6 | 5.63 |
| 56 | ethoxy | alpha | methyl | benzyl | 503.7 | 5.89 |
| 57 | ethoxy | alpha | 2-(3,4-Dimethoxy-phenyl)-ethyl | H | 577.8 | 5.60 |
| 58 | ethoxy | alpha | 4-isopropyl-piperazin-1-yl | | 510.7 | 4.56 |
| 59 | ethoxy | alpha | 1H-indazol-6-yl | H | 515.7 | 5.88 |
| 60 | ethoxy | alpha | Benzo[1,3]dioxol-5-yl | H | 519.6 | 5.63 |
| 61 | ethoxy | alpha | 3-Cyano-phenyl | H | 500.6 | 5.74 |
| 62 | ethoxy | alpha | 3,4-Difluoro-phenyl | H | 511.6 | 5.98 |
| 63 | ethoxy | alpha | 5-methyl-thiazol-2-yl | H | 496.7 | 5.58 |
| 64 | ethoxy | alpha | ethyl | ethyl | 455.3 | 5.98 |
| 65 | methoxy | alpha | 3-chloro-phenyl | H | 509.2 | 6.56 |
| 66 | methoxy | alpha | cyclopropyl | H | 425.6 | 4.78 |
| 67 | methoxy | alpha | cyclohexyl | H | 467.6 | 5.42 |
| 68 | methoxy | alpha | Benzo[1,3]dioxol-5-ylmethyl | H | 519.6 | 5.25 |
| 69 | methoxy | alpha | 2-pyridin-2-yl-ethyl | H | 490.6 | 4.72 |
| 70 | methoxy | alpha | pyridin-3-ylmethyl | H | 476.6 | 4.63 |
| 71 | methoxy | alpha | benzyl | H | 475.6 | 5.32 |
| 72 | methoxy | alpha | 2-methoxy-ethyl | H | 443.6 | 4.65 |
| 73 | methoxy | alpha | 2,4-difluorobenzyl | H | 511.6 | 5.46 |

(continued)

| No. | $R^{14}$ | C15 stereo | $R^2$ | $R^4$ | MW | HPLC Rt [min] |
|---|---|---|---|---|---|---|
| | | | 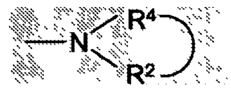 | | | |
| 74 | methoxy | alpha | 3,5-Dimethoxy-benzyl | H | 535.7 | 5.31 |
| 75 | methoxy | alpha | 7-methyl-1H-indol-3-yl | H | 542.7 | 5.48 |
| 76 | methoxy | alpha | 1-methyl-1H-imidazol-4-ylmethyl | H | 479.6 | 4.30 |
| 77 | methoxy | alpha | morpholin-4-yl | | 455.6 | 4.79 |
| 78 | methoxy | alpha | piperidin-1-yl | | 453.6 | 5.35 |
| 79 | methoxy | alpha | methyl | benzyl | 489.7 | 5.63 |
| 80 | methoxy | alpha | ethyl | ethyl | 441.6 | 5.29 |
| 81 | methoxy | alpha | 2-(3,4-Dimethoxy-phenyl)-ethyl | H | 563.7 | 5.35 |
| 82 | methoxy | alpha | 4-isopropyl-piperazin-1-yl | | 496.7 | 4.30 |
| 83 | methoxy | alpha | 1H-indazol-6-yl | H | 501.6 | 5.63 |
| 84 | methoxy | alpha | 5-methyl-thiazol-2-yl | H | 482.6 | 5.34 |
| 85 | methoxy | alpha | 3,4-dihydroxybenzyl | H | 507.3 | 4.87 |
| 86 | methoxy | alpha | Benzo[1,3]dioxol-5-yl | H | 505.3 | 5.71 |
| 87 | methoxy | alpha | 3-Cyano-phenyl | H | 486.3 | 5.85 |
| 88 | methoxy | alpha | 3,4-Difluoro-phenyl | H | 497.2 | 6.13 |
| 89 | methoxy | alpha | 3-chloro-phenyl | H | 495.2 | 6.27 |

**II. Compounds carrying a substitution of the C17 oxo function of the steroidal core**

EXAMPLE 90:

4-(17,17-Difluoro-3-hydroxy-estra-1,3,5(10)-trien-15β-yl)-1-morpholin-4-yl-butan-1-one

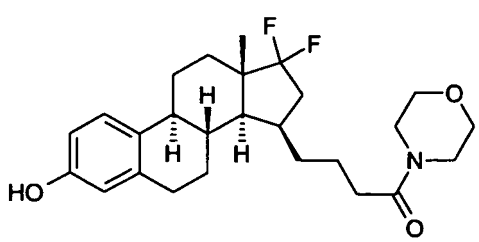

Example 90 was prepared from the Intermediate No. 1 (3-Hydroxy-15β-(4-morpholin-4-yl-4-oxo-butyl)-estra-1,3,5(10)-trien-17-one) according to the reaction depicted in Section D-(I)-(b): To a solution of the estron derivative (106 mg; 250 μmol) in Deoxofluor (0.96 ml; 5.00 mmol) a drop of ethanol was added; the solution was stirred at RT for 5 d. Subsequently, DCM (10 ml) was added and the product was hydrolyzed by addition of sat $NaHCO_3$ solution under ice cooling. For work up, the organic phase was separated off, and the remaining water phase was extracted with DCM. The combined DCM fractions were dried over $MgSO_4$. After evaporation and subsequent purification using column chromatography (DCM/Ether 1:1), 81 mg of a colourless solid were obtained (MW 489.64).

$^{13}C$ NMR (126 MHz, CHLOROFORM-d): δ ppm 17.0 (q, $J_{(C,F)}$, 1 C) 24,5 (t, 1 C) 25.0 (t, 1 C) 27,4 (t, 1 C) 29.3 (t, $J_{(C,F)}$,1 C) 30.7 (t, 1 C) 31.7 (t, 1 C) 32,9 (d, $J_{(C,F)}$,1 C) 34.4 (t, 1 C) 35.9 (d, 1 C) 39.7-40.5 (t, J $_{(C,F)}$, 1 C) 42.0 (t, 1 C) 44.1 (d, 1 C) 45.2 (t, 1 C) 46,1 (s, $J_{(C,F)}$, 1 C) 50,3 (d, $J_{(C,F)}$, 1 C) 66.7 (t, 1 C) 67.0 (t, 1 C) 112.7 (d, 1 C) 115.3 (d, 1

C) 126.0 (d, 1 C) 132.3 (s, 1 C) 138.0 (s, 1 C) 153.9 (s, 1 C) 171.5 (s, 1 C)

$^{1}H$ NMR (501 MHz, CHLOROFORM-*d*): δ ppm 0.99 (s, 3 H) 1.23 - 2.5 (m, 18 H) 2.82 (m, 2 H) 3.43 - 3.52 (m, 2 H) 3.59 - 3.73 (m, 6 H) 5.66 (br. s., 1 H) 6.57 (d, *J*=2.55 Hz, 1 H) 6.63 (dd, *J*=8.5, 2.5 Hz, 1 H) 7.10 (d, *J*=8.5 Hz, 1 H)

EXAMPLE 91:

4-(17,17-Difluoro-3-hydroxy-estra-1,3,5(10)-trien-15a-yl)-1-morpholin-4-yl-butan-1-one

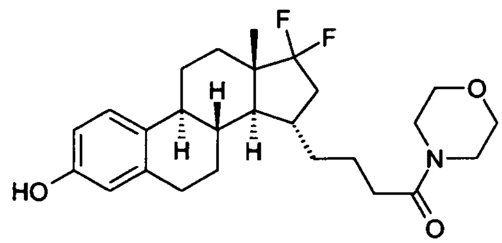

Example 91 was prepared from the Intermediate No. 40 named 3-Hydroxy-15α-(4-morpholin-4-yl-4-oxo-butyl)-estra-1,3,5(10)-trien-17-one as described for Example 90.

$^{13}C$ NMR (126 MHz, CHLOROFORM-*d*): δ ppm 14.9 (q, $J_{(C,F)}$=3.89 Hz, 1 C) 23.8 (t, 1 C) 26.2 (t, 1 C) 27.6 (t, 1 C) 28.6 (t, $J_{(C,F)}$=4.67 Hz, 1 C) 29.7 (t, 1 C) 33.2 (t, 1 C) 35.9 (d, $J_{(C,F)}$=6.75 Hz, 1 C) 36.8 (t, 1 C) 39.4 (d, 1 C) 39.5 - 40.0 (t, $J_{(C,F)}$, 1 C) 42.1 (t, 1 C) 43.8 (d, 1 C) 46.1 (t, 1 C) 46.8 (s, $J_{(C,F)}$=19.98 Hz, 1 C) 53.1 (d, $J_{(C,F)}$=4.15 Hz, 1 C) 66.6 (t, 1 C) 66.9 (t, 1 C) 113.1 (d, 1 C) 115.1 (d, 1 C) 126.8 (d, 1 C) 128.9 - 133.4 (s, $J_{(C,F)}$, 1 C) 131.5 (s, 1 C) 137.5 (s, 1 C) 154.0 (s, 1 C) 171.8 (s, 1 C)

$^{1}H$ NMR (501 MHz, CHLOROFORM-*d*): δ ppm 0.92 (s, 3 H) 1.19 - 1.36 (m, 3 H) 1.38 - 1.50 (m, 1 H) 1.51-1.83 (m, 7H) 1.86-1.99 (m, 2H) 2.13-2.23 (m, 1 H) 2.28 - 2.53 (m, 4 H) 2.70 - 2.83 (m, 2 H) 3.43 - 3.52 (m, 2 H) 3.59 - 3.73 (m, 6 H) 6.06 (br. s., 1 H) 6.55 (d, *J*=2.75 Hz, 1 H) 6.64 (dd, *J*=8.54, 2.75 Hz, 1 H) 7.12 (d, *J*=8.24 Hz, 1 H)

EXAMPLE 92:

4-(17-Fluoro-3-hydroxy-estra-1,3,5(10),16-tetraen-15β-yl)-1-morpholin-4-yl-butan-1-one

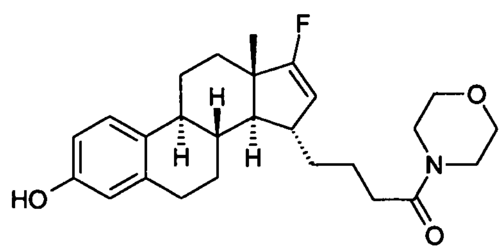

Example 92 was isolated as by-product during the synthesis of Example No. 91.

$^{1}H$ NMR (501 MHz, CHLOROFORM-*d*): δ ppm 0.99 (s, 3 H) 1.23 - 1.85 (m, 9 H) 1.93 - 2.00 (m, 1 H) 2.12 - 2.20 (m, 1 H) 2.25 - 2.48 (m, 5 H) 2.68 - 2.84 (m, 2 H) 3.44 - 3.53 (m, 2 H) 3.63 - 3.70 (m, 6 H) 4.83 - 4.87 (m, 1 H) 6.55 (d, *J*=2.4 Hz, 1 H) 6.63 (dd, *J*=8.5, 2.7 Hz, 1 H) 7.08 (d, *J*=8.5 Hz, 1 H)

$^{13}C$ NMR (126 MHz, CHLOROFORM-*d*): δ ppm 17.1 (q, $J_{C,F}$ =4.2 Hz, 1 C) 23.3 (t, 1 C) 26.4 (t, 1 C) 27.8 (t, 1 C) 29.7 (t, 1 C) 32.9 (t, 1 C) 33.3 (t, 1 C) 34.2 (t, 1 C) 37.8 (d, 1 C) 40.4 (d, $J_{C,F}$ =5.7 Hz, 1 C) 42.1 (t, 1 C) 44.3 (s, $J_{C,F}$ =20.5 Hz, 1 C) 44.4 (d, 1 C) 46.1 (t, 1 C) 57.9 (d, $J_{C,F}$ =5.2 Hz, 1 C) 66.6 (t, 1 C) 66.9 (t, 1 C) 104.2 (d, $J_{C,F}$ =8.0 Hz, 1 C) 112.9 (d, 1 C) 115.1 (d, 1 C) 126.3 (d, 1 C) 131.8 (s, 1 C) 137.4 (s, 1 C) 154.1 (s, 1 C) 170.7 (s, $J_{C,F}$ =289.9 Hz, 1 C) 172.0 (s, 1 C)

EXAMPLE 93:

3-(17,17-Difluoro-3-hydroxy-estra-1,3,5(10)-trien-15β-yl)-N-(5-methyl-thiazol-2-yl)-propionamide

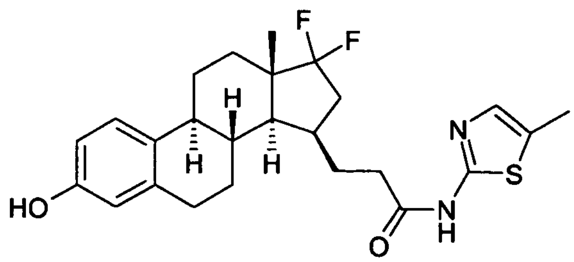

Example 93 was prepared from the Intermediate IVβ-2a-D1 F2 using amide coupling with 2-amino-5-methylthiazole according to general flow diagram Ib and as described for synthesis of Example 4.

$^{1}$H NMR (501 MHz, DMSO-$d_6$): δ ppm 1.01 (s, 3 H) 1.28 - 1.37 (m, 2 H) 1.47 - 1.74 (m, 5 H) 1.86 - 1.96 (m, 1 H) 1.97 - 2.04 (m, 1 H) 2.06 - 2.22 (m, 3 H) 2.24 - 2.39 (m, 6 H) 2.40 - 2.48 (m, 1 H) 2.68 - 2.84 (m, 2 H) 6.47 (d, *J*=2.4 Hz, 1 H) 6.52 (dd, *J*=8.4, 2.6 Hz, 1 H) 7.01 - 7.05 (m, 1 H) 7.09 - 7.11 (m, 1 H) 8.96 - 9.03 (m, 1 H) 11.86 (s, 1 H)

$^{13}$C NMR (126 MHz, DMSO-$d_6$): δ ppm 11.0 (q, 1 C) 16.6 - 16.7 (q, $J_{C,F}$, 1 C) 24.5 (t, 1 C) 26.8 (t, 1 C) 26.8 (t, 1 C) 28.8 (t, 1 C) 30.4 (t, $J_{C,F}$ =4.9 Hz, 1 C) 33.4 (d, $J_{C,F}$ =6.7 Hz, 1 C) 34.2 (t, 1 C) 35.5 (d, 1 C) 38.5 - 38.8 (t, $J_{C,F}$ , 1 C) 43.7 (d, 1 C) 44.5 - 45.0 (s ,$J_{C,F}$, 1 C) 49.7 (d, $J_{C,F}$ =4.9 Hz, 1 C) 112.6 (d, 1 C) 114.9 (d, 1 C) 125.6 (d, 1 C) 125.9 (s, 1 C) 130.1 (s, 1 C) 130.6 - 134.7 (s, $J_{C,F}$, 1 C) 134.6 (d, 1 C) 137.1 (s, 1 C) 155.0 (s, 1 C) 156.1 (s, 1 C) 170.7 (s, 1 C)

EXAMPLE 94:

4-(17-Trifluoromethyl-3-hydroxy-estra-1,3,5(10),16-tetraen-15β-yl)-1-morpholin-4-yl-butan-1-one

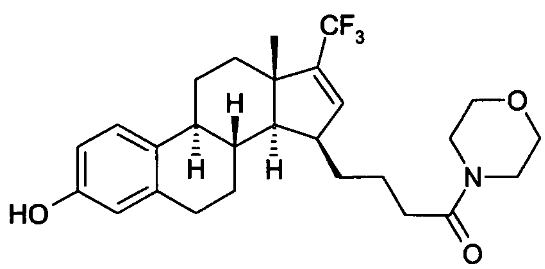

Example 94 was prepared from intermediate Vβ-3a-D-(I)-(d)-$CF_3$ by amide coupling according to general flow diagram Ia: To the solution of Vβ-3a-D-(I)-(d)-$CF_3$ in DCM, a large excess of the Hünig base $N(iPr)_2Et$ and morpholine was added. The solution was stirred over night at ambient temperature. After dilution with further DCM and washing twice with 1 M $KHSO_4$, the organic layer was dried over $Na_2SO_4$ and evaporated to dryness. The crude material was purified by flash chromatography with DCM/EtOAc and by preparative HPLC yielding 15 mg of compound No. 95 as white solid.

$^{13}$C NMR (126 MHz, CHLOROFORM-*d*): δ ppm 22.2 (q, 1 C) 24.7 (t, 1 C) 25.6 (t, 1 C) 27.3 (t, 1 C) 29.1 (t, 1 C) 29.3 (t, 1 C) 33.1 (t, 1 C) 35.1 (d, 1 C) 36.1 (t, 1 C) 42.1 (t, 1 C) 44.2 (d, 1 C) 44.6 (d, 1 C) 46.1 (t, 1 C) 46.3 (s, 1 C) 57.6 (d, 1 C) 66.7 (t, 1 C) 67.0 (t, 1 C) 112.7 (d, 1 C) 115.3 (d, 1 C) 122.4 - 124.8 (s, 1 C) 125.8 (d, 1 C) 132.5 (s, 1 C) 137.9 (s, 1 C) 138.5 (d, *J*=5.7 Hz, 1 C) 143.3 - 144.3 (s, 1 C) 153.9 (s, 1 C) 171.5 (s, 1 C)

$^{1}$H NMR (501 MHz, CHLOROFORM-*d*): δ ppm 1.14 (s, 3 H) 1.32 - 1.78 (m, 8 H) 1.84 (dd, *J*=11.9, 7.3 Hz, 1 H) 1.95 - 2.04 (m, 2 H) 2.27 - 2.36 (m, 4 H) 2.57 - 2.64 (m, 1 H) 2.80 - 2.88 (m, 2 H) 3.44 - 3.51 (m, 2 H) 3.62 - 3.71 (m, 6 H) 5.40 - 5.73 (m, 1 H) 6.45 - 6.48 (m, 1 H) 6.58 (d, *J*=2.7 Hz, 1 H) 6.63 (dd, *J*=8.2, 2.7 Hz, 1 H) 7.09 (d, *J*=8.2 Hz, 1 H)

EXAMPLE 95:

4-(17-Difluoromethylene-3-hydroxy-estra-1,3,5(10)-trien-15β-yl)-1-morpholin-4-yl-butan-1-one

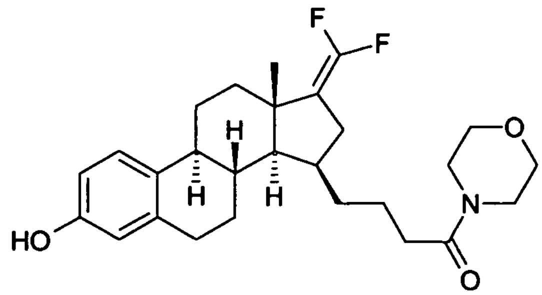

Example 95 was prepared from intermediate Vβ-3a-D-(I)-(a)=$CF_2$ by amide coupling according to general flow diagram Ia: To the solution of Vβ-3a-D-(I)-(a)=$CF_2$ in DCM, a large excess of the Hünig base $N(iPr)_2Et$ and morpholine was added. The solution was stirred over night at ambient temperature. After dilution with further DCM and washing twice with 1 M $KHSO_4$, the organic layer was dried over $Na_2SO_4$ and evaporated to dryness. The crude material was purified by flash chromatography with DCM/EtOAc and by preparative HPLC yielding 15 mg of compound No. 95 as white solid.

$^1$H NMR (501 MHz, CHLOROFORM-*d*): δ ppm 1.05 (s, 3 H) 1.18 - 1.30 (m, 1 H) 1.32 - 1.81 (m, 7 H) 1.87 - 1.97 (m, 1 H) 2.03 - 2.15 (m, 2 H) 2.18 - 2.36 (m, 4 H) 2.53 - 2.68 (m, 1 H) 2.77 - 2.91 (m, 2 H) 3.43 - 3.53 (m, 2 H) 3.59 - 3.72 (m, 6 H) 5.53 (br. s., 1 H) 6.57 (d, *J*=2.44 Hz, 1 H) 6.63 (dd, *J*=8.39, 2.59 Hz, 1 H) 7.11 (d, *J*=8.24 Hz, 1 H)

$^{13}$C NMR (126 MHz, CHLOROFORM-*d*): δ ppm 20.9 (q, $J_{(C,F)}$=2.60 Hz, 1 C) 25.3 (t, 1 C) 26.2 (t, 1 C) 27.5 (t, 1 C) 29.5 (t, 1 C) 31.0 (t, 1 C) 31.3 (t, 1 C) 33.1 (t, 1 C) 35.8 (d, 1 C) 37.7 (d, 1 C) 38.0 (t, $J_{(C,F)}$=4.15 Hz, 1 C) 42.1 (t, 1 C) 42.3 (s, $J_{(C,F)}$=2.98 Hz, 1 C) 44.3 (d, 1 C) 46.1 (t, 1 C) 57.7 (d, 1 C) 66.7 (t, 1 C) 67.0 (t, 1 C) 99.2 (s, $J_{(C,F)}$=17.26 Hz, 1 C) 112.7 (d, 1 C) 115.3 (d, 1 C) 126.0 (d, 1C) 132.5 (s, 1C) 138.0 (s, 1 C) 148.3-153.0 (s, $J_{(C,F)}$, 1 C) 153.8 (s, 1 C) 171.7 (s, 1 C)

EXAMPLE 96:

4-(17-Difluoromethylene-3-hydroxy-estra-1,3,5(10)-trien-15α-yl)-1-morpholin-4-yl-butan-1-one

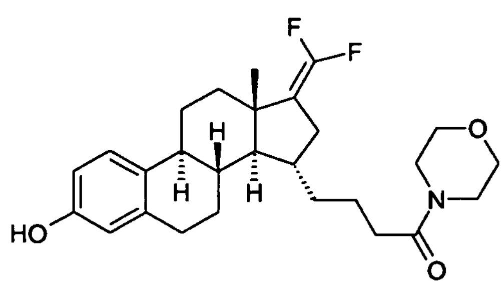

Example 96 can be prepared from the Intermediate No. 40 named 3-Hydroxy-15α-(4-morpholin-4-yl-4-oxo-buty)-estra-1,3,5(10)-trien-17-one of formula (VIα-3a)-40 according to the reaction depicted in Section D-(I)-(a) /1.

EXAMPLE 97:

4-(17-Trifluoromethyl-3-hydroxy-estra-1,3,5(10),16-tetraen-15α-yl)-1-morpholin-4-yl-butan-1-one

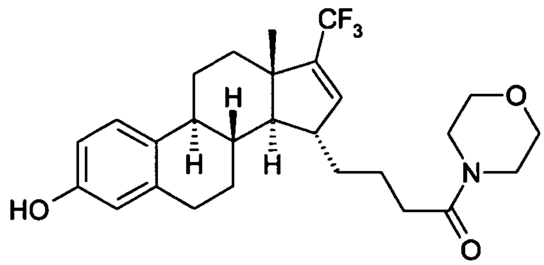

Example 97 can be prepared from the Intermediate No. 40 named 3-Hydroxy-15α-(4-morpholin-4-yl-4-oxo-butyl)-estra-1,3,5(10)-trien-17-one of formula (VIα-3a)-40 according to the reaction depicted in Section D-(I)-(d) /3.

EXAMPLE 98:

4-(17-Trifluoromethyl-3-hydroxy-estra-1,3,5(10)-trien-15α-yl)-1-morpholin-4-yl-butan-1-one

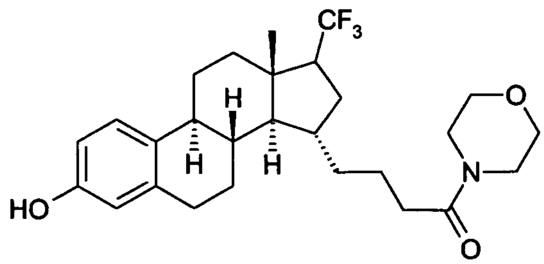

Example 98 can be prepared from Example 16 according to the last reaction step depicted in Section D-(I)-(c) /3.

EXAMPLE 99: 4-(17-Difluoromethyl-2-ethyl-3-hydroxy-estra-1,3,5(10)-trien-15β-yl)-N-(5-methyl-thiazol-2-yl)-butyramide

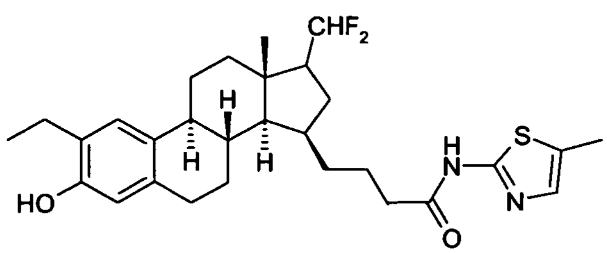

Example 99 can be prepared starting from Example 9 (4-(2-Ethyl-3-hydroxy-17-oxo-estra-1,3,5(10)-trien-15β-yl)-N-(5-methyl-thiazol-2-yl)-butyramide) as educt, according to the reaction depicted in Section D-(I)-(c) /2.

EXAMPLE 100:

4-(17-Difluoromethyl-3-hydroxy-estra-1,3,5(10),16-tetraen-15β-yl)-1-morpholin-4-yl-butan-1-one

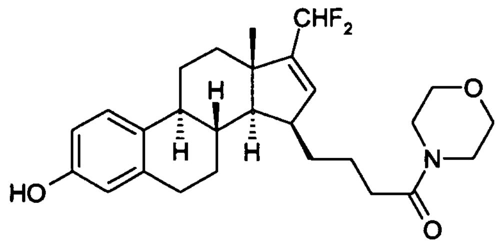

Example 100 can be prepared starting from Example 95 (4-(17-Difluoromethylene-3-hydroxy-estra-1,3,5(10)-trien-15β-yl)-1-morpholin-4-yl-butan-1-one) according to the reaction scheme depicted in Section D-(I)-(d) /2.

FURTHER COMPOUNDS

A variety of compounds numbered 101 to 138 and falling under the scope of general formula (I), in which X-A-Y represents -CO-$NR^4$, $R^1$ represents -H, $R^{14}$ represents -H, and the C17 keto function is replaced by a difluoro group, was prepared by parallel chemistry using a reaction according to general flow diagram Ib starting from the already fluorinated intermediates IVα-3a-D1 F2 and IVβ-2a-D1F2, respectively.

Synthesis Protocol: 0.07 mmol of the individual amine was weight out into a reaction flask. A solution of 0.07 mmol of the respective steroidal building block (IVα-3a-D1F2 and IVβ-2a-D1F2), 0.077 mmol HOBT, 0.231 mmol NMM and 0.154 mmol polymer bound EDCI in 5 ml DCM were added. The reaction mixture was stirred for 24 h at ambient temperature. The solvent was removed in a vacuum centrifuge at 40˚C. Than 4 ml EtOAc and 4 ml $H_2O$ were added. The two phases were stirred vigorously for 2 min, than the organic phase was dried with $Na_2SO_4$ and evaporated in a vacuum centrifuge at 40˚C. After treatment of the crude product with 2 ml THF, 10 mg LiOH and 0.5 ml water, the solvent was evaporated and the residue further extracted (EtOAc and 0.1 M $KHSO_4$). Then, 50 mg polymer bound trisaminoeethlyamine was added yielding after filtration and evaporating to dryness the desired product. If still necessary, products were further purified by flash chromatography (4 g silica gel, eluent EtOAc / cyclohexane).

TABLE 18:

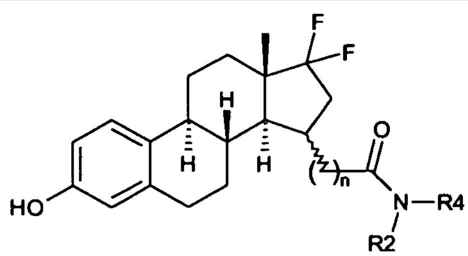

| No | n | C15 stereo | $R^2$ | $R^4$ | MW | HPLC Rt [min] |
|---|---|---|---|---|---|---|
| | | | 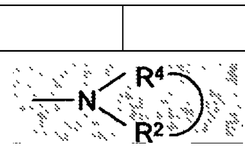 | | | |
| **101** | 3 | alpha | Cyclopropyl | H | 417 | 5.9 |
| **102** | 3 | alpha | Cyclohexyl | H | 459 | 6.58 |
| **103** | 3 | alpha | Benzo[1,3]dioxol-5-ylmethyl | H | 511 | 6.3 |
| **104** | 3 | alpha | 2-Pyridin-2-yl-ethyl | H | 482 | 5.77 |
| **105** | 3 | alpha | Pyridin-3-yl-methyl | H | 468 | 5.67 |
| **106** | 3 | alpha | Benzyl | H | 467 | 6.41 |
| **107** | 3 | alpha | 2-Methoxy-ethyl | H | 435 | 5.76 |
| **108** | 3 | alpha | 2,4-Difluorobenzyl | H | 503 | 6.52 |
| **109** | 3 | alpha | 3,4-dihydroxy-benzyl | H | 499 | 5.65 |

(continued)

| No | n | C15 stereo | $R^2$ | $R^4$ | MW | HPLC Rt [min] |
|---|---|---|---|---|---|---|
| | | | 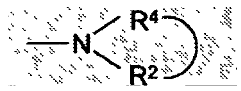 | | | |
| **110** | 3 | alpha | 3,5-Dimethoxy-benzyl | H | 527 | 6.37 |
| **111** | 3 | alpha | 2-(7-Methyl-1H-indol-3-yl)-ethyl | H | 534 | 6.53 |
| **112** | 3 | alpha | 1-Methyl-1H-imidazol-4-ylmethyl | H | 471 | 5.33 |
| **113** | 3 | alpha | Piperidin-1-yl | | 445 | 6.65 |
| **114** | 3 | alpha | Methyl | Benzyl | 481 | 6.84 |
| **115** | 3 | alpha | Ethyl | Ethyl | 433 | 6.54 |
| **116** | 3 | alpha | Methyl | 2-(3,4-Dimethoxyphenyl)-ethyl | 555 | 6.52 |
| **117** | 3 | alpha | 4-Isopropyl-piperazin-1-yl | | 488 | 5.38 |
| **118** | 3 | alpha | Benzo[1,3]dioxol-5-yl | H | 497 | 6.51 |
| **119** | 3 | alpha | 5-methyl-thiazol-2-yl | H | 474 | 6.51 |
| **120** | 2 | beta | Cyclopropyl | H | 403 | 5.76 |
| **121** | 2 | beta | Cyclohexyl | H | 445 | 6.48 |
| **122** | 2 | beta | Benzo[1,3]dioxol-5-ylmethyl | H | 497 | 6.17 |
| **123** | 2 | beta | 2-Pyridin-2-yl-ethyl | H | 468 | 5.6 |
| **124** | 2 | beta | Pyridin-3-yl-methyl | H | 454 | 5.51 |
| **125** | 2 | beta | Benzyl | H | 453 | 6.28 |
| **126** | 2 | beta | 2-Methoxy-ethyl | H | 421 | 5.6 |
| **127** | 2 | beta | 3,4-dihydroxy-benzyl | H | 485 | 5.52 |
| **128** | 2 | beta | 3,5-Dimethoxy-benzyl | H | 513 | 6.25 |
| **129** | 2 | beta | 2-(7-Methyl-1H-indol-3-yl)-ethyl | H | 520 | 6.37 |
| **130** | 2 | beta | 1-Methyl-1H-imidazol-4-ylmethyl | H | 457 | 5.17 |
| **131** | 2 | beta | Piperidin-1-yl | | 431 | 6.47 |
| **132** | 2 | beta | Methyl | Benzyl | 467 | 6.72 |
| **133** | 2 | beta | Ethyl | Ethyl | 419 | 6.42 |
| **134** | 2 | beta | Methyl | 2-(3,4-Dimethoxyphenyl)-ethyl | 541 | 6.4 |
| **135** | 2 | beta | 4-Isopropyl-piperazin-1-yl | | 474 | 5.18 |
| **136** | 2 | beta | Benzo[1,3]dioxol-5-yl | H | 483 | 6.37 |
| **137** | 2 | beta | 5-methyl-thiazol-2-yl | H | 433 | 5.82 |
| **138** | 2 | beta | 2-methoxy-ethyl | 2-methoxy-ethyl | 479.6 | |

**III. Compounds with a heterocyclus fused to the steroidal D-ring**

EXAMPLES 151 to 165 were prepared from the corresponding intermediates (e.g. No. 1, 3A, 39, 1, 40 etc.) using the reaction scheme as depicted in SECTION D-(II). Alternatively, depending on the nature of the C15 side chain, some of the reaction steps had to be carried out after having introduced the heterocyclic ring system, i.e. the 15,16-unsaturated intermediate (X) was derivatized to the appropriate acid or alkenyl intermediate (see e.g. SCHEMES 7B, 7C, 8A and 8B). Then, the heterocyclic ring system was introduced including the C16-C17 carbon atoms attached to the D-ring. The so-obtained intermediates were then used for further modification and amidation of the C15 side chain (introduction of the R2/R4 substituents). Finally the protection group in C3 position was cleaved off.

EXAMPLES 151 and 152:

N-Benzyl-4-(3-hydroxy-(17,16-c)-(1'-methyl)-pyrazolyl-estra-1,3,5(10)-trieno-15β-yl)-butyramide

N-Benzyl-4-(3-hydroxy-(17,16-c)-(2'-methyl)-pyrazolyl-estra-1,3,5(10)-trieno-15β-yl)-butyramide

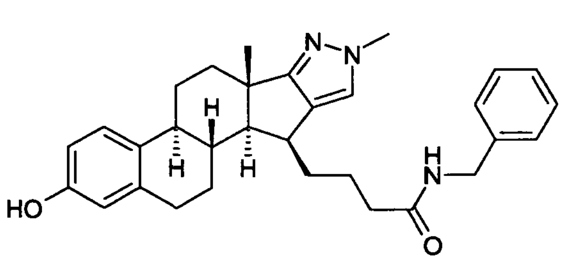

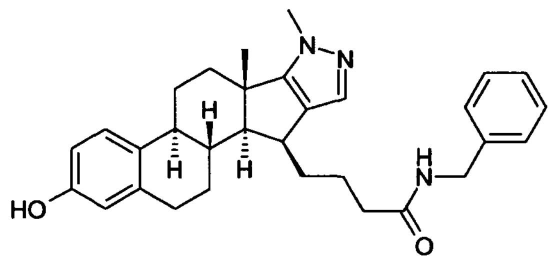

Starting from intermediate compound Xc, an allyl side chain was introduced into C15 position using 1,4-addition of allylbromide according to step 1 of SCHEME 7C, followed by construction of the pyrazol-ring according to D-(II)-(a). Ringdosure with methyl hydrazine gave a mixture of the corresponding isomers. Conversion of the allyl into the N-Benzyl-butyramide side chain was performed according to steps 2 - 4 of SCHEME 7C and to the reaction as depicted in general flow diagram Ib by reaction with benzylamine. Finally the obtained isomers were separated by preparative HPLC.

EXAMPLE 151: $^1$H-NMR (300 MHz, $CDCl_3$): δ 1.10-1.20 (s, 3H), 1.20-2.32 (m, 17H), 2.68-2.88 (m, 3H), 3.72-3.80 (s, 3H), 4.24-4.40 (dd, 2H), 6.48-6.52 (s, 1H), 6.52-6.60 (d, 1 H), 6.96-7.08 (d, 1H), 7.08-7.28 (m, 6H).

EXAMPLE 152: $^1$H-NMR (300 MHz, $CDCl_3$): δ 1.08-1.16 (s, 3H), 1.16-2.40 (m, 17H), 2.68-2.88 (m, 3H), 3.72-3.84 (s, 3H), 4.24-4.44 (dd, 2H), 6.44-6.54 (s, 1H), 6.54-6.60 (d, 1H), 7.00-7.12 (d, 1H), 7.12-7.32 (m, 6H).

EXAMPLES 153 and 154:

3-Hydroxy-15β-(4-morpholin-4-yl-4-oxo-butyl)-estra-1,3,5(10)-trien-[16,17-c]-(1'-methyl)-pyrazole

3-Hydroxy-15β-(4-morpholin-4-yl-4-oxo-butyl)-estra-1,3,5(10)-trien-[16,17-c]-(2'-methyl)-pyrazole

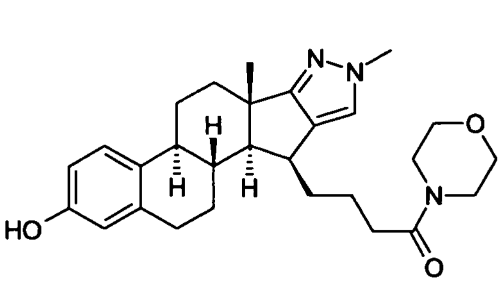

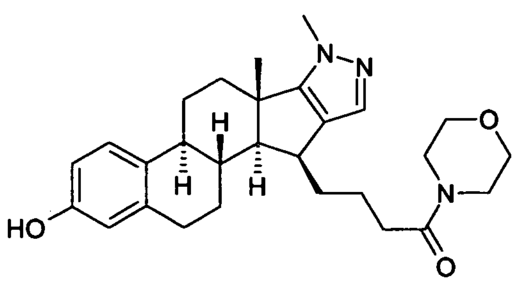

Examples 153 and 154 were prepared according to the procedure described for Examples 151 and 152 using morpholine as amine for the amide coupling step.

EXAMPLE 153: $^1$H-NMR (300 MHz, $CDCl_3$): δ 1.04-1.20 (s, 3H), 1.24-1.84 (m, 9H), 2.00-2.52 (m, 8H), 2.88-2.96 (m, 3H), 3.40-3.70 (m, 8H), 3.70-3.92 (s, 3H), 6.22-6.44 (m, 2H), 6.96-7.12 (d, 1H), 7.20-7.28 (s, 1H).
EXAMPLE 154: $^1$H-NMR (300 MHz, $CDCl_3$): δ 1.16-1.22 (s, 3H), 1.22-1.88 (m, 9H), 2.04-2.56 (m, 8H), 2.76-2.96 (m, 3H), 3.48-3.70 (m, 8H), 3.72-3.84 (s, 3H), 6.48-6.60 (m, 2H), 7.00-7.12 (d, 1H), 7.16-7.24 (s, 1H).

EXAMPLES 155 and 156:

N-Benzyl-4-(3-hydroxy-(17,16-c)-(1'-methyl)-pyrazolyl-estra-1,3,5(10)-trieno-15α-yl)-butyramide

N-Benzyl-4-(3-hydroxy-(17,16-c)-(2'-methyl)-pyrazolyl-estra-1,3,5(10)-trieno-15α-yl)-butyramide

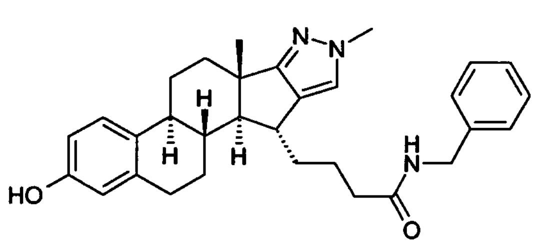
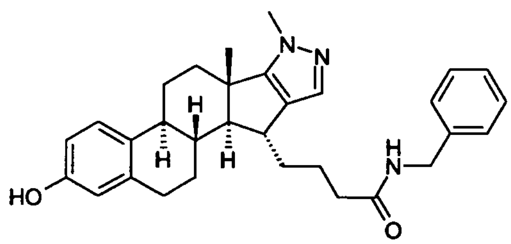

Starting from intermediate compound Xc, an allyl side chain was introduced into C15 position using 1,2-addition of allylbromide and subsequent rearrangement with potassium hydride according to steps 1 and 2 of SCHEME 8B, followed by construction of the pyrazol-ring according to D-(II)-(a). Ringclosure with methyl hydrazine gave a mixture of the corresponding isomers. Conversion of the allyl into the N-Benzyl-butyramide side chain was performed according to steps 3 - 5 of SCHEME 8B and to the reaction as depicted in general flow diagram Ib by reaction with benzylamine. Finally the obtained isomers were separated by preparative HPLC.
EXAMPLE 155: $^1$H-NMR (300 MHz, $CDCl_3$): δ 0.94-1.04 (s, 3H), 1.20-2.50 (m, 17H), 2.60-2.88 (m, 3H), 3.72-3.84 (s, 3H), 4.28-440 (s, 2H), 6.40-6.50 (s, 1H), 6.52-6.60 (d, 1H), 7.04-7.12 (d, 1 H),7.14-7.36 (m, 5H).
EXAMPLE 156: $^1$H-NMR (300 MHz, $CDCl_3$): δ 0.96-1.08 (s, 3H), 1.24-2.48 (m, 17H), 2.64-2.88 (m, 3H), 3.72-3.84 (s, 3H), 4.28-4.44 (s, 2H), 6.40-6.50 (s, 1H), 6.50-6.60 (d, 1H), 7.00-7.12 (d, 1H), 7.12-7.36 (m, 5H).

EXAMPLES 157 and 158:

3-Hydroxy-15α-(4-morpholin-4-yl-4-oxo-butyl)-estra-1,3,5(10)-trien-[16,17-c]-(1'-methyl)-pyrazole

3-Hydroxy-15α-(4-morpholin-4-yl-4-oxo-butyl)-estra-1,3,5(10)-trien-[16,17-c]-(2'-methyl)-pyrazole

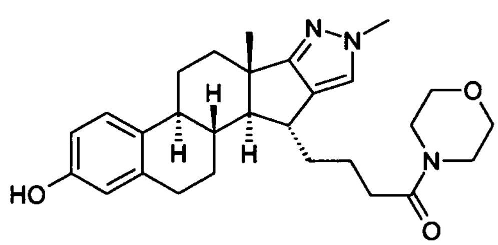
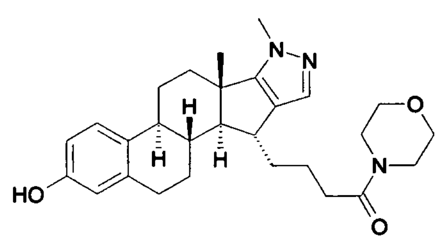

Examples 157 and 158 were prepared according to the procedure described for Examples 155 and 156 using morpholine as amine for the amide coupling step.
EXAMPLE 157: $^1$H-NMR (300 MHz, $CDCl_3$): δ 0.96-1.08 (s, 3H), 1.12-2.36 (m, 17H), 2.72-2.96 (m, 3H), 3.50-3.72 (m, 8H), 3.76-3.86 (s, 3H), 6.44-6.50 (s, 1 H), 6.52-6.60 (d, 1 H), 7.04-7.12 (d, 1H), 7.24-7.30 (s, 1 H).
EXAMPLE 158: $^1$H-NMR (300 MHz, $CDCl_3$): δ 0.92-1.08 (s, 3H), 1.24-2.56 (m, 17H), 2.68-2.92 (m, 3H), 3.22-3.70 (m, 8H), 370-3.88 (s, 3H), 6.40-6.48 (s, 1H), 6.48-6.60 (d, 1H), 7.00-7.10 (d, 1H), 7.12-7.22 (s, 1 H).

EXAMPLES 159 and 160

4-(3-Hydroxy-(17,16-c)-(1'-methyl)-pyrazolyl-estra-1,3,5(10)-trieno-15α-yl)-N-(5-methylthiazol-2-yl)-butyramide

4-(3-Hydroxy-(17,16-c)-(2'-methyl)-pyrazolyl-estra-1,3,5(10)-trieno-15α-yl)-N-(5-methylthiazol-2-yl)-butyramide

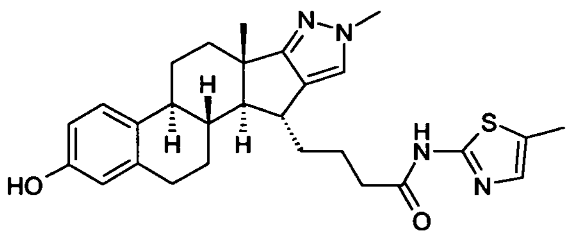 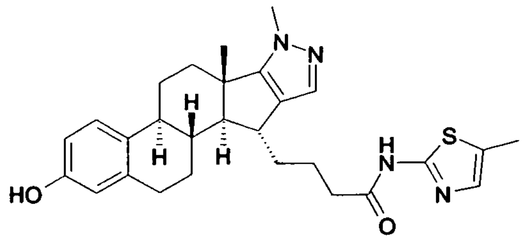

Examples 159 and 160 were prepared according to the procedure described for Examples 155 and 156 using 2-amino-5-methylthiazole for the amide coupling step.

EXAMPLE 159: $^1$H-NMR (300 MHz, $CDCl_3$): δ 0.80-0.90 (s, 3H), 0.90-2.48 (m, 20 H), 2.56-2.80 (m, 3H), 3.60-3.68 (s, 3H), 6.36-6.42 (s, 1H), 6.44-6.52 (d, 1 H), 6.84-6.92 (s, 1H), 6.96-7.04 (m, 2H).

EXAMPLE 160: $^1$H-NMR (300 MHz, $CDCl_3$): δ 0.84-0.96 (s, 3H), 0.96-2.48 (m, 20H), 2.58-2.80 (m, 3H), 3.60-3.72 (s, 3H), 6.36-6.44 (s, 1H), 6.44-6.56 (d, 1H), 6.84-6.92 (s, 1H), 6.96-7.04 (d, 1H), 7.04-7.08 (s, 1 H).

EXAMPLE 161:

3-Hydroxy-15β-(4-morpholin-4-yl-4-oxo-butyl)-estra-1,3,5(10)-trien-[16,17-c]-isoxazole

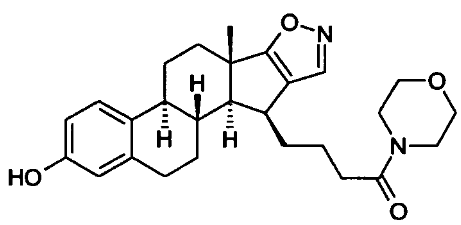

Starting from intermediate compound Xc, an allyl side chain was introduced into C15 position using 1,4-addition of allylbromide according to step 1 of SCHEME 7C, followed by construction of the oxazole-ring according to D-(II)-(c) using hydroxylamine for the ringclosure. Conversion of the allyl into the 4-morpholin-4-yl-4-oxo-butyl side chain can be performed according to steps 2 - 4 of SCHEME 7C and to the reaction as depicted in general flow diagram Ib by amide coupling with morpholine.

EXAMPLE 162:

3-Hydroxy-15β-(4-morpholin-4-yl-4-oxo-butyl)-estra-1,3,5(10)-trien-[16,17-c]-pyrazole

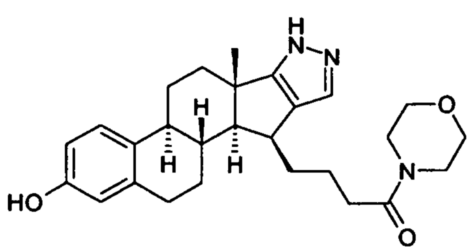

Starting from intermediate compound Xc, an allyl side chain was introduced into C15 position using 1,4-addition of allylbromide according to step 1 of SCHEME 7C, followed by construction of the pyrazol-ring according to D-(II)-(a) using benzylhydrazine for the ringclosure to give a protected pyrazol. Conversion of the allyl into the 4-morpholin-4-yl-4-oxo-butyl side chain was performed according to steps 2 (metathesis) and 3 (saponification) of SCHEME 7C, followed by amide coupling with morpholine according to general flow diagram Ib. Finally, reduction of the double bond and debenzylation gave the desired endproduct No. 162.

$^1$H-NMR (300 MHz, $CDCl_3$): δ 1.08-1.20 (s, 3H), 1.22-2.48 (m, 17H), 2.72-3.00 (m, 3H), 3.40-3.76 (m, 8H), 6.52-6.56 (s, 1H), 6.56-6.60 (d, 1H), 7.00-7.12 (d, 1 H), 7.22-7.28 (s, 1 H).

## IV. Compounds carrying a sulphamate, carbamate, phosphonate, thiophosphonate, sulphonate, phosphate or sulphate group in R1

EXAMPLE 163: 3-Sulphamate-15β-(4-morpholin-4-yl-4-oxo-butyl)-estra-1,3,5(10)-trien-17-one

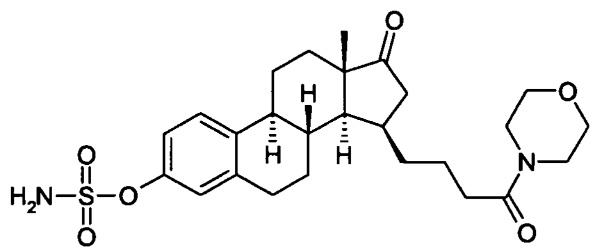

Example 163 was prepared from the Intermediate No. 1 named 3-Hydroxy-15β-(4-morpholin-4-yl-4-oxo-butyl)-estra-1,3,5(10)-trien-17-one of formula (VIβ-3a)-1 using sulfamoyl chloride as sulfamoylating agent.

EXAMPLE 164: 3-Sulphate-15β-(4-morpholin-4-yl-4-oxo-butyl)-estra-1,3,5(10)-trien-17-one

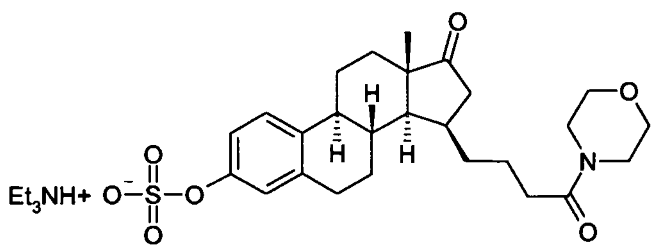

Example 164 was prepared from the Intermediate No. 1 named 3-Hydroxy-15β-(4-morpholin-4-yl-4-oxo-butyl)-estra-1,3,5(10)-trien-17-one of formula (VIβ-3a)-1 using sulfur trioxide - triethylamine complex: The estron derivative (VIβ-3a)-1 (0.25 mmol) and sulfur trioxide - triethylamine complex (54.4 mg, 0.30 mmol, "Fluka") were stirred in anhydrous DMF (1 ml) at RT overnight. Ca. 0.3 g silica gel (for column chromatography) was added, and the solvent was removed in high vacuum at 35°C. The remaining powder was loaded on the column prepacked with ca. 6 g silica gel. Flash chromatography with afforded the desired triethylammonium phenol sulfate.

BIOLOGICAL TESTING MATERIALS AND METHODS

1. Inhibition of the 17β-hydroxysteroid dehydrogenase type 1 enzyme

**17β-HSD1 purification:** Recombinant baculovirus was generated by the "Bac to Bac Expression System" (Invitrogen). Recombinant bacmid was transfected to Sf9 insect cells using "Cellfectin Reagent" (Invitrogen). 60h later cells were harvested; the microsomal fraction was isolated as described by Puranen et al. (1994). Aliquots were stored frozen until determination of enzymatic activity.

**Assay - Inhibition of recombinant human 17β-hydroxysteroid dehydrogenase type 1:** Recombinant protein (0.1μg/ml) was incubated in 20 mM $KH_2PO_4$ pH 7.4 with 30 nM 3H-estrone and 1 mM NADPH for 30 min at RT, in

the presence of potential inhibitors at concentrations of 1 μM or 0.1 μM. Inhibitor stock solutions were prepared in DMSO. Final concentration of DMSO was adjusted to 1 % in all samples. The enzyme reaction was stopped by addition of 10 % trichloroacetic acid (final concentration). Samples were centrifuged in a microtiter plate at 4000 rpm for 10 min. Supernatants were applied to reverse phase HPLC on a Waters Symmetry C18 column, equipped with a Waters Sentry Guard column. Isocratic HPLC runs were performed at RT at a flow rate of 1 ml/min of acetonitrile:water 48:52 as running solvent. Radioactivity was monitored in the eluate by a Packard Flow Scintillation Analyzer. Total radioactivity for estrone and estradiol were determined in each sample and percent conversion of estrone to estradiol was calculated according to the following formula:

$$\underline{\%\ \text{conversion}} = 100 \times \frac{\{(\text{cpm estradiol in sample with inhibitor}) / [(\text{cpm estrone in sample with inhibitor}) + (\text{cpm estradiol in sample with inhibitor})]\}}{\{(\text{cpm estradiol in sample without inhibitor}) / [(\text{cpm estrone in sample without inhibitor}) + (\text{cpm estradiol in sample without inhibitor})]\}}.$$

Percent inhibition was calculated as follows: $\underline{\%\ \text{inhibition}} = 100 - \%\text{conversion}$

The values "% inhibition" were determined for exemplified compounds, and the results are summarized in Table 19.

Table 19: Inhibition of 17β-HSD enzyme type I

| Compound No. | Compound Structure | Inhibition of rec 17β-HSD1 | |
|---|---|---|---|
| | | 100 nM | 1 μm |
| 2 | 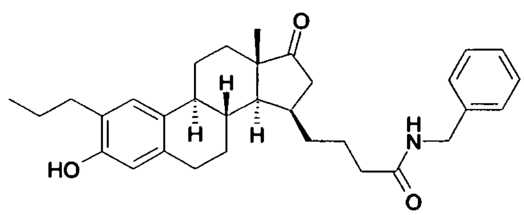 | 54.8 | 74.8 |
| 3 | 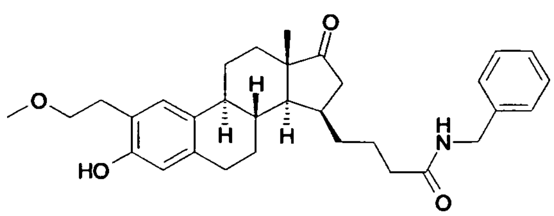 | 19.6 | 71.2 |
| 6 | 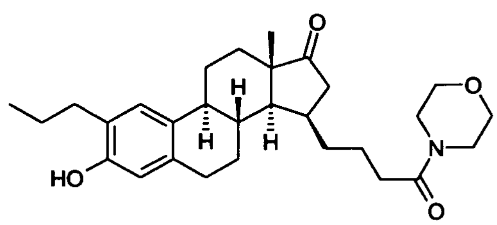 | 37.9 | 79.3 |
| 30 | 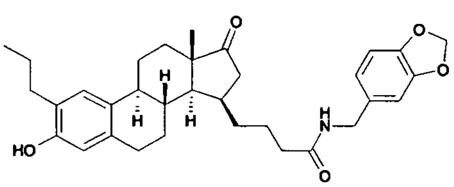 | 39.8 | 73.6 |

(continued)

| Compound No. | Compound Structure | Inhibition of rec 17β-HSD1 | |
|---|---|---|---|
| | | 100 nM | 1 μm |
| 32 | 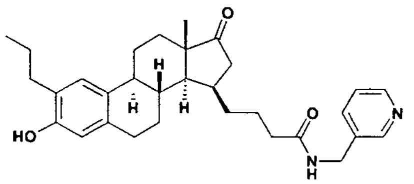 | 50.0 | 79.0 |
| 34 | 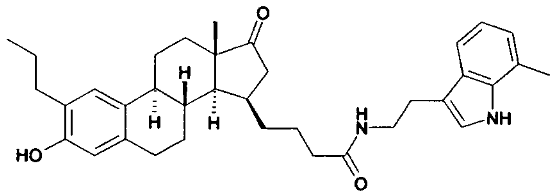 | 68.8 | 71.9 |
| 36 | 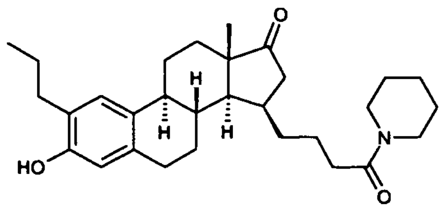 | 56.9 | 78.1 |
| 37 | 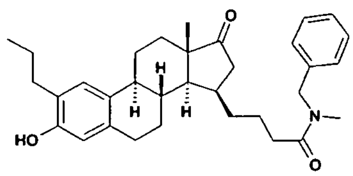 | 64.0 | 74.7 |
| 38 | 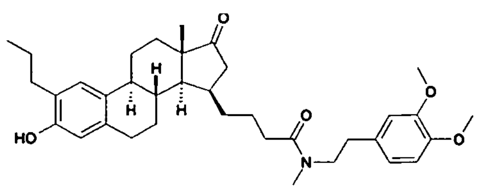 | 51.0 | 76.7 |
| 40 | 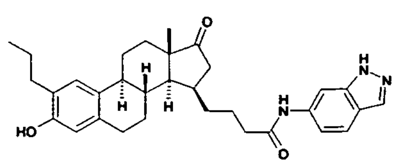 | 72.0 | 75.8 |
| 41 | 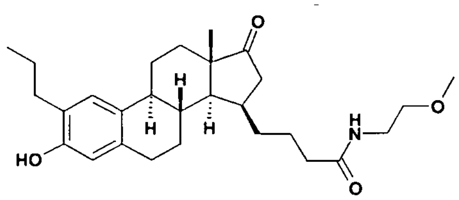 | 25.9 | 70.4 |
| 42 | 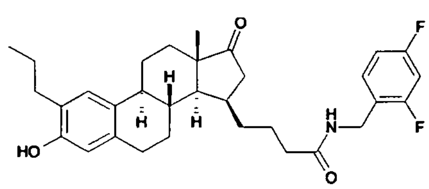 | 53.3 | 71.7 |

(continued)

| Compound No. | Compound Structure | Inhibition of rec 17β-HSD1 | |
|---|---|---|---|
| | | 100 nM | 1 μm |
| 44 | 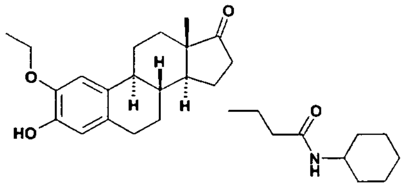 | 55.5 | 75.0 |
| 45 | 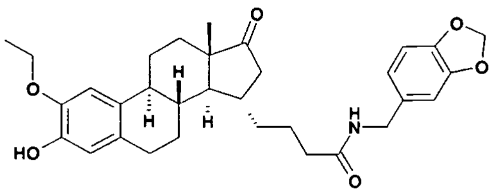 | 54.0 | 75.7 |
| 52 | 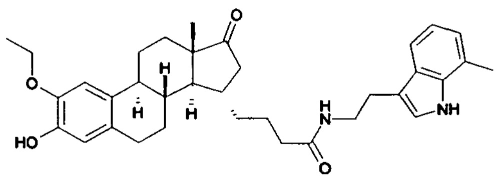 | 50.0 | 72.2 |
| 55 | 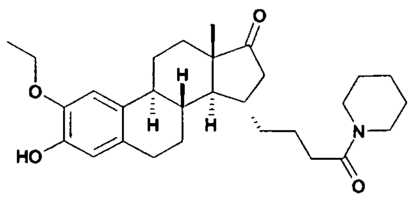 | 42.6 | 71.9 |
| 59 | 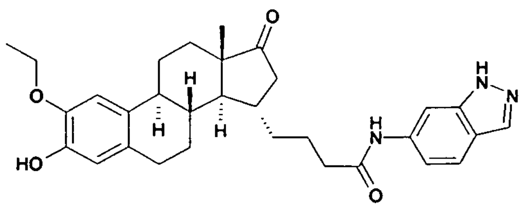 | 56.1 | 69.7 |
| 67 | 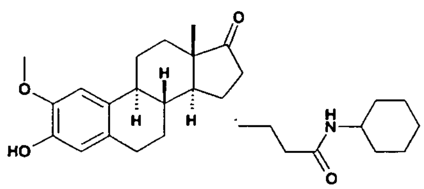 | 42.5 | 73.2 |
| 71 | 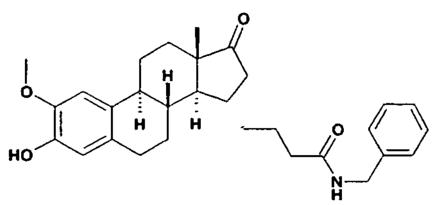 | 45.8 | 70.6 |

(continued)

| Compound No. | Compound Structure | Inhibition of rec 17β-HSD1 | |
|---|---|---|---|
| | | 100 nM | 1 μm |
| 78 | 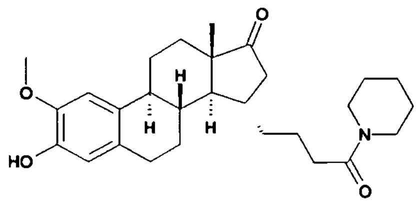 | 40.6 | 72.0 |
| 83 | 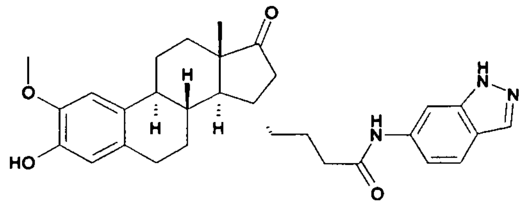 | 53.9 | 71.6 |
| 90 | 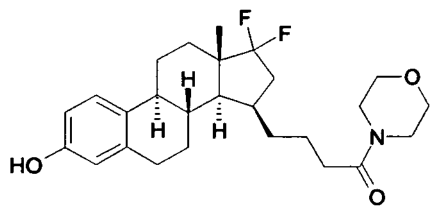 | 63.5 | 81.6 |
| 91 | 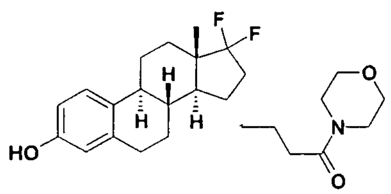 | 32.0 | 85.0 |
| 93 | 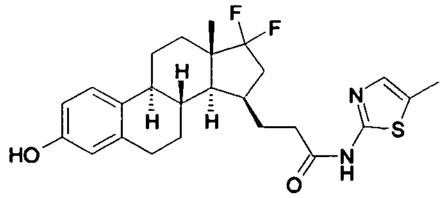 | 59.5 | 86.7 |
| 102 | 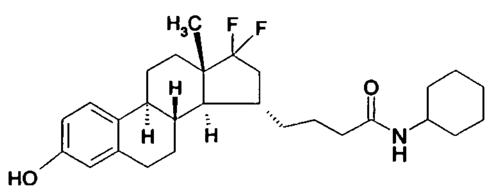 | 41.6 | 70.6 |
| 106 | 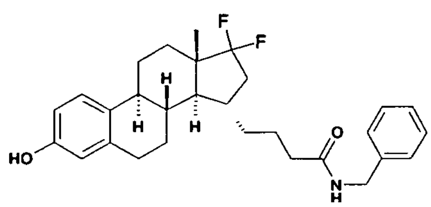 | 36.7 | 68.0 |

(continued)

| Compound No. | Compound Structure | Inhibition of rec 17β-HSD1 | |
|---|---|---|---|
| | | 100 nM | 1 μm |
| 109 | 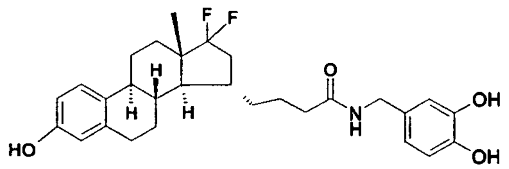 | 35.1 | 70.7 |
| 111 | 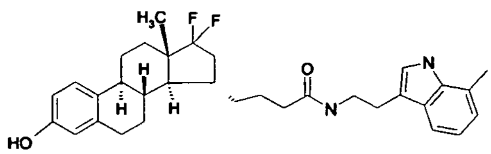 | 50.6 | 68.3 |
| 113 | 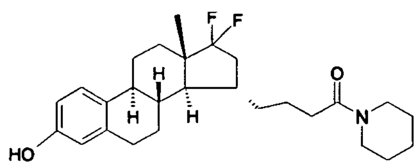 | 47.2 | 76.9 |
| 116 | 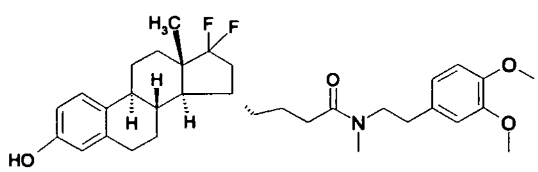 | 49.3 | 82.4 |
| 120 | 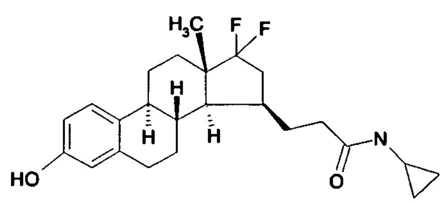 | 37.3 | 85.0 |
| 121 | 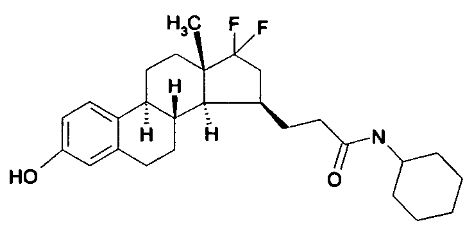 | 63.6 | 92.5 |
| 122 | 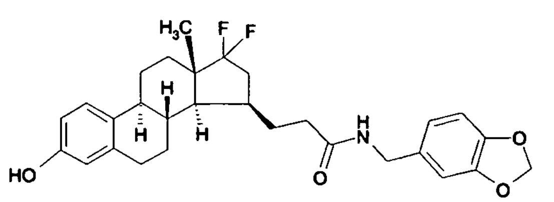 | 34.0 | 77.9 |
| 125 | 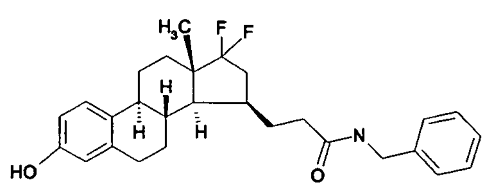 | 29.2 | 76.9 |

(continued)

| Compound No. | Compound Structure | Inhibition of rec 17β-HSD1 | |
|---|---|---|---|
| | | 100 nM | 1 μm |
| 127 | 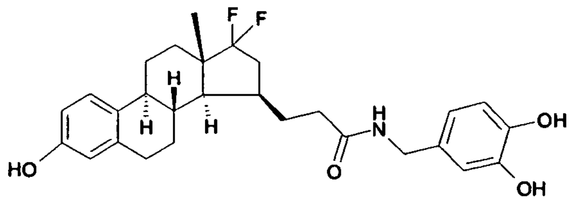 | 21.1 | 81.3 |
| 128 | 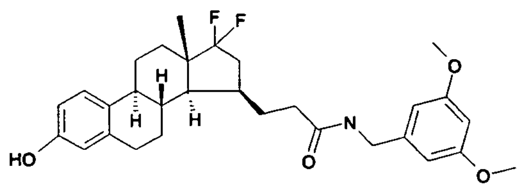 | 21.2 | 71.7 |
| 129 | 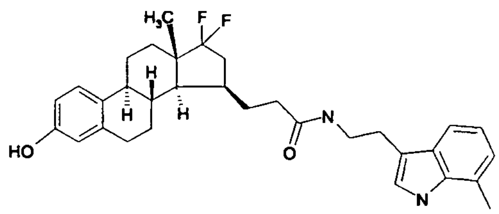 | 33.1 | 76.9 |
| 131 | 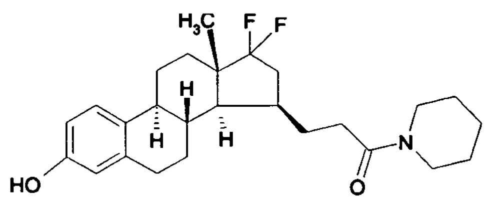 | 19.8 | 71.9 |
| 133 | 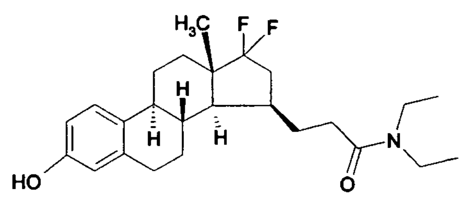 | 71.4 | 90.6 |
| 134 | 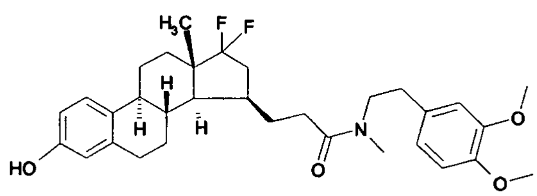 | 50.5 | 88.6 |
| 161’ | 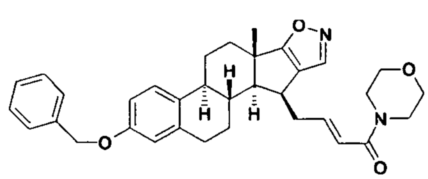 | 21.0 | 74.0 |

(continued)

| Compound No. | Compound Structure | Inhibition of rec 17β-HSD1 | |
|---|---|---|---|
| | | 100 nM | 1 μm |
| 162 | 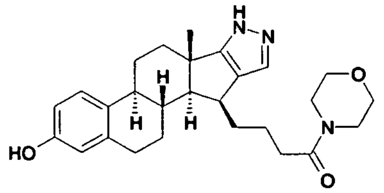 | 63.0 | 93.0 |
| 163 | 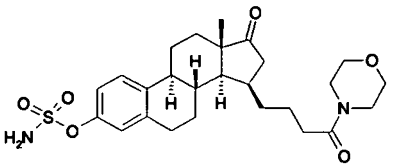 | 10.0 | 43.0 |
| 164 | 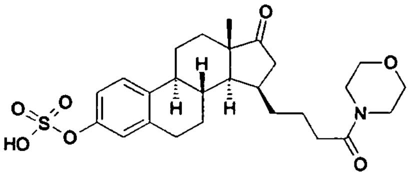 | 3.0 | 27.0 |

2. Estrogen Receptor Binding Assay

The binding affinity of the compounds of the invention to the estrogen receptor α and to the estrogen receptor β may be determined according to the in vitro ER binding assays described by Koffman et al (1991). Alternatively, an estrogen receptor binding assay may be performed according to international patent application WO 00/07996.

3. Estrogen Receptor Transactivation Assays

Compounds of the invention showing binding affinity towards the estrogen receptor may be further tested with regard to their individual estrogenic or anti-estrogenic potential (agonistic binding or antagonistic binding to the ERα or ERβ). The determination of the estrogen receptor agonist activity may be performed according to an in vitro assay system using the MMTV-ERE-LUC reporter system which is for example described within published US patent application US 2003/0170292:

To assay estrogen receptor agonist activity, Hela cells are grown in 24-well microtiter plates and then transiently co-transfected with two plasmids using lipofectamine. The first plasmid comprises DNA encoding human estrogen receptor (either ER-alpha or ER-beta), and the second plasmid comprises an estrogen-driven reporter system comprising: a luciferase reporter gene (LUC) whose transcription is under the control of upstream regulatory elements comprising 4 copies of the vitellogenin estrogen response element (ERE) cloned into the mouse mammary tumor virus (MMTV) promoter (the full name for the reporter system being "MMTV-ERE-LUC"). Cells are exposed to the compounds of the invention in RPMI 1640 medium, supplemented with 10% charcoal-treated fetal calf serum, 2 mM L-glutamine, 0.1 mM non-essential amino acids and 1 mM sodium pyruvate for 42-48 h at 37˚C in a 5% carbon dioxide incubator. Concurrently, cells exposed to estradiol (1 nM) serve as positive controls. Replicate wells exposed to the solvent in which the compounds of the invention are dissolved (i.e. ethanol or methanol) are used as negative controls. After the 42-48 h incubation period, cells are rinsed with phosphate buffered saline (PBS), lysis buffer (Promega Corp) is added, and cell lysates are collected for measurement of luciferase activity with a luminometer. Estrogenic activity of the compounds of the invention is expressed as fold-increase in luciferase activity as compared to that observed in negative control cells.

Alternatively, the determination of the estrogen receptor transactivation activity (estrogenicity assay or agonist assay) and of the inhibitory potency of transactivation activity (anti-estrogenicity assay or antagonist assay) may be performed according to international patent application WO 00/07996.

4. STS ASSAY- INHIBITION OF STEROID SULPHATASE ACTIVITY IN MCF-7 CELLS

Steroid sulphate activity is measured in vitro using intact MCF-7 human breast cancer cells. This hormone dependent cell line is widely used to study the control of human breast cancer cell growth. It possesses significant steroid sulphate activity and is available in the U.S.A. form the American Type Culture Collection (ATCC) and in the U.K. (e.g. from The Imperial Cancer Research Fund).

Cells are maintained in Minimal Essential Medium (MEM) (Flow Laboratories, Irvine, Scotland) containing 20 mM HEPES, 5% foetal bovine serum, 2 mM glutamin, non-essential amino acids and 0.075% sodium bicarbonate. Up to 30 replicate 25 $cm^2$ tissue culture flasks are seeded with approximately $1x10^5$ cells/flask using the above medium. Cells are grown to 80% confluency and the medium is changed every third day.

Intact monolayers of MCF-7 cells in triplicate 25 $cm^2$ tissue culture flasks are washed with Earle's Balanced Salt Solution (EBSS from ICN Flow, High Wycombe, U.K.) and incubated for 3-4 h at 37˚C with 5 pmol ($7x10^5$ dpm) [6,7-$^3$H] oestrone-3-sulphate (specific activity 60 Ci/mmol from New England Nuclear, Boston, Mass., U.S.A.) in serum-free MEM (2.5 ml) together with oestrone-3-sulphamate (11 concentrations: 0; 1fM; 0.01pM; 0.1pM; 1pM; 0.01 nM; 0.1nM; 1nM; 0.01mM; 0.1mM; 1mM). After incubation each flask is cooled and the medium (1ml) is pipette into separate tubes containing [$^{14}$C] oestrone ($7x10^3$ dpm) (specific activity 97 Ci/mmol from Amersham International Radiochemical Centre, Amersham, U.K.). The mixture is shaken thoroughly for 30 seconds with toluene (5 ml). Experiments have shown that >90% [$^{14}$C] oestrone and <0.1% [$^3$H] oestrone-3-sulphate is removed from the aqueous phase by this treatment. A portion (2ml) of the organic phase is removed, evaporated and the $^3$H and $^{14}$C content of the residue determined by scintillation spectrometry. The mass of oestrone-3-sulphate hydrolyse was calculated from the $^3$H counts obtained (corrected for the volumes of the medium and organic phase used, and for recovery of [$^{14}$C] oestrone added) and the specific activity of the substrate. Each batch of experiments includes incubations of microsomes prepared from a sulphatase-positive human placenta (positive control) and flasks without cells (to assess apparent non-enzymatic hydrolysis of the substrate). The number of cell nuclei per flask is determined using a Coulter Counter after treating the cell monolayers with Zaponin. One flask in each batch is used to assess cell membrane status and viability using the Trypan Blue exclusion method.

Results for steroid sulphate activity are expressed as the mean $\pm$ 1 S.D. of the total product (oestrone + oestradiol) formed during the incubation period (20 h) calculated for 106 cells and, for values showing statistical significance, as a percentage reduction (inhibition) over incubations containing no oestrone-3-sulphamate. Unpaired Student's t-test was used to test the statistical significance of resutts.

5. CHO/STS ASSAY

CHO cells stably transfected with human steroid sulfatase (CHO/STS) are seeded into microtiter plates. After reaching approximately 90% confluency, they are incubated overnight with graded concentrations of test substances (e. g. compounds of the present invention or compounds for use in the present invention). They are then fixed with 4% paraformaldehyde for 10 min at RT and washed 4 times with PBS, before incubation with 100 μl/well 0.5mM 4-methylumbelliferyl sulfate (MUS), dissolved in 0.1 M Tris-HCl, pH 7.5. The enzyme reaction is carried out at 37˚C for 30 min. Then 50μl/well stop solution (1 M Tris-HCl, pH 10.4) are added. The enzyme reaction solutions are transferred to white plates (Microfluor, Dynex, Chantilly, VA) and read in a Fluoroskan II or Tecan fluorescence microtiter plate reader. Reagent blanks are subtracted from all values. Optionally, for drug testing, the fluorescence units (FU) can be divided by the optical density readings after staining cellular protein with sulforhodamine B ($OD_{550}$), in order to correct for variations in cell number. $IC_{50}$ values are determined by linear interpolation between two bracketing points. In each assay with inhibitors, estrone 3-O-sulfamate is run as a reference compound, and the IC50 values are normalized to estrone 3-O-sulfamate (relative $IC_{50}$ = $IC_{50}$ compound/$IC_{50}$ estrone 3-O-sulfamate).

6. STS INHIBITION IN PLACENTA MICROSOMES

Sulphatase-positive human placenta from normal term pregnancies are thoroughly minced with scissors and washed once with cold phosphate buffer (pH 7.4, 50 mM) then re-suspended in cold phosphate buffer (5 ml/g tissue). Homogenisation is accomplished with an Ultra-Turrax homogeniser, using three 10 second bursts separated by 2 min cooling periods in ice. Nuclei and cell debris are removed by centrifuging (4˚C) at 2000g for 30 min and portions (2 ml) of the supernatant are stored at 20˚C. The protein concentration of the supernatants is determined by the method of Bradford [Anal. Biochem. 72:248-254 (1976)].

Incubations (1 ml) are carried out using a protein concentration of 100 mg/ml, substrate concentration of 20 mM [6,7-$^3$H] oestrone-3-sulphate (specific activity 60 Ci/mmol from New England Nuclear, Boston, Mass., U.S.A.) and an incubation time of 20 min at 37˚C. If necessary eight concentrations of compounds are employed: 0 (i.e. control); 0.05mM; 0.1 mM; 0.2mM; 0.4mM; 0.6mM; 0.8mM; and 1.0mM. After incubation each sample is cooled and the medium

(1 ml) was pipetted into separate tubes containing [$^{14}C$] oestrone ($7x10^3$ dpm) (specific activity 97 Ci/mmol from Amersham International Radiochemical Centre, Amersham, U.K.). The mixture is shaken thoroughly for 30 seconds with toluene (5 ml). Experiments have shown that >90% [$^{14}C$] oestrone and <0.1% [3H] oestrone-3-sulphate is removed from the aqueous phase by this treatment.

A portion (2 ml) of the organic phase was removed, evaporated and the $^3H$ and $^{14}C$ content of the residue determined by scintillation spectrometry. The mass of estrone-3-sulphate hydrolyse is calculated from the $^3H$ counts obtained (corrected for the volumes of the medium and organic phase used, and for recovery of [$^{14}C$] oestrone added) and the specific activity of the substrate.

7. ANIMAL ASSAY MODEL FOR DETERMINING STS ACTIVITY

The inhibition of STS activity in vivo may be determined by using the compounds of the present invention in an animal model, in particular in ovariectomised rats. In this model compounds which are estrogenic stimulate uterine growth. The compound (10 mg/Kg/day for five days) was administered orally to rats with another group of animals receiving vehicle only (propylene glycol). A further group received the compound EMATE subcutaneously in an amount of 10 $\mu$g/day for five days. At the end of the study samples of liver tissue were obtained and oestrone sulphate activity assayed using 3H oestrone sulphate as the substrate as previously described (see international application WO 96/15257).

CITED LITERATURE

- Akanni & Marples (1993) "Preparation of 16-formylestradiol and the 16-(alpha-methylenebutanolide) derivative" Steroids 58(5):234-8.
- Cushman et al (1995) "Synthesis, antitubulin and antimitotic activity, and cytotoxicity of analogs of 2-methoxyestradiol, an endogenous mammalian metabolite of estradiol that inhibits tubulin polymerization by binding to the colchicine binding site." J Med Chem. 38(12):2041-9.
- Cushman et al (2002) "The effect of exchanging various substituents at the 2-position of 2-methoxyestradiol on cytotoxicity in human cancer cell cultures and inhibition of tubulin polymerization." J Med Chem. 45(21):4748-54.
- Day et al (2003) "The effects of 2-substituted oestrogen sulphamates on the growth of prostate and ovarian cancer cells." J Steroid Biochem Mol Biol. 2003 84(2-3):317-25.
- Edwards et al. (1990) "Difluoromethyldiphenylphosphine oxide. A new reagent for conversion of carbonyl compounds to 1,1-difluoroolefins." Tetrahedron Lett 31(39):5571-5574
- EP0367576 - Estrogen nucleus derivatives for use in the inhibition of sex steroid activity
- Gonzalez et al (1982) "Synthesis and pharmacological evaluation of 8$\alpha$-estradiol derivatives" Steroids 40(2):171-188
- Koffman et al (1991) "Evidence for involvement of tyrosine in estradiol binding by rat uterus estrogen receptor." J. Steroid. Biochem. Mol. Biol. 38(2):135
- Labaree et al (2003) "Synthesis and Evaluation of B-, C- and D-ring substituted estradiol carboxylic acid esters as locally active estrogens" J. Med. Chem. 46:1886-1904
- Labrie et al (1997) "The key role of 17 beta-hydroxysteroid dehydrogenases in sex steroid biology." Steroids, 62: 148-58
- Lawrence et al (2005) "Novel and potent 17beta-hydroxysteroid dehydrogenase type 1 inhibitors." J Med Chem. 48 (8):2759-62.
- Ley et al (1994) "Tetrapropylammonium perruthenate, Pr4N+Ru04-, TPAP: a catalytic oxidant for organic synthesis" Synthesis. 07:639-666
- Liu et al (1992) "Synthesis of high affinity fluorine-substituted ligands for the androgen receptor. Potential agents for imaging prostatic cancer by positron emission tomography." J Med Chem. 35(11):2113-29
- Lunn & Farkas (1968) "The adamantyl carbonium ion as a dehydrogenating agent, its reactions with estrone" Tetrahedron 24(23):6773-6776.
- Mindnich et al (2004) "The role of 17 beta-hydroxysteroid dehydrogenases" Mol Cell Endocrinol. 218(1-2):7-20. Review
- Mohanakrishnan & Cushman (1999) "Pd(0)-Mediated Cross Coupling of 2-lodoestradiol with Organozinc Bromides: A General Route to the Synthesis of 2-Alkynyl, 2-Alkenyl and 2-Alkylestradiol Analogs"Synlett 1999(07):1097-1099
- Nambara et al. (1976) "Synthesis of Estetrol Monoglucuronides" Steroids 27:111-122
- Nussbaumer & Billich (2003) "Steroid sulfatase inhibitors." Expert Opin. Ther. Patents 13(5):605-625
- Nussbaumer & Billich (2004) "Steroid sulfatase inhibitors." Med Res Rev. 24(4):529-76
- Oda et al (1989) "The hydrogenation of alpha-hydroxymethylene-ketone derivatives to alpha-hydroxymethyl-ketone

derivatives with a cell-free system of Streptomyces cinereocrocatus" Chem Pharm Bull (Tokyo) 37(2):502-5.

- Page et al (1990) "Efficient regioselective a-ring functionalization of oestrogens" Tetrahedron 46(6):2059-2068
- Pelletier & Poirier (1996) "Synthesis and evaluation of estradiol derivatives with 16α-(bromoalkylamide), 16α-(bromoalkyl) or 16α-(bromoalkynyl) side chain as inhibitors of 17β-hydroxysteroid dehydrogenase type 1 without estrogenic activity" Bioorg Med Chem, 4(10):1617-1628.
- Poirier (2003) "Inhibitors of 17 beta-hydroxysteroid dehydrogenases" Curr Med Chem. 10:453-77
- Poirier et al. (1991) "Synthesis of 17β-estradiol derivatives with N-Butyl, N-methyl alkylamide side chain at position 15." Tetrahedron, 47(37):7751-7766
- Poirier et al. (1996) "D-Ring alkylamine derivatives of estradiol: effect on ER-binding affinity and antiestrogenic activity" Bioorg Med Chem Lett 6(21):2537-2542.
- Poirier et al (1998) "A 6β-(Thiaheptanamide) Derivative of Estradiol as inhibitor of 17β-Hydroxysteroid Dehydrogenase Type 1", J. Steroid Biochem. Molec. Biol., 64:83-90
- Puranen et al (1994) "Site-directed mutagenesis of the putative active site of human 17 beta-hydroxysteroid dehydrogenase type 1" Biochem. J. 304:289-93.
- Rao & Cessac (2002) "A new, practical synthesis of 2-methoxyestradiols." Steroids. 67(13-14):1065-70.
- Reed et al (2005) "Steroid sulfatase: molecular biology, regulation, and inhibition." Endocr Rev. 26(2):171-202.
- Sam et al. (1998) "C16 and C17 Derivatives of Estradiol as Inhibitors of 17β-Hydroxysteroid Dehydrogenase Type 1: Chemical Synthesis and Structure-Activity Relationships", Drug Design and Discovery, 15:157-180
- Schneider et al (1983) "A convenient method for the formation of 16-methylene-17-keto steroids" Synthesis, 8:665-9.
- Schwarz et al (2001) "Studies on modified estrogens: Towards the synthesis of novel 14,15-cyclopropa[a]estra-1,3,5(10),8-tetraenes" Pharmazie 56(11):843-849
- Tamaya et al. (1985) "Comparison of cellular levels of steroid receptors in uterine leiomyoma and myometrium." Acta Obstet Gynecol Scand., 64:307-9
- Tremblay & Poirier (1998) "Overview of a Rational Approach to Design Type I 17β-Hydroxysteroid Dehydrogenase Inhibitors Without Estrogenic Activity: Chemical Synthesis and Biological Evaluation", J. Steroid Biochem. Molec. Biol., 66:179-191
- US 2003/0170292
- US 3,275,623
- US 3,347,878
- US 3,413,321
- US 6,043,236
- Verdier-Pinard et al (2000) "A steroid derivative with paclitaxel-like effects on tubulin polymerization." Mol Pharmacol. 57(3):568-75.
- Wang & Ruan (1994) "Trifluoromethylation of steroidal ketones" J. Fluorine Chem. 69(1):1-3
- WO 93/05063
- WO 96/15257
- WO 96/28462
- WO 00/07996
- WO 02/32409
- WO 03/017973
- WO 2004/080271
- WO 2004/085345
- WO 2004/085457
- WO 2004/085459
- WO 2005/047303
- WO 2006/003012 (also published as US2006052461)
- WO 2006/003013 (also published as US2006009434)
- WO 2006/027347
- Wölfling et al (2003) "Synthesis and receptor-binding examinations of the normal and 13-epi-D-homoestrones and their 3-methyl ethers" Steroids 68:277-288
- Xenos & Catsoulacos (1985) "Synthesis of 16,17-pyrazolo-fused derivatives of A-homo-steroidal ring A lactams" Synthesis 3:307-9
- Yoshikawa et al. (2002) "Diastereo- and Enantioselective Direct Catalytic Aldol Reaction of 2-Hydroxyacetophenones with Aldehydes Promoted by a Heteropolymetallic Complex: Catalytic Asymmetric Synthesis of anti-1,2-Diols" J. Org. Chem. 67(8); 2556-2565.

## Claims

1. A compound of the general formula I,

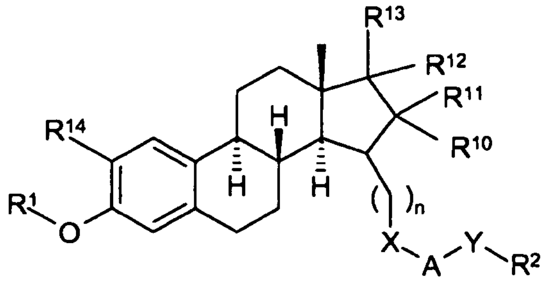

(I)

wherein -X-A-Y- together represent a group selected from

(a) -CO-$NR^4$-,
(b) -CO-O-,
(c) -CO-,
(d) -CO-NH-$NR^4$-,
(e) -NH-CO-NH-,
(f) -NH-CO-O-,
(g) -NH-CO-,
(h) -NH-CO-NH-$SO_2$-,
(i) -NH-$SO_2$-NH-,
(j) -NH-$SO_2$-O-,
(k) -NH-$SO_2$-
(l) -O-CO-NH-,
(m) -O-CO-,
(n) -O-CO-NH-$SO_2$-$NR^4$-, and
(o) -O-;

n represents 1, 2, 3, 4, 5 or 6, or, if -X-A-Y- represents -CO-$NR^4$-, -CO-O-, -CO-, or -CO-NH-$NR^4$-, then n may also represent 0;
$R^1$ is selected from:

(a) -H,
(b) -($C_1$-$C_6$)alkyl, which is optionally substituted with halogen, nitril, -$OR^6$, -$SR^6$, or -$COOR^6$; the number of said substituents being 1, 2 or 3 for halogen, and 1 or 2 for any combination of said halogen, nitril, -$OR^6$, -$SR^6$, or -$COOR^6$ moieties,
(c) -phenyl, which is optionally substituted with halogen, nitril, -$OR^6$, -$SR^6$, -$R^6$, or -$COOR^6$, the number of said substituents being up to perhalo for halogen, and 1 or 2 for any combination of said halogen, nitril, -$OR^6$, -$SR^6$, -$R^6$ or -$COOR^6$ moieties,
(d) -($C_1$-$C_4$)alkyl-phenyl, in which the alkyl portion is optionally substituted with up to three halogens; and the phenyl portion is optionally substituted with halogen, nitril, -$OR^6$, -$SR^6$, -$R^6$ or -$COOR^6$, the number of substituents on said phenyl portion being up to perhalo for halogen, and 1 or 2 for any combination of said halogen, nitril, -$OR^6$, -$SR^6$, -$R^6$ or - $COOR^6$ moieties,
(e) -$SO_2$-$NR^3R^{3'}$,
(f) -CO-$NR^3R^{3'}$,
(g) -PO($OR^{16}$)-$R^3$,
(h) -PS($OR^{16}$)-$R^3$,
(i) -PO($OR^{16}$)-O-$R^3$
(j) -$SO_2$-$R^3$, and
(k) -$SO_2$-O- $R^3$;

wherein
$R^6$ represents H, -($C_1$-$C_4$)alkyl or halogenated -($C_1$-$C_4$)alkyl;

$R^3$ and $R^{3'}$ are independently selected from H, alkyl, aryl and arylalkyl, or $R^3$ and $R^{3'}$ form together with the nitrogen atom, to which $R^3$ and $R^{3'}$ are attached, a heterocyclic 4-, 5-, 6-, 7-or 8-memberred ring, which is optionally saturated, partly unsaturated, or aromatic; which optionally contains up to three additional heteroatoms selected from N, O and S, the number of additional N atoms being 0, 1, 2 or 3 and the number of O and S atoms each being 0, 1 or 2; and
$R^{16}$ represents -H, alkyl, or arylalkyl;
$R^2$ and $R^4$ are independently selected from:

(a) -H,
(b) optionally substituted alkyl,
(c) optionally substituted acyl, under the proviso that -X-A-Y- represent -CO-NH-$NR^4$-,
(d) optionally substituted aryl,
(e) optionally substituted heteroaryl, and
(f) optionally substituted cycloheteroalkyl,

or $R^2$ and $R^4$ form together with the nitrogen atom, to which $R^2$ and $R^4$ are attached, a heterocyclic 4-, 5-, 6-, 7- or 8-memberred ring, which is optionally saturated, partly unsaturated, or aromatic; which optionally contains up to three additional heteroatoms selected from N, O and S, the number of additional N atoms being 0, 1, 2 or 3 and the number of O and S atoms each being 0, 1 or 2; and which ring is optionally part of a multiple condensed ring-system, wherein the ring or the ring-system is optionally substituted;
the substituents $R^{10}$, $R^{11}$, $R^{12}$ and $R^{13}$ together with the carbon atoms, to which they are attached, form a structure

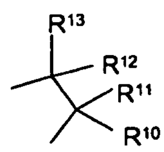,

which is selected from the group of

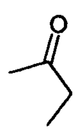 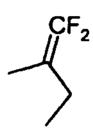 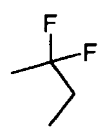 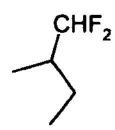 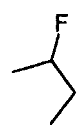

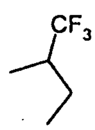 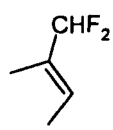 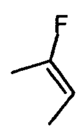 and 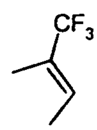

or, the substituents $R^{10}$, $R^{11}$, $R^{12}$ and $R^{13}$ together with the carbon atoms, to which they are attached, form a heterocyclic 5- or 6-membered ring, which is partly unsaturated or aromatic, which contains one, two or three heteroatoms independently selected from N, O and S, the number of N atoms being 0, 1, 2 or 3 and the number of O and S atoms each being 0, 1 or 2, wherein one heteroatom is directly attached to the C17 C-atom of the steroidal core; and which ring is optionally substituted with an alkyl group;
$R^{14}$ represents an alkyl, alkoxy, or alkoxy-alkyl group, or
$R^{14}$ may also represent -H, under the proviso that at least

(i) $R^1$ represents -$SO_2$-$NR^3R^{3'}$, -CO-$NR^3R^{3'}$, -PO($OR^{16}$)-$R^3$, -PS($OR^{16}$)-$R^3$, -PO($OR^{16}$)-$OR^3$, -$SO_2$-$R^3$, or -$SO_2$-$OR^3$; or
(ii)

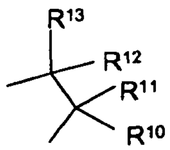 is different from 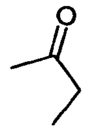

and all pharmacologically acceptable salts thereof.

**2.** A compound according to claim 1, wherein $R^2$ and $R^4$ are independently selected from:

(a) -H, wherein, if -X-A-Y- together represents -CO-O- or -CO-, then $R^2$ is different from -H,

(b) -($C_1$-$C_{12}$)alkyl, optionally substituted with up to five substituents independently selected from the group consisting of halogen, hydroxyl, thiol, nitrile, alkoxy, aryloxy, arylalkyloxy, amino, amido, alkylthio, arylthio, arylalkylthio, sulfamoyl, sulfonamide, acyl, carboxyl, acylamino,

aryl, which aryl is optionally substituted with up to three substituents independently selected from the group consisting of halogen, hydroxyl, ($C_1$-$C_6$)alkoxy, ($C_1$-$C_6$)alkyl, halogenated ($C_1$-$C_6$)alkyl, halogenated ($C_1$-$C_6$) alkoxy, carboxyl-($C_1$-$C_6$)alkyl, thiol, nitrile, sulfamoyl, sulfonamide, carboxyl, aryloxy, arylalkyloxy, ($C_1$-$C_6$) alkylthio, arylthio, arylalkylthio, amino, amido, acyl, acylamino and heteroaryl; or which aryl is optionally substituted by two groups which are attached to adjacent carbon atoms and are combined into a saturated or partly unsaturated cyclic 5, 6, 7, or 8-membered ring system, optionally containing up to three heteroatoms selected from N, O and S, the number of N atoms being 0, 1, 2 or 3 and the number of O and S atoms each being being 0, 1 or 2;

heteroaryl, which heteroaryl is optionally substituted with up to three substituents independently selected from the group consisting of halogen, hydroxyl, ($C_1$-$C_6$)alkoxy, ($C_1$-$C_6$)alkyl, halogenated ($C_1$-$C_6$)alkyl, halogenated ($C_1$-$C_6$)alkoxy, carboxyl-($C_1$-$C_6$)alkyl, thiol, nitrile, sulfamoyl, sulfonamide, carboxyl, aryloxy, arylalkyloxy, ($C_1$-$C_6$)alkylthio, arylthio, arylalkylthio, amino, amido, acyl, acylamino, aryl-($C_1$-$C_4$)-alkyl and aryl;

whereby each aryl group is optionally substituted with up to three substituents independently selected from the group consisting of hydroxyl, halogen, ($C_1$-$C_6$)alkoxy, ($C_1$-$C_6$)alkyl, halogenated ($C_1$-$C_6$)alkyl and halogenated ($C_1$-$C_6$)alkoxy; and

cycloheteroalkyl, which cycloheteroalkyl group is optionally substituted with up to three substituents independently selected from the group consisting of oxo, ($C_1$-$C_8$)-alkyl, aryl, aryl-($C_1$-$C_4$)-alkyl, hydroxyl, ($C_1$-$C_6$)alkoxy, carboxyl-($C_1$-$C_6$)alkyl, thiol, nitrile, sulfamoyl, sulfonamide, carboxyl, aryloxy, arylalkyloxy, ($C_1$-$C_6$)alkylthio, arylthio, arylalkylthio, amino, amido, acyl, and acylamino,

whereby each aryl group is optionally substituted with up to three substituents independently selected from the group consisting of hydroxyl, halogen, ($C_1$-$C_4$)-alkyl, ($C_1$-$C_4$)-alkoxy, halogenated ($C_1$-$C_4$)-alkyl, and halogenated ($C_1$-$C_4$)-alkoxy);

(c) acyl -(C=O)-R', wherein R' represents hydrogen, ($C_1$-$C_4$)alkyl, aryl, or aryl-($C_1$-$C_4$)alkyl, or heteroaryl-($C_1$-$C_4$) alkyl;

which aryl or aryl-($C_1$-$C_4$)alkyl is optionally substituted in the aryl moiety with up to three substituents independently selected from the group consisting of hydroxyl, halogen, ($C_1$-$C_4$)alkoxy, ($C_1$-$C_4$)-alkyl or halogenated ($C_1$-$C_4$)alkyl;

(d) aryl

which aryl is optionally substituted with up to three substituents independently selected from the group consisting of halogen, hydroxyl, ($C_1$-$C_6$)a)koxy, ($C_1$-$C_6$)alkyl, halogenated ($C_1$-$C_6$)alkyl, halogenated ($C_1$-$C_6$)alkoxy, carboxyl-($C_1$-$C_6$)alkyl, thiol, nitrile, nitro, sulfamoyl, sulfonamide, carboxyl, aryloxy, arylalkyloxy, ($C_1$-$C_6$)alkylsulfonyl, arylsulfonyl, ($C_1$-$C_6$)alkylthio, arylthio, arylalkylthio, amino, amido, acyl, acylamino and heteroaryl; or which aryl is optionally substituted by two groups which are attached to adjacent carbon atoms and are combined into a saturated or partly unsaturated cyclic 5, 6, 7, or 8-membered ring system, optionally containing up to three heteroatoms selected from N, O and S, the number of N atoms being 0, 1, 2 or 3 and the number of O and S atoms each being 0, 1 or 2;

(e) heteroaryl,

which heteroaryl is optionally substituted with up to three substituents independently selected from the group consisting of halogen, hydroxyl, ($C_1$-$C_6$)alkoxy, ($C_1$-$C_6$)alkyl, halogenated ($C_1$-$C_6$)alkyl, halogenated ($C_1$-$C_6$) alkoxy, carboxyl-($C_1$-$C_6$)alkyl, thiol, nitrile, sulfamoyl, sulfonamide, arylsulfoxy, carboxyl, aryloxy, arylalkyloxy, ($C_1$-$C_6$)alkylsulfonyl, arylsulfonyl, ($C_1$-$C_6$)alkylthio, arylthio, arylalkylthio, amino, amido, acyl, acylamino, aryl-($C_1$-$C_4$)-alkyl and aryl,

whereby each aryl group is optionally substituted with up to three substituents independently selected from the group consisting of hydroxyl, halogen, $(C_1-C_6)$alkoxy, $(C_1-C_6)$alkyl, halogenated $(C_1-C_6)$alkyl and halogenated $(C_1-C_6)$alkoxy; or

(f) cycloheteroalkyl,

which cycloheteroalkyl is optionally substituted with up to three substituents independently selected from the group consisting of oxo, $(C_1-C_{14})$-alkyl, aryl, aryl-$(C_1-C_4)$-alkyl, hydroxyl, $(C_1-C_6)$alkoxy, carboxyl-$(C_1-C_6)$alkyl, thiol, nitrile, sulfamoyl, sulfonamide, carboxyl, aryloxy, arylalkyloxy, $(C_1-C_6)$alkylthio, arylthio, arylalkylthio, amino, amido, acyl, and acylamino,

whereby each aryl group is optionally further substituted with up to three substituents independently selected from the group consisting of hydroxyl, halogen, $(C_1-C_4)$-alkyl, $(C_1-C_4)$-alkoxy, halogenated $(C_1-C_4)$-alkyl, and halogenated $(C_1-C_4)$-alkoxy;

or wherein $R^2$ and $R^4$ form together with the nitrogen atom, to which $R^2$ and $R^4$ are attached, a heterocyclic 4-, 5-, 6-, 7- or 8-memberred ring, which is optionally saturated or partly unsaturated; which optionally contains up to three additional heteroatoms selected from N, O and S, the number of additional N atoms being 0, 1, 2 or 3 and the number of O and S atoms each being 0, 1 or 2; and which ring is optionally part of a multiple condensed ring-system, wherein the ring or the ring-system is optionally substituted

(i) with up to three substituents independently selected from the group consisting of $(C_1-C_8)$-alkyl, halogen, hydroxyl, carboxyl, thiol, nitrile, $(C_1-C_6)$-alkoxy, carboxyl-$(C_1-C_6)$alkyl, aryloxy, arylalkyloxy, amino, amido, alkylthio, arylthio, arylalkylthio, sulfamoyl, sulfonamide, aryl, aryl-$(C_1-C_4)$-alkyl, heteroaryl, and cycloheteroalkyl, wherein the $(C_1-C_8)$-alkyl group is optionally substituted with up to three substituents independently selected among hydroxyl, halogen, $(C_1-C_4)$-alkoxy, or halogenated $(C_1-C_4)$-alkoxy,

whereby the alkyl-chain of the $(C_1-C_4)$-alkoxy moiety is optionally substituted with hydroxyl;

wherein the aryl group or aryl moiety is optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, halogenated $(C_1-C_4)$-alkoxy and carboxyl-$(C_1-C_6)$alkyl, or wherein the aryl moiety is optionally substituted by two groups which are attached to adjacent carbon atoms and are combined into a saturated or partly unsaturated cyclic 5, 6, 7, or 8 membered ring system, optionally containing up to three heteroatoms selected from N, O and S, the number of N atoms being 0, 1, 2 or 3 and the number of O and S atoms each being 0, 1 or 2;

wherein the heteroaryl group is optionally substituted with up to three substituents independently selected from the group consisting of hydroxyl, halogen, $(C_1-C_4)$-alkyl, $(C_1-C_4)$-alkoxy, halogenated $(C_1-C_4)$-alkyl, halogenated $(C_1-C_4)$-alkoxy) and carboxyl-$(C_1-C_6)$alkyl;

wherein the cycloheteroalkyl is optionally substituted with up to three substituents independently selected from the group consisting of oxo, $(C_1-C_8)$-alkyl, aryl, aryl-$(C_1-C_4)$-alkyl, hydroxyl, $(C_1-C_6)$alkoxy, carboxyl-$(C_1-C_6)$ alkyl, and carboxyl,

whereby each aryl group is optionally further substituted with up to three substituents independently selected from the group consisting of hydroxyl, halogen, $(C_1-C_4)$-alkyl, $(C_1-C_4)$-alkoxy, halogenated $(C_1-C_4)$-alkyl, and halogenated $(C_1-C_4)$-alkoxy); or

(ii) by two groups which are attached to the same carbon atom and are combined into a saturated or partly unsaturated cyclic 4, 5, 6, 7, or 8-membered ring system, optionally containing up to three heteroatoms selected from N, O and S, the number of N atoms being 0, 1, 2 or 3 and the number of O and S atoms each being 0, 1 or 2, whereby the cyclic ring system is optionally substituted by up to two substituents independently selected from oxo, $(C_1-C_6)$-alkyl, aryl and aryl-$(C_1-C_4)$-alkyl;

and wherein n represents

(a) 1, 2, 3, 4, 5 or 6, if -X-A-Y- together represent -NH-CO-$NR^4$-, -NH-CO-O-, -NH-CO-, -NH-CO-NH-$SO_2$-, -NH-$SO_2$-$NR^4$-, -NH-$SO_2$-O-, -NH-$SO_2$-, -O-CO-$NR^4$-,-O-CO-, -O-CO-NH-$SO_2$-$NR^4$-, or -O-, or

(b) 0, 1, 2, 3, 4, or 5, if -X-A-Y- together represent -CO-$NR^4$-, -CO-O-, -CO-, or -CO-NH-$NR^4$-.

**3.** A compound of the general formula I according to claim 1 or 2, which is an optically pure enantiomer having the formula (II)

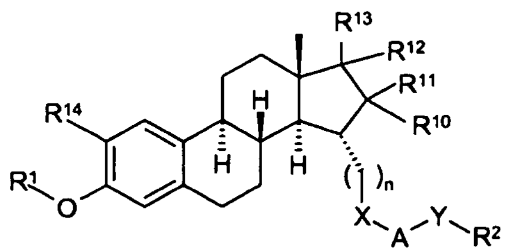

(II)

or a physiologically acceptable salt thereof.

**4.** A compound of the general formula I according to claims 1 or 2, which is an optically pure enantiomer having the formula (III)

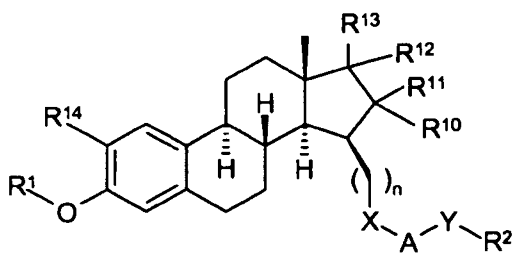

(III)

or a physiologically acceptable salt thereof.

**5.** A compound according to any of the preceding claims 1 to 4, wherein
$R^1$ is selected from:

(a) $-SO_2-NR^3R^{3'}$,
(b) $-CO-NR^3R^{3'}$,
(c) $-PO(OR^{16})-R^3$,
(d) $-PS(OR^{16})-R^3$,
(e) $-PO(OR^{16})-O-R^3$,
(f) $-SO_2-R^3$; and
(g) $-SO_2-O-R^3$;

wherein
$R^3$ and $R^{3'}$ are independently selected from -H, $-(C_1-C_8)$alkyl, phenyl and $-(C_1-C_4)$alkylphenyl, or
$R^3$ and $R^{3'}$ together with the nitrogen atom, to which $R^3$ and $R^{3'}$ are attached, form a heterocyclic 4-, 5-, 6-, 7- or 8-memberred ring, which is selected from the group consisting of

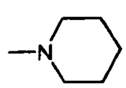 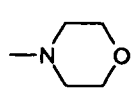 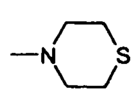 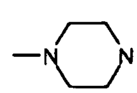

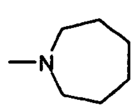 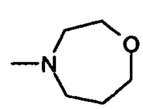 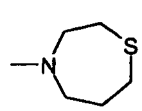 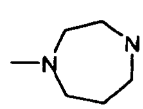

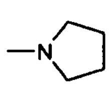 and 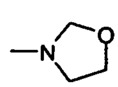

,

$R^{16}$ represents -H, $-(C_1-C_4)$alkyl , or-$(C_1-C_4)$alkyl-phenyl,

$R^{10}$ and $R^{11}$ both represent -H and $R^{12}$ and $R^{13}$ together represent =O; and $R^{14}$ represents -H, -($C_1$-$C_8$)alkyl, -O-($C_1$-$C_8$)alkyl, or -($C_1$-$C_8$)alkyl-O-(C1-$C_8$)alkyl.

**6.** A compound according to claim 5, wherein $R^1$ represents -$SO_2$-$NR^3R^{3'}$, wherein $R^3$ and $R^{3'}$ together with the nitrogen atom, to which $R^3$ and $R^{3'}$ are attached, form a heterocyclic ring, which is selected from the group consisting of morpholine, thiomorpholine and piperazyl, or wherein $R^1$ represents -$SO_2$-$NH_2$, and $R^{14}$ represents -H.

**7.** A compound according to any of the preceding claims 1 to 4, wherein $R^1$ represents -H, ($C_1$-$C_4$)alkyl, or-($C_1$-$C_4$)alkyl-phenyl; and $R^{10}$ and $R^{11}$ both represent -H and $R^{12}$ and $R^{13}$ together represent =O; and $R^{14}$ represents -($C_1$-$C_8$)alkyl, -O-($C_1$-$C_8$)alkyl, or-($C_1$-$C_8$)alkyl-O-($C_1$-$C_8$)alkyl.

**8.** A compound according to claim 7, wherein $R^{14}$ represents -($C_1$-$C_4$)alkyl, -O-($C_1$-$C_4$)alkyl, or -($C_1$-C4)alkyl-O-($C_1$-$C_4$)alkyl.

**9.** A compound according to claim 8, wherein $R^1$ represents -H, and $R^{14}$ represents ethyl, propyl, methoxyethyl or methoxy.

**10.** A compound according to any of the preceding claims 1 to 4, wherein $R^1$ represents -H, ($C_1$-$C_4$)alkyl, or -($C_1$-$C_4$)alkyl-phenyl; the substituents $R^{10}$, $R^{11}$, $R^{12}$ and $R^{13}$ together with the carbon atoms, to which they are attached, form a structure

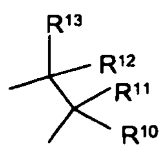

which is selected from the group of

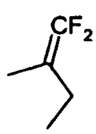 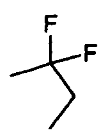 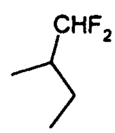 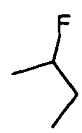

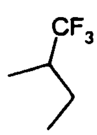 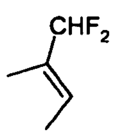 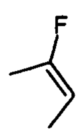 and 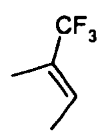 ,

and $R^{14}$ represents -H, -($C_1$-$C_8$)alkyl, -O-($C_1$-$C_8$)alkyl, or-($C_1$-$C_8$)alkyl-O-($C_1$-$C_8$)alkyl.

**11.** A compound according to claim 10, wherein the substituents $R^{10}$, $R^{11}$, $R^{12}$ and $R^{13}$ together with the carbon atoms, to which they are attached, form a structure

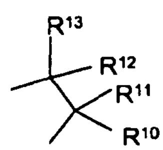 ,

which is selected from the group of

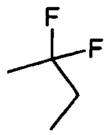 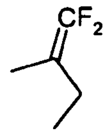 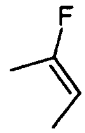 and 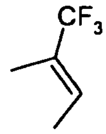.

**12.** A compound according to claim 11, wherein $R^1$ and $R^{14}$ each individually represent-H.

**13.** A compound according to any of the preceding claims 1 to 4, wherein
$R^1$ represents -H, $(C_1\text{-}C_4)$alkyl, or $-(C_1\text{-}C_4)$alkyl-phenyl:
the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring, which is partly unsaturated or aromatic, which contains one, two or three heteroatoms independently selected from N, O and S, the number of N atoms being 0, 1, 2 or 3 and the number of O and S atoms each being 0, 1 or 2, wherein one heteroatom is directly attached to the C17 C-atom of the steroidal core; and which ring is optionally substituted with an alkyl group; and
$R^{14}$ represents -H, $-(C_1\text{-}C_8)$alkyl, $-O-(C_1\text{-}C_8)$alkyl, or $-(C_1\text{-}C_8)$alkyl-$O-(C_1\text{-}C_8)$alkyl.

**14.** A compound according to claim 12, wherein
the substituents $R^{10}$, $R^{11}$, $R^{12}$ and $R^{13}$ together with the carbon atoms, to which $R^{10}$, $R^{11}$, $R^{12}$ and $R^{13}$ are attached, form a heterocyclic 5- or 6-membered ring to provide a compound of one of the following formulas

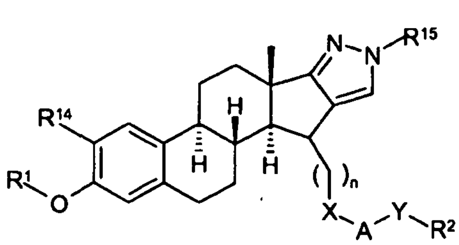, 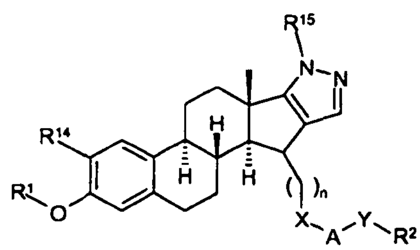,

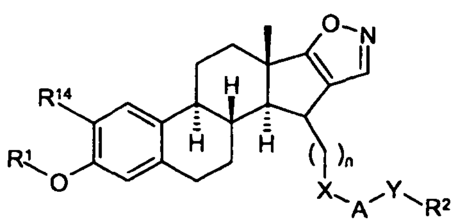, or 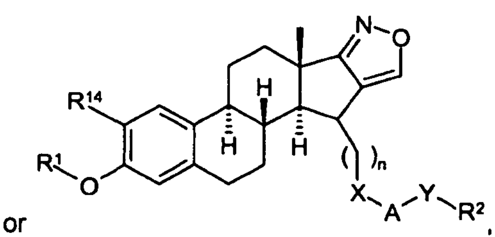,

wherein $R^{15}$ represents -H or $-(C_1\text{-}C_4)$alkyl.

**15.** A compound according to claim 14, wherein $R^1$ and $R^{14}$ each individually represent -H.

**16.** A compound according to any of the preceding claims 1 to 4, wherein
$R^1$ represents -H or $-SO_2\text{-}NH_2$,
the substituents $R^{10}$, $R^{11}$, $R^{12}$ and $R^{13}$ together with the carbon atoms, to which they are attached, form a structure

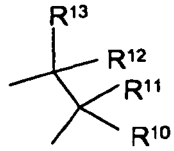,

which is selected from the group of

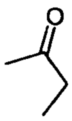 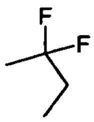 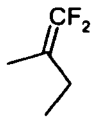 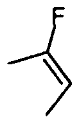 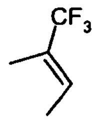 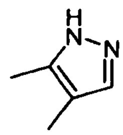 and 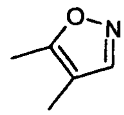 ;

and
$R^{14}$ represents -H, -($C_1$-$C_4$)alkyl, -O-($C_1$-$C_4$)alkyl, or -($C_1$-$C_4$)alkyl-O-($C_1$-$C_4$)alkyl.

**17.** A compound according to any of the preceding claims 1 to 16, wherein -X-A-Y- together represent a group selected from -CO-$NR^4$-, -CO-O-, -CO-, and -CO-NH-$NR^4$-; and n represents 0, 1, 2, 3, 4, or 5.

**18.** A compound according to claim 17, wherein -X-A-Y- together represent -CO-$NR^4$-.

**19.** A compound according to claim 18, wherein
$R^2$ represents a -($C_1$-$C_4$)alkyl-phenyl or a thiazolyl group, optionally substituted with -($C_1$-$C_4$)-alkyl, and $R^4$ represents -H; or
$R^2$ and $R^4$ form together with the nitrogen atom, to which $R^2$ and $R^4$ are attached, a morpholinyl group, and n represents 3.

**20.** A compound according to claim 1, selected from the group consisting of exemplary compounds:

N-Benzyl-4-(2-ethyl-3-hydroxy-17-oxo-estra-1,3,5(10)-trien-15β-yl)-butyramide
N-Benzyl-4-(3-hydroxy-17-oxo-2-propyl-estra-1,3,5(10)-trien-15β-yl)-butyramide
N-Benzyl-4-(3-hydroxy-2-(2-methoxy-ethyl)-17-oxo-estra-1,3,5(10)-trien-15β-yl)-butyramide
N-Benzyl-4-(3-hydroxy-2-methoxy-17-oxo-estra-1,3,5(10)-trien-15β-yl)-butyramide
2-Ethyl-3-hydroxy-15β-(4-morpholin-4-yl-4-oxo-butyl)-estra-1,3,5(10)-trien-17-one
3-Hydroxy-15β-(4-morpholin-4-yl-4-oxo-butyl)-2-propyl-estra-1,3,5(10)-trien-17-one
3-Hydroxy-2-(2-methoxy-ethyl)-15β-(4-morpholin-4-yl-4-oxo-butyl)-estra-1,3,5(10)-trien-17-one
3-Hydroxy-2-methoxy-15β Sp-(4-morpholin-4-yl-4-oxo-butyl)-estra-1,3,5(10)-trien-17-one
4-(2-Ethyl-3-hydroxy-17-oxo-estra-1,3,5(10)-trien-15β-yl)-N-(5-methyl-thiazol-2-yl)-butyramide
4-(3-Hydroxy-2-methoxy-17-oxo-estra-1,3,5(10)-trien-15β-yl)-N-(5-methyl-thiazol-2-yl)-butyramide
4-(17,17-Difluoro-3-hydroxy-estra-1,3,5(10)-trien-15β-yl)-1-morpholin-4-yl-butan-1-one
4-(17-Fluoro-3-hydroxy-estra-1,3,5(10),16-tetraen-15β-yl)-1-morpholin-4-yl-butan-1-one
4-(17-Difluoromethylene-3-hydroxy-estra-1,3,5(10)-trien-15β-yl)-1-morpholin-4-yl-butan-1-one
N-Benzyl-4-(17,17-difluoro-3-hydroxy-estra-1,3,5(10)-trien-15α-yl)-butyramide
3-Hydroxy-15β-(4-morpholin-4-yl-4-oxo-butyl)-estra-1,3,5(10)-trien-[16,17-c]-pyrazole
3-Sulphamate-15β-(4-morpholin-4-yl-4-oxo-butyl)-estra-1,3,5(10)-trien-17-one,
3-Sulphate-15β-(4-morpholin-4-yl-4-oxo-butyl)-estra-1,3,5(10)-trien-17-one,

and any physiologically acceptable salt thereof.

**21.** A compound according to any of the claims 1 to 20, for use as a medicament.

**22.** A pharmaceutical composition comprising as active agent a compound of formula (I) accord-22. A pharmaceutical composition comprising as active agent a compound of formula (I) according to any of the claims 1 to 20, and at least one pharmaceutically acceptable carrier.

**23.** A compound of formula (I) according to any of the preceding claims 1 to 20 for the treatment or prevention of a steroid hormone dependent disease or disorder in a mammal.

**24.** A compound of formula (I) according to claim 23 wherein the steroid hormone dependent disease or disorder is an estradiol dependent disease or disorder.

**25.** A compound of formula (I) according to claim 24, wherein the estradiol dependent disease or disorder is malign and is selected from the group consisting of breast cancer, ovarian cancer, uterine cancer, endometrial cancer, and endometrial hyperplasia.

**26.** A compound of formula (I) according to claim 25, wherein the malign disease or disorder is **characterized by** a detectable level of 17β-HSD1 and/or STS expression within a cancer tissue sample.

**27.** A compound of formula (I) according to claim 25 or 26, wherein the estradiol dependent disease is breast cancer and the mammal is a human post-menopausal female.

**28.** A compound of formula (I) according to claim 24, wherein the estradiol dependent disease or disorder is benign and is selected from the group consisting of endometriosis, uterine fibroids, uterine leiomyoma, adenomyosis, dysmenorrhea, menorrhagia, metrorrhagia, and urinary dysfunction.

**29.** A compound of formula (I) according to claim 28, wherein the mammal is a human pre- or peri-menopausal female.

**30.** A compound of formula (I) according to claim 23, wherein steroid hormone dependent disease or disorder is an androgen-dependent disease or disorder.

**31.** A compound of formula (I) according to claim 30, wherein the androgen-dependent disease or disorder is selected from the group consisting of acne, seborrhea, androgenetic alopecia, hirsutism, and prostate cancer.

**32.** A compound of formula (I) according to claim 23, wherein the steroid hormone dependent disease or disorder is an estrogen- or androgen dependent disease or disorder requiring the lowering of the endogeneous estrogen or androgen concentration in a generalized or tissue-specific manner.

**33.** A compound of formula (I) according to claim 32, wherein the disease or disorder is selected from the group consisting of prostadynia, benign prostatic hyperplasia, urinary dysfunction, lower urinary tract syndrome, squamous cell carcinoma, rheumatoid arthritis, type I and II diabetes, systemic lupus erythematosus, multiple sclerosis, myastenia gravis, thyroiditis, vasculitis, ulcerative colitis, Crohn's disease, psoriasis, contact dermatitis, graft versus host disease, eczema, asthma, organ rejection following transplantation, colon cancer, tissue wounds, skin wrinkles, cataracts, cognitive dysfunctions, senile dementia and Alzheimer's disease.

**34.** Use of a compound of formula (I) according to any of the preceding claims 1 to 20 for the manufacture of a medicament for the treatment or prevention of a steroid hormone dependent disease or disorder in a mammal.

**35.** Use of a compound of formula (I) according to claim 34, wherein the steroid hormone dependent disease or disorder is an estradiol dependent disease or disorder.

**36.** Use of a compound of formula (I) according to claim 35, wherein the estradiol dependent disease or disorder is malign and is selected from the group consisting of breast cancer, ovarian cancer, uterine cancer, endometrial cancer, and endometrial hyperplasia.

**37.** Use of a compound of formula (I) according to claim 36, wherein the malign disease or disorder is **characterized by** a detectable level of 17β-HSD1 and/or STS expression within a cancer tissue sample.

**38.** Use of a compound of formula (I) according to claim 36 or 37, wherein the estradiol dependent disease is breast cancer and the mammal is a human post-menopausal female.

**39.** Use of a compound of formula (I) according to claim 35, wherein the estradiol dependent disease or disorder is benign and is selected from the group consisting of endometriosis, uterine fibroids, uterine leiomyoma, adenomyosis, dysmenorrhea, menorrhagia, metrorrhagia, and urinary dysfunction.

**40.** Use of a compound of formula (I) according to claim 39, wherein the mammal is a human pre- or peri-menopausal female.

**41.** Use of a compound of formula (I) according to claim 34, wherein steroid hormone dependent disease or disorder is an androgen-dependent disease or disorder.

**42.** Use of a compound of formula (I) according to claim 41, wherein the androgen-dependent disease or disorder is selected from the group consisting of acne, seborrhea, androgenetic alopecia, hirsutism, and prostate cancer.

**43.** Use of a compound of formula (I) according to claim 34, wherein the steroid hormone dependent disease or disorder is an estrogen- or androgen dependent disease or disorder requiring the lowering of the endogeneous estrogen or androgen concentration in a generalized or tissue-specific manner.

**44.** Use of a compound of formula (I) according to claim 43, wherein the disease or disorder is selected from the group consisting of prostadynia, benign prostatic hyperplasia, urinary dysfunction, lower urinary tract syndrome, squamous cell carcinoma, rheumatoid arthritis, type I and II diabetes, systemic lupus erythematosus, multiple sclerosis, myastenia gravis, thyroiditis, vasculitis, ulcerative colitis, Crohn's disease, psoriasis, contact dermatitis, graft versus host disease, eczema, asthma, organ rejection following transplantation, colon cancer, tissue wounds, skin wrinkles, cataracts, cognitive dysfunctions, senile dementia and Alzheimer's disease.

**Patentansprüche**

**1.** Verbindung der allgemeinen Formel I,

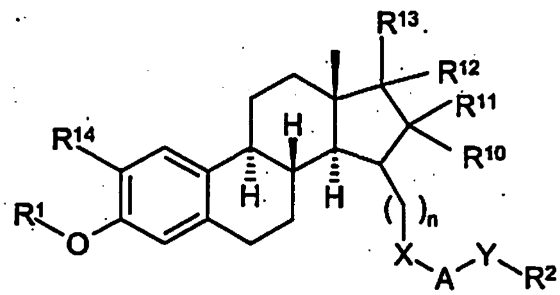

(I)

wobei -X-A-Y- zusammen eine Gruppe, ausgewählt aus

(a) -CO-$NR^4$-,
(b) -CO-O-,
(c) -CO-,
(d) -CO-NH-$NR^4$-,
(e) -NH-CO-NH-,
(f) -NH-CO-O-,
(g) -NH-CO-,
(h) -NH-CO-NH-$SO_2$-,
(i) -NH-$SO_2$-NH-,
(j) -NH-$SO_2$-O-,
(k) -NH-$SO_2$-
(l) -O-CO-NH-,
(m) -O-CO-,
(n) -O-CO-NH-$SO_2$-$NR^4$- und
(o) -O-

darstellt;
n 1, 2, 3, 4, 5 oder 6 darstellt oder, wenn -X-A-Y- -CO-$NR^4$-, -CO-O-, -CO- oder -CO-NH-$NR^4$-darstellt, n auch 0 darstellen kann;
$R^1$ ausgewählt ist aus:

(a) -H,
(b) -($C_1$-$C_6$) Alkyl, das gegebenenfalls mit Halogen, Nitril, -$OR^6$, -$SR^6$ oder -$COOR^6$ substituiert ist; wobei die Anzahl der Substituenten 1, 2 oder 3 für Halogen ist und 1 oder 2 für jede Kombination der Halogen-, Nitril-, -$OR^6$-, -$SR^6$- und -$COOR^6$-Einheiten,
(c) -Phenyl, das gegebenenfalls mit Halogen, Nitril, -$OR^6$, -$SR^6$, -$R^6$ oder -$COOR^6$ substituiert ist; wobei die Anzahl der Substituenten bis zu Perhalogen für Halogen ist und 1 oder 2 für jede Kombination der Halogen-, Nitril-, -$OR^6$-, -$SR^6$-, -$R^6$- und -$COOR^6$-Einheiten,

(d) -($C_1$-$C_4$)Alkylphenyl, worin der Alkylteil gegebenenfalls mit bis zu drei Halogenen substituiert ist; und der Phenylteil gegebenenfalls mit Halogen, Nitril, -$OR^6$, -$SR^6$, -$R^6$ oder -$COOR^6$ substituiert ist, wobei die Anzahl der Substituenten an dem Phenylteil bis zu Perhalogen für Halogen ist und 1 oder 2 für jede Kombination der Halogen-, Nitril-, -$OR^6$-, -$SR^6$-, -$R^6$- und -$COOR^6$-Einheiten,
(e) -$SO_2$-$NR^3R^{3'}$,
(f) -CO-$NR^3R^{3'}$,
(g) -PO ($OR^{16}$)-$R^3$,
(h) -PS ($OR^{16}$) -$R^3$,
(i) -PO($OR^{16}$)-O-$R^3$,
(j) -$SO_2$-$R^3$ und
(k) -$SO_2$-O- $R^3$;

wobei
$R^6$ H, - ($C_1$-$C_4$)Alkyl oder halogeniertes -($C_1$-$C_4$)Alkyl darstellt;
$R^3$ und $R^{3'}$ unabhängig ausgewählt sind aus H, Alkyl, Aryl und Arylalkyl oder $R^3$ und $R^{3'}$ zusammen mit dem Stickstoffatom, an das $R^3$ und $R^{3'}$ gebunden sind, einen heterocyclischen 4-, 5-, 6-, 7- oder 8-gliedrigen Ring bilden, der gegebenenfalls gesättigt, teilweise nichtgesättigt oder aromatisch ist; der gegebenenfalls bis zu drei zusätzliche Heteroatome enthält, ausgewählt aus N, 0 und S, wobei die Anzahl der zusätzlichen N-Atome 0, 1, 2 oder 3 ist und die Anzahl der 0- und S-Atome jeweils 0, 1 oder 2 ist; und
$R^{16}$ -H, Alkyl oder Arylalkyl darstellt;
$R^2$ und $R^4$ unabhängig ausgewählt sind aus:

(a) -H,
(b) gegebenenfalls substituiertem Alkyl,
(c) gegebenenfalls substituiertem Acyl, mit der Maßgabe, dass -X-A-Y- -CO-NH-$NR^4$-darstellt,
(d) gegebenenfalls substituiertem Aryl,
(e) gegebenenfalls substituiertem Heteroaryl und
(f) gegebenenfalls substituiertem Cycloheteroalkyl,

oder $R^2$ und $R^4$ zusammen mit dem Stickstoffatom, an das $R^2$ und $R^4$ gebunden sind, einen heterocyclischen 4-, 5-, 6-, 7- oder 8-gliedrigen Ring bilden, der gegebenenfalls gesättigt, teilweise nichtgesättigt oder aromatisch ist; der gegebenenfalls bis zu drei zusätzliche Heteroatome enthält, ausgewählt aus N, O und S, wobei die Anzahl der zusätzlichen N-Atome 0, 1, 2 oder 3 ist und die Anzahl der 0-und S-Atome jeweils 0, 1 oder 2 ist; und welcher Ring gegebenenfalls Teil eines mehrfachen anellierten Ringsystems ist, wobei der Ring oder das Ringsystem gegebenenfalls substituiert ist;
die Substituenten $R^{10}$, $R^{11}$, $R^{12}$ und $R^{13}$ zusammen mit den Kohlenstoffatomen, an die sie gebunden sind, eine Struktur

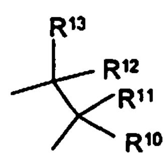

bilden, die ausgewählt ist aus der Gruppe von

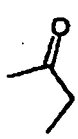
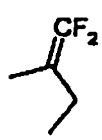
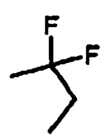
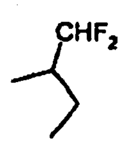
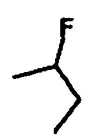

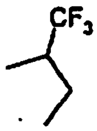 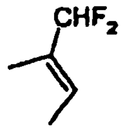 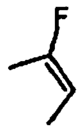 und 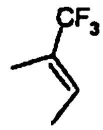

oder die Substituenten $R^{10}$, $R^{11}$, $R^{12}$ und $R^{13}$ zusammen mit den Kohlenstoffatomen, an die sie gebunden sind, einen heterocyclischen 5- oder 6-gliedrigen Ring bilden, der teilweise nichtgesättigt oder aromatisch ist, der ein, zwei oder drei Heteroatome enthält, unabhängig ausgewählt aus N, O und S, wobei die Anzahl der N-Atome 0, 1, 2 oder 3 ist und die Anzahl der 0- und S-Atome jeweils 0, 1 oder 2 ist, wobei ein Heteroatom direkt an das C17-C-Atom des Steroidkerns gebunden ist; und welcher Ring gegebenenfalls mit einer Alkylgruppe substituiert ist;
$R^{14}$ eine Alkyl-, Alkoxy- oder Alkoxyalkylgruppe darstellt oder
$R^{14}$ auch -H darstellen kann, mit der Maßgabe, dass wenigstens

(i) $R^1$ $-SO_2-NR^3R^{3'}$, $-CO-NR^3R^{3'}$, $-PO(OR^{16})-R^3$, $-PS(OR^{16})-R^3$, $-PO(OR^{16})-OR^3$, $-SO_2-R^3$ oder $-SO_2-OR^3$ darstellt; oder
(ii)

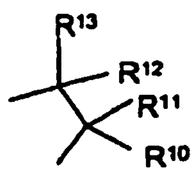 verschieden ist von 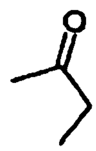

und alle pharmazeutisch verträglichen Salze davon.

**2.** Verbindung gemäß Anspruch 1,
wobei $R^2$ und $R^4$ unabhängig ausgewählt sind aus:

(a) -H, wobei, wenn -X-A-Y- zusammen -CO-O-oder -CO- darstellt, $R^2$ von -H verschieden ist,
(b) $-(C_1-C_{12})$Alkyl, gegebenenfalls substituiert mit bis zu fünf Substituenten, unabhängig ausgewählt aus der Gruppe bestehend aus Halogen, Hydroxy, Thiol, Nitril, Alkoxy, Aryloxy, Arylalkyloxy, Amino, Amido, Alkylthio, Arylthio, Arylalkylthio, Sulfamoyl, Sulfonamid, Acyl, Carboxy, Acylamino,
Aryl, welches Aryl gegebenenfalls mit bis zu drei Substituenten substituiert ist, unabhängig ausgewählt aus der Gruppe bestehend aus Halogen, Hydroxy, $(C_1-C_6)$ Alkoxy, $(C_1-C_6)$Alkyl, halogeniertem $(C_1-C_6)$Alkyl, halogeniertem $(C_1-C_6)$Alkoxy, Carboxy-$(C_1-C_6)$alkyl, Thiol, Nitril,
Sulfamoyl, Sulfonamid, Carboxy, Aryloxy, Arylalkyloxy, $(C_1-C_6)$Alkylthio, Arylthio, Arylalkylthio, Amino, Amido, Acyl, Acylamino und Heteroaryl; oder welches Aryl gegebenenfalls mit zwei Gruppen substituiert ist, die an benachbarte Kohlenstoffatome gebunden sind und zu einem gesättigten oder teilweise nichtgesättigten cyclischen 5-, 6-, 7-oder 8-gliedrigen Ringsystem kombiniert sind, das gegebenenfalls bis zu drei Heteroatome enthält, ausgewählt aus N, O und S, wobei die Anzahl der N-Atome 0, 1, 2 oder 3 ist und die Anzahl der 0-und S-Atome jeweils 0, 1 oder 2 ist;
Heteroaryl, welches Heteroaryl gegebenenfalls mit bis zu drei Substituenten substituiert ist, unabhängig ausgewählt aus der Gruppe bestehend aus Halogen, Hydroxy, $(C_1-C_6)$Alkoxy, $(C_1-C_6)$Alkyl, halogeniertem $(C_1-C_6)$ Alkyl, halogeniertem $(C_1-C_6)$Alkoxy, Carboxy-$(C_1-C_6)$alkyl, Thiol, Nitril, Sulfamoyl, Sulfonamid, Carboxy, Aryloxy, Arylalkyloxy, $(C_1-C_6)$Alkylthio, Arylthio, Arylalkylthio, Amino, Amido, Acyl, Acylamino, Aryl-$(C_1-C_4)$alkyl und Aryl;
wobei jede Arylgruppe gegebenenfalls mit bis zu drei Substituenten substituiert ist, unabhängig ausgewählt aus der Gruppe bestehend aus Hydroxy, Halogen, $(C_1-C_6)$Alkoxy, $(C_1-C_6)$Alkyl, halogeniertem $(C_1-C_6)$Alkyl und halogeniertem $(C_1-C_6)$Alkoxy ; und
Cycloheteroalkyl, welche Cycloheteroalkylgruppe gegebenenfalls mit bis zu drei Substituenten substituiert ist, unabhängig ausgewählt aus der Gruppe bestehend aus Oxo, $(C_1-C_8)$Alkyl, Aryl, Aryl-$(C_1-C_4)$alkyl, Hydroxy, $(C_1-C_6)$Alkoxy, Carboxy-$(C_1-C_6)$alkyl, Thiol, Nitril, Sulfamoyl, Sulfonamid, Carboxy, Aryloxy, Arylalkyloxy, $(C_1-C_6)$Alkylthio, Arylthio, Arylalkylthio, Amino, Amido, Acyl und Acylamino,
wobei jede Arylgruppe gegebenenfalls mit bis zu drei Substituenten substituiert ist, unabhängig ausgewählt aus der Gruppe bestehend aus Hydroxy, Halogen, $(C_1-C_4)$Alkyl, $(C_1-C_4)$Alkoxy, halogeniertem $(C_1-C_4)$Alkyl und halogeniertem $(C_1-C_4)$Alkoxy ;

(c) Acyl -(C=O)-R', wobei R' Wasserstoff, $(C_1-C_4)$Alkyl, Aryl, Aryl-$(C_1-C_4)$alkyl oder Heteroaryl-$(C_1-C_4)$alkyl darstellt;

welches Aryl oder Aryl-$(C_1-C_4)$alkyl in der Aryleinheit gegebenenfalls mit bis zu drei Substituenten substituiert ist, unabhängig ausgewählt aus der Gruppe bestehend aus Hydroxy, Halogen, $(C_1-C_4)$Alkoxy, $(C_1-C_4)$Alkyl oder halogeniertem $(C_1-C_4)$Alkyl;

(d) Aryl

welches Aryl gegebenenfalls mit bis zu drei Substituenten substituiert ist, unabhängig ausgewählt aus der Gruppe bestehend aus Halogen, Hydroxy, $(C_1-C_6)$Alkoxy, $(C_1-C_6)$Alkyl, halogeniertem $(C_1-C_6)$Alkyl, halogeniertem $(C_1-C_6)$Alkoxy, Carboxy-$(C_1-C_6)$alkyl, Thiol, Nitril, Nitro, Sulfamoyl, Sulfonamid, Carboxy, Aryloxy, Arylalkyloxy, $(C_1-C_6)$Alkylsulfonyl, Arylsulfonyl, $(C_1-C_6)$Alkylthio, Arylthio, Arylalkylthio, Amino, Amido, Acyl, Acylamino und Heteroaryl; oder

welches Aryl gegebenenfalls mit zwei Gruppen substituiert ist, die an benachbarte Kohlenstoffatome gebunden sind und zu einem gesättigten oder teilweise nichtgesättigten cyclischen 5-, 6-, 7- oder 8-gliedrigen Ringsystem kombiniert sind, das gegebenenfalls bis zu drei Heteroatome enthält, ausgewählt aus N, O und S, wobei die Anzahl der N-Atome 0, 1, 2 oder 3 ist und die Anzahl der 0- und S-Atome jeweils 0, 1 oder 2 ist;

(e) Heteroaryl,

welches Heteroaryl gegebenenfalls mit bis zu drei Substituenten substituiert ist, unabhängig ausgewählt aus der Gruppe bestehend aus Halogen, Hydroxy, $(C_1-C_6)$Alkoxy, $(C_1-C_6)$Alkyl, halogeniertem $(C_1-C_6)$Alkyl, halogeniertem $(C_1-C_6)$Alkoxy, Carboxy-$(C_1-C_6)$alkyl, Thiol, Nitril, Sulfamoyl, Sulfonamid, Arylsulfoxy, Carboxy, Aryloxy, Arylalkyloxy, $(C_1-C_6)$Alkylsulfonyl, Arylsulfonyl, $(C_1-C_6)$Alkylthio, Arylthio, Arylalkylthio, Amino, Amido, Acyl, Acylamino, Aryl-$(C_1-C_4)$alkyl und Aryl,

wobei jede Arylgruppe gegebenenfalls mit bis zu drei Substituenten substituiert ist, unabhängig ausgewählt aus der Gruppe bestehend aus Hydroxy, Halogen, $(C_1-C_6)$Alkoxy, $(C_1-C_6)$Alkyl, halogeniertem $(C_1-C_6)$Alkyl und halogeniertem $(C_1-C_6)$Alkoxy; oder

(f) Cycloheteroalkyl,

welches Cycloheteroalkyl gegebenenfalls mit bis zu drei Substituenten substituiert ist, unabhängig ausgewählt aus der Gruppe bestehend aus Oxo, $(C_1-C_{14})$Alkyl, Aryl, Aryl-$(C_1-C_4)$alkyl, Hydroxy, $(C_1-C_6)$Alkoxy, Carboxy-$(C_1-C_6)$alkyl, Thiol, Nitril, Sulfamoyl, Sulfonamid, Carboxy, Aryloxy, Arylalkyloxy, $(C_1-C_6)$Alkylthio, Arylthio, Arylalkylthio, Amino, Amido, Acyl und Acylamino,

wobei jede Arylgruppe gegebenenfalls mit bis zu drei Substituenten weiter substituiert ist, unabhängig ausgewählt aus der Gruppe bestehend aus Hydroxy, Halogen, $(C_1-C_4)$Alkyl, $(C_1-C_4)$Alkoxy, halogeniertem $(C_1-C_4)$Alkyl und halogeniertem $(C_1-C_4)$Alkoxy;

oder wobei $R^2$ und $R^4$ zusammen mit dem Stickstoffatom, an das $R^2$ und $R^4$ gebunden sind, einen heterocyclischen 4-, 5-, 6-, 7- oder 8-gliedrigen Ring bilden, der gegebenenfalls gesättigt oder teilweise nichtgesättigt ist; der gegebenenfalls bis zu drei zusätzliche Heteroatome enthält, ausgewählt aus N, O und S, wobei die Anzahl der zusätzlichen N-Atome 0, 1, 2 oder 3 ist und die Anzahl der 0- und S-Atome jeweils 0, 1 oder 2 ist; und welcher Ring gegebenenfalls Teil eines mehrfach anellierten Ringsystems ist,

wobei der Ring oder das Ringsystem gegebenenfalls substituiert ist

(i) mit bis zu drei Substituenten, unabhängig ausgewählt aus der Gruppe bestehend aus $(C_1-C_8)$Alkyl, Halogen, Hydroxy, Carboxy, Thiol, Nitril, $(C_1-C_6)$Alkoxy, Carboxy-$(C_1-C_6)$alkyl, Aryloxy, Arylalkyloxy, Amino, Amido, Alkylthio, Arylthio, Arylalkylthio, Sulfamoyl, Sulfonamid, Aryl, Aryl-$(C_1-C_4)$alkyl, Heteroaryl und Cycloheteroalkyl, wobei die $(C_1-C_9)$Alkylgruppe gegebenenfalls mit bis zu drei Substituenten substituiert ist, unabhängig ausgewählt aus Hydroxy, Halogen, $(C_1-C_4)$Alkoxy oder halogeniertem $(C_1-C_4)$Alkoxy,

wobei die Alkylkette der $(C_1-C_4)$Alkoxyeinheit gegebenenfalls mit Hydroxy substituiert ist;

wobei die Arylgruppe oder Aryleinheit gegebenenfalls mit bis zu drei Substituenten substituiert ist, unabhängig ausgewählt aus der Gruppe bestehend aus Hydroxy, Halogen, $(C_1-C_4)$Alkyl, $(C_1-C_4)$Alkoxy , halogeniertem $(C_1-C_4)$Alkyl, halogeniertem $(C_1-C_4)$Alkoxy und Carboxy-$(C_1-C_6)$alkyl, oder wobei die Aryleinheit gegebenenfalls mit zwei Gruppen substituiert ist, die an benachbarte Kohlenstoffatome gebunden sind und zu einem gesättigten oder teilweise nichtgesättigten cyclischen 5-, 6-, 7- oder 8-gliedrigen Ringsystem kombiniert sind, das gegebenenfalls bis zu drei Heteroatome enthält, ausgewählt aus N, 0 und S, wobei die Anzahl der N-Atome 0, 1, 2 oder 3 ist und die Anzahl der O- und S-Atome jeweils 0, 1 oder 2 ist;

wobei die Heteroarylgruppe gegebenenfalls mit bis zu drei Substituenten substituiert ist, unabhängig ausgewählt aus der Gruppe bestehend aus Hydroxy, Halogen, $(C_1-C_4)$Alkyl, $(C_1-C_4)$Alkoxy, halogeniertem $(C_1-C_4)$Alkyl, halogeniertem $(C_1-C_4)$Alkoxy und Carboxy-$(C_1-C_6)$alkyl ;

wobei das Cycloheteroalkyl gegebenenfalls mit bis zu drei Substituenten substituiert ist, unabhängig ausgewählt aus der Gruppe bestehend aus Oxo, $(C_1-C_8)$Alkyl, Aryl, Aryl-$(C_1-C_4)$alkyl, Hydroxy, $(C_1-C_6)$Alkoxy, Car-

boxy-($C_1$-$C_6$)alkyl und Carboxy,
wobei jede Arylgruppe gegebenenfalls mit bis zu drei Substituenten weiter substituiert ist, unabhängig ausgewählt aus der Gruppe bestehend aus Hydroxy, Halogen, ($C_1$-$C_4$)Alkyl, ($C_1$-$C_4$)Alkoxy, halogeniertem ($C_1$-$C_4$) Alkyl und halogeniertem ($C_1$-$C_4$)Alkoxyl oder
(ii) mit zwei Gruppen, die an das gleiche Kohlenstoffatom gebunden sind und zu einem gesättigten oder teilweise nichtgesättigten cyclischen 4-, 5-, 6-, 7- oder 8-gliedrigen Ringsystem kombiniert sind, das gegebenenfalls bis zu drei Heteroatome enthält, ausgewählt aus N, O und S, wobei die Anzahl der N-Atome 0, 1, 2 oder 3 ist und die Anzahl der 0- und S-Atome jeweils 0, 1 oder 2 ist;
wobei das cyclische Ringsystem gegebenenfalls mit bis zu zwei Substituenten substituiert ist, unabhängig ausgewählt aus Oxo, ($C_1$-$C_6$)Alkyl, Aryl und Aryl-($C_1$-$C_4$)alkyl;

und wobei n

(a) 1, 2, 3, 4, 5 oder 6 darstellt, wenn -X-A-Y- zusammen -NH-CO-$NR^4$-, -NH-CO-O-, -NH-CO-, -NH-CO-NH-$SO_2$-, -NH-$SO_2$-$NR^4$-, -NH-$SO_2$-O-, -NH-$SO_2$-, -O-CO-$NR^4$-, -O-CO-, -O-CO-NH-$SO_2$-$NR^4$- oder -O- darstellt, oder
(b) 0, 1, 2, 3, 4, oder 5 darstellt, wenn -X-A-Y- zusammen -CO-$NR^4$-, -CO-O-, -CO-oder -CO-NH-$NR^4$- darstellt.

**3.** Verbindung der allgemeinen Formel I gemäß Anspruch 1 oder 2, die ein optisch reines Enantiomer mit der Formel (II) ist,

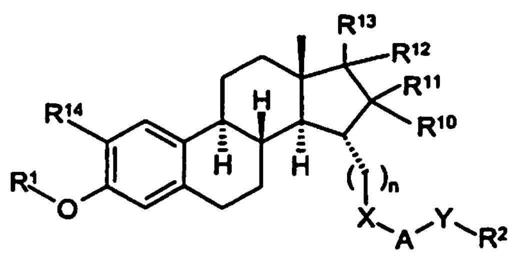

(II)

oder ein physiologisch verträgliches Salz davon.

**4.** Verbindung der allgemeinen Formel I gemäß Anspruch 1 oder 2, die ein optisch reines Enantiomer mit der Formel (III) ist,

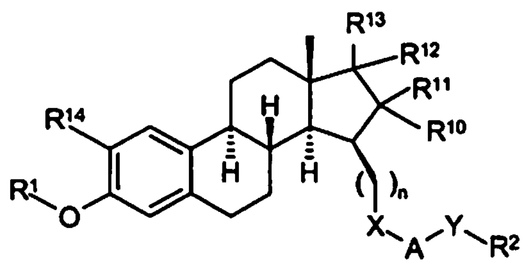

(III)

oder ein physiologisch verträgliches Salz davon.

**5.** Verbindung gemäß einem der vorstehenden Ansprüche 1 bis 4, wobei $R^1$ ausgewählt ist aus:

(a) -$SO_2$-$NR^3R^{3'}$,
(b) -CO-$NR^3R^{3'}$,
(c) -PO($OR^{16}$)-$R^3$,
(d) -PS($OR^{16}$)-$R^3$,
(e) -PO($OR^{16}$)-O-$R^3$,
(f) -$SO_2$-$R^3$; und
(g) -$SO_2$-O- $R^3$;

wobei
$R^3$ und $R^{3'}$ unabhängig ausgewählt sind aus -H, -($C_1$-$C_8$)Alkyl, Phenyl und -($C_1$-$C_4$)Alkylphenyl, oder
$R^3$ und $R^{3'}$ zusammen mit dem Stickstoffatom, an das $R^3$ und $R^{3'}$ gebunden sind, einen heterocyclischen 4-, 5-, 6-, 7- oder 8-gliedrigen Ring bilden, der ausgewählt ist aus der Gruppe bestehend aus

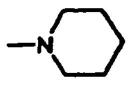 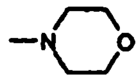 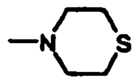 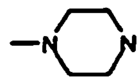

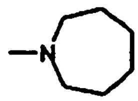 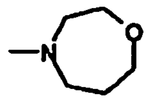 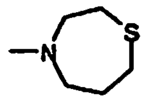 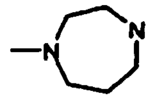

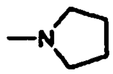 und 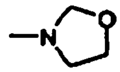

$R^{16}$ -H, -($C_1$-$C_4$)Alkyl oder -($C_1$-$C_4$)Alkylphenyl darstellt,
$R^{10}$ und $R^{11}$ beide -H darstellen und $R^{12}$ und $R^{13}$ zusammen =O darstellen; und
$R^{14}$ -H, -$C_1$-$C_8$)Alkyl, -O-($C_1$-$C_8$)Alkyl oder -($C_1$-$C_8$)Alkyl-O-($C_1$-$C_8$)alkyl darstellt.

**6.** Verbindung gemäß Anspruch 5, wobei
$R^1$ -$SO_2$-$NR^3R^{3'}$ darstellt, wobei $R^3$ und $R^{3'}$ zusammen mit dem Stickstoffatom, an das $R^3$ und $R^{3'}$ gebunden sind, einen heterocyclischen Ring bilden, der ausgewählt ist aus der Gruppe bestehend aus Morpholin, Thiomorpholin und Piperazyl, oder wobei $R^1$ -$SO_2$-$NH_2$ darstellt, und
$R^{14}$ -H darstellt.

**7.** Verbindung gemäß einem der vorstehenden Ansprüche 1 bis 4, wobei
$R^1$ -H, ($C_1$-$C_4$) Alkyl oder -($C_1$-$C_4$) Alkylphenyl darstellt; und
$R^{10}$ und $R^{11}$ beide -H darstellen und $R^{12}$ und $R^{13}$ zusammen =O darstellen; und
$R^{14}$ -($C_1$-$C_8$)Alkyl, -O-($C_1$-$C_8$)Alkyl oder -($C_1$-$C_8$)Alkyl-O-($C_1$-$C_8$)alkyl darstellt.

**8.** Verbindung gemäß Anspruch 7, wobei
$R^{14}$ - ($C_1$-$C_4$) Alkyl, -0- ($C_1$-$C_4$) Alkyl oder -($C_1$-$C_4$)Alkyl-O-($C_1$-$C_4$)alkyl darstellt.

**9.** Verbindung gemäß Anspruch 8, wobei
$R^1$ -H darstellt und
$R^{14}$ Ethyl, Propyl, Methoxyethyl oder Methoxy darstellt.

**10.** Verbindung gemäß einem der vorstehenden Ansprüche 1 bis 4, wobei
$R^1$ -H, ($C_1$-$C_4$)Alkyl oder -($C_1$-$C_4$)Alkylphenyl darstellt;
die Substituenten $R^{10}$, $R^{11}$, $R^{12}$ und $R^{13}$ zusammen mit den Kohlenstoffatomen, an die sie gebunden sind, eine Struktur

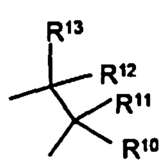

bilden, die ausgewählt ist aus der Gruppe von

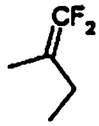 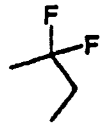 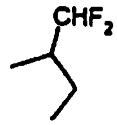 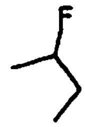

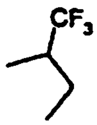 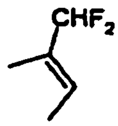 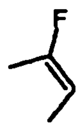 und 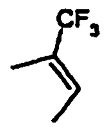 ,

und
$R^{14}$ -H, -($C_1$-$C_8$)Alkyl, -O-($C_1$-$C_8$)Alkyl oder - ($C_1$-$C_8$) Alkyl-O- ($C_1$-$C_8$) alkyl darstellt.

**11.** Verbindung gemäß Anspruch 10, wobei die Substituenten $R^{10}$, $R^{11}$, $R^{12}$ und $R^{13}$ zusammen mit den Kohlenstoffatomen, an die sie gebunden sind, eine Struktur

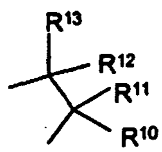

bilden, die ausgewählt ist aus der Gruppe von

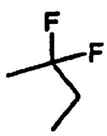 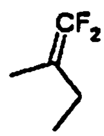 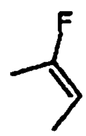 und 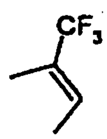 .

**12.** Verbindung gemäß Anspruch 11, wobei $R^1$ und $R^{14}$ jeweils -H darstellen.

**13.** Verbindung gemäß einem der vorstehenden Ansprüche 1 bis 4, wobei
$R^1$ -H, ($C_1$-$C_4$) Alkyl oder -($C_1$-$C_4$) Alkylphenyl darstellt;
die Substituenten $R^{10}$, $R^{11}$, $R^{12}$ und $R^{13}$ zusammen mit den Rohlenstoffatomen, an die $R^{10}$, $R^{11}$, $R^{12}$ und $R^{13}$ gebunden sind, einen heterocyclischen 5- oder 6-gliedrigen Ring bilden, der teilweise nichtgesättigt oder aromatisch ist, der ein, zwei oder drei Heteroatome enthält, unabhängig ausgewählt aus N, O und S, wobei die Anzahl der N-Atome 0, 1, 2 oder 3 ist und die Anzahl der 0-und S-Atome jeweils 0, 1 oder 2 ist, wobei ein Heteroatom direkt an das C-Atom C17 des Steroidkerns gebunden ist; und welcher Ring gegebenenfalls mit einer Alkylgruppe substituiert ist; und
$R^{14}$ -H, -($C_1$-$C_8$)Alkyl, -O-($C_1$-$C_8$)Alkyl oder -($C_1$-$C_8$)Alkyl-O-($C_1$-$C_8$)alkyl darstellt.

**14.** Verbindung gemäß Anspruch 12, wobei die Substituenten $R^{10}$, $R^{11}$, $R^{12}$ und $R^{13}$ zusammen mit den Kohlenstoffatomen, an die $R^{10}$, $R^{11}$, $R^{12}$ und $R^{13}$ gebunden sind, einen heterocyclischen 5- oder 6-gliedrigen Ring bilden, um eine Verbindung gemäß einer der folgenden Formeln zu ergeben,

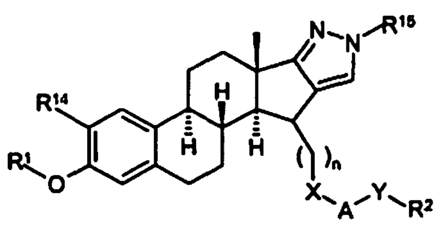,
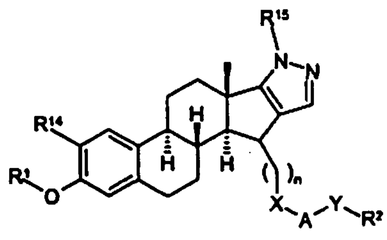,

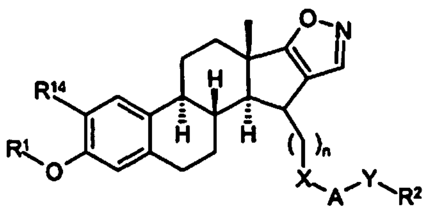
oder
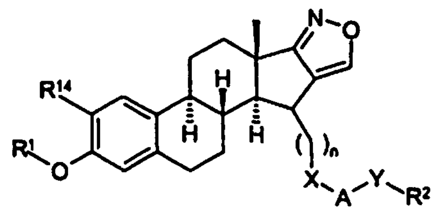,

wobei $R^{15}$ -H oder -($C_1$-$C_4$)Alkyl darstellt.

**15.** Verbindung gemäß Anspruch 14, wobei $R^1$ und $R^{14}$ jeweils -H darstellen.

**16.** Verbindung gemäß einem der vorstehenden Ansprüche 1 bis 4, wobei
$R^1$ -H oder -$SO_2$-$NH_2$ darstellt,
die Substituenten $R^{10}$, $R^{11}$, $R^{12}$ und $R^{13}$ zusammen mit den Kohlenstoffatomen, an die sie gebunden sind, eine Struktur

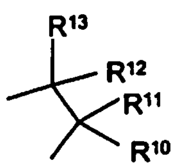

bilden, die ausgewählt ist aus der Gruppe von

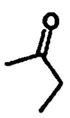 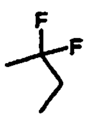 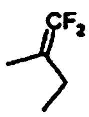 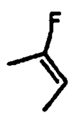 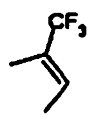 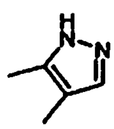 und 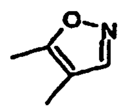 ;

und
$R^{14}$ -H, -($C_1$-$C_4$)Alkyl, -O-($C_1$-$C_4$)Alkyl oder -($C_1$-$C_4$)Alkyl-O-($C_1$-$C_4$)alkyl darstellt.

**17.** Verbindung gemäß einem der vorstehenden Ansprüche 1 bis 16, wobei -X-A-Y- zusammen eine Gruppe darstellt, ausgewählt aus -CO-$NR^4$-, -CO-O-, -CO- und -CO-NH-$NR^4$- ; und n 0, 1, 2, 3, 4 oder 5 darstellt.

**18.** Verbindung gemäß Anspruch 17, wobei -X-A-Y-zusammen -CO-$NR^4$- darstellt.

**19.** Verbindung gemäß Anspruch 18, wobei
$R^2$ ein - ($C_1$-$C_4$)Alkylphenyl oder eine Thiazolylgruppe darstellt, gegebenenfalls substituiert mit -($C_1$-$C_4$)Alkyl, und $R^4$ -H darstellt; oder
$R^2$ und $R^4$ zusammen mit dem Stickstoffatom, an das $R^2$ und $R^4$ gebunden sind, eine Morpholinylgruppe bilden, und n 3 darstellt.

**20.** Verbindung gemäß Anspruch 1, ausgewählt aus der Gruppe bestehend aus den Beispielverbindungen

N-Benzyl-4-(2-ethyl-3-hydroxy-17-oxoestra-1,3,5(10)-trien-15β-yl)butyramid
N-Benzyl-4-(3-hydroxy-17-oxo-2-propylestra-1,3,5(10)-trien-15β-yl)butyramid
N-Benzyl-4-(3-hydroxy-2-(2-methoxyethyl)-17-oxoestra-1,3,5(10)-trien-15β-yl)butyramid
N-Benzyl-4-(3-hydroxy-2-methoxy-17-oxoestra-1,3,5(10)-trien-15β-yl)butyramid
2-Ethyl-3-hydroxy-15β-(4-morpholin-4-yl-4-oxobutyl)estra-1,3,5(10)-trien-17-on
3-Hydroxy-15β-(4-morpholin-4-yl-4-oxobutyl)-2-propylestra-1,3,5(10)-trien-17-on
3-Hydroxy-2-(2-methoxyethyl)-15β-(4-morpholin-4-yl-4-oxobutyl)estra-1,3,5(10)-trien-17-on
3-Hydroxy-2-methoxy-15β-(4-morpholin-4-yl-4-oxobutyl)estra-1,3,5(10)-trien-17-on
4-(2-Ethyl-3-hydroxy-17-oxoestra-1,3,5(10)-trien-15β-yl)-N-(5-methylthiazol-2-yl)butyramid
4-(3-Hydroxy-2-methoxy-17-oxoestra-1,3,5(10)-trien-15β-yl)-N-(5-methylthiazol-2-yl)butyramid
4-(17,17-Difluor-3-hydroxyestra-1,3,5(10)-trien-15β-yl)-1-morpholin-4-ylbutan-1-on
4-(17-Fluor-3-hydroxyestra-1,3,5(10),16-tetraen-15β-yl)-1-morpholin-4-ylbutan-1-on
4-(17-Difluormethylen-3-hydroxyestra-1,3,5(10)-trien-15β-yl)-1-morpholin-4-ylbutan-1-on
N-Benzyl-4-(17,17-difluor-3-hydroxyestra-1,3,5(10)-trien-15α-yl)butyramid
3-Hydroxy-15β-(4-morpholin-4-yl-4-oxobutyl)estra-1,3,5(10)-trien-[16,17-c]pyrazol
3-Sulfamat-15β-(4-morpholin-4-yl-4-oxobutyl)estra-1,3,5(10)-trien-17-on
3-Sulfat-15β-(4-morpholin-4-yl-4-oxobutyl)estra-1,3,5(10)-trien-17-on
und jedes physiologisch verträgliche Salz davon.

**21.** Verbindung gemäß einem der Ansprüche 1 bis 20 für die Verwendung als Medikament.

**22.** Pharmazeutische Zusammensetzung, umfassend als Wirkstoff eine Verbindung der Formel (I) gemäß einem der Ansprüche 1 bis 20 und wenigstens einen pharmazeutisch verträglichen Träger.

**23.** Verbindung der Formel (I) gemäß einem der vorstehenden Ansprüche 1 bis 20 für die Behandlung oder Vorbeugung einer Steroidhormon-abhängigen Erkrankung oder Störung bei einem Säuger.

**24.** Verbindung der Formel (I) gemäß Anspruch 23, wobei die Steroidhormon-abhängige Erkrankung oder Störung eine Estradiol-abhängige Erkrankung oder Störung ist.

**25.** Verbindung der Formel (I) gemäß Anspruch 24, wobei die Estradiol-abhängige Erkrankung oder Störung malign ist und ausgewählt ist aus der Gruppe bestehend aus Brustkrebs, Ovarialkrebs, Uteruskrebs, Endometriumkrebs und Endometriumhyperplasie.

**26.** Verbindung der Formel (I) gemäß Anspruch 25, wobei die maligne Erkrankung oder Störung durch eine nachweisbare Höhe von 17β-HSD1- und/oder STS-Expression in einer Krebsgewebeprobe gekennzeichnet ist.

**27.** Verbindung der Formel (I) gemäß Anspruch 25 oder 26, wobei die Estradiol-abhängige Erkrankung Brustkrebs ist und der Säuger eine menschliche, postmenaupausale Frau ist.

**28.** Verbindung der Formel (I) gemäß Anspruch 24, wobei die Estradiol-abhängige Erkrankung oder Störung benign ist und ausgewählt ist aus der Gruppe bestehend aus Endometriose, Uterusmyomen, Uterusleiomyom, Adenomyose, Dysmenorrhoe, Menorrhagie, Metrorrhagie und Harntrakt-Dysfunktion.

**29.** Verbindung der Formel (I) gemäß Anspruch 28, wobei der Säuger eine menschliche, prä- oder postmenaupausale Frau ist.

**30.** Verbindung der Formel (I) gemäß Anspruch 23, wobei die Steroidhormon-abhängige Erkrankung oder Störung eine Androgen-abhängige Erkrankung oder Störung ist.

**31.** Verbindung der Formel (I) gemäß Anspruch 30, wobei die Androgen-abhängige Erkrankung oder Störung ausgewählt ist aus der Gruppe bestehend aus Akne, Seborrhoe, androgener Alopezie, Hirsutismus und Prostatakrebs.

**32.** Verbindung der Formel (I) gemäß Anspruch 23, wobei die Steroidhormon-abhängige Erkrankung oder Störung eine Estrogen- oder Androgen-abhängige Erkrankung oder Störung ist, die das Absenken der endogenen Estrogen- oder Androgenkonzentration auf generalisierte oder gewebespezifische weise erfordert.

**33.** Verbindung der Formel (I) gemäß Anspruch 32, wobei die Erkrankung oder Störung ausgewählt ist aus der Gruppe

bestehend aus Prostadynie, benigner Prostatahyperplasie, Harntrakt-Dysfunktion, Syndrom des unteren Harntrakts, Plattenepithelkarzinom, rheumatoider Arthritis, Typ-I- und Typ-II-Diabetes, systemischem Lupus erythematodes, Multiple Sklerose, Myasthenia gravis, Thyroiditis, Vaskulitis, Colitis ulcerosa, Morbus Crohn, Psoriasis, Kontaktdermatitis, Graftversus-Host-Erkrankung, Ekzem, Asthma, Organabstoßung nach Transplantation, Colonkrebs, Gewebewunden, Hautfalten, Katarakte, kognitiven Dysfunktionen, seniler Demenz und Alzheimer-Erkrankung.

**34.** Verwendung einer Verbindung der Formel (I) gemäß einem der vorstehenden Ansprüche 1 bis 20 für die Herstellung eines Medikaments für die Behandlung oder Vorbeugung einer Steroidhormon-abhängigen Erkrankung oder Störung bei einem Säuger.

**35.** Verwendung einer Verbindung der Formel (I) gemäß Anspruch 34, wobei die Steroidhormon-abhängige Erkrankung oder Störung eine Estradiol-abhängige Erkrankung oder Störung ist.

**36.** Verwendung einer Verbindung der Formel (I) gemäß Anspruch 35, wobei die Estradiol-abhängige Erkrankung oder Störung malign ist und ausgewählt ist aus der Gruppe bestehend aus Brustkrebs, Ovarialkrebs, Uteruskrebs, Endometriumkrebs und Endometriumhyperplasie.

**37.** Verwendung einer Verbindung der Formel (I) gemäß Anspruch 36, wobei die maligne Erkrankung oder Störung durch eine nachweisbare Höhe von 17β-HSD1-und/oder STS-Expression in einer Krebsgewebeprobe gekennzeichnet ist.

**38.** Verwendung einer Verbindung der Formel (I) gemäß Anspruch 36 oder 37, wobei die Estradiol-abhängige Erkrankung Brustkrebs ist und der Säuger eine menschliche, postmenaupausale Frau ist.

**39.** Verwendung einer Verbindung der Formel (I) gemäß Anspruch 35, wobei die Estradiol-abhängige Erkrankung oder Störung benign ist und ausgewählt ist aus der Gruppe bestehend aus Endometriose, Uterusmyomen, Uterusleiomyom, Adenomyose, Dysmenorrhoe, Menorrhagie, Metrorrhagie und Harntrakt-Dysfunktion.

**40.** Verwendung einer Verbindung der Formel (I) gemäß Anspruch 39, wobei der Säuger eine menschliche, prä- oder postmenaupausale Frau ist.

**41.** Verwendung einer Verbindung der Formel (I) gemäß Anspruch 34, wobei die Steroidhormon-abhängige Erkrankung oder Störung eine Androgen-abhängige Erkrankung oder Störung ist.

**42.** Verwendung einer Verbindung der Formel (I) gemäß Anspruch 41, wobei die Androgen-abhängige Erkrankung oder Störung ausgewählt ist aus der Gruppe bestehend aus Akne, Seborrhoe, androgener Alopezie, Hirsutismus und Prostatakrebs.

**43.** Verwendung einer Verbindung der Formel (I) gemäß Anspruch 34, wobei die Steroidhormon-abhängige Erkrankung oder Störung eine Estrogen- oder Androgen-abhängige Erkrankung oder Störung ist, die das Absenken der endogenen Estrogen- oder Androgenkonzentration auf generalisierte oder gewebespezifische Weise erfordert.

**44.** Verwendung einer Verbindung der Formel (I) gemäß Anspruch 43, wobei die Erkrankung oder Störung ausgewählt ist aus der Gruppe bestehend aus Prostadynie, benigner Prostatahyperplasie, Harntrakt-Dysfunktion, Syndrom des unteren Harntrakts, Plattenepithelkarzinom, rheumatoider Arthritis, Typ-I- und Typ-II-Diabetes, systemischem Lupus erythematodes, Multiple Sklerose, Myasthenia gravis, Thyroiditis, Vaskulitis, Colitis ulcerosa, Morbus Crohn, Psoriasis, Kontaktdermatitis, Graft-versus-Host-Erkrankung, Ekzem, Asthma, Organabstoßung nach Transplantation, Colonkrebs, Gewebewunden, Hautfalten, Katarakte, kognitiven Dysfunktionen, seniler Demenz und Alzheimer-Erkrankung.

## Revendications

**1.** Composé de formule générale I,

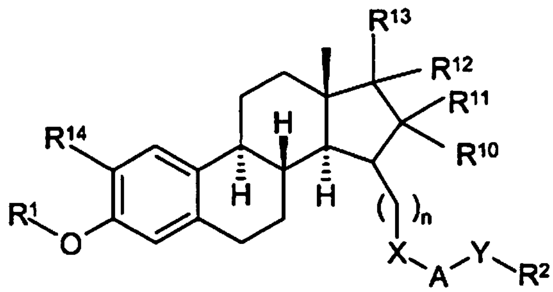

(I)

dans laquelle -X-A-Y- représente ensemble un groupement sélectionné parmi

(a) -CO-$NR^4$-,
(b) -CO-O-,
(c) -CO-,
(d) -CO-NH-$NR^4$-,
(e) -NH-CO-NH-,
(f) -NH-CO-O-,
(g) -NH-CO-,
(h) -NH-CO-NH-$SO_2$-,
(i) -NH-$SO_2$-NH-,
(j) -NH-$SO_2$-O-,
(k) -NH-$SO_2$-
(l) -O-CO-NH-,
(m) -O-CO-,
(n) -O-CO-NH-$SO_2$-$NR^4$-, et
(o) -O- ;

n représente 1, 2, 3, 4, 5 ou 6, ou, si -X-A-Y-représente -CO-$NR^4$-, -CO-O-, -CO-, ou -CO-NH-$NR^4$-, alors n peut également représenter 0 ;
$R^1$ est sélectionné parmi:

(a) -H,
(b) -($C_1$-$C_6$)alkyle, qui est éventuellement substitué par halogène, nitrile, -$OR^6$, -$SR^6$, ou -$COOR^6$ ; le nombre desdits substituants étant égal à 1, 2 ou 3 pour halogène, et égal à 1 ou 2 pour une association quelconque desdits motifs halogène, nitrile, -$OR^6$, -$SR^6$, ou -$COOR^6$,
(c) -phényle, qui est éventuellement substitué par halogène, nitrile, -$OR^6$, -$SR^6$, -$R^6$, ou -$COOR^6$, le nombre desdits substituants allant jusqu'à perhalogéno pour halogène, et à 1 ou 2 pour une association quelconque desdits motifs halogène, nitrile, -$OR^6$, -$SR^6$, -$R^6$ ou -$COOR^6$,
(d) -($C_1$-$C_4$)alkylphényle, dans lequel la partie alkyle est éventuellement substituée par jusqu'à trois halogènes ; et la partie phényle est éventuellement substituée par halogène, nitrile, -$OR^6$, -$SR^6$, -$R^6$ ou -$COOR^6$, le nombre de substituants sur ladite partie phényle allant jusqu'à perhalogéno pour halogène, et à 1 ou 2 pour une association quelconque desdits motifs halogène, nitrile, -$OR^6$, -$SR^6$, -$R^6$ ou -$COOR^6$,
(e) -$SO_2$-$NR^3R^{3'}$,
(f) -CO-$NR^3R^{3'}$,
(g) -PO($OR^{16}$)-$R^3$,
(h) -PS($OR^{16}$)-$R^3$,
(i) -PO($OR^{16}$)-O-$R^3$
(j) -$SO_2$-$R^3$, et
(k) -$SO_2$-O- $R^3$ ;

dans lesquels
$R^6$ représente H, -($C_1$-$C_4$)alkyle ou -($C_1$-$C_4$)alkyle halogéné ;
$R^3$ et $R^{3'}$ sont sélectionnés indépendamment parmi H, alkyle, aryle et arylalkyle, ou $R^3$ et $R^{3'}$ forment, conjointement avec l'atome d'azote auquel $R^3$ et $R^{3'}$ sont liés, un cycle hétérocyclique de 4, 5, 6, 7 ou 8 chaînons, qui est éventuellement saturé, partiellement insaturé, ou aromatique ; qui contient éventuellement jusqu'à trois hétéroatomes supplémentaires sélectionnés parmi N, O et S, le nombre d'atomes de N supplémentaires étant égal à 0, 1, 2 ou 3

et le nombre d'atomes de O et S étant chacun égal à 0, 1 ou 2 ; et
$R^{16}$ représente -H, alkyle, ou arylalkyle ;
$R^2$ et $R^4$ sont sélectionnés indépendamment parmi:

(a) -H,
(b) alkyle éventuellement substitué,
(c) acyle éventuellement substitué, à condition que -X-A-Y- représente -CO-NH-$NR^4$-,
(d) aryle éventuellement substitué,
(e) hétéroaryle éventuellement substitué, et
(f) cyclohétéroalkyle éventuellement substitué,

ou $R^2$ et $R^4$ forment, conjointement avec l'atome d'azote auquel $R^2$ et $R^4$ sont liés, un cycle hétérocyclique à 4, 5, 6, 7 ou 8 chaînons, qui est éventuellement saturé, partiellement insaturé, ou aromatique ; qui contient éventuellement jusqu'à trois hétéroatomes supplémentaires sélectionnés parmi N, O et S, le nombre d'atomes de N supplémentaires étant égal à 0, 1, 2 ou 3 et le nombre d'atomes de 0 et S étant chacun égal à 0, 1 ou 2 ; et lequel cycle fait éventuellement partie d'un système de cycles condensés multiples, où le cycle ou le système de cycles est éventuellement substitué ;
les substituants $R^{10}$, $R^{11}$, $R^{12}$ et $R^{13}$, conjointement avec les atomes de carbone auxquels ils sont liés, forment une structure

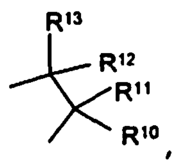

qui est sélectionnée dans le groupe constitué par

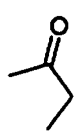
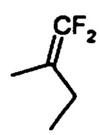
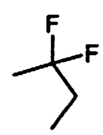
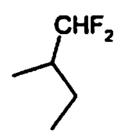
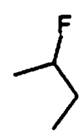

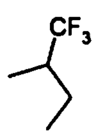
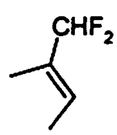
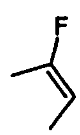
et
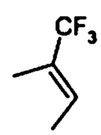

ou, les substituants $R^{10}$, $R^{11}$, $R^{12}$ et $R^{13}$, conjointement avec les atomes de carbone auxquels ils sont liés, forment un cycle hétérocyclique de 5 ou 6 chaînons, qui est partiellement insaturé ou aromatique, qui contient un, deux ou trois hétéroatomes sélectionnés indépendamment parmi N, O et S, le nombre d'atomes de N étant égal à 0, 1, 2 ou 3 et le nombre d'atomes de O et S étant chacun égal à 0, 1 ou 2, où un hétéroatome est lié directement à l'atome de C C17 du noyau stéroïdien ; et lequel cycle est éventuellement substitué par un groupement alkyle ;
$R^{14}$ représente un groupement alkyle, alcoxy ou alcoxyalkyle, ou
$R^{14}$ peut également représenter -H, à condition qu'au moins

(i) $R^1$ représente $-SO_2-NR^3R^{3'}$, $-CO-NR^3R^{3'}$, $-PO(OR^{16})-R^3$, $-PS(OR^{16})-R^3$, $-PO(OR^{16})-OR^3$, $-SO-R^3$,ou $-SO_2OR^3$ ; ou
(ii)

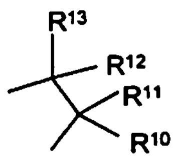 est différent de 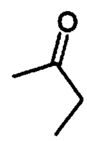

ainsi que tous les sels pharmacologiquement acceptables de celui-ci.

**2.** Composé selon la revendication 1,
dans lequel $R^2$ et $R^4$ sont sélectionnés indépendamment parmi:

(a) -H, où, si -X-A-Y- représente ensemble -CO-O- ou -CO-, alors $R^2$ est différent de -H,
(b) $-(C_1-C_{12})$alkyle, éventuellement substitué par jusqu'à cinq substituants sélectionnés indépendamment dans le groupe constitué par halogène, hydroxyle, thiol, nitrile, alcoxy, aryloxy, arylalkyloxy, amino, amido, alkylthio, arylthio, arylalkylthio, sulfamoyle, sulfonamide, acyle, carboxyle, acylamino,
aryle, lequel aryle est éventuellement substitué par jusqu'à trois substituants sélectionnés indépendamment dans le groupe constitué par halogène, hydroxyle, $(C_1-C_6)$alcoxy, $(C_1-C_6)$alkyle, $(C_1.C_6)$alkyle halogéné, $(C_1-C_6)$ alcoxy halogéné, carboxy- $(C_1-C_6)$ alkyle, thiol, nitrile, sulfamoyl, sulfonamide, carboxyle, aryloxy, arylalkyloxy, $(C_1-C_6)$alkylthio, arylthio, arylalkylthio, amino, amido, acyle, acylamino et hétéroaryle ; ou lequel aryle est éventuellement substitué par deux groupements qui sont liés à des atomes de carbone adjacents et sont associés en un noyau cyclique de 5, 6, 7 ou 8 chaînons saturé ou partiellement insaturé, contenant éventuellement jusqu'à trois hétéroatomes sélectionnés parmi N, 0 et S, le nombre d'atomes de N étant égal à 0, 1, 2 ou 3 et le nombre d'atomes de O et S étant chacun égal à 0, 1 ou 2 ;
hétéroaryle, lequel hétéroaryle est éventuellement substitué par jusqu'à trois substituants sélectionnés indépendamment dans le groupe constitué par halogène, hydroxyle, $(C_1-C_6)$alcoxy, $(C_1-C_6)$alkyle, $(C_1-C_6)$alkyle halogéné, $(C_1-C_6)$ alcoxy halogéné, carboxy-$(C_1-C_6)$alkyle, thiol, nitrile, sulfamoyle, sulfonamide, carboxyle, aryloxy, arylalkyloxy, $(C_1-C_6)$alkylthio, arylthio, arylalkylthio, amino, amido, acyle, acylamino, aryl-$(C_1-C_4)$-alkyle et aryle ;
ce par quoi chaque groupement aryle est éventuellement substitué par jusqu'à trois substituants sélectionnés indépendamment dans le groupe constitué par hydroxyle, halogène, $(C_1-C_6)$ alcoxy, $(C_1-C_6)$alkyle, $(C_1-C_6)$ alkyle halogéné et $(C_1-C_6)$alcoxy halogéné ; et
cyclohétéroalkyle, lequel groupement cyclohétéroalkyle est éventuellement substitué par jusqu'à trois substituants sélectionnés indépendamment dans le groupe constitué par oxo, $(C_1-C_8)$-alkyle, aryle, aryl-$(C_1-C_4)$-alkyle, hydroxyle, $(C_1-C_6)$alcoxy, carboxy-$(C_1-C_6)$alkyle, thiol, nitrile, sulfamoyle, sulfonamide, carboxyle, aryloxy, arylalkyloxy, $(C_1-C_6)$alkylthio, arylthio, arylalkylthio, amino, amido, acyle, et acylamino,
ce par quoi chaque groupement aryle est éventuellement substitué par jusqu'à trois substituants sélectionnés indépendamment dans le groupe constitué par hydroxyle, halogène, $(C_1-C_4)$-alkyle, $(C_1-C_4)$-alcoxy $(C_1-C_4)$-alkyle halogéné, et $(C_1-C_4)$-alcoxy) halogéné ;
(c) acyle -(C=O)-R', où R' représente hydrogène, $(C_1-C_4)$alkyle, aryle, ou aryl-$(C_1$-C4)alkyle, ou hétéroaryl-$(C_1-C_4)$alkyle ;
lequel aryle ou aryl-$(C_1-C_4)$alkyle est éventuellement substitué dans le motif aryle par jusqu'à trois substituants sélectionnés indépendamment dans le groupe constitué par hydroxyle, halogène, $(C_1-C_4)$ alcoxy, $(C_1-C_4)$-alkyle ou $(C_1-C_4)$alkyle halogéné ;
(d) aryle
lequel aryle est éventuellement substitué par jusqu'à trois substituants sélectionnés indépendamment dans le groupe constitué par halogène, hydroxyle, $(C_1-C_6)$alcoxy, $(C_1-C_6)$alkyle, $(C_1-C_6)$alkyle halogéné, $(C_1-C_6)$alcoxy halogéné, carboxy- $(C_1-C_6)$alkyle, thiol, nitrile, nitro, sulfamoyle, sulfonamide, carboxyle, aryloxy, arylalkyloxy, $(C_1-C_6)$alkylsulfonyle, arylsulfonyle, $(C_1-C_6)$alkylthio, arylthio, arylalkylthio, amino, amido, acyle, acylamino et hétéroaryle ; ou
lequel aryle est éventuellement substitué par deux groupements qui sont liés à des atomes de carbone adjacents et sont associés en un noyau cyclique de 5, 6, 7 ou 8 chaînons saturé ou partiellement insaturé, contenant éventuellement jusqu'à trois hétéroatomes sélectionnés parmi N, O et S, le nombre d'atomes de N étant égal à 0, 1, 2 ou 3 et le nombre d'atomes de 0 et S étant chacun égal à 0, 1 ou 2 ;
(e) hétéroaryle,
lequel hétéroaryle est éventuellement substitué par jusqu'à trois substituants sélectionnés indépendamment dans le groupe constitué par halogène, hydroxyle, $(C_1-C_6)$ alcoxy, $(C_1-C_6)$alkyle, $(C_1-C_6)$alkyle halogéné, $(C_1-C_6)$alcoxy halogène, carboxy-$(C_1-C_6)$alkyle, thiol, nitrile, sulfamoyl, sulfonamide, arylsulfoxy, carboxyle,

aryloxy, arylalkyloxy, ($C_1$-$C_6$)alkylsulfonyle, arylsulfonyle, ($C_1$-$C_6$)alkylthio, arylthio, arylalkylthio, amino, amido, acyle, acylamino, aryl-($C_1$-$C_4$)-alkyle et aryle,
ce par quoi chaque groupement aryle est éventuellement substitué par jusqu'à trois substituants sélectionnés indépendamment dans le groupe constitué par hydroxyle, halogène, ($C_1$-$C_6$) alcoxy, ($C_1$-$C_6$)alkyle, ($C_1$-$C_6$) alkyle halogéné et ($C_1$-$C_6$)alcoxy halogéné ; ou
(f) cyclohétéroalkyle,
lequel cyclohétéroalkyle est éventuellement substitué par jusqu'à trois substituants sélectionnés indépendamment dans le groupe constitué par oxo, ($C_1$-$C_{14}$) -alkyle, aryle, aryl-($C_1$-$C_4$)-alkyle, hydroxyle, ($C_1$-$C_6$)alcoxy, carboxy-($C_1$-$C_6$)alkyle, thiol, nitrile, sulfamoyle, sulfonamide, carboxyle, aryloxy, arylalkyloxy, ($C_1$-$C_6$)alkylthio, arylthio, arylalkylthio, amino, amido, acyle, et acylamino,
ce par quoi chaque groupement aryle est éventuellement substitué davantage par jusqu'à trois substituants sélectionnés indépendamment dans le groupe constitué par hydroxyle, halogène, ($C_1$-$C_4$)-alkyle, ($C_1$-$C_4$)-alcoxy, ($C_1$-$C_4$)-alkyle halogéné, et ($C_1$-$C_4$)-alcoxy halogéné ;

ou où $R^2$ et $R^4$ forment, conjointement avec l'atome d'azote auquel $R^2$ et $R^4$ sont liés, un cycle hétérocyclique de 4, 5, 6, 7 ou 8 chaînons, qui est éventuellement saturé ou partiellement insaturé ; qui contient éventuellement jusqu'à trois hétéroatomes supplémentaires sélectionnés parmi N, 0 et S, le nombre d'atomes de N supplémentaires étant égal à 0, 1, 2 ou 3 et le nombre d'atomes de 0 et S étant chacun égal à 0, 1 ou 2 ; et lequel cycle fait éventuellement partie d'un système de cycles condensés multiples,
où le cycle ou le système de cycles est éventuellement substitué

(i) par jusqu'à trois substituants sélectionnés indépendamment dans le groupe constitué par ($C_1$-$C_8$)-alkyle, halogène, hydroxyle, carboxyle, thiol, nitrile, ($C_1$-$C_6$)-alcoxy, carboxy-($C_1$-$C_6$)alkyle, aryloxy, arylalkyloxy, amino, amido, alkylthio, arylthio, arylalkylthio, sulfamoyle, sulfonamide, aryle, aryl-($C_1$-$C_4$)-alkyle, hétéroaryle, et cyclohétéroalkyle,
où le groupement ($C_1$-$C_8$)-alkyle est éventuellement substitué par jusqu'à trois substituants sélectionnés indépendamment parmi hydroxyle, halogène, ($C_1$-$C_4$)-alcoxy, ou ($C_1$-$C_4$)-alcoxy halogéné,
ce par quoi la chaîne alkyle du motif ($C_1$-$C_4$)-alcoxy est éventuellement substituée par hydroxyle ;
où le groupement aryle ou motif aryle est éventuellement substitué par jusqu'à trois substituants sélectionnés indépendamment dans le groupe constitué par hydroxyle, halogène, ($C_1$-$C_4$) -alkyle, ($C_1$-$C_4$)-alcoxy, ($C_1$-$C_4$) -alkyle halogéné, ($C_1$-$C_4$)-alcoxy halogéné et carboxy-($C_1$-$C_6$)alkyle, ou où le motif aryle est éventuellement substitué par deux groupements qui sont liés à des atomes de carbone adjacents et sont associés en un noyau cyclique de 5, 6, 7 ou 8 chaînons saturé ou partiellement insaturé, contenant éventuellement jusqu'à trois hétéroatomes sélectionnés parmi N, O et S, le nombre d'atomes de N étant égal à 0, 1, 2 ou 3 et le nombre d'atomes de 0 et S étant chacun égal à 0, 1 ou 2 ;
où le groupement hétéroaryle est éventuellement substitué par jusqu'à trois substituants sélectionnés indépendamment dans le groupe constitué par hydroxyle, halogène, ($C_1$-$C_4$)-alkyle, ($C_1$-$C_4$)-alcoxy, ($C_1$-$C_4$) -alkyle halogéné, ($C_1$-$C_4$)-alcoxy halogéné et carboxy-($C_1$-$C_6$)alkyle ;
où le cyclohétéroalkyle est éventuellement substitué par jusqu'à trois substituants sélectionnés indépendamment dans le groupe constitué par oxo, ($C_1$-$C_8$)-alkyle, aryle, aryl-($C_1$-$C_4$-alkyle, hydroxyle, ($C_1$-$C_6$)alcoxy, carboxy-($C_1$-$C_6$) alkyle, et carboxyle,
ce par quoi chaque groupement aryle est éventuellement substitué davantage par jusqu'à trois substituants sélectionnés indépendamment dans le groupe constitué par hydroxyle, halogène, ($C_1$-$C_4$)-alkyle, ($C_1$-$C_4$)-alcoxy, ($C_1$-$C_4$)-alkyle halogéné, et ($C_1$-$C_4$)-alcoxy halogéné ; ou
(ii) par deux groupements qui sont liés au même atome de carbone et sont associés en un noyau cyclique de 4, 5, 6, 7 ou 8 chaînons saturé ou partiellement insaturé, contenant éventuellement jusqu'à trois hétéroatomes sélectionnés parmi N, O et S, le nombre d'atomes de N étant égal à 0, 1, 2 ou 3 et le nombre d'atomes de 0 et S étant chacun égal à 0, 1 ou 2,
ce par quoi le système de cycles est éventuellement substitué par jusqu'à deux substituants sélectionnés indépendamment parmi oxo, ($C_1$-$C_6$)-alkyle, aryle et aryl-($C_1$-$C_4$)-alkyle ;

et où n représente

(a) 1, 2, 3, 4, 5 ou 6, si -X-A-Y- représente ensemble -NH-CO-$NR^4$-, -NH-CO-O-, -NH-CO-, -NH-CO-NH-$SO_2$-, -NH-$SO_2$-$NR^9$-, -NH-$SO_2$-O-, -NH-$SO_2$-, -O-CO-$NR^4$-, -O-CO-, -O-CO-NH-$SO_2$-$NR^4$-, ou -O-, ou
(b) 0, 1, 2, 3, 4, ou 5, si -X-A-Y- représente ensemble -CO-$NR^4$-, -CO-O-, -CO-, ou -CO-NH-$NR^4$-.

**3.** Composé de formule générale I selon la revendication 1 ou 2, qui est un énantiomère optiquement pur répondant

à la formule (II)

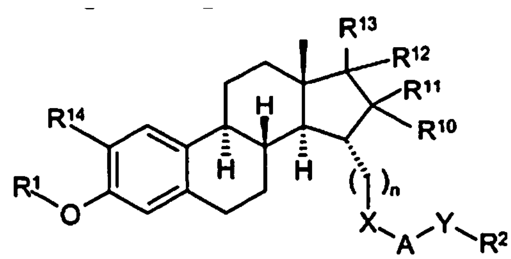

(II)

ou un sel physiologiquement acceptable de celui-ci.

**4.** Composé de formule générale I selon la revendication 1 ou 2, qui est un énantiomère optiquement pur répondant à la formule (III)

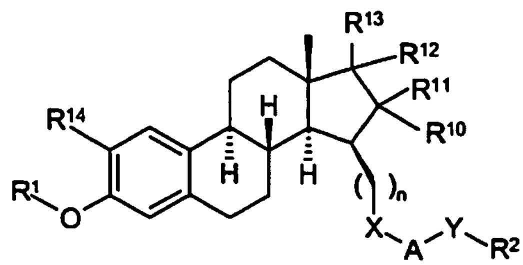

(III)

ou un sel physiologiquement acceptable de celui-ci.

**5.** Composé selon l'une quelconque des revendications précédentes 1 à 4, dans lequel
$R^1$ est sélectionné parmi:

(a) $-SO_2-NR^3R^{3'}$,
(b) $-CO-NR^3R^{3'}$,
(c) $-PO(OR^{16})-R^3$,
(d) $-PS(OR^{16})-R^3$,
(e) $-PO(OR^{16})-O-R^3$,
(f) $-SO_2-R^3$ ; et
(g) $-SO_2-O-R^3$ ;

où
$R^3$ et $R^{3'}$ sont sélectionnés indépendamment parmi -H, $-(C_1-C_8)$alkyle, phényle et $-(C_1-C_4)$-alkylphényle, ou
$R^3$ et $R^{3'}$, conjointement avec l'atome d'azote auquel $R^3$ et $R^{3'}$ sont liés, forment un cycle hétérocyclique de 4, 5, 6, 7 ou 8 chaînons, qui est sélectionné dans le groupe constitué par

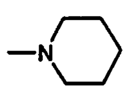 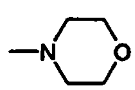 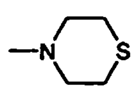 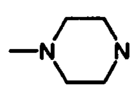

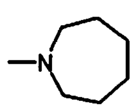 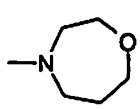 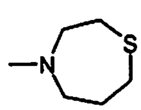 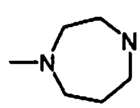

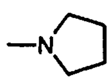 et 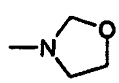 ,

$R^{16}$ représente -H, -($C_1$-$C_4$alkyle, ou -($C_1$-$C_4$)alkylphényle,
$R^{10}$ et $R^{11}$ représentent tous les deux -H et $R^{12}$ et $R^{13}$ représentent ensemble =O ; et
$R^{14}$ représente -H, -($C_1$-$C_8$)alkyle, -O-($C_1$-$C_8$)alkyle, ou -($C_1$-$C_8$)alkyl-O-($C_1$-$C_8$)alkyle.

**6.** Composé selon la revendication 5, dans lequel $R^1$ représente -$SO_2$-$NR^3R^{3'}$, où $R^3$ et $R^{3'}$, conjointement avec l'atome d'azote auquel $R^3$ et $R^{3'}$ sont liés, forment un cycle hétérocyclique, qui est sélectionné dans le groupe constitué par morpholine, thiomorpholine et pipérazyle, ou où $R^1$ représente -$SO_2$-$NH_2$, et
$R^{14}$ représente -H.

**7.** Composé selon l'une quelconque des revendications précédentes 1 à 4, dans lequel
$R^1$ représente -H, ($C_1$-$C_4$) alkyle, ou -($C_1$-$C_4$)-alkylphényle ; et
$R^{10}$ et $R^{11}$ représentent tous les deux -H et $R^{12}$ et $R^{13}$ représentent ensemble =0 ; et
$R^{14}$ représente -($C_1$-$C_8$)alkyle, -O-($C_1$-$C_8$)alkyle, ou - ($C_1$-$C_8$) alkyl-O-($C_1$-$C_8$) alkyle.

**8.** Composé selon la revendication 7, dans lequel $R^{14}$ représente -($C_1$-$C_4$)alkyle, -O-($C_1$-$C_4$)alkyle, ou - ($C_1$-$C_4$)alkyl-O-($C_1$-$C_4$)alkyle.

**9.** Composé selon la revendication 8, dans lequel $R^1$ représente -H, et
$R^{14}$ représente éthyle, propyle, méthoxyéthyle ou méthoxy.

**10.** Composé selon l'une quelconque des revendications précédentes 1 à 4, dans lequel
$R^1$ représente -H, ($C_1$-$C_4$)alkyle, ou -($C_1$-$C_4$)-alkylphényle ;
les substituants $R^{10}$, $R^{11}$, $R^{12}$ et $R^{13}$, conjointement avec les atomes de carbone auxquels ils sont liés, forment une structure

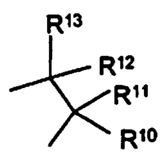

qui est sélectionnée dans le groupe constitué par

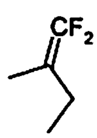 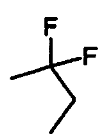 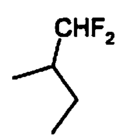 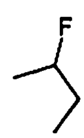

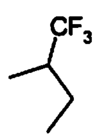 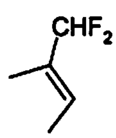 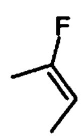 et 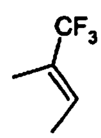 ,

et
$R^{14}$ représente -H, -($C_1$-$C_8$)alkyle, -O-($C_1$-$C_8$)alkyle,
ou -($C_1$-$C_8$)alkyl-O-($C_1$-$C_8$)alkyle.

**11.** Composé selon la revendication 10, dans lequel les substituants $R^{10}$, $R^{11}$, $R^{12}$ et $R^{13}$, conjointement avec les atomes de carbone auxquels ils sont liés, forment une structure

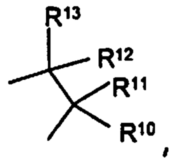

qui est sélectionnée dans le groupe constitué par

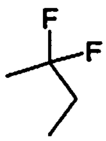

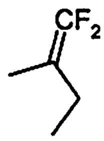

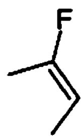

et

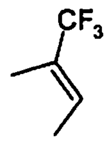

.

**12.** Composé selon la revendication 11, dans lequel $R^1$ et $R^{14}$ représentent chacun individuellement -H.

**13.** Composé selon l'une quelconque des revendications précédentes 1 à 4, dans lequel
$R^1$ représente -H, $(C_1-C_4)$ alkyle, ou $-(C_1-C_4)$-alkylphényle ;
les substituants $R^{10}$, $R^{11}$, $R^{12}$ et $R^{13}$, conjointement avec les atomes de carbone auxquels $R^{10}$, $R^{11}$, $R^{12}$ et $R^{13}$ sont liés, forment un cycle hétérocyclique de 5 ou 6 chaînons, qui est partiellement insaturé ou aromatique, qui contient un, deux ou trois hétéroatomes sélectionnés indépendamment parmi N, O et S, le nombre d'atomes de N étant égal à 0, 1, 2 ou 3 et le nombre d'atomes de O et S étant chacun égal à 0, 1 ou 2, où un hétéroatome est lié directement à l'atome de C C17 du noyau stéroïdien ; et lequel cycle est éventuellement substitué par un groupement alkyle ; et
$R^{14}$ représente -H, $-(C_1-C_8)$alkyle, $-O-(C_1-C_8)$alkyle, ou $-(C_1-C_8)$alkyl-$O-(C_1-C_8)$alkyle.

**14.** Composé selon la revendication 12, dans lequel
les substituants $R^{10}$, $R^{11}$, $R^{12}$ et $R^{13}$, conjointement avec les atomes de carbone auxquels $R^{10}$, $R^{11}$, $R^{12}$ et $R^{13}$ sont liés, forment un cycle hétérocyclique de 5 ou 6 chaînons pour fournir un composé répondant à l'une des formules suivantes

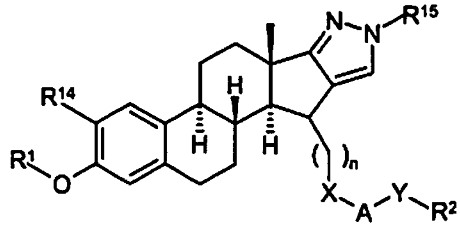

,

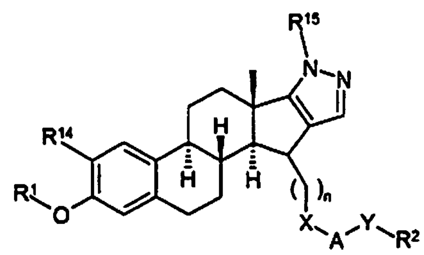

,

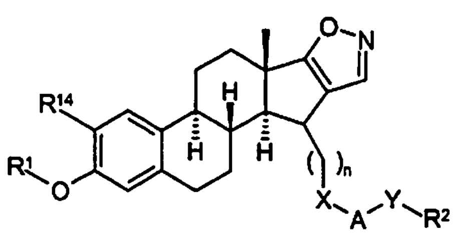

, ou

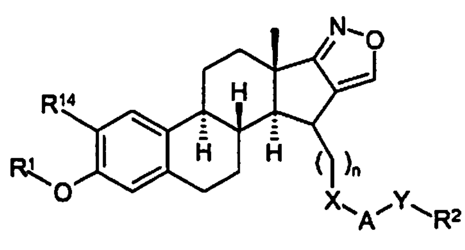

,

où $R^{15}$ représente -H ou $-(C_1-C_4)$alkyle.

**15.** Composé selon la revendication 14, dans lequel $R^1$ et $R^{14}$ représentent chacun individuellement -H.

**16.** Composé selon l'une quelconque des revendications précédentes 1 à 4, dans lequel
$R^1$ représente -H ou $-SO_2-NH_2$,

les substituants $R^{10}$, $R^{11}$, $R^{12}$ et $R^{13}$, conjointement avec les atomes de carbone auxquels ils sont liés, forment une structure

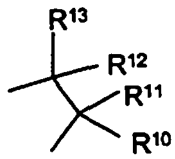,

qui est sélectionnée dans le groupe constitué par

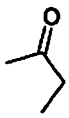 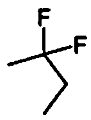 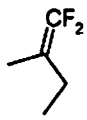 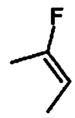 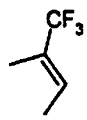 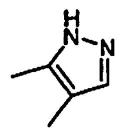 et 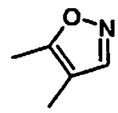 ;

et
$R^{14}$ représente -H, -($C_1$-$C_4$)alkyle, -O-($C_1$-$C_4$)alkyle, ou -($C_1$-$C_4$)alkyl-O-($C_1$-$C_4$)alkyle.

**17.** Composé selon l'une quelconque des revendications précédentes 1 à 16, dans lequel -X-A-Y- représente ensemble un groupement sélectionné parmi -CO-$NR^4$-, -CO-O-, -CO-, et -CO-NH-$NR^4$- ; et n représente 0, 1, 2, 3, 4, ou 5.

**18.** Composé selon la revendication 17, dans lequel -X-A-Y- représente ensemble -CO-$NR^4$-.

**19.** Composé selon la revendication 18, dans lequel
$R^2$ représente un groupement -($C_1$-$C_4$)alkylphényle ou thiazolyle, éventuellement substitué par -($C_1$-$C_4$)-alkyle, et $R^4$ représente -H ; ou
$R^2$ et $R^4$ forment, conjointement avec l'atome d'azote auquel $R^2$ et $R^4$ sont liés, un groupement morpholinyle, et n représente 3.

**20.** Composé selon la revendication 1, sélectionné dans le groupe constitué par les composés illustratifs:

le N-benzyl-4-(2-éthyl-3-hydroxy-17-oxo-estra-1,3,5(10)trién-15β-yl)butyramide
le N-benzyl-4-(3-hydroxy-17-oxo-2-propyl-estra-1,3,5(10)trién-15β-yl)butyramide
le N-benzyl-4-(3-hydroxy-2-(2-méthoxyéthyl)-17-oxo-estra-1,3,5(10)trién-15β-yl)butyramide
le N-benzyl-4-(3-hydroxy-2-méthoxy-17-oxo-estra-1,3,5(10)trién-15β-yl)butyramide
la 2-éthyl-3-hydroxy-15β-(4-morpholin-4-yl-4-oxobutyl)-estra-1,3,5(10)trién-17-one-
la 3-hydroxy-15β-(4-morpholin-4-yl-4-oxobutyl)-2-propyl-estra-1,3,5(10)trién-17-one
la 3-hydroxy-2-(2-méthoxyéthyl)-15β-(4-morpholin-4-yl-4-oxobutyl)-estra-1,3,5(10)trién-17-one
la 3-hydroxy-2-méthoxy-15β-(4-morpholin-4-yl-4-oxobutyl)-estra-1,3,5(10)trién-17-one
le 4-(2-éthyl-3-hydroxy-17-oxo-estra-1,3,5(10)trién-15β-yl)-N-(5-méthylthiazol-2-yl)butyramide
le 4-(3-hydroxy-2-méthoxy-17-oxo-estra-1,3,5(10)trién-15β-yl)-N-(5-méthylthiazol-2-yl)butyramide
la 4-(17,17-difluoro-3-hydroxy-estra-1,3,5(10)trién-15β-yl)-1-morpholin-4-ylbutan-1-one
la 4-(17-fluoro-3-hydroxy-estra-1,3,5(10),16-tétraén-15β-yl)-1-morpholin-4-ylbutan-1-one
la 4-(17-difluorométhylène-3-hydroxy-estra-1,3,5(10)trién-15β-yl)-1-morpholin-4-yl-butan-1-one
le N-benzyl-4-(17,17-difluoro-3-hydroxy-estra-1,3,5(10)trién-15α-yl)butyramide
le 3-hydroxy-15α-(4-morpholin-4-yl-4-oxobutyl)-estra-1,3,5(10)triène[16,17-c]pyrazole
la 3-sulfamate-15β-(4-morpholin-4-yl-4-oxobutyl)-estra-1,3,5(10)trién-17-one,
la 3-sulfate-15α-(4-morpholin-4-yl-4-oxobutyl)-estra-1,3,5(10)trién-17-one,
ainsi que tout sel physiologiquement acceptable de celui-ci.

**21.** Composé selon l'une quelconque des revendications 1 à 20, pour une utilisation comme médicament.

**22.** Composition pharmaceutique comprenant comme agent actif un composé de formule (I) selon l'une quelconque des revendications 1 à 20, ainsi qu'au moins un véhicule pharmaceutiquement acceptable.

**23.** Composé de formule (I) selon l'une quelconque des revendications précédentes 1 à 20, pour le traitement ou la prévention d'une maladie ou d'un trouble dépendant d'une hormone stéroïdienne chez un mammifère.

**24.** Composé de formule (I) selon la revendication 23, où la maladie ou le trouble dépendant d'une hormone stéroïdienne est une maladie ou un trouble dépendant de l'oestradiol.

**25.** Composé de formule (I) selon la revendication 24, où la maladie ou le trouble dépendant de estradiol est malin et est sélectionné dans le groupe constitué par le cancer du sein, le cancer de l'ovaire, le cancer de l'utérus, le cancer de l'endomètre, et l'hyperplasie de l'endomètre.

**26.** Composé de formule (I) selon la revendication 25, où la maladie maligne ou le trouble malin est caractérisé(e) par un taux détectable de l'expression de 17β-HSD1 et/ou de STS au sein d'un échantillon de tissu cancéreux.

**27.** Composé de formule (I) selon la revendication 25 ou 26, où la maladie dépendant de l'oestradiol est le cancer du sein et le mammifère est une femelle humaine post-ménopausée.

**28.** Composé de formule (I) selon la revendication 24, où la maladie ou le trouble dépendant de l'oestradiol est bénin et est sélectionné dans le groupe constitué par l'endométriose, les fibroïdes utérins, les léiomyomes utérins, l'adénomyose, la dysménorrhée, la ménorragie, la métrorragie, et les dysfonctionnements urinaires.

**29.** Composé de formule (I) selon la revendication 28, où le mammifère est une femelle humaine pré- ou péri-ménopausée.

**30.** Composé de formule (I) selon la revendication 23, la maladie ou le trouble dépendant d'une hormone stéroïdienne est une maladie ou un trouble dépendant de l'androgène.

**31.** Composé de formule (I) selon la revendication 30, où la maladie ou le trouble dépendant de l'androgène est sélectionné dans le groupe constitué par l'acné, la séborrhée, l'alopécie androgénétique, l'hirsutisme, et le cancer de la prostate.

**32.** Composé de formule (I) selon la revendication 23, où la maladie ou le trouble dépendant d'une hormone stéroïdienne est une maladie ou un trouble estrogène- ou androgène-dépendant(e) nécessitant l'abaissement de la concentration en estrogène ou androgène endogène de manière généralisée ou spécifique de tissus.

**33.** Composé de formule (I) selon la revendication 32, où la maladie ou le trouble est sélectionné(e) dans le groupe constitué par la prostatodynie, l'hyperplasie bénigne de la prostate, les dysfonctionnements urinaires, le syndrome du tractus urinaire inférieur, le carcinome des cellules squameuses, la polyarthrite rhumatoïde, le diabète de type I et II, le lupus érythémateux disséminé, la sclérose en plaques, la myasthénie gravis, la thyroïdite, l'angéite, la rectocolite hémorragique, la maladie de Crohn, le psoriasis, la dermite de contact, la réaction de greffe contre hôte, l'eczéma, l'asthme, le rejet d'organe suite à une greffe, le cancer du côlon, les plaies tissulaires, les rides cutanées, la cataracte, les dysfonctionnements cognitifs, la démence sénile et la maladie d'Alzheimer.

**34.** Utilisation d'un composé de formule (I) selon l'une quelconque des revendications précédentes 1 à 20, pour la fabrication d'un médicament destiné au traitement ou à la prévention d'une maladie ou d'un trouble dépendant d'une hormone stéroïdienne chez un mammifère.

**35.** Utilisation d'un composé de formule (I) selon la revendication 34, où la maladie ou le trouble dépendant d'une hormone stéroïdienne est une maladie ou un trouble dépendant de l'oestradiol.

**36.** Utilisation d'un composé de formule (I) selon la revendication 35, où la maladie ou le trouble dépendant de l'oestradiol est malin(gne) et est sélectionné(e) dans le groupe constitué par le cancer du sein, le cancer de l'ovaire, le cancer de l'utérus, le cancer de l'endomètre, et l'hyperplasie de l'endomètre.

**37.** Utilisation d'un composé de formule (I) selon la revendication 36, où la maladie maligne ou le trouble malin est caractérisé(e) par un taux détectable de l'expression de 17β-HSD1 et/ou de STS au sein d'un échantillon de tissu cancéreux.

**38.** Utilisation d'un composé de formule (I) selon la revendication 36 ou 37, où la maladie dépendant de estradiol est

le cancer du sein et le mammifère est une femelle humaine post-ménopausée.

**39.** Utilisation d'un composé de formule (I) selon la revendication 35, où la maladie ou le trouble dépendant de l'oestradiol est bénin(gne) et est sélectionné(e) dans le groupe constitué par l'endométriose, les fibroïdes utérins, les léiomyomes utérins, l'adénomyose, la dysménorrhée, la ménorragie, la métrorragie, et les dysfonctionnements urinaires.

**40.** Utilisation d'un composé de formule (I) selon la revendication 39, où le mammifère est une femelle humaine pré- ou péri-ménopausée.

**41.** Utilisation d'un composé de formule (I) selon la revendication 34, où la maladie ou le trouble dépendant d'une hormone stéroïdienne est une maladie ou un trouble dépendant de l'androgène.

**42.** Utilisation d'un composé de formule (I) selon la revendication 41, où la maladie ou le trouble dépendant de l'androgène est sélectionné(e) dans le groupe constitué par l'acné, la séborrhée, l'alopécie androgénétique, l'hirsutisme, et le cancer de la prostate.

**43.** Utilisation d'un composé de formule (I) selon la revendication 34, où la maladie ou le trouble dépendant d'une hormone stéroïdienne est une maladie ou un trouble estrogène- ou androgène-dépendant(e) nécessitant l'abaissement de la concentration en estrogène ou androgène endogène de manière généralisée ou spécifique de tissus.

**44.** Utilisation d'un composé de formule (I) selon la revendication 43, où la maladie ou le trouble est sélectionné(e) dans le groupe constitué par la prostatodynie, l'hyperplasie bénigne de la prostate, les dysfonctionnements urinaires, le syndrome du tractus urinaire inférieur, le carcinome des cellules squameuses, la polyarthrite rhumatoïde, le diabète de type I et II, le lupus érythémateux disséminé, la sclérose en plaques, la myasthénie gravis, la thyroïdite, l'angéite, la rectocolite hémorragique, la maladie de Crohn, le psoriasis, la dermite de contact, la réaction de greffe contre hôte, l'eczéma, l'asthme, le rejet d'organe suite à une greffe, le cancer du côlon, les plaies tissulaires, les rides cutanée, la cataracte, les dysfonctionnements cognitifs, la démence sénile et la maladie d'Alzheimer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004080271 A
- WO 03017973 A
- WO 0232409 A
- EP 0367576 A
- WO 2004085457 A
- WO 2005047303 A
- WO 2006003012 A
- WO 2006003013 A
- WO 2004085345 A
- WO 2006027347 A
- WO 9305064 A
- WO 2004085459 A
- WO 9950453 A
- WO 9628462 A
- US 3413321 A
- US 3347878 A
- US 3275623 A
- WO 200485457 A
- WO 200485457 A2
- WO 9305063 A
- US 6043236 A
- WO 200547303 A
- WO 0007996 A
- US 20030170292 A
- WO 9615257 A
- US 2006052461 A
- US 2006009434 A


### Non-patent literature cited in the description


- **Bradford.** *Anal. Biochem.,* 1976, vol. 72, 248-254
- **Akanni ; Marples.** Preparation of 16-formylestradiol and the 16-(alpha-methylenebutanolide) derivative. *Steroids,* 1993, vol. 58 (5), 234-8
- **Cushman et al.** Synthesis, antitubulin and antimitotic activity, and cytotoxicity of analogs of 2-methoxyestradiol, an endogenous mammalian metabolite of estradiol that inhibits tubulin polymerization by binding to the colchicine binding site. *J Med Chem.,* 1995, vol. 38 (12), 2041-9
- **Cushman et al.** The effect of exchanging various substituents at the 2-position of 2-methoxyestradiol on cytotoxicity in human cancer cell cultures and inhibition of tubulin polymerization. *J Med Chem.,* 2002, vol. 45 (21), 4748-54
- **Day et al.** The effects of 2-substituted oestrogen sulphamates on the growth of prostate and ovarian cancer cells. *J Steroid Biochem Mol Biol.,* 2003, vol. 84 (2-3), 317-25
- **Edwards et al.** Difluoromethyldiphenylphosphine oxide. A new reagent for conversion of carbonyl compounds to 1,1-difluoroolefins. *Tetrahedron Lett,* 1990, vol. 31 (39), 5571-5574
- **Gonzalez et al.** Synthesis and pharmacological evaluation of 8$\alpha$-estradiol derivatives. *Steroids,* 1982, vol. 40 (2), 171-188
- **Koffman et al.** Evidence for involvement of tyrosine in estradiol binding by rat uterus estrogen receptor. *J. Steroid. Biochem. Mol. Biol.,* 1991, vol. 38 (2), 135
- **Labaree et al.** Synthesis and Evaluation of B-, C- and D-ring substituted estradiol carboxylic acid esters as locally active estrogens. *J. Med. Chem.,* 2003, vol. 46, 1886-1904
- **Labrie et al.** The key role of 17 beta-hydroxysteroid dehydrogenases in sex steroid biology. *Steroids,* 1997, vol. 62, 148-58
- **Lawrence et al.** Novel and potent 17beta-hydroxysteroid dehydrogenase type 1 inhibitors. *J Med Chem.,* 2005, vol. 48 (8), 2759-62
- **Ley et al.** Tetrapropylammonium perruthenate, Pr4N+Ru04-, TPAP: a catalytic oxidant for organic synthesis. *Synthesis,* 1994, vol. 07, 639-666
- **Liu et al.** Synthesis of high affinity fluorine-substituted ligands for the androgen receptor. Potential agents for imaging prostatic cancer by positron emission tomography. *J Med Chem.,* 1992, vol. 35 (11), 2113-29
- **Lunn ; Farkas.** The adamantyl carbonium ion as a dehydrogenating agent, its reactions with estrone. *Tetrahedron,* 1968, vol. 24 (23), 6773-6776
- **Mindnich et al.** The role of 17 beta-hydroxysteroid dehydrogenases. *Mol Cell Endocrinol.,* 2004, vol. 218 (1-2), 7-20

- **Mohanakrishnan ; Cushman.** Pd(0)-Mediated Cross Coupling of 2-Iodoestradiol with Organozinc Bromides: A General Route to the Synthesis of 2-Alkynyl, 2-Alkenyl and 2-Alkylestradiol Analogs. *Synlett,* 1999, 1097-1099
- **Nambara et al.** Synthesis of Estetrol Monoglucuronides. *Steroids,* 1976, vol. 27, 111-122
- **Nussbaumer ; Billich.** Steroid sulfatase inhibitors. *Expert Opin. Ther. Patents,* 2003, vol. 13 (5), 605-625
- **Nussbaumer ; Billich.** Steroid sulfatase inhibitors. *Med Res Rev.,* 2004, vol. 24 (4), 529-76
- **Oda et al.** The hydrogenation of alpha-hydroxymethylene-ketone derivatives to alpha-hydroxymethyl-ketone derivatives with a cell-free system of Streptomyces cinereocrocatus. *Chem Pharm Bull,* 1989, vol. 37 (2), 502-5
- **Page et al.** Efficient regioselective a-ring functionalization of oestrogens. *Tetrahedron,* 1990, vol. 46 (6), 2059-2068
- **Pelletier ; Poirier.** Synthesis and evaluation of estradiol derivatives with 16α-(bromoalkylamide), 16α-(bromoalkyl) or 16α-(bromoalkynyl) side chain as inhibitors of 17β-hydroxysteroid dehydrogenase type 1 without estrogenic activity. *Bioorg Med Chem,* 1996, vol. 4 (10), 1617-1628
- **Poirier.** Inhibitors of 17 beta-hydroxysteroid dehydrogenases. *Curr Med Chem.,* 2003, vol. 10, 453-77
- **Poirier et al.** Synthesis of 17β-estradiol derivatives with N-Butyl, N-methyl alkylamide side chain at position 15. *Tetrahedron,* 1991, vol. 47 (37), 7751-7766
- **Poirier et al.** D-Ring alkylamine derivatives of estradiol: effect on ER-binding affinity and antiestrogenic activity. *Bioorg Med Chem Lett,* 1996, vol. 6 (21), 2537-2542
- **Poirier et al.** A 6β-(Thiaheptanamide) Derivative of Estradiol as inhibitor of 17β-Hydroxysteroid Dehydrogenase Type 1. *J. Steroid Biochem. Molec. Biol.,* 1998, vol. 64, 83-90
- **Puranen et al.** Site-directed mutagenesis of the putative active site of human 17 beta-hydroxysteroid dehydrogenase type 1. *Biochem. J.,* 1994, vol. 304, 289-93
- **Rao ; Cessac.** A new, practical synthesis of 2-methoxyestradiols. *Steroids,* 2002, vol. 67 (13-14), 1065-70
- **Reed et al.** Steroid sulfatase: molecular biology, regulation, and inhibition. *Endocr Rev.,* 2005, vol. 26 (2), 171-202
- **Sam et al.** C16 and C17 Derivatives of Estradiol as Inhibitors of 17β-Hydroxysteroid Dehydrogenase Type 1: Chemical Synthesis and Structure-Activity Relationships. *Drug Design and Discovery,* 1998, vol. 15, 157-180
- **Schneider et al.** A convenient method for the formation of 16-methylene-17-keto steroids. *Synthesis,* 1983, vol. 8, 665-9
- **Schwarz et al.** Studies on modified estrogens: Towards the synthesis of novel 14,15-cyclopropa[a]estra-1,3,5(10),8-tetraenes. *Pharmazie,* 2001, vol. 56 (11), 843-849
- **Tamaya et al.** Comparison of cellular levels of steroid receptors in uterine leiomyoma and myometrium. *Acta Obstet Gynecol Scand.,* 1985, vol. 64, 307-9
- **Tremblay ; Poirier.** Overview of a Rational Approach to Design Type I 17β-Hydroxysteroid Dehydrogenase Inhibitors Without Estrogenic Activity: Chemical Synthesis and Biological Evaluation. *Steroid Biochem. Molec. Biol.,* 1998, vol. 66, 179-191
- **Verdier-Pinard et al.** A steroid derivative with paclitaxel-like effects on tubulin polymerization. *Mol Pharmacol.,* 2000, vol. 57 (3), 568-75
- **Wang ; Ruan.** Trifluoromethylation of steroidal ketones. *J. Fluorine Chem.,* vol. 69 (1), 1-3
- **Wölfling et al.** Synthesis and receptor-binding examinations of the normal and 13-epi-D-homoestrones and their 3-methyl ethers. *Steroids,* vol. 68, 277-288
- **Xenos ; Catsoulacos.** Synthesis of 16,17-pyrazolo-fused derivatives of A-homo-steroidal ring A lactams. *Synthesis,* 1985, vol. 3, 307-9
- **Yoshikawa et al.** Diastereo- and Enantioselective Direct Catalytic Aldol Reaction of 2-Hydroxyacetophenones with Aldehydes Promoted by a Heteropolymetallic Complex: Catalytic Asymmetric Synthesis of anti-1,2-Diols. *J. Org. Chem.,* 2002, vol. 67 (8), 2556-2565